# NEW EUROPEAN PATENT SPECIFICATION

(11) **EP 1 227 323 B2**
(45) Date of publication and mention of the opposition decision: **19.01.2011**
(45) Mention of the grant of the patent: 22.11.2006
(21) Application number: 02006640.3
(22) Date of filing: 23.08.1991
(51) Int. Cl.: G01N 33/576, A61K 39/29

(54) **Non-A, non-B hepatitis virus antigen, and diagnostic methods**
Nicht-A, nicht-B Hepatitis Virus Antigen, und diagnostische Verfahren.
Antigène du virus de l'hépatite non-A, non B et procédé diagnostique

(30) Priority: 27.08.1990 US 573643; 21.11.1990 US 616369; 21.08.1991 US 748564
(43) Date of publication of application: 31.07.2002
(62) Divisional of application: 91920080.8
(73) Proprietor: Bioprocess Pty Ltd, Eveleigh, NSW 1430 (AU); F. Hoffmann-La Roche Ltd., 4070 Basel (CH)
(72) Inventor: Zebedee, Suzanne, Carlsbad, CA 92009-7113 (US); Inchauspe, Geneviève, 69424 Lyon Cédex 03 (FR); Nasoff, Marc S., San Diego, CA 92131-2386 (US); Prince, Alfred M., Pound Ridge, NY 10576-0154 (US)
(74) Representative: Vossius & Partner

(56) References cited:
- EP-A- 0 363 025
- EP-A- 0 377 303
- OKAMOTO H. ET AL.: "The 5'-terminal sequence of the hepatitis C virus genome" JAPANESE JOURNAL OF EXPERIMENTAL MEDECINE, vol. 60, no. 3, June 1990 (1990-06), pages 163-177, XP002034219
- NASOFF M S ET AL: "Identification of an immunodominant epitope within the capsid protein of hepatitis C virus" PROCEEDINGS OF THE NATIONAL ACADEMY OF SCIENCES OF USA, vol. 88, no. 12, 15 June 1991 (1991-06-15), pages 5462-5466, XP000310531

## Description

### Technical Field

The present invention relates to compositions containing a NANBV structural protein useful in diagnostic methods, kits comprising said composition as well as the use of said kits and compositions for diagnostic purposes.

### Background of the Invention

Non-A, non-B hepatitis (NANBH) is believed to re caused by a transmissible virus that has been referred to as both hepatitis C virus (HCV) and non-A, non-B hepatitis virus (NANBV). Although the transmissible disease was discovered years ago, a complete characterization of the causative agent is still being developed.

Isolates of NANBV have been obtained and portions or all of the viral genome of the various isolates were molecularly cloned and sequenced. Choo et al, Science, 244:359-362 (1989); Choo et al., Proc. Natl. Acad. Sci. USA, 88:2451-2455 (1991) ; Takamizawa et al., J. Virol., 65:1105-1113 (1991); Kato et al., Proc. Natl. Acad. Sci. USA, 87:9524-9528 (1990); and Takeuchi et al., Nucl. Acids Res., 18:4626 (1990). Similarities in nucleotide base sequence between the different isolates of NANBV suggest that they are a part of a family of related viruses. Okamoto et al, Japan-J. Exp. Med., 60:163-177 (1990); and Ogata et al., Proc. Natl. Acad. Sci. USA, 88:3392-3396 (1991). Properties of the NANBV genome suggest that NANBV may be a very distant relative of the flavivirus family. However, similarities in both the size and hydropathicity of the structural proteins suggest that NANB viruses may also be distantly related to the pestivirus family. Miller et al., Proc; Natl. Acad. Sci., 87:2057-2061 (1990) ; and Okamoto et al., Japan J. Exp. Med., 60:163-177 (1990).

The difficulties in characterizing the NANBV isolates taxonomically and the lack of information regarding the proteins encoded by the NANBV genome have made it difficult to identify relevant gene products useful for diagnostic markers.

The NANBV genome is comprised of a positive stranded RNA molecule that codes for a single polyprotein. The gene products of NANBV are believed to include both structural and non structural proteins, based on homologies to characteri-zed, related viruses. From these homologies, it is predicted that NANBV expresses a single polyprotein gene product from the complete viral genome, which is then cleaved into functionally distinct structural and nonstructural proteins. This type of viral morphogenesis precludes positive identification of the individual mature viral proteins until they have been physically isolated and characterized. Since no in vitro culturing system to propagate the virus has been developed for NANBV, no NANBV structural or nonstructural gene products (proteins) have been isolated from biological specimens or NANBV-infected cells. Thus, the identification of NANBV proteins, of their role in the viral life cycle, and of their role in disease, have yet to be determined. In particular, antigenic markers for NANBV-induced disease have yet to be fully characterized.

One NANBV gene product, namely the antigen C100-3, derived from portions of the nonstructural genes designated NS3 and NS4, has been expressed as a fusion protein and used to detect anti-C100-3 antibodies in patients with various forms of NANB hepatitis. See, for example, Kuo et al, Science, 244:362-364 (1989); and International Application No. PCT/US88/04125. A diagnostic assay based on C100-3 antigen is commercially available from Ortho Diagnostics, Inc. (Raritan, NJ). This C100-3 assay currently represents the state of the art in detecting NANBV infections. However, the C100-3 antigen-based immunoassay has been reported to preferentially detect antibodies in sera from chronically infected patients. C100-3 seroconversion generally occurs from four to six months after the onset of hepatitis, and in some cases C100-3 fails to detect any antibody where an NANBV infection is present. Alter et al., New Eng. J. Med., 321:1538-39 (1989); Alter et al., New Eng. J. Med., 321:1494-1500 (1989); and Weiner et al., Lancet, 335:1-3 (1990). McFarlane et al., Lancet, 335:754-757 (1990), described false positive results when the C100-3-based immunoassay was used to measure antibodies in patients with autoimmune chronic active hepatitis. Using the C100-3-based immunoassay, Grey et al., Lancet, 335:609-610 (1990), describe false positive results on sera from patients with liver disease caused by a variety of conditions other than NANBV.

A NANBV immunoassay that could accurately detect seroconversion at early times after infection, or that could identify an acute NANBV infection, is not presently available.

The Hutch strain of HCV is a clinically interesting isolate compared to the Donn strain (HCV-1) because HCV-H grows to extremely high titers in the patient.

### Summary of the Invention

One Hutch strain (HCV-H) of non-A, non-B hepatitis virus (NANBV) designated the Hutch c59 isolate (or HCV-Hc59) has been propagated through passage in animals and the entire viral genome has been cloned and sequenced. When using the term "subgroup" the present specification refers to a group of NANBVs which is serologically defined by particular strains, such as the Hutch c59 strain. Sequence data shows differences at both the nucleotide and amino acid level when compared to previously reported NANBV strains. See, the sequences of the following HCV isolates, where the isolate designation is shown in parenthesis for comparison, Okamoto et al, Japan J. Exp. Med., 60:163-177, 1990 (HC-J1, HC-J4); Takeuchi et al., Nucleic Acids Res., 18:4626, 1990 (HCV-JH); Choo et al., Proc. Natl. Acad. Sci, USA, 88:2451-2455, 1991 (HCV-1); Kato et al., Proc, Natl, Acad. Sci. USA, 87:9524-9528, 1990 (HCV-J); Takamizawa et al., J. Virol., 65:1105-1113, 1991 (HCV-BK); United States Patent No. 5,032,511 to Takahashi et al.; Ogata et al., Proc. Natl. Acad. Sci. USA, 88:3392-3396, 1991 (HCV-Hh); and International Application No. PCT/US88/04125.

The identified sequences have been shown herein to encode structural proteins of NANBV. The NANBV structural proteins are also shown herein to include antigenic epitopes useful for diagnosis of antibodies immunoreactive with structural proteins of NANBV, and for use in vaccines to induce neutralizing antibodies against NANBV. In particular, the NANBV antigens of this Invention are Hutch c59 isolate NANBV antigens.

The nucleotide sequence that codes for the amino terminal polyprotein portion of the structural genes of the Hutch strain of NANBV is contained in SEQ ID NO:1. By comparison to other NANBV isolates, to flavivirus, and to pestivirus, the nucleotide sequence contained in SEQ ID NO:1 is believed to encode structural proteins of NANBV, namely capsid and portions of envelope.

The structural antigens described herein are present in the putative capsid protein contained in SEQ ID NO:1 from amino acid residue positions 1-120, and are present in the amino terminal portion of the putative envelope protein contained in SEQ ID NO:1 from residue positions 121 to 326.

Nucleotide and amino acid residue sequences are defined herein from a starting base or amino acid residue position number to an end base or residue position number. It is understood that all such sequences include both the starting and end position numbers.

The complete sequence of the genome of the Hutch c59 isolate has also been determined and is described. Thus, the specification describes a DNA segment encoding the viral genome of the Hutch c59 isolate of NANBV contained in SEQ ID NO:46 from nucleotide position 1 to 9416.

The specification also describes a DNA segment encoding a NANBV structural protein that comprises a NANBV structural antigen, preferably capsid antigen. A particularly preferred capsid antigen includes an amino acid residue sequence represented by SEQ ID NO:1 from residue 1 to residue 20, from residue 21 to residue 40, from residue 2 to residue 40, or from residue 1 to residue 74. The DNA segment described herein preferably includes the nucleotide base sequence represented by SEQ ID NO:1 from base position 1 to base position 60, from base position 61 to base position 120, from base position 4 to base position 120, or from base position 1 to base position 222, respectively.

A polynucleotide is also described herein comprising a nucleotide sequence that encodes portions of the Hutch c59 isolate polyprotein, particularly portions of the sequence-specific regions of c59 in the V, V₁, V₂ or V₃ region.

Also described is a recombinant DNA molecule comprising a vector, preferably an expression vector, operatively linked to a DNA segment of the present invention. A preferred recombinant DNA molecule is pGEX-3X-690:691, pGEX-3X-690:694, pGEX-3X-693:691, PGEX-3X-15:17, pGEX-3X-15:18, pGEX-2T-15:17, pGEX-2T-CAP-A, pGEX-2T-CAP-B or pGEX-2T-CAP-A-B.

A NANBV structural protein is contemplated that comprises an amino acid residue sequence that defines a NANBV structural antigen, preferably a capsid antigen, and more preferably one that includes the amino acid residue sequence contained in SEQ ID NO:1 from residue 1 to residue 20, from residue 21 to residue 40, from residue 2 to residue 40, or from residue 1 to residue 74. Fusion proteins comprising a NANBV structural protein of this invention are also contemplated.

The invention provides for a method for detecting seroconversion associated with NANBV infection at early times after infection comprising:
(a) forming an aqueous immunoreaction admixture by admixing a body fluid sample with a NANBV capsid antigen together with C100-3 antigen;
(b) maintaining said aqueous immunoreaction admixture for a time period sufficient for allowing antibodies against the NANBV capsid antigen present in the body fluid sample to immunoreact with said NANBV capsid antigen to form an immunoreaction product; and
(c) detecting the presence of said immunoreaction product formed and thereby detecting early sero conversion.

The invention also relates to the use of NANBV capsid antigen for the preparation of a diagnostic composition for diagnosing seroconversion associated with NANBV infection at early times after infection.

Furthermore, described is a kit to be used for diagnosing seroconversion associated with NANBV infection at early times after infection in a body fluid sample comprising an NANBV capsid antigen selected from the group consisting of:
(a) a NANBV capsid antigen CAP-A having the amino acid sequence from the residue 1 to 20 of SEQ ID NO:1;
(b) a NANBV capsid antigen CAP-B having the amino acid sequence from the residue 21 to 40 of SEQ ID NO:1; and
(c) a NANBV capsid antigen CAP-N having the amino acid sequence from the residue 1 to 74 of SEQ ID NO:1.

The specification also describes antibody molecules that immunoreact with the Hutch c59 isolate of NANBV, but do not immunoreact with NANBV isolates HCV-1, HCV-BK, HCV-J, HC-J1, HC-J4, HCV-JH or HCV-Hh, i.e., c59-specific antibody molecules.

Further described is a culture of cells transformed with a recombinant DNA molecule described herein and methods of producing a NANBV structural protein of this invention using the culture.

Also contemplated is a composition comprising a NANBV structural protein. The composition is preferably characterized as being essentially free of (a) procaryotic antigens, and (b) other NANBV-related proteins.

Still further described is a diagnostic system in kit form comprising, in an amount sufficient to perform at least one assay, a NANBV structural protein composition, a polypeptide or a fusion protein of this invention, as a separately packaged reagent. Preferably, the diagnostic system contains the fusion protein affixed to a solid matrix.

Further contemplated is a method, preferably an in vitro method, of assaying a body fluid sample for the presence of antibodies against at least one of the NANBV structural antigens described herein. The method comprises forming an immunoreaction admixture by admixing (contacting) the body fluid sample with an immunological reagent such as a NANBV structural protein, polypeptide or fusion protein of this invention. The immunoreaction admixture is maintained for a time period sufficient for any of the antibodies present to immunoreact with the admixed immunological reagent to form an immunoreaction product, which product, when detected, is indicative of the presence of anti-NANBV structural protein antibodies. Preferably, the immunological reagent is affixed to a solid matrix when practicing the method.

The specification also describes an inoculum (or a vaccine) comprising an immunologically effective amount of a NANBV structural protein, polypeptide or fusion protein of this invention dispersed in a pharmaceutically acceptable carrier and/or diluent. The inoculum is essentially free of (a) procaryotic antigens, and (b) other NANBV-related proteins.

A prophylactic method for treating infection, which method comprises administering an inoculum of as described herein, is also disclosed.

### Brief Description of the Drawings

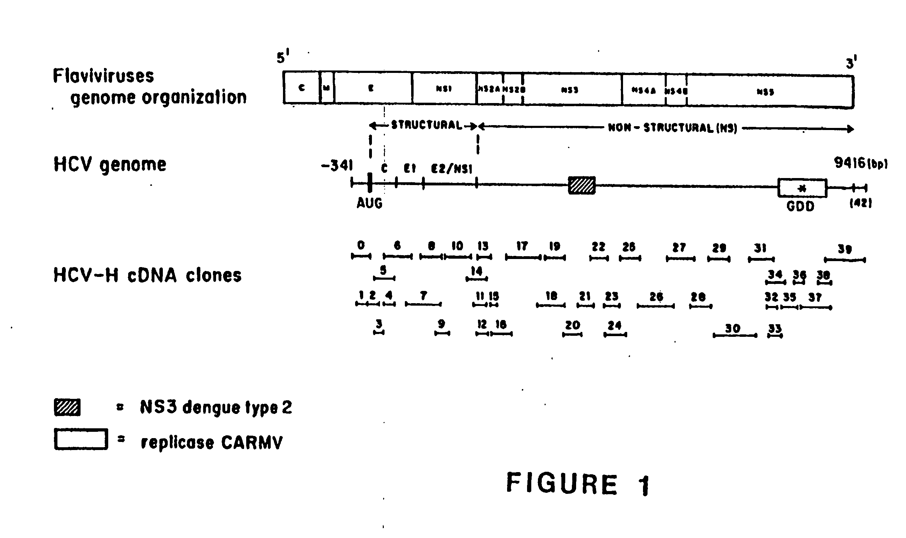

Figure 1 is a schematic representation of the HCv-Hc59 genome and location of HCV-Hc59 cDNA clones numbered from zero to 39. Alignment with the protein encoded by flaviviruses is shown as well as the putative domains in the HCV encoded genome. Regions of amino acid homology with the Dengue Type 2 Ns3 virus and the Carnation Mottle virus (CARMv) are indicated by striped and empty boxes, respectively.

### Detailed Description of the Invention

### A. Definitions

Amino Acid: All amino acid residues identified herein are in the natural L-configuration. In keeping with standard polypeptide nomenclature, J. Biol. Chem., 243:3557-59, (1969), abbreviations for amino acid residues are as shown in the following Table of Correspondence:

**TABLE OF CORRESPONDENCE**

| SYMBOL 3-Letter | AMINO ACID |
|---|---|
| Tyr | L-tyrosine |
| Gly | L-glycine |
| Phe | L-phenylalanine |
| Met | L-methionine |
| Ala | L-alanine |
| Ser | L-serine |
| Ile | L-isoleucine |
| Leu | L-leucine |
| Thr | L-threonine |
| Val | L-valine |
| Pro | L-proline |
| Lys | L-lysine |
| His | L-histidine |
| Gln | L-glutamine |
| Glu | L-glutamic acid |
| Glx | Gln or Glu |
| Trp | L-tryptophan |
| Arg | L-arginine |
| Asp | L-aspartic acid |
| Asn | L-asparagine |
| Asx | Asp or Asn |
| Cys | L-cysteine |
| Xaa | Unknown or other |

It should be noted that all amino acid residue sequences, typically referred to herein as "residue sequences", are represented herein by formulae whose left to right orientation is in the conventional direction of amino-terminus to carboxy-terminus. Antigen: A polypeptide or protein that is able to specifically bind to (immunoreact with) an antibody and form an immunoreaction product (immunocomplex). The site on the antigen with which the antibody binds is referred to as an antigenic determinant or epitope.

Nucleotide: a monomeric unit of DNA or RNA consisting of a sugar moiety (pentose), a phosphate, and a nitrogenous heterocyclic base. The base is linked to the sugar moiety via the glycosidic carbon (1' carbon of the pentose) and that combination of base and sugar is a nucleoside. When the nucleoside contains a phosphate group bonded to the 3' or 5' position of the pentose it is referred to as a nucleotide. A sequence of operatively linked nucleotides is typically referred to herein as a "base sequence", and is represented herein by a formula whose left to right orientation is in the conventional direction of 5'-terminus to 3'-terminus.

Duplex DNA: A double-stranded nucleic acid molecule comprising two strands of substantially complementary polynucleotide hybridized together by the formation of a hydrogen bond between each of the complementary nucleotides present in a base pair of the duplex. Because the nucleotides that form a base pair can be either a ribonucleotide base or a deoxyribonucleotide base, the phrase "duplex DNA" refers to either a DNA-DNA duplex comprising two DNA strands (ds DNA), or an RNA-DNA duplex comprising one DNA and one RNA strand.

Base Pair (bp): a partnership of adenine (A) with thymine (T), or of cytosine (C) with guanine (G) in a double stranded DNA molecule. In RNA, uracil (U) is substituted for thymine.

Complementary Nucleotide Sequence: a sequence of nucleotides in a single-stranded molecule of DNA or RNA that is sufficiently complementary to that on another single strand to specifically (non-randomly) hybridize to it with consequent hydrogen bonding.

Hybridization: the pairing of complementary nucleotide sequences (strands of nucleic acid) to form a duplex, heteroduplex or complex containing more than two single-stranded nucleic acids by the establishment of hydrogen bonds between/among complementary base pairs. It is a specific, i.e. non-random, interaction between/among complementary polynucleotides that can be competitively inhibited.

Hybridization Product: The product formed when a polynucleotide hybridizes to a single or double-stranded nucleic acid. When a polynucleotide hybridizes to a double-stranded nucleic acid, the hybridization product formed is referred to as a triple helix or triple-stranded nucleic acid molecule. Moser et al, Science, 238:645-50 (1987).

Nucleotide Analog: a purine or pyrimidine nucleotide that differs structurally from a A, T, G, C, or U, but is sufficiently similar to substitute for the normal nucleotide in a nucleic acid molecule. Inosine (I) is a nucleotide that can hydrogen bond with any of the other nucleotides, A, T, G, C, or U. In addition, methylated bases are known that can participate in nucleic acid hybridization.

### B. DNA Segments

In living organisms, the amino acid residue sequence of a protein or polypeptide is directly related via the genetic code to the deoxyribonucleic acid (DNA) sequence of the structural gene that codes for the protein. Thus, a structural gene can be defined in terms of the amino acid residue sequence, i.e., protein or polypeptide, for which it codes.

An important and well known feature of the genetic code is its redundancy. That is, for most of the amino acids used to make proteins, more than one coding nucleotide triplet (codon) can code for or designate a particular amino acid residue. Therefore, a number of different nucleotide sequences may code for a particular amino acid residue sequence. Such nucleotide sequences are considered functionally equivalent since they can result in the production of the same amino acid residue sequence in all organisms. Occasionally, a methylated variant of a purine or pyrimidine may be incorporated into a given nucleotide sequence. However, such methylations do not affect the coding relationship in any way.

The specification also describes an isolated DNA segment that comprises a nucleotide base sequence that encodes a NANBV structural protein comprising a NANBV structural antigen such as a capsid antigen, an envelope antigen, or both. Preferably, the structural antigen is immunologically related to the Hutch strain of NANBV.

More preferably, the encoded NANBV structural antigen has an amino acid residue sequence that corresponds, and preferably is identical, to the amino acid residue sequence contained in SEQ ID NO:1.

In one embodiment, the putative capsid antigen includes an amino acid residue sequence contained in SEQ ID NO:1 from residue 1 to residue 20, from residue 21 to residue 40, from residue 2 to residue 40, or from residue 1 to residue 74. In another embodiment, the capsid antigen includes the sequence contained in SEQ ID NO:1 from residue 69 to residue 120.

DNA segments disclosed herein include a base sequence represented by the base sequence contained in SEQ ID NO:1 from base position 1 to base position 222, from base position 205 to base position 360, from base position 361 to base position 528, or from base position 361 to base position 978.

The length of the nucleotide base sequence disclosed herein is no more than about 3, 000 bases, preferably no more than about 1,000 bases.

The amino acid residue sequence of a particularly preferred NANBV structural protein is contained in SEQ ID NO:2 from residue 1 to residue 315, in SEQ ID NO:3 from residue 1 to residue 252, in SEQ ID NO:4 from residue 1 to residue 252 and in SEQ ID NO:6 from residue 1 to residue 271.

A purified DNA segment as disclosed herein is substantially free of other nucleic acids that do not contain the nucleotide base sequences specified herein for a DNA segment of this invention, whether the DNA segment is present in the form of a composition containing the purified DNA segment, or as a solution suspension or particulate formulation. By substantially free is meant that the DNA segment is present as at least 10% of the total nucleic acid present by weight, preferably greater than 50 percent, and more preferably greater than 90 percent of the total nucleic acid by weight.

A DNA segment disclosed herein contains a nucleotide base sequence that defines a structural gene capable of expressing a fusion protein. The phrase "fusion protein" refers to a protein having a polypeptide portion operatively linked by a peptide bond to a second polypeptide portion defining a NANBV structural antigen as disclosed herein.

A preferred first polypeptide portion has an amino acid residue sequence corresponding to a sequence as contained in SEQ ID NO:2 from about residue 1 to about residue 221, and is derived from the protein glutathione-S-transferase (GST).

A preferred second polypeptide portion defining a NANBV structural antigen in a fusion protein includes an amino acid residue sequence represented by the sequence contained in SEQ ID NO:1 from residue 1 to residue 20, from residue 21 to residue 40, from residue 2 to residue 40, from residue 1 to residue 74 or from residue 69 to residue 120.

A fusion protein as disclosed herein can contain more than one polypeptide portion defining a NANBV structural antigen, as for example the combination of two polypeptide portions representing different structural antigens as shown by the amino acid residue sequence contained in SEQ ID NO:1 from residue 1 to residue 120, or in SEQ ID NO:1 from residue 1 to residue 326.

As disclosed herein, that portion of a fusion protein encoding DNA segment that codes for the polypeptide portion defining a NANBV capsid antigen includes a nucleotide base sequence corresponding to a sequence that codes for an amino acid residue sequence as contained in SEQ ID NO:1 from residue 1 to residue 20, from residue 21 to residue 40, from residue 2 to residue 40, or from residue 1 to residue 74, and more preferably includes a nucleotide base sequence corresponding to a base sequence as contained in SEQ ID NO:1 from base 1 to base 60, from base 61 to base 120, from base 4 to base 120, or from base 1 to base 222, respectively.

It is also disclosed herein that a DNA segment is bound to a complementary DNA segment, thereby forming a double stranded DNA segment. In addition, it should be noted that a double stranded DNA segment of this invention preferably has a single stranded cohesive tail at one or both of its termini.

It is also disclosed that a DNA segment comprises a nucleotide base sequence that encodes the genome of the Hutch isolate of NANBV. Preferably, the DNA segment has a nucleotide base sequence that encodes the amino acid residue sequence of the polyprotein produced by the genome of the Hutch c59 isolate, which amino acid residue sequence is shown in SEQ ID NO:46 from residue 1 to residue 3011. More preferably, the DNA segment in this embodiment has the nucleotide sequence shown in SEQ ID NO:46 from base 1 to base 9416.

A DNA segment encoding the c59 isolate genome is useful for the preparation of a hybridization standard or control in diagnostic methods based on nucleic acid hybridization using the polynucleotides, for the preparation of NANBV structural antigens or fusion proteins by recombinant DNA methods, for the preparation of infectious NANBV c59 isolate particles in culture, and the like, all of which are described herein.

Also disclosed herein is a fragment of a DNA segment of this invention corresponding to a portion of a NANBV genome or encoding a portion of a NANBV structural antigen. These fragments, when present in single stranded form or specified in the context of one strand of a double stranded DNA segment, are referred to herein as polynucleotides.

Where the polynucleotide is used to encode a NANBV structural antigen, or region of the Hutch c59 isolate polyprotein, the polynucleotide corresponds to the coding strand of a NANBV genome as described herein.

Thus, the specification describes a polynucleotide that comprises a nucleotide base sequence that includes a nucleotide base sequence that encodes an amino acid residue sequence corresponding to a portion of the polyprotein expressed by the Hutch isolate of NANBV. Preferably the polynucleotide encodes a sequence that corresponds to a portion of the amino acid residue sequence of the c59 isolate shown in SEQ ID NO:46 from residue 1 to residue 3011.

Particularly preferred are regions of the Hutch c59 isolate which are unique and thereby provide a means to distinguish the Hutch isolate, and more preferably the c59 isolate, from other isolates of NANBV on the basis of amino acid residue or nucleotide base sequence differences. Regions of the genome of the c59 isolate useful for distinguishing isolates contain differences in nucleotide base sequence, and preferably define differences in the encoded amino acid residue sequence, when compared to the nucleotide or amino acid residue sequence of the isolate to be distinguished.

Representative comparisons to identify Hutch isolate sequence differences are shown herein in the Examples, and particularly in Table 11.

A DNA segment or polynucleotide described herein can easily be prepared from isolated virus obtained from the blood of a NANBV-infected individual such as described herein or can be synthesized de novo by chemical techniques.

De novo chemical synthesis of a DNA segment or a polynucleotide can be conducted using any suitable method, such as, for example, the phosphotriester or phosphodiester methods. See Narang et al., Meth. Enzymol., 68:90, (1979); U.S. Patent No. 4,356,270; Itakura et al., Ann. Rev. Biochem., 53:323-56 (1989); Brown et al., Meth. Enzymol., 68:109, (1979); and Matteucci et al., J. Am. Chem. Soc., 103:3185 (1981).

Of course, by chemically synthesizing the structural gene portion, any desired modifications can be made simply by substituting the appropriate bases for those encoding a native amino acid residue. However, DNA segments including sequences identical to a segment contained in SEQ ID NOS 1, 2, 3, 4 or 6 are preferred.

Derivation of a polynucleotide from nucleic acids involves the cloning of a nucleic acid into an appropriate host by means of a cloning vector, replication of the vector and therefore multiplication of the amount of the cloned nucleic acid, and then the isolation of subfragments of the cloned nucleic acids. For a description of subcloning nucleic acid fragments, see Maniatis et al., Molecular Cloning: A Laboratory Manual, Cold Spring Harbor Laboratory, pp 390-401 (1982); and see U.S. Patents No. 4,416,988 and No. 4,403,036.

In preferred embodiments a polynucleotide probe has a size of less than about 200 nucleotides in length, preferably less than 100 nucleotides, and more preferably less than 30 nucleotides.

By "substantially complementary" and its grammatical equivalents in relation to a probe is meant that there is sufficient nucleotide base sequence similarity between a subject polynucleotide probe and a specific nucleic acid sequence present in a gene of interest that the probe is capable of hybridizing with the specific sequence under hybridizing conditions and form a duplex comprised of the probe and the specific sequence.

Therefore, the polynucleotide probe sequence may not reflect the exact sequence of the target sequence so long as the probe contains substantial complementarity with the target sequence. For example, a non-complementary polynucleotide can be attached to the one end of the probe, with the remainder of the probe sequence being substantially complementary to the target sequence. Such non-complementary polynucleotides might code for an endonuclease restriction site or a site for protein binding. Alternatively, non-complementary bases or longer sequences can be interspersed into the probe, provided the probe sequence has sufficient complementarity with the sequence of the target strand as to non-randomly hybridize therewith and thereby form a hybridization product under hybridization conditions.

The polynucleotide probe is provided in single-stranded form for maximum efficiency, but may alternatively be double stranded. If double stranded, the polynucleotide probe is first treated to separate its strands before being used in hybridization to

In addition, a DNA segment can be prepared by first synthesizing oligonucleotides that correspond to portions of the DNA.segment, which oligonucleotides are then assembled by hybridization and ligation into a complete DNA segment. Such methods are also well known in the art. See for example, Paterson et al., Cell, 48:441-452 (1987); and Lindley et al., Proc. Natl. Acad. Sci., 85:9199-9203 (1988), where a recombinant peptide, neutrophil-activated factor, was produced from the expression of a chemically synthesized gene in E. coli.

A DNA segment as described herein can be used for the preparation of rDNA molecules, in the construction of vectors for expressing a NANBV structural protein or fusion protein of this invention, or as a hybridization probe for detecting the presence of NANBV specific nucleic acid sequences in samples.

Where the use of a DNA segment is for preparing proteins, the specified amino acid residue is considered important, and the nucleotide base sequence of the DNA segment can vary based on the redundancy of the genetic code, as is well known, to provide for the desired amino acid residue sequence.

### C. Recombinant DNA Molecules

The present specification further describes a recombinant DNA (rDNA) that includes a DNA segment of the present invention operatively linked to a vector. A preferred rDNA of the present invention is characterized as being capable of directly expressing, in a compatible host, a NANBV structural protein or fusion protein of this invention. Preferred DNA segments for use in a rDNA are those described herein above.

By "directly expressing" is meant that the mature polypeptide chain of the protein is formed by translation alone as opposed to proteolytic cleavage of two or more terminal amino acid residues from a larger translated precursor protein. Preferred rDNAs of the present invention are the plasmids pGEX-3X-690:694 , pGEX-3X-693:691, pGEX-3X-690:691, pGEX-3X-15:17, pGEX-3X-15:18, pGEX-2T-15:17, pGEX-2T-CAP-A, pGEX-2T-CAP-B, and pGEX-2T-CAP-A-B described in Example 1.

A recombinant DNA molecule (rDNA) as described herein can be produced by operatively linking a vector to a DNA segment of the present invention. Exemplary rDNA molecules and the methods for their preparation are described in Example 1.

In another embodiment, a rDNA molecule as described herein comprises a vector operatively linked to a DNA segment comprising a nucleotide base sequence that encodes the genome of the Hutch isolate of NANBV. Preferably, the rDNA molecule includes a nucleotide base sequence that encodes the amino acid residue sequence of the polyprotein produced by the genome of the Hutch c59 isolate, which amino acid residue sequence is shown in SEQ ID NO:46 from residue 1 to residue 3011. More preferably, the rDNA molecule in this embodiment includes a nucleotide base sequence shown in SEQ ID NO:46 from base 1 to base 9416.

As used herein, the term "vector" refers to a DNA molecule capable of autonomous replication in a cell and to which another DNA segment can be operatively linked so as to bring about replication of the attached segment. Typical vectors are plasmids, bacteriophages and the like. Vectors capable of directing the expression of a NANBV structural protein or fusion protein are referred to herein as "expression vectors". Thus, a recombinant DNA molecule (rDNA) is a hybrid DNA molecule comprising at least two nucleotide sequences not normally found together in nature.

The choice of vector to which a DNA segment of the present invention is operatively linked depends directly, as is well known in the art, on the functional properties desired, e.g., protein expression, and the host cell to be transformed, these being limitations inherent in the art of constructing recombinant DNA molecules. However, a vector as described herein is at least capable of directing the replication, and preferably also expression, of the recombinant or fusion protein structural gene included in DNA segments to which it is operatively linked.

In preferred embodiments, a vector described herein includes a prokaryotic replicon (ori); i.e., a DNA sequence having the ability to direct autonomous replication and maintenance of the recombinant DNA molecule extrachromosomally in a procaryotic host cell, such as a bacterial host cell, transformed therewith. Such replicons are well known in the art. In addition, those embodiments that include a procaryotic replicon also typically include a gene whose expression confers drug resistance to a bacterial host transformed therewith. Typical bacterial drug resistance genes for use in these vectors are those that confer resistance to ampicillin or tetracycline. Typical of such vector plasmids are pUC8, pUC9, pBR322 and pBR329 available from Biorad Laboratories, (Richmond, CA).

Those vectors that include a procaryotic replicon can also include a procaryotic promoter capable of directing the expression (transcription and translation) of the gene encoding a NANBV structural protein or fusion protein in a bacterial host cell, such as E. coli, transformed therewith. A promoter is an expression control element formed by a DNA sequence that permits binding of RNA polymerase and subsequent transcription initiation to occur. Promoter sequences compatible with bacterial hosts are typically provided in plasmid vectors containing convenient restriction sites for insertion of a DNA segment of the present invention. A typical vector is pPL-lambda available from Pharmacia, (Piscataway, NJ).

Vector plasmids having a bacterial promoter that is inducible with IPTG are the pTTQ plasmids available from Amersham (Arlington Heights, IL), and the pKK223-3 plasmid available from Pharmacia. Additional expression vectors for producing in procaryotes a cloned gene product in the form of a fusion protein are well known and commercially available.

Although the expression vectors pGEX-3X and pGEX-2T have been used as exemplary in producing the fusion proteins described herein, other functionally equivalent expression vectors can be used. Functionally equivalent vectors contain an expression promoter that is inducible by IPTG for fusion protein expression in E. coli, and a configuration such that upon insertion of the DNA segment into the vector a fusion protein is produced. Commercially available vectors functionally equivalent to the vectors pGEX-3X and pGEX-2T used herein include the pGEMEX-1 plasmid vector from Promega (Madison, WI) that produces a fusion between the amino terminal portion of the T7 gene 10 protein and the cloned insert gene, the pMAL plasmid vectors from New England Biolabs (Beverly, MA) that produce a fusion with the maltose binding protein (MBP) encoded by the mal E gene, and the pGEX-3X and pGEX-2T plasmids from Pharmacia that produce a fusion with the enzyme glutathione-s-transferase (GST) and the cloned insert gene, respectively.

The construction and use of the pGEX-3X and PGEX-2T vectors have been described by Smith et al., Gene, 67:31-40 (1988).

In particularly preferred embodiments, a fusion protein contains a GST derived polypeptide-portion as an added functional domain operatively linked to a NANBV structural antigen of this invention. Any inducible promoter driven vector, such as the vectors pTTQ, pKK223-3, pGEX-3X or pGEX-2T described above and the like, can be used to express a GST-NANBV structural protein, referred to herein as a GST:NANBV fusion protein. Thus, although the pGEX-3X and pGEX-2T vectors are described as exemplary, the DNA molecules described herein are not to be construed as limited to these vectors, because the disclosure in one embodiment is directed to an rDNA for expression of a protein having NANBV structural antigens fused to GST and not drawn to the vector per se.

A variety of methods have been developed to operatively link DNA segments to vectors via complementary cohesive termini. For instance, complementary homopolymer tracts can be added to the DNA segment to be inserted and to the vector DNA. The vector and DNA segment are then joined by hydrogen bonding between the complementary homopolymeric tails to form recombinant DNA molecules.

Synthetic linkers containing one or more restriction sites provide an alternative method of joining the DNA segment to vectors. A DNA segment generated by endonuclease restriction digestion is treated with bacteriophage T4 DNA polymerase or E. coli DNA polymerase I, enzymes that remove protruding, 3', single-stranded termini with their 3'-5' exonucleolytic activities and fill in recessed 3' ends with their polymerizing activities.

The combination of these activities therefore generates blunt-ended DNA segments. The blunt-ended segments are then incubated with a large molar excess of linker molecules in the presence of an enzyme that is able to catalyze the ligation of blunt-ended DNA molecules, such as bacteriophage T4 DNA ligase. Thus, the products of the reaction are DNA segments carrying polymeric linker sequences at their ends. These DNA segments are then cleaved with the appropriate restriction enzyme and ligated to an expression vector that has been cleaved with an enzyme that produces termini compatible with those of the DNA segment.

Synthetic linkers containing a variety of restriction endonuclease sites are commercially available from a number of sources including International Biotechnologies, Inc., New Haven, CN.

Also contemplated by the present invention are RNA equivalents of the above described recombinant DNA molecules.

### D. Transformed Cells and Cultures

The specification also discloses a host cell transformed with a recombinant DNA molecule described herein.

The term "host cell" includes both eukaryotic and prokaryotic hosts. Preferred rDNA molecules for use in a transformed cell are those described herein above and preferably are rDNAs capable of expressing a recombinant or fusion protein. Specific preferred embodiments of transformed cells are those which contain an rDNA molecule having one of the preferred DNA segments described herein above, and particularly cells transformed with the rDNA plasmid pGEX-3X-690:694, pGEX-3X-693:691, pGEX-3X-690:691, pGEX-3X-15:17, pGEX-3X-15:18, pGEX-2T-15:17, pGEX-2T-CAP-A, pGEX-2T-CAP-B, or pGEX-2T-CAP-A-B.

Bacterial cells are preferred procaryotic host cells and typically are a strain of E. coli, such as, for example, the E. coli strain DH5α available from Bethesda Research Laboratories, Inc., Bethesda, MD. Transformation of appropriate cell hosts with a recombinant DNA molecule of the present invention is accomplished by well known methods that typically depend on the type of vector used. With regard to transformation of procaryotic host cells, see, for example, Cohen et al., Proc. Natl. Acad. Sci. USA, 69:2110 (1972); and Sambrook et al., Molecular Cloning, A Laboratory Manual, 2nd Ed., Cold Spring Harbor Laboratory, Cold Spring Harbor, NY (1989).

Successfully transformed cells, i.e., cells that contain a recombinant DNA molecule described herein can be identified by well known techniques. For example, cells resulting from the introduction of an rDNA described herein can be isolated as single colonies. Cells from those colonies can be harvested, lysed and their DNA content examined for the presence of the rDNA using a method such as that described by Southern, J. Mol. Biol., 98:503 (1975) or Berent et al., Biotech., 3:208 (1985).

In addition to directly assaying for the presence of rDNA, cells transformed with the appropriate rDNA can be identified by well known immunological methods when the rDNA is capable of directing the expression of a NANBV structural protein. For example, cells successfully transformed with an expression vector disclosed herein produce proteins displaying NANBV structural protein antigenicity. Samples of cells suspected of being transformed are harvested and assayed for the presence of a NANBV structural antigen using antibodies specific for that antigen, such antibodies being described further herein.

Thus, in addition to the transformed host cells themselves, the specification also describes a culture of those cells, preferably a monoclonal (clonally homogeneous) culture, or a culture derived from a monoclonal culture, in a nutrient medium. Preferably, the culture also contains a protein displaying NANBV structural protein antigenicity.

Nutrient media useful for culturing transformed host cells are well known in the art and can be obtained from several commercial sources.

### E. Methods for Producing NANBV Structural Proteins, Polypeptides and Fusion Proteins

Further described herein is a method for producing recombinant proteins and fusion proteins of this invention.

The present method entails initiating a culture comprising a nutrient medium containing host cells, preferably E. coli cells, transformed with a recombinant DNA molecule that is capable of expressing a NANBV structural protein or a fusion protein. The culture is maintained for a time period sufficient for the transformed cells to express the NANBV structural protein or fusion protein. The expressed protein is then recovered from the culture.

Expression vectors and expression vector culturing conditions for producing NANBV structural proteins are generally well known in the art. Such vectors and culturing conditions can be altered without affecting the spirit of the present invention. However, preferred are the vectors designed specifically for the production of proteins not normally found in the host cell used to express a NANBV structural protein. Exemplary are the vectors that contain inducible promoters for directing the expression of DNA segments that encode the NANBV structural protein. Vectors with promoters inducible by IPTG are also well known. See for example plasmids pTTQ and pKK223-3 available from Amersham and Pharmacia respectively. Particularly preferred are the promoters inducible by IPTG present in the pGEX vectors pGEX-3X and pGEX-2T described herein.

Using vectors with inducible promoters, expression of NANBV structural proteins requires an induction phase at the beginning of the above described maintenance step for expressing the protein, as is known and described in detail in Example 2.

Methods for recovering an expressed protein from a culture are well known in the art and include fractionation of the protein-containing portion of the culture using well known biochemical techniques. For instance, the methods of gel filtration, gel chromatography, ultrafiltration, electrophoresis, ion exchange, affinity chromatography and the like, such as are known for protein fractionations, can be used to isolate the expressed proteins found in the culture. In addition, immunochemical methods, such as immunoaffinity, immunoadsorption and the like can be performed using well known methods.

Particularly preferred are isolation methods that utilize the presence of the polypeptide portion defining glutathione-S-transferase (GST) as a means to separate the fusion protein from complex mixtures of protein. Affinity adsorption of a GST-containing fusion protein to a solid phase containing glutathione affixed thereto can be accomplished as described by Smith et al., Gene, 67:31 (1988). Alternatively, the GST-containing polypeptide portion of the fusion protein can be separated from the NANBV structural antigen by selective cleavage of the fusion protein at a specific proteolytic cleavage site, according to the methods of Smith et al., Gene, 67:31 (1988). Exemplary isolation methods are described in Examples 5 and 6.

In addition to its preparation by the use of a rDNA expression vector, a NANBV structural protein comprising a NANBV structural antigen can be prepared in the form of a synthetic polypeptide.

Polypeptides can be synthesized by any of the techniques that are known to those skilled in the polypeptide art. Synthetic chemistry techniques, such as a solid-phase Merrifield-type synthesis, are preferred for reasons of purity, antigenic specificity, freedom from undesired side products, ease of production and the like, and can be carried out according to the methods described in Merrifield et al., J. Am. Chem. Soc., 85:2149-2154 (1963) and Houghten et al., Int. J. Pept, Prot. Res., 16:311-320 (1980). An excellent summary of the many techniques available can be found in J.M. Steward and J.D. Young, "Solid Phase Peptide synthesis", W.H. Freeman Co., San Francisco, 1969; M. Bodanszky, et al., "Peptide Synthesis", John Wiley & Sons, Second Edition, 1976 and J. Meienhofer, "Hormonal Proteins and Peptides", Vol. 2, p. 46, Academic Press (NY), 1983 for solid phase peptide synthesis, and E. Schroder and K. Kubke, "The peptides", Vol. 1, Academic Press (New York), 1965 for classical solution synthesis,

Appropriate protective groups usable in such synthesis are described in the above texts and in J.F.W. McOmie, "Protective Groups in Organic Chemistry", Plenum Press, New York, 1973.

A subject polypeptide includes any chemical derivative of a polypeptide whose amino acid residue sequence is shown herein. Therefore, a present polypeptide can be subject to various changes where such changes provide for certain advantages in its use.

"Chemical derivative" refers to a subject polypeptide having one or more residues chemically derivatized by reaction of a functional side group. Such derivatized molecules include for example, those molecules in which free amino groups have been derivatized to form amine hydrochlorides, p-toluene sulfonyl groups, carbobenzoxy groups, t-butyloxycarbonyl groups, chloroacetyl groups or formyl groups. Free carboxyl groups may be derivatized to form salts, methyl and ethyl esters or other types of esters or hydrazides. Free hydroxyl groups may be derivatized to form 0-acyl or O-alkyl derivatives. The imidazole nitrogen of histidine may be derivatized to form N-im-benzylhistidine. Also included as chemical derivatives are those peptides which contain one or more naturally occurring amino acid derivatives of the twenty standard amino acids. For examples: 4-hydroxyproline may be substituted for proline; 5-hydroxylysine may be substituted for lysine; 3-methylhistidine may be substituted for histidine; homoserine may be substituted for serine; and ornithine may be substituted for lysine. Polypeptides of the present invention also include any polypeptide having one or more additions relative to the sequence of a polypeptide whose sequence is shown herein, so long as the requisite activity is maintained.

Additional residues may also be added at either terminus for the purpose of providing a "linker" by which the polypeptides of this invention can be conveniently affixed to a label or solid matrix, or carrier. Preferably the linker residues do not form NANBV structural antigens.

Labels, solid matrices and carriers that can be used with the polypeptides of this invention are described herein below.

Amino acid residue linkers are usually at least one residue and can be 40 or more residues, more often 1 to 10 residues, but do not form NANBV epitopes. Typical amino acid residues used for linking are tyrosine, cysteine, lysine, glutamic and aspartic acid, or the like. In addition, a subject polypeptide can differ, unless otherwise specified, from the natural sequence of the NANBV polyprotein by the sequence being modified by terminal-NH₂ acylation, e.g., acetylation, or thioglycolic acid amidation, by terminal-carboxlyamidation, e.g., with ammonia, methylamine, and the like.

When coupled to a carrier to form what is known in the art as a carrier-hapten conjugate, a polypeptide of the present invention is capable of inducing antibodies that immunoreact with NANBV. In view of the well established principle of immunologic cross-reactivity, the present invention therefore contemplates antigenically related variants of the polypeptides described herein. An "antigenically related variant" is a subject polypeptide that is capable of inducing antibody molecules that immunoreact with a polypeptide described in this specification and with NANBV.

Any peptide described herein may be used in the form of a pharmaceutically acceptable salt. Suitable acids which are capable of forming salts with the peptides of the present invention include inorganic acids such as hydrochloric acid, hydrobromic acid, perchloric acid, nitric acid, thiocyanic acid, sulfuric acid, phosphoric acetic acid, propionic acid, glycolic acid, lactic acid, pyruvic acid, oxalic acid, malonic acid, succinic acid, maleic acid, fumaric acid, anthranilic acid, cinnamic acid, naphthalene sulfonic acid, sulfanilic acid or the like.

Suitable bases capable of forming salts with the peptides of the present invention include inorganic bases such as sodium hydroxide, ammonium hydroxide, potassium hydroxide and the like; and organic bases such as mono-, di- and tri-alkyl and aryl amines (e.g. triethylamine, diisopropyl amine, methyl amine, dimethyl amine and the like) and optionally substituted ethanolamines (e.g. ethanolamine, diethanolamine and the like).

In general, the solid-phase synthesis methods contemplated comprise the sequential addition of one or more amino acid residues or suitably protected amino acid residues to a growing peptide chain. Normally, either the amino or carboxyl group of the first amino acid residue is protected by a suitable, selectively removable protecting group. A different, selectively removable protecting group is utilized for amino acids containing a reactive side group such as lysine.

Using a solid phase synthesis as exemplary, the protected or derivatized amino acid is attached to an inert solid support through its unprotected carboxyl or amino group. The protecting group of the amino or carboxyl group is then selectively removed and the next amino acid in the sequence having the complimentary (amino or carboxyl) group suitably protected is admixed and reacted under conditions suitable for forming the amide linkage with the residue already attached to the solid support. The protecting group of the amino or carboxyl group is then removed from this newly added amino acid residue, and the next amino acid (suitably protected) is then added, and so forth. After all the desired amino acids have been linked in the proper sequence, any remaining terminal and side group protecting groups (and solid support) are removed sequentially or concurrently, to afford the final polypeptide.

### F. NANBV Structural Protein and Fusion Protein Compositions

In another embodiment, the present specification describes a composition containing a NANBV structural protein, preferably isolated, comprising an amino acid residue sequence that defines a NANBV structural antigen described herein.

By isolated is meant that a NANBV structural protein of this invention is present in a composition as a major protein constituent, typically in amounts greater than 10% of the total protein in the composition, but preferably in amounts greater than 90% of the total protein in the composition.

A NANBV structural antigen, as used herein, is a structural protein coded by the genome of NANBV and has the properties of an antigen as defined herein, namely, to be able to immunoreact specifically with an antibody. NANBV structural proteins have been tentatively designated as capsid and envelope, and have been partially characterized as described herein to contain the NANBV structural antigens capsid and envelope, respectively.

NANBV capsid antigen as described herein comprises an amino acid residue sequence that is immunologically related in sequence to the putative Hutch strain NANBV capsid antigen, whose sequence is contained in SEQ ID NO:1 from residue 1 to residue 120.

By "immunologically related" is meant that sufficient homology in amino acid sequence is present in the two protein sequences being compared that antibodies specific for one protein immunoreact (cross-react) with the other protein. Immunological cross-reactivity can be measured by methods well known including the immunoassay methods described herein.

As used herein, the phrase "recombinant protein" refers to a protein of at least 20 amino acid residues in length, and preferably at least 50 residues, that includes an amino acid residue sequence that corresponds, and preferably is identical, to a portion of the NANBV structural protein contained in SEQ ID NO:1.

In preferred embodiments a NANBV structural protein includes an amino acid residue sequence that is immunologically related to, and preferably is identical to, the sequence contained in SEQ ID NO:1 from residue 1 to residue 20, from residue 21 to residue 40, from residue 2 to residue 40, or from residue 1 to residue 74. The NANBV structural protein with the indicated sequence is particularly preferred for use in diagnostic methods and systems because the capsid antigens contained therein were demonstrated herein to be particularly useful in detecting acute NANBV infection. Related NANBV structural proteins include a sequence contained in SEQ XD NO:1 from residue 1 to residue 120, from residue 1 to residue 176, and from residue 1 to residue 326. Exemplary are the proteins described herein having a sequence contained in SEQ ID NO:2 from residue 1 to residue 315, in SEQ ID NO:3 from residue 1 to residue 252, in SEQ ID NO:4 from residue 1 to residue 252, or in SEQ ID NO:6 from residue 1 to residue 271.

In another embodiment a NANBV structural protein includes an amino acid residue sequence that is immunologically related to, and preferably is identical to, the sequence contained in SEQ ID NO:1 from residue 69 to residue 120. An exemplary NANBV structural protein has the sequence of the expressed protein coded for by the rDNA plasmid pGEX-3X-693:691.

In another embodiment, a NANBV structural protein is contemplated that comprises an amino acid residue sequence according to a polypeptide described herein.

In preferred embodiments a NANBV structural protein is essentially free of both procaryotic antigens (i.e., host cell-specific antigens) and other NANBV-related proteins. By "essentially free" is meant that the ratio of NANBV structural antigen to foreign antigen, such as procaryotic antigen, or other NANBV-related protein is at least 10:1, preferably is 100:1, and more preferably is 200:1.

The presence and amount of contaminating protein in a NANBV structural protein preparation can be determined by well known methods. Preferably, a sample of the composition is subjected to sodium dodecyl sulfate-polyacrylamide gel electrophoresis (SDS-PAGE) to separate the NANBV structural protein from any protein contaminants present. The ratio of the amounts of the proteins present in the sample is then determined by densitometric soft laser scanning, as is well known in the art. See Guilian et al., Anal, Biochem., 129:277-287 (1983).

A NANBV structural protein can be prepared as an isolated protein, and more preferably essentially free of procaryotic antigens or NANBV non-structural antigens by the methods disclosed herein for producing NANBV structural proteins. Particularly preferred are methods which rely on the properties of a polypeptide region of a fusion protein, which region is present in the fusion protein to facilitate separation of the fusion protein from host cell proteins on the basis of affinity. Exemplary are the GST-containing fusion proteins whose amino acid residue sequences are contained in SEQ ID NOS:2, 3, 4 or 6 wherein the GST polypeptide region of each provides the fusion protein with a functional domain having an affinity to bind to the normal substrate for GST, namely glutathione. The purification of a fusion protein having a GST polypeptide region is described further herein.

In a related embodiment, the invention describes a polypeptide that defines a NANBV antigen. Thus, the invention contemplates a polypeptide corresponding to a region of the NANBV polyprotein that defines an antigenic determinant of the virus that is useful as a NANBV antigen in serological assays or in an inoculum to induce anti-NANBV antisera, as described herein.

A polypeptide described herein comprises a sequence of amino acids of about 7 to about 200 residues in length, preferably about 20 to 150 residues in length, that comprises an amino acid residue sequence defined by the nucleotide sequence of a polynucleotide disclosed herein.

A preferred polypeptide comprises an amino acid residue sequence that includes an amino acid residue sequence selected from the group of sequences consisting of residue 391 to residue 404 of SEQ ID NO:46, residue 246 to residue 256 of SEQ ID NO:46, residue 461 to residue 466 of SEQ ID NO:46, residue 473 to residue 482 of SEQ ID NO:46, and residue 2356 to residue 2379 of SEQ ID NO:46. In particularly preferred embodiments the polypeptide has an amino acid residue sequence that corresponds to the sequence shown in SEQ ID NO:46.

Insofar as a polypeptide is useful to distinguish Hutch isolates, the specification describes a polypeptide having a length from about 7 to about 200 amino acid residues and comprising an amino acid residue sequence that corresponds to a portion of the sequence of the Hutch c59 isolate of NANBV shown in SEQ ID NO:46. In this embodiment, the polypeptide has at least one amino acid residue difference in sequence when compared to the amino acid residue sequence of an isolate of NANBV selected from the group consisting of HCV-1, HCV-BK, HCV-J, HC-J1, HC-J4, HCV-JH and HCV-Hh.

Preferably, a polypeptide is immunoreactive with anti-Hutch strain NANBV antisera when measured in standard serological immunoassays such as are described herein.

In another embodiment, a composition comprising an isolated fusion protein is also contemplated by the present invention that comprises a NANBV structural protein of this invention operatively linked at one or both termini to another polypeptide by a peptide bond. The added polypeptide can be any polypeptide designed to increase the functional domains present on the fusion protein. The added functional domains are included to provide additional immunogenic epitopes, to add mass to the fusion protein, to alter the solubility of the fusion protein, to provide a means for affinity-based isolation of the fusion protein, and the like. Exemplary added functional domains are the Thrombin or Factor Xa specific cleavage sites provided when a subject fusion protein is produced in the vector pGEX-3X or pGEX-2T, respectively, as described herein. An additional exemplary domain is the GST-derived protein domain that allows rapid isolation using affinity chromatography to a solid phase containing glutathione affixed thereto.

A Thrombin or Factor Xa cleavage Site-containing domain is used herein, in one embodiment, to allow production of an NANBV structural protein free of the GST function domain. Exemplary is the protein produced in Example 6 having an amino acid residue sequence contained in SEQ ID NO:2 from residue 226 to residue 315. The Factor Xa cleavage site-containing domain is also used in the commercially available fusion protein expression vector pMAL available from New England Biolabs (Beverly, MA) described herein.

In a related embodiment a NANBV structural protein is produced by Thrombin cleavage of a protein produced using the pGEX-2T vector, such as a protein having an amino acid residue sequence contained in SEQ ID NO:3 from residue 225 to residue 252, in SEQ ID NO:4 from residue 225 to residue 252, or in SEQ ID NO:6 from residue 225 to residue 271.

A fusion protein disclosed herein includes an amino acid residue sequence corresponding from its amino-terminus to its carboxy-terminus to the amino acid residue sequence contained in SEQ ID NO:1 from residue 1 to residue 20, from residue 21 to residue 40, from residue 2 to residue 40, from residue 1 to residue 74, from residue 69 to residue 120, from residue 121 to residue 176, or from residue 121 to residue 326. A preferred fusion protein has a sequence corresponding to, and more preferably is identical to, the amino acid residue sequence in SEQ ID NO:2 from residue 1 to residue 315, in SEQ ID NO:3 from residue 1 to residue 252, in SEQ ID NO:4 from residue 1 to residue 252, or in SEQ ID NO:6 from residue 1 to residue 271. Other preferred fusion proteins are defined by the amino acid residue sequence of the expressed protein coding sequence present in the rDNA plasmids pGEX-3X-690:694, pGEX-3X-690:691, pGEX-3X-693:691, pGEX-3X-15:17, pGEX-3X-15:18, pGEX-2T-15:17, pGEX-2T-CAP-A, pGEX-2T-CAP-B, and pGEX-2T-CAP-A-8.

The phrase "fusion protein", when used herein refers to an isolated protein as it was defined for a NANBV structural protein as described herein. Thus, an isolated fusion protein is a composition having a fusion protein as described herein in amounts greater than 10 percent of the total protein in the composition, and preferably greater than 90 percent of the total protein in the composition.

A preferred fusion protein is a heterologous fusion protein, that is, a fusion protein that contains a polypeptide portion derived from a protein originating in a heterologous species of virus, organism, pathogen or animal, i.e., a non-NANBV protein. Preferably a heterologous fusion protein contains a non-NANBV polypeptide portion that is not immunologically related to a NANBV structural antigen described herein.

A fusion protein may contain a functional domain that provides an immunogenic or antigenic epitope other than the NANBV structural antigen defined herein and is preferably derived from a separate pathogen, or from several pathogens. The functional domain is immunogenic where that domain is present to form a polyvalent vaccine or immunogen for the purpose of inducing antibodies immunoreactive with both NANBV structural protein and a second pathogen. The functional domain is antigenic where that domain is present to form a polyvalent antigen for use in diagnostic systems and methods for detecting at least two species of antibodies.

Of particular interest in this embodiment are fusion proteins designed to include a functional domain that is derived from other hepatitis-causing viruses, such as Hepatitis B virus, and Hepatitis A virus. These viruses have been well characterized to contain antigenic determinants and immunogenic determinants suitable for use in the fusion protein described herein, and provide the advantage of multipurpose biochemical reagents in both diagnostic and vaccine applications. Additionally, the included functional domain can contain amino acid sequences from other pathogens, preferably those which may also infect individuals with NANBV hepatitis, such as HIV.

Preferred NANBV structural proteins or fusion proteins comprising a NANBV structural antigen of the present invention are in non-reduced form, i.e., are substantially free of sulfhydryl groups because of intramolecular Cys-Cys bonding.

In preferred compositions, the NANBV structural protein or fusion protein as described herein, is present, for example, in liquid compositions such as sterile suspensions or solutions, or as isotonic preparations containing suitable preservatives.

One such composition useful for inducing anti-NANBV structural protein antibodies in a mammal is referred to as a vaccine and contains a NANBV structural protein or fusion protein described herein.

### H. Antibody Compositions

An antibody to be employed in context of this invention is a composition containing antibody molecules that immunoreact with a NANBV structural antigen, with the Hutch isolate of NANBV, preferably the c59 isolate, and with a NANBV structural protein, polypeptide or fusion protein of the present invention (anti-NANBV structural protein antibody molecules). A preferred antibody contains antibody molecules that immunoreact with an epitope present on a polypeptide having an amino acid residue sequence contained in SEQ ID NO:1 from residue 1 to residue 326, preferably that immunoreacts with a polypeptide having the sequence contained in SEQ ID NO:1 from residue 1 to residue 20, from residue 21 to residue 40, from residue 2 to residue 40, from residue 1 to residue 74, from residue 49 to residue 120, or from residue 121 to residue 326.

In addition, it is preferred that anti-NANBV structural protein antibody molecules do not immunoreact with the NANBV isolates HCV-1, HCV-BK, HCV-J, HC-J1, HC-J4, HCV-JH or HCV-Hh, or with the C-100-3 antigen described herein, and available in the commercial assay available from Ortho Diagnostics, Inc.

An antibody may typically be produced by immunizing a mammal with an inoculum containing Hutch c59 isolate or a NANBV structural protein or polypeptide of this invention and thereby induce in the mammal antibody molecules having immunospecificity for the NANBV structural antigens described herein. The antibody molecules are then collected from the mammal and isolated to the extent desired by well known techniques such as, for example, by using DEAE Sephadex to obtain the IgG fraction.

To enhance the specificity of the antibody, the antibodies may be purified by immunoaffinity chromatography using solid phase-affixed immunizing NANBV structural protein. The antibody is contacted with the solid phase-affixed NANBV structural protein for a period of time sufficient for the NANBV structural protein to immunoreact with the antibody molecules to form a solid phase-affixed immunocomplex. The bound antibodies are separated from the complex by standard techniques.

To produce an antibody composition that does not immunoreact with the C-100-3 antigen or the NANBV isolates identified above, immunoadsorption methods are used to remove the undesirable immunospecificities. Immunoadsorption methods to remove immunospecificities are generally well known and involve first contacting the antibody composition with a solid phase having affixed thereto one or more of the antigens or NANBV isolates to form an immunoadsorption admixture. Preferably, there is an excess of antigen or NANBV in the solid phase in proportion to the antibodies in the composition having the undesirable immunospecificities in the immunoadsorption admixture.

The immunoadsorption admixture is then maintained under immunoreaction conditions and for a time period sufficient for an immunocomplex to form in the solid phase. Thereafter, the liquid and solid phases are separated, and the liquid phase is retained having the undesirable antibody molecules immunoadsorbed away onto the solid phase.

Particularly preferred is an antibody composition containing c59 isolate specific antisera, formed by immunization with Hutch c59 isolate, or preferably with a polypeptide described herein selected as defined herein to have an amino acid residue sequence unique to c59 and preferably derived from the V, V1, V2 or V3 variable regions of NANBV. Thereafter, the produced antibody composition is immunoadsorbed to remove antibodies immunoreactive with NANBV isolates other than c59 as described herein.

The antibody so produced can be used, inter alia, in the diagnostic methods and systems of the present invention to detect NANBV structural antigens as described herein present in a body sample.

The word "inoculum" in its various grammatical forms is used herein to describe a composition containing a NANBV structural antigen of this invention as an active ingredient used for the preparation of antibodies immunoreactive with NANBV structural antigens.

The preparation and use of an inoculum for production of an antibody of this invention largely parallels the descriptions herein for a vaccine insofar as the vaccine is also designed to induce the production of antibodies and is exemplary of the preparation and use of an inoculum. A key difference is that the inoculum is formulated for use on an animal rather than a human, as is well known.

A preferred antibody is a monoclonal antibody and can be used in the same manner as disclosed herein for antibodies described herein.

A monoclonal antibody is typically composed of antibodies produced by clones of a single cell called a hybridoma that secretes (produces) but one kind of antibody molecule. The hybridoma cell is formed by fusing an antibody-producing cell and a myeloma or other self-perpetuating cell line. The preparation of such antibodies were first described by Kohler and Milstein, Nature 256:495-497 (1975).

The hybridoma supernates so prepared can be screened for immunoreactivity with a NANBV structural antigen such as the NANBV structural protein used in the inoculum to induce the antibody-producing cell. Other methods of producing monoclonal antibodies, the hybridoma cell, and hybridoma cell cultures are also well known.

Also contemplated by this invention is the hybridoma cell, and cultures containing a hybridoma cell that produce a monoclonal antibody of this invention.

It should be understood that in addition to the aforementioned carrier ingredients the pharmaceutical formulation described herein can include, as appropriate, one or more additional carrier ingredients such as diluents, buffers, binders, surface active agents, thickness, lubricants, preservatives (including antioxidants) and the like, and substances included for the purpose of rendering the formulation isotonic with the blood of the intended recipient. Typically, a preservative such as merthiolate (at a 1:5000 dilution of a 1% solution) is added to eliminate the risk of microbial contamination, even if sterile techniques were employed in the manufacture of the inoculum.

### I. Diagnostic Systems and Methods

### 1. Diagnostic Systems

The present invention contemplates a diagnostic system for assaying for the presence of anti-NANBV antibodies or NANBV structural antigens in a body sample according to the diagnostic methods described herein.

A diagnostic system in kit form includes, in an amount sufficient for at least one assay according to the methods described herein, a NANBV structural protein, polypeptide or fusion protein or a combination thereof described herein, or an anti-NANBV antibody composition disclosed herein, as a separately packaged reagent. Instructions for use of the packaged reagent are also typically included.

"Instructions for use" typically include a tangible expression describing the reagent concentration or at least one assay method parameter such as the relative amounts of reagent and sample to be admixed, maintenance time periods for reagent/ sample admixtures, temperature, buffer conditions and the like.

In preferred embodiments, a diagnostic system of the present invention further includes a label or indicating means capable of signaling the formation of a complex containing a NANBV structural antigen, a recombinant protein or an anti-NANBV antibody.

As used herein, the terms "label" and "indicating means" in their various grammatical forms refer to single atoms and molecules that are either directly or indirectly involved in the production of a detectable signal to indicate the presence of a complex. Any label or indicating means can be linked to or incorporated in a reagent species such as an antibody or monoclonal antibody, or can be used separately, and those atoms or molecules can be used alone or in conjunction with additional reagents. Such labels are themselves well-known in clinical diagnostic chemistry.

The label can be a fluorescent labeling agent that chemically binds to antibodies or antigens without denaturing them to form a fluorochrome (dye) that is a useful immunofluorescent tracer. Suitable fluorescent labeling agents are fluorochromes such as fluorescein isocyanate (FIC), fluorescein isothiocyanite (FITC), 5-dimethylamine-1-naphthalenesulfonyl chloride (DANSC), tetramethylrhodamine isothiocyanate (TRITC), lissamine, rhodamine 8200 sulfonyl chloride (RB 200 SC), a chelate-lanthanide bound (e.g., Eu, Tb, Sm) and the like. A description of immunofluorescence analysis techniques is found in DeLuca, "Immunofluorescence Analysis", in Antibody As a Tool, Marchalonis, et al., eds., John Wiley & Sons, Ltd., pp. 189-231 (1982).

In preferred embodiments, the label is an enzyme, such as horseradish peroxidase (HRP), glucose oxidase, alkaline phosphatase or the like. In such cases where the principal label is an enzyme such as HRP or glucose oxidase, additional reagents are required to visualize the fact that an antibody-antigen complex (immunoreactant) has formed. Such additional reagents for HRP include hydrogen peroxide and an oxidation dye precursor such as diaminobenzidine. An additional reagent useful with HRP is 2,2'-azino-di-(3-ethyl-benzthiazoline-6-sulfonic acid) (ABTS).

Radioactive elements are also useful labeling agents and are used illustratively herein. An exemplary radiolabeling agent is a radioactive element that produces gamma ray emissions. Elements which themselves emit gamma rays, such as ¹²⁴I, ¹²⁵I, ¹²⁸I, ¹³¹I and ⁵¹Cr represent one class of gamma ray emission-producing radioactive element indicating groups. Particularly preferred is ¹²⁵I. Another group of useful labeling means are those elements such as ¹¹C, ¹⁸F, ¹⁵O and ¹³N which themselves emit positrons. The positrons so emitted produce gamma rays upon encounters with electrons present in the animal's body. Also useful is a beta emitter, such as ¹¹¹ indium, ³H, ³⁵S, ¹⁴C, or ³²P.

Additional labels have been described in the art and are suitable for use in the diagnostic systems of this invention. For example, the specific affinity found between pairs of molecules can be used, one as a label affixed to the specific binding agent and the other as a means to detect the presence of the label. Exemplary pairs are biotin:avidin, where biotin is the label, and peroxidase:anti-peroxidase (PAP), where peroxidase is the label.

The linking of labels, i.e., labeling of, polypeptides and proteins is well known in the art. For instance, antibody molecules produced by a hybridoma can be labeled by metabolic incorporation of radioisotope-containing amino acids provided as a component in the culture medium. See, for example, Galfre et al., Meth. Enzymol., 73:3-46 (1981). The techniques of protein conjugation or coupling through activated functional groups are particularly applicable. See, for example, Aurameas, et al., Scand. J. Immunol., Vol. 8 Suppl. 7:7-23 (1978), Rodwell et al., Biotech., 3:889-894 (1984), and U.S. Pat. No. 4,493,795.

The diagnostic systems can also include, preferably as a separate package, a specific binding agent. A "specific binding agent" is a molecular entity capable of selectively binding a reagent species, which in turn is capable of reacting with a product described herein but is not itself a protein expression product described herein. Exemplary specific binding agents are antibody molecules such as anti-human IgG or anti-human IgM, complement proteins or fragments thereof, protein A, and the like. Preferably the specific binding agent can bind the anti-NANBV antibody to be detected when the antibody is present as part of an immunocomplex.

In preferred embodiments the specific binding agent is labeled. However, when the diagnostic system includes a specific binding agent that is not labeled, the agent is typically used as an amplifying means or reagent. In these embodiments, the labeled specific binding agent is capable of specifically binding the amplifying means when the amplifying means is bound to a reagent species-containing complex.

The diagnostic kits of the present invention can be used in an "ELISA" format to detect the presence or quantity of antibodies in a body fluid sample such as serum, plasma or saliva. "ELISA" refers to an enzyme-linked immunosorbent assay that employs an antibody or antigen bound to a solid phase and an enzyme-antigen or enzyme-antibody conjugate to detect and quantify the amount of an antigen or antibody present in a sample. A description of the ELISA technique is found in Chapter 22 of the 4th Edition of Basic and Clinical Immunology by D.P. Sites et al., published by Lange Medical Publications of Los Altos, CA in 1982 and in U.S. Patents No. 3,654,090; No. 3,850,752; and No. 4,016,043.

Thus, in preferred embodiments, the NANBV structural protein, polypeptide, fusion protein or anti-NANBV antibody described herein can be affixed to a solid matrix to form a solid support that is separately packaged in the subject diagnostic systems.

The reagent is typically affixed to the solid matrix by adsorption from an aqueous medium although other modes of affixation, well known to those skilled in the art, can be used.

Useful solid matrices are well known in the art. Such materials include the cross-linked dextran available under the trademark SEPHADEX from Pharmacia Fine Chemicals (Piscataway, NJ); agarose; beads of polystyrene about 1 micron to about 5 millimeters in diameter available from Abbott Laboratories of North Chicago, IL; polyvinyl chloride, polystyrene, cross-linked polyacrylamide, nitrocellulose- or nylon-based webs such as sheets, strips or paddles; or tubes, plates or the wells of a microtiter plate such as those made from polystyrene or polyvinylchloride.

The NANBV structural protein, polypeptide, fusion protein, anti-NANBV antibody, polynucleotides, labeled specific binding agent or amplifying reagent of any diagnostic system described herein can be provided in solution, as a liquid dispersion or as a substantially dry power, e.g., in lyophilized form. Where the indicating means is an enzyme, the enzyme's substrate can also be provided in a separate package of a system. A solid support such as the before-described microtiter plate and one or more buffers can also be included as separately packaged elements in this diagnostic assay system.

The packages discussed herein in relation to diagnostic systems are those customarily utilized in diagnostic systems. Such packages include glass and plastic (e.g., polyethylene, polypropylene and polycarbonate) bottles, vials, plastic and plastic-foil laminated envelopes and the like.

### 2. Diagnostic Methods

The present invention contemplates any diagnostic method that results in detecting anti-NANBV structural protein antibodies or NANBV structural antigens in a body sample using a NANBV structural protein, polypeptide, fusion protein or anti-NANBV structural antigen antibody described herein as an immunochemical reagent to form an immunoreaction product whose amount relates, either directly or indirectly, to the amount of material to be detected in the sample. Those skilled in the art will understand that there are numerous well known clinical diagnostic chemistry procedures in which an immunochemical reagent of this invention can be used to form an immunoreaction product whose amount relates to the amount of specific antibody or antigen present in a body sample.

Various heterogenous and homogenous protocols, either competitive or noncompetitive, can be employed in performing an assay method of this invention.

To detect the presence of anti-NANBV structural protein antibodies in a patient, a body sample, and preferably a body fluid sample such as blood, plasma, serum, urine or saliva from the patient, is contacted by admixture under biological assay conditions with a NANBV antigenic molecule described herein such as a NANBV structural protein, and preferably with a polypeptide or fusion protein of the present invention, to form an immunoreaction admixture. The admixture is then maintained for a period of time sufficient to allow the formation of a NANBV antigenic molecule-antibody molecule immunoreaction product (immunocomplex). The presence, and preferably the amount, of complex can then be detected as described herein. The presence of the complex is indicative of anti-NANBV antibodies in the sample.

In preferred embodiments the presence of the immunoreaction product formed between NANBV antigenic molecules and a patient's antibodies is detected by using a specific binding reagent as discussed herein. For example, the immunoreaction product is first admixed with a labeled specific binding agent to form a labeling admixture. A labeled specific binding agent comprises a specific binding agent and a label as described herein. The labeling admixture is then maintained under conditions compatible with specific binding and for a time period sufficient for any immunoreaction product present to bind with the labeled specific binding agent and form a labeled product. The presence, and preferably amount, of labeled product formed is then detected to indicate the presence or amount of immunoreaction product.

In preferred embodiments the diagnostic methods of the present invention are practiced in a manner whereby the immunocomplex is formed and detected in a solid phase, as disclosed for the diagnostic systems herein.

Thus, in a preferred diagnostic method, the NANBV structural protein or polypeptide is affixed to a solid matrix to form the solid phase. It is further preferred that the specific binding agent is protein A, or an anti-human Ig, such as IgG or IgM, that can complex with the anti-NANBV structural protein antibodies immunocomplexed in the solid phase with the NANBV structural protein. Most preferred is the use of labeled specific binding agents where the label is a radioactive isotope, an enzyme, biotin or a fluorescence marker such as lanthanide as described for the diagnostic systems, or detailed by references shown below.

In this solid phase embodiment, it is particularly preferred to use a recombinant protein that contains the antigen defined by the amino acid residue sequence contained in SEQ ID NO:1 from residue 1 to residue 20, from residue 21 to residue 40, from residue 2 to residue 40, or from residue 1 to residue 74, as embodied in the fusion proteins as described in Example 7.

In another preferred diagnostic method, the NANBV antigenic molecule of the invention is affixed to solid matrix as described above, and dilutions of the biological sample are subjected to the immunocomplexing step by contacting dilutions of sample with the solid surface and removing non-bound materials. Due to the multivalence of antibodies present in biological samples from infected individuals (bivalent for IgG, pentavalent for IgM) subsequent addition of labeled NANBV structural protein, polypeptide or fusion protein disclosed herein to this admixture will become attached to the solid phase by the sample antibody serving as bridge between the solid phase NANBV antigenic molecules as disclosed and the soluble, labeled molecules. The presence of label in the solid phase indicates the presence and preferably the amount of specific antibody in the sample. One skilled in the art can determine a range of dilutions and determine therefrom a concentration of labeled antigen in the solid phase. The biological sample and the labeled NANBV antigenic molecules described herein can be admixed prior to, or simultaneously with contacting the biological sample with the solid phase allowing the trimolecular complex to form at the solid phase by utilizing the bridging property of bivalent or multivalent specific antibody. As a particularly useful label, biotinylated NANBV antigenic molecules described herein can be the labeled antigen, allowing the subsequent detection by addition of an enzyme-streptavidin, or an enzyme-avidin complex, followed by the appropriate substrate. Enzymes such as horseradish peroxidase, alkaline phosphatase, B-galactosidase or urease are frequently used and these, and other, along with several appropriate substrates are commercially available. Preferred labels with a marker which allows direct detection of the formed complex include the use of a radioactive isotope, such as, eg., iodine, or a lanthanide chelate such as Europium.

Enzyme immunoassay techniques, whether direct or competition assays using homogenous or heterogenous assay formats, have been extensively described in the art: Exemplary techniques can be found in Maggio, Enzyme Immunoassay, CRC Press, Cleveland, OH (1981); and Tijssen, "Practice and Theory of Enzyme Immunoassays", Elsevier, Amsterdam (1988).

Biological assay conditions are those that maintain the biological activity of the NANBV antigenic molecules and the anti-NANBV structural protein antibodies in the immunoreaction admixture. Those conditions include a temperature range of about 4°C to about 45°C, preferably about 37°C, a pH value range of about 5 to about 9, preferably about 7, and an ionic strength varying from that of distilled water to that of about one molar sodium chloride, preferably about that of physiological saline. Methods for optimizing such conditions are well known in the art.

Also contemplated are immunological assays capable of detecting the presence of immunoreaction product formation without the use of a label. Such methods employ a "detection means", which means are themselves well-known in clinical diagnostic chemistry.

Exemplary detection means include methods known as biosensors and include biosensing methods based on detecting changes in the reflectivity of a surface (surface plasmon resonance), changes in the absorption of an evanescent wave by optical fibers or changes in the propagation of surface acoustical waves.

Another embodiment contemplates detection of the immunoreaction product employing time resolved fluorometry (TR-FIA), where the label used is able to produce a signal detectable by TR-FIA. Typical labels suitable for TR-FIA are metal-complexing agents such as a lanthanide chelate formed by a lanthanide and an aromatic beta-diketone, the lanthanide being bound to the antigen or antibody via an EDTA-analog so that a fluorescent lanthanide complex is formed.

The principle of time-resolved fluorescence is described by Soini et al, Clin. Chem., 25:353-361 (1979), and has been extensively applied to immunoassay. See for example, Halonen et al., Current Topics in Microbiology and Immunology, 104: 133-146 (1985); Suonpaa et al., Clinica Chimica Acta, 145:341-348 (1985): Lovgren et al., Talanta, 31:909-916 (1984); U.S. Patent Nos. 4,374,120 and 4,569,790; and published International Patent Application Nos. EPO 139 675 and WO87/02708. A preferred lanthanide for use in TR-FIA is Europium.

Regents and systems for practicing the TR-FIA technology are available through commercial suppliers (Pharmacia Diagnostics, Uppsala, Sweden).

Particularly preferred are the solid phase immunoassays described herein in Example 7, performed as a typical "Western Blot".

The present diagnostic methods may be practiced in combination with other separate methods for detecting the appearance of anti-NANBV antibodies in species infected with NANBV. For example, a composition of this invention may be used together with commercially available C100-3 antigen (Ortho Diagnostics, Inc., Raritan, N.J.) in assays to determine the presence of either or both antibody species immunoreactive with the two antigens.

### Examples

The following examples are given for illustrative purposes only and do not in any way limit the scope of the invention.

### Example 1. Production of Recombinant DNA Molecules

### A. Isolation of NANBV Clones and Sequence Analysis

### (1) Isolation of NANBV RNA and Preparation of cDNA

As a source for NANB virions, blood was collected from a chimpanzee infected with the Hutchinson (Hutch) strain exhibiting acute phase NANBH. Plasma was clarified by centrifugation and filtration. NANB virions were then isolated from the clarified plasma by immunoaffinity chromatography on a column of NANBV IgG (Hutch strain) coupled to protein G sepharose. NANBV RNA was eluted from the sepharose beads by soaking in guanidinium thiocyanate and the eluted RNA was then concentrated through a cesium chloride (CsCl) cushion. Sambrook et al., Molecular Cloning: A Laboratory Manual, Sambrook et al., eds. Second Edition, Cold Spring Harbor Laboratory Press, NY (1989).

The purified NANBV RNA in picogram amounts was used as a template in a primer extension reaction admixture containing random and oligo dT primers, dNTPs, and reverse transcriptase to form first strand cDNAs. The resultant first strand cDNAs were used as templates for synthesis of second strand cDNAs in a reaction admixture containing DNA polymerase I and RNAse H to form double stranded (ds) cDNAs (Sambrook et al., supra). The synthesized ds cDNAs were amplified using an asymmetric synthetic primer-adaptor system wherein sense and anti-sense primers were annealed to each other and ligated to the ends of the double stranded NANBV cDNAs with T4 ligase under blunt-end conditions to form cDNA-adaptor molecules. Polymerase chain reaction (PCR) amplification was performed as described below by admixing the cDNA-adaptor molecules with the same positive sense adaptor primers, dNTPs and TAQ polymerase (Promega Biotec, Madison, WI) to prepare amplified NANBV cDNAs. The resultant amplified NANBV cDNA sequences were then used as templates for subsequent amplification in a PCR reaction with specific NANBV oligonucleotide primers.

### (2) Synthesis of Oligonucleotides for Use in NANBV cloning

Oligonucleotides were selected to correspond to the 5' sequence of Hepatitis C which putatively encodes the NANBV structural-capsid and envelope proteins (HCJl sequence: Okamoto et al., Jap. J. Exp. Med., 60:167-177, 1990). The selected oligonucleotides were synthesized on a Pharmacia Gene Assembler according to the manufacturer's instruction, purified by polyacrylamide gel electrophoresis and have nucleotide base sequences and consecutive SEQ ID NOs beginning with 15 and ending with 23 as shown in Table 1.

**TABLE 1**

| SYNTHETIC OLIGONUCLEOTIDES | | | |
|---|---|---|---|
| Oligonucleotide Designation^{a} | Putative NANBV Region | Oligonucleotide Sequence | SEQ ID NO |
| 690 (+) | Capsid 1-21 | ATGAGCACGATTCCCAAACCT | 15 |
| 693 (+) | Capsid 146-162 | GAGGAAGACTTCCGAGC | 16 |
| 694 (-) | Capsid 208-224 | GTCCTGCCCTCGGGCCG | 17 |
| 691 (-) | Capsid 340-359 | ACCCAAATTGCGCGACCTACG | 18 |
| 14 (+) | Envelope 356-374 | TGGGTAAGGTCATCGATAC | 19 |
| 15 (+) | Envelope 361-377 | AAGGTCATCGATACCCT | 20 |
| 18 (-) | Envelope 512-529 | AGATAGAGAAAGAGCAAC | 21 |
| 16 (-) | Envelope 960-981 | GGACCAGTTCATCATCATATAT | 22 |
| 17 (-) | Envelope 957-976 | CAGTTCATCATCATATCCCA | 23 |

| | | | |
|---|---|---|---|
| a The oligonucleotides are numerically defined and their polarity is indicated as (+) and (-) indicating the sequence corresponds to the sense and anti-sense coding strand, respectively. All sequences are listed in the 5' to 3' orientation. | | | |

### (3) PCR Amplification of NANBV cDNA

PCR amplification was performed by admixing the primer-adapted amplified cDNA sequences prepared in Example 1A(1) with the synthetic oligonucleotides 690 and 694 as primer (primer pairs 690:694). The resulting PCR reaction admixture contained the primer-adapted amplified cDNA template, oligonucleotides 690 and 694, dNTPs, salts (KCl and MgCl₂) and TAQ polymerase. PCR amplification of the cDNA was conducted by maintaining the admixture at a 37°C annealing temperature for 30 cycles. Aliquots of samples from the first round of amplification were reamplified at a 55°C annealing temperature for 30 cycles under similar conditions.

### (4) Preparation of Vectors containing PCR Amplified ds DNA

Aliquots from the second round of PCR amplification were subjected to electrophoresis on a 5% acrylamide gel. After separation of the PCR reaction products, the region of the gel containing DNA fragments corresponding to the expected 690:694 amplified product of approximately 224 bp was excised and purified following standard electroelution techniques (Sambrook et al., supra). The purified fragments were kinased and cloned into the pUC18 plasmid cloning vector at the Sma I polylinker site to form a plasmid containing the DNA segment 690:694 operatively linked to pUC18.

The resulting mixture containing pUC18 and a DNA segment corresponding to the 690:694 sequence region was then transformed into the E. coli strain JM83. Plasmids containing inserts were identified as lac' (white) colonies on X-gal medium containing ampicillin. pUC18 plasmids which contained the 690:694 DNA segment were identified by restriction enzyme analysis and subsequent electrophoresis on agarose gels, and were designated pUC18 690:694 rDNA molecules.

### (5) Sequencing of Hepatitis Clones that Encode the Putative Capsid Protein

Two independent colonies believed to contain a pUC18 vector having the NANBV Hutch strain 690:694 DNA segment (pUC18 690:694) that codes for the amino terminus of the putative capsid protein were amplified and used to prepare plasmid DNA by CsCl density gradient centrifugation by standard procedures (Sambrook et al., supra). The plasmids were sequenced using ³⁵S dideoxy procedures with pUC 18 specific primers. The two plasmids were independently sequenced on both DNA strands to assure the accuracy of the sequence. The resulting sequence information is presented as base 1 to base 224 of SEQ ID NO:1.

Plasmid pUC18 690:694 contains a NANBV DNA segment that is 224 bp in length and when compared to the HCJ1 prototype sequence reveals two nucleotide substitutions and one amino acid residue difference in the amino terminal region of the putative capsid protein.

### (6) Preparation of NANBV Clones from the 5' End of the Genome

To obtain the sequence of the NANBV Hutch genome encoding the remainder of the capsid region (Okamoto et al., supra), the oligonucleotides 693 and 691 (described in Table 1) were used in PCR reactions. cDNA was prepared as described in Example 1A(1) to viral NANBV RNA from Hutch and used in PCR amplification as described in Example 1A(3) with the oligonucleotide pair 693:691. The resultant PCR amplified ds DNA was then cloned into pUC18 cloning vectors and screened for inserts as described in Example 1A(4) to form pUC 18 693:691. Clones were then sequenced with pUC18 specific primers as described in Example 1A(5).

Plasmid pUC18 693:691 contains a NANBV DNA segment that is 157 bp in length and spans nucleotide bases 203 to 360 of SEQ ID NO:1. The segment does not extend to the sequence of the 693 primer used for generating the fragment. The sequence of this fragment reveals three nucleotide differences when compared to the known sequence of HCJ1 and does not have any corresponding amino acid changes to the HCJ1 sequence.

To obtain the sequence of the NANBV Hutch genome encoding the putative envelope region (Okamoto et al., supra), the oligonucleotide primers 14 through 18 (described in Table 1) were used in various combinations with NANBV Hutch RNA samples. As a source of NANBV RNA, a liver biopsy specimen from a chimpanzee inoculated with the Hutch strain at 4 weeks post-inoculation and exhibiting acute infection was used. The biopsied sample was first frozen and then ground. The resultant powder was the treated with guanidine isothiocyanate for the extraction of RNA. RNA was extracted from the guanidium-treated liver samples with phenol in the presence of SDS at 65°C. The liver samples were extracted a second time, and then extracted with chloroform. The extracted RNA was precipitated at -20°C with isopropanol and sodium acetate.

The purified liver-derived RNA was used as a template in primer extension reactions with the oligonucleotides 18 and 16 to generate NANBV specific-cDNAs. To prepare cDNA to the Hutch strain amino-terminal protein coding sequences, anti-sense oligonucleotides, 18 and 16, were annealed to liver-derived Hutch RNA in the presence of dNTPs and reverse transcriptase at 42°C to form primer extension products. The first round of PCR amplification of the two cDNAs was performed by admixing the primer extension reaction products with separate pairs of oligonucleotides 14:16 (16 primed cDNA) and 14:18 (18 primed cDNA) for 30 cycles at 55°C annealing temperature. The PCR reactions were performed on the above admixture as in 1A(3). Aliquots from the 14:16 and 14:18 amplifications were used as templates for the second round of amplification in which the oligonucleotide pairs 15:17 and 15:18, respectively, were used as primers.

PCR reaction products from each of the primer pair reactions were analyzed by electrophoresis on low melt agarose gels. Following separation, the regions of the gel containing DNA fragments corresponding to the expected 15:17 and 15:18 amplified products of approximately 617 bp and 168 bp, respectively, were excised and eluted from the gel slices at 65°C. The resultant eluted fragments were purified by phenol and chloroform extractions. To clone the 15:17 and 15:18 fragments, the purified fragments were separately treated with the Klenow fragment of DNA polymerase and kinase for subsequent subcloning into the SmaI site of the pBluescript plasmid vector (Stratagene Cloning Systems, La Jolla, CA). Transformed E. coli DH5 colonies were analyzed for plasmid insert by restriction enzyme analysis as described in Example 1A(4).

pBluescript plasmid containing 15:17 or 15:18 DNA segments were purified using large scale CsCl plasmid preparation protocols. The DNA segments present in the amplified and purified plasmids were each sequenced as described in Example 1A(5).

The sequence of the 15:17 DNA segment is contained in SEQ ID NO:1 from nucleotide 361 to 978. The sequence of the 15:18 DNA segment is also presented in SEQ ID NO:1 from nucleotide 361 to 529. These two clones overlap by 168 bp of the 15:18 DNA segment.

The sequence results indicate that the 15:17 DNA segment differs by 30 nucleotides when compared to the HCJ1 sequence (Okamoto et al., supra) and also differs by ten amino acid residues. The 15:18 DNA segment differs by seven nucleotides and by three amino acid residues when compared to HCJ1. In the overlap region, the two DNA segments differ at two nucleotide bases, namely, bases 510 and 511, where DNA segment 15:18 contains a C in place of a T and an A in place of a G, respectively, which results in a change of a serine in place of a glycine amino acid residue, at residue 171 of SEQ ID NO:1. The reason for these differences is unknown and may be due to a PCR artifact.

### B. Production of Recombinant DNA (rDNA) at Encodes a Fusion Protein

### (1) Isolation of the 690:694 Fragment from the pUC 18 Clone and Introduction of the Fragment into the pGEX-3X Expression Vector

The pUC18 vector containing the 690:694 DNA segment was subjected to restriction enzyme digestion with Eco RI and Bam HI to release the DNA segment that includes a sequence contained in SEQ ID NO:1 from base 1 to base 224 from the pUC18 vector. The released DNA segment was subjected to acrylamide gel electrophoresis and the DNA segment containing the 224 bp NANBV insert plus portions of the pUC 18 polylinker was then excised and eluted from the gel as described in Example 1A(4). The eluted DNA segment was extracted with a mixture of phenol and chloroform, and precipitated.

The precipitated DNA segment was resuspended to a concentration of 25 µg/ml in water and treated with the Klenow fragment of DNA polymerase I and dNTP to fill in the staggered ends created by the restriction digestion. The resultant blunt-ended 690:694 segment was admixed with the bacterial expression vector, pGEX-3X, (available from Pharmacia Inc., Piscataway, NJ) which was linearized with the blunt end restriction enzyme Sma I. The admixed DNAs were then covalently linked (ligated) by maintaining the admixture overnight at 16°C in the presence of ligase buffer and 5 units of T4 DNA ligase to form a plasmid of 690:694 DNA segment operatively linked to pGEX-3X.

### (2) Selection and Verification of Correctly Oriented Ligated Insert

The ligation mixture containing the pGEX-3X vector and the 690:694 DNA segment was transformed into host E. coli strain W3110. Plasmids containing inserts were identified by selection of host bacteria containing vector in Luria broth (LB) media containing ampicillin. Bacterial cultures at stationary phase were subjected to alkaline lysis protocols to form a crude DNA preparation. The DNA was digested with the restriction enzyme Xho I. The single Xho I site, which cleaves within the 690:694 DNA segment between nucleotide positions 173 to 178 of SEQ ID NO:1, but not within the pGEX-3X vector, was used to screen for vector containing the 690:694 DNA segment.

Several 690:694 DNA segment-containing vectors were amplified and the resultant amplified vector DNA was purified by CsCl density gradient centrifugation. The DNA was sequenced across the inserted DNA segment ligation junctions by ³⁵S dideoxy methods with a primer that hybridized to the pGEX-3X sequence at nucleotide positions 614 to 633 contained in SEQ ID NO:2. Vectors containing 690:694 DNA segment having the correct coding sequence for in-frame translation of a NANBV structural protein were thus identified and selected to form pGEX-3X-690:694.

### (3) Structure of the Fusion Protein

The pGEX-3X vector is constructed to allow for inserts to be placed at the C terminus of Sj26, a 26-kDa glutathione S-transferase (GST; EC 2.5.1.18) encoded by the parasitic helminth Schistosoma japonicum. Insertion of the 690:694 NANBV fragment in-frame behind Sj26 allows for the synthesis of the Sj26-NANBV fusion polypeptide. The NANBV polypeptide can be cleaved from the GST carrier by digestion with the site-specific protease factor Xa (Smith et al., Gene, 67:31-40, 1988).

The nucleotide and predicted amino acid sequence of the pGEX-3X-690:694 fusion transcript from the GST sequence through the 690:694 insert is presented in SEQ ID NO:2. The resulting rDNA molecule, pGEX-3X-690:694, is predicted to encode a NANBV fusion protein having the amino acid residue sequence contained in SEQ ID NO:2 from amino acid residue 1 to residue 315. The resulting protein product generated from the expression of the plasmid is referred to as both the GST:NANBV 690:694 fusion protein and the CAP-N fusion protein.

### C. Production of Recombinant DNAs (rDNAs) that Encode NANBV Capsid and Envelope Fusion Proteins

pGEX-3X-693:691: Plasmid pGEX-3X-693:691 was formed by first subjecting the plasmid pUC 18 693:691 prepared in Example 1A(6) to restriction enzyme digestion with Eco RI and Bam HI as performed in Example 1B(1). The resultant released DNA segment having a sequence contained in SEQ ID NO:1 from base 205 to base 360 was purified as performed in Example 1B(1). The purified DNA segment was admixed with and ligated to the pGEX-3X vector which was linearized by restriction enzyme digestion with Eco RI and Bam HI in the presence of T₄ ligase at 16°C to form the plasmid pGEX-3X-693:691.

A pGEX-3X plasmid containing a 693:691 DNA segment was identified by selection as performed in Example 1B(2) with the exception that crude DNA preparations were digested with Eco RI and Bam HI to release the 693:691 insert. A pGEX-3X vector containing a 693:691 DNA segment having the correct coding sequence for in-frame translation of a NANBV structural protein was identified by sequence analysis as performed in Example 1B(2) and selected to form pGEX-3X-693:691.

The resulting vector encodes a fusion protein (GST:NANBV 693:691) that is comprised of an amino-terminal polypeptide portion corresponding to residues 1 to 221 of GST as contained in SEQ ID NO:2, an intermediate polypeptide portion corresponding to residues 222 to 225 and defining a cleavage site for the protease Factor Xa, a linker protein corresponding to residues 226 to 230 consisting of the amino acid residue sequence (SEQ ID NO:25):
Gly Ile Pro Asn Ser
encoded by the nucleotide base sequence (SEQ ID NO:24):
GGG ATC CCC AAT TCA, respectively;
a carboxy-terminal polypeptide portion corresponding to residues 231 to 282 defining a NANBV capsid antigen having the amino acid residue sequence 69 to 120 in SEQ ID NO:1, and a carboxy-terminal linker portion corresponding to residues 283 to 287 consisting of the amino acid residue sequence (SEQ ID NO:27):
Asn Ser Ser END
encoded by the nucleotide base sequence (SEQ ID NO:26):
AAT TCA TCG TGA, respectively.

pGEX-3X-15:18: Plasmid pGEX-3X-15:18 was formed by first subjecting the plasmid Bluescript 15:18 prepared in Example 1A(6) to restriction enzyme digestion with Eco RV and Bam HI and the Bam HI cohesive termini were filled in as performed in Example 1B(1). The resultant released DNA segment having a sequence contained in SEQ ID NO:1 from base 361 to base 528 was purified as performed in Example 1B(1). The purified DNA segment was admixed with and ligated to the pGEX-3X vector which was linearized by restriction enzyme digestion with Sma I as performed in 1B(1) to form the plasmid pGEX-3X-15:18.

A pGEX-3X plasmid containing a 15:18 DNA segment was identified by selection as performed in Example 1B(2) and crude DNA preparations were cut with Eco RI and Bam HI to release the 15:18 inserts. A pGEX-3X vector containing a 15:18 DNA segment having the correct coding sequence for in-frame translation of a NANBV structural protein was identified as performed in Example 1B(2) and selected to form pGEX-3X-15:18.

The resulting vector encodes a fusion protein (GST:NANBV 15:18) that is comprised of an amino-terminal polypeptide portion corresponding to residues 1 to 221 of GST, an intermediate polypeptide portion corresponding to residues 222 to 225 and defining a cleavage site for the protease Factor Xa, a linker protein corresponding to residues 226 to 234 consisting of the amino acid residue sequence (SEQ ID NO:29):
Gly Ile Pro Ile Glu Phe Leu Gln Pro,
encoded by the nucleotide base sequence (SEQ ID NO:28):
GGG ATC CCC ATC GAA TTC CTG CAG CCC,
respectively; a carboxy-terminal polypeptide portion corresponding to residues 235 to 290 defining a NANBV envelope antigen having the amino acid residue sequence 121 to 176 in SEQ ID NO:1, and a carboxy-terminal linker portion corresponding to residues 291 to 298 consisting of a amino acid residue sequence (SEQ ID NO:31):
Trp Gly Ile Gly Asn Ser Ser END
encoded by the nucleotide base sequence (SEQ ID NO:30):
TGG GGG ATC GGG AAT TCA TCG TGA, respectively.

pGEX-3X-15:17: Plasmid pGEX-3X-15:17 was formed by first subjecting the plasmid Bluescript 15:17 prepared in Example 1A(6) to restriction enzyme digestion with Eco RI and Bam HI and the cohesive termini were filled in as performed in Example 1B(1). The resultant released DNA segment having a sequence contained in SEQ ID NO:1 from base 361 to base 978 was purified as performed in Example 1B(1). The purified DNA segment was admixed with and ligated to the pGEX-3X vector which was linearized by restriction enzyme digestion with Sma I as performed in Example 1B(1) to form the plasmid pGEX-3X-15:17.

A pGEX-3X plasmid containing a 15:17 DNA segment was identified by selection as performed in Example 1B(2) and DNA preparations were digested with Eco RI and Bam HI as indicated above. pGEX-3X vector containing a 15:17 DNA segment having the correct coding sequence for in-frame translation of a NANBV structural protein was identified as performed in Example 1B(2) and selected to form pGEX-3X-15:17.

The resulting vector encodes a fusion protein (GST:NANBV 15:17) that is comprised of an amino-terminal polypeptide portion corresponding to residues 1 to 221 of GST, an intermediate polypeptide portion corresponding to residues 222 to 225 and defining a cleavage site for the protease Factor Xa, a linker protein corresponding to residues 226 to 233 consisting of the amino acid residue sequence (SEQ ID NO:33):
Gly Ile Pro Asn Ser Cys Ser Pro
encoded by the nucleotide base sequence (SEQ ID NO:32):
GGG ATC CCC AAT TCC TGC AGC CCT, respectively; a carboxy-terminal polypeptide portion corresponding to residues 234 to 439 defining a NANBV envelope antigen having the amino acid residue sequence 121 to 326 in SEQ ID NO:1, and a carboxy-terminal linker portion corresponding to residues 440 to 446 consisting of the amino acid residue sequence (SEQ ID NO:35):
Gly Ile Gly Asn Ser Ser END
encoded by the nucleotide base sequence (SEQ ID NO:34):
GGG ATC GGG AAT TCA TCG TGA, respectively.

pGEX-2T-15:17: Plasmid pGEX-2T-15:17 was formed by first subjecting the plasmid Bluescript 15:17 prepared in Example 1A(6) to restriction enzyme digestion with Eco RV and Bam HI and the Bam HI cohesive termini were filled in as performed in Example 1B(1). The resultant released DNA segment having a sequence contained in SEQ ID NO:1 from base 361 to base 978 was purified as performed in Example 1B(1). The purified DNA segment was admixed with and ligated to the pGEX-2T vector (Pharmacia, INC.) which was linearized by restriction enzyme digestion with Sma I as performed in Example 1B(1) to form the plasmid pGEX-2T-15:17.

A pGEX-2T plasmid containing a 15:17 DNA segment was identified by selection as performed in Example 1B(2) and by digestion of crude DNA preparations with Eco RI and Bam HI. A pGEX-2T vector containing a 15:17 DNA segment having the correct coding sequence for in-frame translation of a NANBV structural protein was identified as performed in Example 1B(2) and selected to form pGEX-2T-15:17.

The resulting vector encodes a fusion protein (GST:NANBV 15:17) that is comprised of an amino-terminal polypeptide portion corresponding to residues 1 to 221 of GST, an intermediate polypeptide portion corresponding to residues 222 to 226 and defining a cleavage site for the protease Thrombin consisting of the amino acid residue sequence (SEQ ID NO:37):
Val Pro Arg Gly Ser
encoded by the nucleotide base sequence (SEQ ID NO:36):
GTT CCG CGT GGA TCC, respectively;
a linker protein corresponding to residues 227 to 233 consisting of an amino acid residue sequence (SEQ ID NO:39):
Pro Ser Asn Ser Cys Ser Pro
encoded by a nucleotide base sequence (SEQ ID NO:38):
CCA TCG AAT TCC TGC AGC CCT,
respectively; a carboxy-terminal polypeptide portion corresponding to residues 234 to 439 defining a NANBV envelope antigen, and a carboxy-terminal linker portion corresponding to residues 440 to 446 consisting of the amino acid residue sequence (SEQ ID NO:-41):
Gly Ile His Arg Asp END
encoded by the nucleotide base sequence (SEQ ID NO:40):
GGA ATT CAT CGT GAC TGA, respectively.

pGEX-3X-690:691: To obtain a DNA segment corresponding to the NANBV Hutch sequence shown from SEQ ID NO:1 from base 1 to base 360, the oligonucleotides 690:691 are used in PCR reactions as performed in Example 1A(6). The resultant PCR amplified ds DNA is then cloned into pUC 18 cloning vectors as described in Example 1A(4) to form pUC18 690:691. Clones are then sequenced with pUC18 primers as described in Example 1A(5) to identify a plasmid containing the complete sequence. The resulting identified plasmid is selected, is designated pUC18 690:691, and contains a NANBV DNA segment that is 361 bp in length and spans nucleotides 1 to 360 of SEQ ID NO:1.

Plasmid pGEX-3X-690:691 is formed by first subjecting the plasmid pUC18 690:691 to restriction enzyme digestion with Eco RI and Bam HI as performed in Example 1B(1). The resultant released DNA segment having a sequence contained in SEQ ID NO:1 from base 1 to base 360 with pUC18 polylinker sequence is purified as performed in Example 1B(1). The purified DNA segment is admixed with and ligated to the pGEX-3X vector which is linearized by restriction enzyme digestion with Sma I as performed in Example 1B(1) to form the plasmid pGEX-3X-690:691.

A pGEX-3X plasmid containing a 690:691 DNA segment is identified by selection as performed in Example 1B(2). pGEX-3X vector containing a 690:691 DNA segment having the correct coding sequence for in-frame translation of a NANBV structural protein is identified as performed in Example 1B(2) and selected to form pGEX-3X-690:691.

The resulting vector encodes a fusion protein (GST:NANBV 690:691) that is comprised of an amino-terminal polypeptide portion corresponding to residues 1 to 221 of GST, an intermediate polypeptide portion corresponding to residues 222 to 225 and defining a cleavage site for the protease Factor Xa, a linker protein corresponding to residues 226 to 234 consisting of the amino acid residue sequence (SEQ ID NO:43):
Gly Ile Pro Asn Ser Ser Ser Val Pro
encoded by the nucleotide base sequence (SEQ ID NO:42):
GGG ATC CCC AAT TCG AGC TCG GTA CCC
respectively; a carboxy-terminal polypeptide portion corresponding to residues 235 to 355 defining a NANBV capsid antigen, and a carboxy-terminal linker portion corresponding to residues 356 to 363 consisting of the amino acid residue sequence (SEQ ID NO:45):
Thr Gly Ile Gly Asn Ser Ser END
encoded by the nucleotide base sequence (SEQ ID NO:44):
ACG GGG ATC GGG AAT TCA TCG TGA, respectively.

pGEX-2T-CAP-A: Oligonucleotides 1-20(+) and 1-20(-) for constructing the vector pGEX-2T-CAP-A for expressing the CAP-A fusion protein were prepared as described in Example 1A(2) having nucleotide base sequences corresponding to SEQ ID NO:7 and SEQ ID NO:8, respectively.

Oligonucleotides 1-20 (+) and 1-20 (-) were admixed in equal amounts with the expression vector pGEX-2T (Pharmacia) that had been predigested with Eco RI and Bam HI and maintained under annealing conditions to allow hybridization of the complementary oligonucleotides and to allow the cohesive termini of the resulting double-stranded (ds) oligonucleotide product to hybridize with pGEX-2T at the Eco RI and Bam HI cohesive termini. After ligation the resulting plasmid designated pGEX-2T-CAP-A contains a single copy of the ds oligonucleotide product and a structural gene coding for a fusion protein designated CAP-A having an amino acid residue sequence shown in SEQ ID NO:3 from residue 1 to residue 252.

The pGEX-2T vector is similar to the pGEX-3X vector described above, except that the resulting fusion protein is cleavable by digestion with the site specific protease thrombin.

pGEX-2T-CAP-B: Oligonucleotides 21-40(+) and 21-40(-) for constructing the vector pGEX-2T-CAP-B for expressing the CAP-B fusion protein were prepared as described in Example 1A(2) having nucleotide base sequences corresponding to SEQ ID NO:9 and SEQ ID NO:10, respectively.

Oligonucleotides 21-40 (+) and 21-40 (-) were admixed in equal amounts with the pGEX-2T expression vector that had been predigested with Eco RI and Bam HI and maintained under annealing conditions to allow hybridization of the complementary oligonucleotides and to allow the cohesive termini of the resulting double-stranded oligonucleotide product to hybridize with pGEX-2T at the Eco RI and Bam HI cohesive termini. After ligation the resulting plasmid designated as pGEX-2T-CAP-B contains a single copy of the ds oligonucleotide product and contains a structural gene coding for a fusion protein designated CAP-B having an amino acid residue sequence shown in SEQ ID NO:4 from residue 1 to residue 252.

pGEX-2T-CAP C: Oligonucleotides 41-60(+) and 41-60(-) for constructing the vector pGEX-2T-CAP-C for expressing the CAP-C fusion protein were prepared as described in Example 1A(2) having nucleotide base sequences corresponding to SEQ ID NO:11 and SEQ ID NO:12, respectively.

Oligonucleotides 41-60 (+) and 41-60 (-) were admixed in equal amounts with the pGEX-2T expression vector that had been predigested with Eco RI and Bam HI and maintained under annealing conditions to allow hybridization of the complementary oligonucleotides and to allow the cohesive termini of the resulting double-stranded oligonucleotide product to hybridize with pGEX-2T at the Eco RI and Bam HI cohesive termini. After ligation the resulting plasmid designated as pGEX-2T-CAP-C contains a single copy of the double-stranded oligonucleotide product and contains a structural gene coding for a fusion protein designated CAP-C having an amino acid residue sequence shown in SEQ ID NO:5 from residue 1 to residue 252.

pGEX-2T-CAP-A-B: Oligonucleotides for constructing the vector pGEX-2T-CAP-A-B for expressing the CAP-A-B fusion protein were prepared as described in Example lA(2) having nucleotide base sequences corresponding to SEQ ID NO:13 and SEQ ID NO:14, respectively.

Oligonucleotides according to SEQ ID NO:13 and SEQ ID NO:14 were admixed in equimolar amounts with the plasmid pGEX-3X-690:694 described in Example 1B(2). The admixture was combined with the reagents for a polymerase chain reaction (PCR) and the two admixed oligonucleotides were used as primers on the admixed pGEX-3X-690:694 as template in a PCR reaction to form a PCR extension product consisting of a double-stranded nucleic acid molecule that encodes the amino acid residue sequence contained in SEQ ID NO:1 from residue 2 to 40 and also includes PCR-added restriction sites for Bam HI at the 5' terminus and Eco RI at the 3' terminus. The PCR extension product was then cleaved with the restriction enzymes Bam HI and Eco RI to produce cohesive termini on the PCR extension product. The resulting product with cohesive termini was admixed in equal amounts with the pGEX-2T expression vector that had been predigested with Eco RI and Bam HI and maintained under annealing conditions to allow the cohesive termini of the double-stranded PCR extension product to hybridize with pGEX-2T at the Eco RI and Bam HI cohesive termini. After ligation the resulting plasmid designated pGEX-2T-CAP-A-B contains a single copy of the double-stranded PCR extension product and contains a structural gene coding for a fusion protein designated CAP-A-B having an amino acid residue sequence shown in SEQ ID NO:6 from residue 1 to residue 271.

### Example 2. Expression of the NANBV 690:694 Fusion Protein Using rDNA

The bacterial colonies which contain the pGEX-3X-690:694 plasmid in the correct orientation were selected to examine the properties of the fusion protein. Bacterial cultures of pGEX-3X-690:694 were grown to a stationary phase in the presence of ampicillin (50 µg/ml final concentration) at 37°C. This culture was inoculated at a 1:50 dilution into fresh LB medium at 37°C in the presence of ampicillin and maintained at 37°C with agitation at 250 rpm until the bacteria reached an optical density of 0.5 when measured using a spectrometer with a 550 nm wavelength light source detector. Isopropylthio-beta-D-galactoside (IPTG) was then admixed to the bacterial culture at a final concentration of 1 mM to initiate (induce) the synthesis of the fusion protein under the control of the tac promoter in the pGEX-3X vector.

Beginning at zero time and at one hour intervals thereafter for three hours-following admixture with IPTG (i.e., the induction phase), the bacterial culture was maintained as above to allow expression of recombinant protein. During this maintenance phase, the optical density of the bacterial culture was measured and 1 ml aliquots were removed for centrifugation. Each resultant cell pellet containing crude protein lysate was resuspended in Laemmli dye mix containing 1% beta-mercaptoethanol at a final volume of 50 µl for each 0.5 OD 550 unit. Samples were boiled for 15 minutes and 10 µl of each sample was electrophoresed on a 10% SDS-PAGE Laemmli gel.

Other GST:NANBV fusion proteins were also expressed in bacteria by transformation with the appropriate expression vector and induction as described above.

### Example 3. Detection of Expressed Fusion Proteins

To visualize the IPTG-induced fusion proteins, the Laemmli gels were stained with Coomassie Blue and destained in acetic acid and methanol. Induced proteins from separate clones were examined and compared on the basis of the increase of a protein band in the predicted size range from time zero to time three hours post-IPTG treatment. Expression of fusion protein was observed in clones that exhibited an increase from zero time in the intensity of a protein band corresponding to the fusion protein.

The GST:NANBV fusion proteins CAP-A, CAP-B, and CAP-C, when analyzed on a 12.5% PAGE Laemmli gel as described in Example 2, exhibited an apparent molecular weight of about 30,000 daltons.

### Example 4. Western Blot Analysis

Samples from IPTG inductions containing a GST:NANBV fusion protein of this invention were separated by gel electrophoresis and were transferred onto nitrocellulose for subsequent immunoblotting analysis. The nitrocellulose filter was admixed with antibody blocking buffer (20 mM sodium phosphate, pH 7.5, 0.5 M sodium chloride, 1% bovine serum albumin, and 0.05% Tween 40) for 3 to 12 hours at room temperature. Sera from humans or chimpanzees with NANB hepatitis believed to contain antibody immunoreactive with NANBV structural protein was diluted 1:500 in the antibody blocking buffer and admixed with the nitrocellulose and maintained for 12 hours at room temperature to allow the formation of an immunoreaction product on the solid phase. The nitrocellulose was then washed three times in excess volumes of antibody blocking buffer. The washes were followed by admixture of the nitrocellulose with 50 µl of ¹²⁵I protein A (New England Nuclear, Boston, MA) at a 1:500 dilution in antibody blocking buffer for one hour at room temperature to allow the labeled protein A to bind to any immunoreaction product present in the solid phase on the nitrocellulose. The nitrocellulose was then washed as described herein, dried and exposed to X-ray film for one to three hours at -70°C in order to visualize the label and therefore any immunoreaction product on the nitrocellulose.

Results of the Western blot immunoassay are shown in Tables 2 through 7. Samples prepared using pGEX-3X vector that produces control GST were also prepared as above and tested using the Western blot procedure as a control. The expressed GST protein was not detectable as measured by immunoreactivity using the sera shown to immunoreact with a fusion protein of this invention (e.g., GST:NANBV 690:694 fusion protein).

### Example 5. Purification of Expressed GST:NANBV Fusion Proteins

Cultures of E. coli strain W3110 transformed with recombinant pGEX-3X-690:694 plasmids prepared in Example 2 were cultured for 3 hours following IPTG induction treatment. The cells were then centrifuged to form a bacterial cell pellet, the cells were resuspended in 1/200 culture volume in lysis buffer (MTPBS: 150 mM NaCl, 16 mM Na₂HPO₄, 4 mM NaH₂PO₄, pH 7.3), and the cell suspension was lysed with a French pressure cell. Triton X-100 was admixed to the cell lysate to produce a final concentration of 1%. The admixture was centrifuged at 50,000 X g for 30 minutes at 4°C. The resultant supernatant was collected and admixed with 2 ml of 50% (w/v) glutathione agarose beads (Sigma, St. Louis, MO) preswollen in MTPBS. After maintaining the admixture for 5 minutes at 25°C to allow specific affinity binding between GST and glutathione in the solid phase, the beads were collected by centrifugation at 1000 X g and washed in MTPBS three times.

The GST:NANBV 690:694 fusion protein was eluted from the washed glutathione beads by admixture and incubation of the glutathione beads with 2 ml of 50 mM Tris HCl, pH 8.0, containing 5 mM reduced glutathione for 2 minutes at 25°C to form purified GST:NANBV 690:694 fusion protein.

The above affinity purification procedure produced greater than 95% pure fusion protein as determined by SDS PAGE. That is, the purified protein was essentially free of procaryotic antigen and non-structural NANBV antigens as defined herein.

Alternatively, GST:NANBV 690:694 fusion protein was purified by anion exchange chromatography. Cultures were prepared as described above and cell pellets were resuspended in 8M guanidine and maintained overnight at 4°C to solubilize the fusion protein. The cell suspension was then applied to an S-300 sepharose chromatography column and peak fractions containing the GST:NANBV 690:694 fusion protein were collected, pooled, dialyzed in 4 M urea and subjected to anion exchange chromatography to form purified fusion protein.

Other GST:NANBV fusion proteins described herein were also expressed in cultures of E.coli strain W3110 as described above using the GST fusion protein vectors produced in Example 1 after their introduction by transformation into the E.coli host. After induction and lysis of the cultures, the GST fusion proteins were purified as described above using glutathione agarose affinity chromatography to yield greater than 95% pure fusion protein as determined by SDS-PAGE. Thus, CAP-A, CAP-B and CAP-C fusion proteins were all expressed and purified as above using the pGEX-2T-CAP-A vector, the pGEX-2T-CAP-B vector, or the pGEX-2T-CAP-C vector, respectively, and CAP-A-B fusion protein is expressed and purified using the PGEX-2T-CAP-A-B vector.

### Example 6. Protease Cleavage of Purified GST:NANBV 690:694 Fusion Protein

Purified GST:NANBV 690:694 fusion protein prepared in Example 5 is subjected to treatment with activated Factor (Xa) (Sigma) to cleave the GST carrier from the NANBV 690:694 fusion protein (Smith et al., supra). Seven µg of Factor X are activated prior to admixture with purified fusion proteins by admixture and maintenance with 75 nanograms (ng) activation enzyme, 8 mM Tris-HCl (pH 8.0), 70 mM NaCl and 8 mM CaCl₂ at 37°C for 5 minutes. Fifty µg of purified fusion protein are then admixed with 500 ng activated human factor Xa in the elution buffer described in Example 5 containing 50 mM Tris HCl, 5 mM reduced glutathione, 100 mM NaCl, and 1 mM CaCl₂, and maintained at 25°C for 30 minutes. The resulting cleavage reaction products are then absorbed on glutathione-agarose beads prepared in Example 5 to affinity bind and separate free GST from any cleaved NANBV structural antigen-containing protein. Thereafter the liquid phase is collected to form a solution containing purified NANBV structural protein having an amino acid residue sequence contained in SEQ ID NO:2 from residue 226 to residue 315.

### Example 7. Immunological Detection of Anti-NANBV structural Protein Antibodies

NANBV Hutch strain virus was injected in chimpanzees and blood samples were collected at various weekly intervals post to inoculation (INOC) to analyze the immunological response to NANBV by five different diagnostic assays. Chimpanzees were categorized as either being in the acute or chronic phase of infection. The assays utilized in the evaluation of the immune response include: 1) alanine aminotransferase (ALT) enzyme detection (Alter et al., JAMA, 246:630-634, 1981; and Aach et al., N. Engl. J. Med., 304:989-994, 1981); 2) histological evaluation for NANBV virions by electron microscopy (EM); 3) detection of anti-HCV antibodies using the commercially available kit containing C100-3 antigen (Ortho Diagnostics, Inc.); 4) detection of anti-CAP-N antibodies by immunoblot analysis as described in Example 4 using the CAP-N fusion protein; and 5) Detection of virus by PCR amplification as described in Example 1.

In Table 2, results are presented from ALT, EM, anti-HCV (anti-C100-3), anti-CAP-N, and PCR assays on sera from a chimpanzee with acute NANB Hepatitis.

**TABLE 2**

| CHIMP 59 - ACUTE NANB HEPATITIS | | | | | |
|---|---|---|---|---|---|
| WEEK POST INOC¹ | ALT | EM | ANTI HCV | ANTI CAP-N² | PCR 690-691 |
| 8 | 26 | ++ | - | - | - |
| 10 | 26 | + | - | + | - |
| 12 | 107 | + | - | + | - |
| 14 | 115 | + | + | + | - |
| 16 | 26 | + | + | + | + |
| 18 | 17 | ND | + | + | (+) |
| 20 | 11 | ND | + | + | - |

| | | | | | |
|---|---|---|---|---|---|
| ¹ Week after inoculation. ² A plus (+) indicates immunoreaction was observed between admixed serum and the fusion protein, designated "CAP-N" because it corresponds to the amino terminal of the putative NANBV capsid protein, using the Western blot immunoassay described in Example 4. | | | | | |

The results in Table 2 show immunoreaction between fusion protein and anti-NANBV structural protein antibodies in the sera tested. Furthermore, seroconversion is detectable by the immunoassay using fusion protein containing capsid antigen at times earlier than when the same sera is assayed in the C100-3-based immunoassay.

In Table 3, results are presented from ALT, anti-HCV (anti-C100-3) and anti-CAP-N assays on sera collected from a human with definitive NANB Hepatitis.

**TABLE 3**

| NYU - 169 - DEFINITIVE NANB HEPATITIS | | | |
|---|---|---|---|
| Week Post Infect | ALT | Anti HCV | Anti CAP-N |
| 2 | 34 | - | - |
| 6 | 8 | - | - |
| 10 | 150 | - | - |
| 12 | 118 | - | - |
| 14 | 183 | - | + |
| 16 | 317 | - | + |
| 19 | 213 | - | + |
| 23 | 53 | - | + |

The results in Table 3 show that in the human series 169 seroconversion sera samples, the CAP-N antigen present in the fusion protein detects NANBV-specific antibodies as early as 14 weeks post inoculation, whereas the C100-3-based immunoassay does not detect any anti-NANBV antibody at the times studied.

In Table 4, results are presented from ALT, EM, anti-HCV, and anti-CAP-N assays on sera from a chimpanzee with a self limited infection presented.

**TABLE 4**

| CHIMP 213 - SELF LIMITED INFECTION | | | | |
|---|---|---|---|---|
| Week Post Inoc | ALT | EM | Anti HCV | Anti CAP-N |
| 4 | 24 | + | - | + |
| 6 | 34 | + | - | + |
| 8 | 38 | + | - | + |
| 13 | 28 | ND | - | + |
| 16 | 25 | ND | - | + |
| 18 | 23 | ND | + | + |
| 20 | 25 | - | + | + |

The results in Table 4 show that the CAP-N antigen detects anti-NANBV antibodies earlier than the C100-3 antigen when using sera sampled during the course of a self-limiting NANBV infection.

In Table 5, results are presented from ALT, anti-HCV and anti-CAP-N assays on sera from a chimpanzee that converted from an acute infection profile to a chronic one.

**TABLE 5**

| CHIMP 10 - ACUTE/CHRONIC NANB HEPATITIS | | | | |
|---|---|---|---|---|
| Symptoms | Week Post Inoc | Peak ALT | Anti HCV | Anti CAP-N |
| acute | 2 | 223 | - | + |
| chronic | 40 | 223 | + | + |
| chronic | 42 | 223 | + | + |
| chronic | 44 | 223 | + | + |
| chronic | 51 | 223 | + | - |

The results in Table 5. indicate that the CAP-N antigen preferentially detects anti-NANBV antibodies in acute stages of NANBV infection.

In Table 6, results are presented from ALT, EM, anti-HCV (anti-C100-3) and anti-CAP-N assays on sera collected at various intervals from several chimpanzees with acute or chronic NANB Hepatitis.

**TABLE 6**

| ADDITIONAL ACUTE SERA | | | | |
|---|---|---|---|---|
| Week Post Inoc | Week Post Alt Elev | Peak ALT | Anti HCV | Anti CAP-N |
| 2 | +1 | 73 | - | + |
| 14 | +2 | 66 | - | + |
| 6 | +2 | 197 | - | + |
| 11 | +1 | 151 | - | - |
| 8 | +4 | 125 | - | + |
| 15 | +1 | 82 | - | + |
| 12 | -4 | 73 | ND | + |

| ADDITIONAL CHRONIC SERA | | | | |
|---|---|---|---|---|
| 156 | +131 | 110 | + | + |
| 156 | - | 89 | + | + |
| 160 | - | 89 | + | + |

The results in Table 6 indicate that the CAP-N antigen more often detected anti-NANBV antibodies in sera from acutely infected individuals than did the C100-3 antigen.

The results of Tables 2-6 show that the NANBV structural protein of the invention, in the form of a fusion protein containing CAP-N antigen and produced by the vector pGEX-3X-690:694, detects antibodies in defined seraconversion series at times in an infected patient or chimpanzee earlier than detectable by present state of the art methods using the C100-3 antigen. In addition, the results show that CAP-N antigen is particularly useful to detect acute NANBV infection early in the infection.

Taken together, the results indicate that patients infected with NANBV contain circulating antibodies in their blood that are immunospecific for NANBV antigen designated herein as structural antigens, and particularly are shown to immunoreact with the putative capsid antigen defined by CAP-N. These antibodies are therefore referred to as anti-NANBV structural protein antibodies and are to be distinguished from the class of antibodies previously detected using the NANBV non-structural protein antigen C100-3.

In Table 7, comparative results are presented from anti-HCV capsid fusion protein assays according to the basic immunoblot assay described in Example 4 using various chimp and human sera on the following HCV capsid fusion proteins: CAP-N, CAP-A, CAP-B and CAP-C.

**TABLE 7**

| SERA | TYPE^{a} | CAP-N^{b} | CAP-A^{c} | CAP-B^{d} | CAP-C^{e} |
|---|---|---|---|---|---|
| C18 | Chimp 10 (A) | +++ | + | + | - |
| C10 | Chimp 194(A) | +++ | +++ | +++ | - |
| 59-16 | Chimp 59 (A) | +++ | + | +++ | ND |
| 59-12 | Chimp 59 (A) | ND^{f} | ++ | +++ | - |
| C9 | Chimp 181(A) | +++ | - | +++ | - |
| 213-18 | Chimp 213(A) | ND | + | + | - |
| C2 | Chimp 10 (C) | ++ | - | - | - |
| C1 | Chimp 10 (C) | +++ | - | - | - |
| C19 | Chimp 10 (C) | +++ | - | - | - |
| C4 | Chimp 68 (C) | +++ | +++ | +++ | ND |
| 169-16 | Human | ND | +++ | +++ | - |
| 169-23 | Human | ND | +++ | +++ | - |
| 191-1 | Human | + | + | + | ND |
| 191-2 | Human | + | + | ++ | ND |
| 191-3 | Human | + | + | + | ND |
| 216-1 | Human | - | +/- | +/- | ND |
| 216-2 | Human | + | + | + | ND |
| 216-3 | Human | + | + | + | ND |

| | | | | | |
|---|---|---|---|---|---|
| a The type of sera tested is indicated by the species (chimp or human), a chimp identification number if the sample is from a chimp, and a designation (in parenthesis) if the sera donor exhibits acute (A) or chronic (C) HCV infection at the time the sera was sampled. b CAP-N indicates the GST:NANBV 690:694 fusion protein produced in Example 5 that includes HCV capsid protein residues 1 to 74. c CAP-A indicates the GST:NANBV fusion protein produced in Example 5 that includes HCV capsid protein residues 1 to 20. d CAP-B indicates the GST:NANBV fusion protein produced in Example 5 that includes HCV capsid protein residues 21 to 40. e CAP-C indicates the GST:NANBV fusion protein produced in Example 5 that includes HCV capsid protein residues 41 to 60. f +, ++ and +++ indicate relative amounts of anti-HCV capsid antibody immunization product detected by the Western blot assay, where + indicates a weak band after overnight exposure of the x-ray film, ++ indicates a strong band after overnight exposure of the x-ray film, +++ indicates a strong band after 1 to 2 hours exposure of the X-ray film, and +/- or - indicates a faint or no band, respectively, after overnight exposure of the X-ray film g "ND" indicates not tested. | | | | | |

The results shown in Table 7 indicate that fusion proteins containing the CAP-A antigen or CAP-B antigen are immunoreactive with antibodies present in sera from HCV-infected humans or chimps. In addition, CAP-C antigen does not significantly immunoreact with sera from HCV infected humans or chimps.

### Example 8. Characterization of NANBV Genomic RNA Sequence

### A. Characterization of cDNA Clones and Primary Structure of NANBV

### (1) Isolation of NANBV Viral RNA.

NANBV, also referred to as hepatitis C virus (HCV), was isolated from two tissue sources from a HCV-infected chimpanzee, number 59 (c59), that had been inoculated with the Hutch (H) strain of HCV (designated HCV-Hc59) as described in Example 1A(1). Chimpanzee liver was biopsied during the acute phase of infection (4 weeks post-inoculation) and chimpanzee plasma was taken 13 weeks post-inoculation. Extraction of nucleic acids from liver was performed as described by Ogata et al., Proc. Natl. Acad. Sci., USA, 88:3392-3396 (1991) and in Example 1A(6). HC virions were isolated from plasma having viral titers of 10^{5.5} to 10^{6.5} CID₅₀/ml. HCV RNA was purified from the plasma samples by either immunoaffinity chromatography as described in Example 1A(1) or by isopropanol precipitation.

Briefly, 50 µl of plasma was diluted with an ice cold buffer solution containing 4.2 M guanidinium isothiocyanate, 0.5% sarcosyl and 0.025 M Tris-HCl at pH 8.0. The diluted plasma was then admixed with 50 µl of extraction buffer containing 100 mM Tris-HCl at pH 8.0, 10 mM EDTA and 1% SDS to form an extraction admixture. The admixture was vortexed and maintained at 5 minutes at 65°C to initiate extraction. Serum proteins were then removed from the admixture with phenol/chloroform at 65°C followed by one extraction with chloroform alone. HCV RNA was then precipitated from the protein-free admixture by admixing two volumes of ice cold isopropanol and one-tenth volume of 3 M sodium acetate and maintaining the admixture overnight at -20°C. After pelleting by centrifugation in an Eppendorf centrifuge at 1400 rpm for 30 minutes at 4°C, HCV RNA was washed once with 70% ethanol, vacuum dried and then resuspended in 9 µl RNAse-free water. Purified HCV RNA samples were heated for 5 minutes at 65°C prior to cDNA synthesis performed as described below and in Example 1A(1).

### (2) Cloning of HCV-Hc59 cDNA.

Five µg of purified liver or plasma derived HCV RNA was used per cDNA priming reaction. Specific nucleotide primers derived from published HCV sequences and spanning the entire reported genomic sequences were used to prime the reaction. See Okamoto et al., Japan. J. Exp. Med., 60:167-177 (1990); Kato et al., Proc. Natl. Acad. Sci., USA, 87:9524-9528 (1990); Han et al., Proc. Natl. Acad. Sci.. USA, 88:1711-1715 (1991); and Houghton et al., European Patent Application Number 88310922.5 and Publication Number 318216. Selected target sequences were amplified using a PCR-based approach using a variety of nucleotide primers as described in Example 1A(3). The nucleotide sequences of the primers are listed in Table 8 below and have been identified by primer number and corresponding SEQ ID NOs.

**TABLE 8**

| NUCLEOTIDE PRIMERS USED IN CLONING HCV-HC59 CDNA | | | |
|---|---|---|---|
| PRIMER (#) | SEQ ID NO. | NUCLEOTIDE SEQUENCE (5'-3') | POLARITY^{a} |
| 1 | 47 | CAGCCCCCTGATGGGGGCGAC | + |
| 22 | 48 | ACTCGCAAGCACCCTATCA | - |
| 21 | 49 | CTGTGAGGAACTACTGTCT | + |
| 690 | 50 | ATGAGCACGAATCCTCAAACCT | + |
| 694 | 51 | GTCCTGCCCTCGGGCC | - |
| 693 | 52 | CGAGGAAGACTTCCGAGC | + |
| 691 | 53 | ACCCAAATTGCGCGACCTAC | - |
| 15 | 54 | TAAGGTCATCGATACCCT | + |
| 17 | 55 | CAGTTCATCATCATATCCCA | - |
| 18 | 56 | AGATAGAGAAAGAGCAAC | - |
| 23 | 57 | AGACTTCCGAGCGGTCGCAA | + |
| 717 | 58 | GACCTGTGCGGGTCTGTC | + |
| 567 | 59 | GGGTCGGCAGCTGGCTAGCCTCTCA | - |
| 801 | 60 | TCCTGGCGGGCATAGCGT | + |
| 8 | 61 | CCCCAGCCCTGGTCAAAATCGGTAA | - |
| 568 | 62 | TGAGAGGCTAGCCAGCTGCCGACCC | + |
| 745 | 63 | CTGTCGGTCGTTCCCACCA | - |
| 626 | 64 | CCGCGAAGAGTGTGTGTGGT | + |
| 627 | 65 | CAATGTTCTGGTGGAGGTG | - |
| 617 | 66 | GCCATTAAGTGGGAGTACGTCGTTCTCC | + |
| 652 | 67 | CGAGGAAGGATACAAGACC | - |
| 628 | 68 | TGCTTGTGGATGATGCTACT | + |
| 629 | 69 | CACACGTGCAGTTGCGCT | - |
| 701 | 70 | CTGCTGACCACTACACAG | + |
| 654 | 71 | GACCAGAGTGGAAGCGCAA | + |
| 653 | 72 | TACCAGAGTCGGGTGTACAG | - |
| 500 | 73 | CTAGGAGGCCCCTTGTCTGC | - |
| 688 | 74 | CTCGGGCCAGCCGATGGA | + |
| 633 | 75 | GGGGACCTCATGGTTGTCT | - |
| 846 | 76 | CCCGTGGAGTGGCTAAGG | + |
| 831 | 77 | CTCCTCGATGTTGGGATGG | - |
| 830 | 78 | CAGAGCTTCCAGGTGGCTC | + |
| 795 | 79 | CGGGCTCCGTCACTGTG | + |
| 794 | 80 | GTATTGCAGTCTATCACCGAG | - |
| 464 | 81 | GGCTATACCGGCGACTTCGA | + |
| 40 | 82 | CGTTGAGTGCGGGAGACAG | - |
| 463 | 83 | TCACCATTGAGACAATCACG | + |
| 788 | 84 | GTAAGGAAGGTTCTCCCCACTC | - |
| 571 | 85 | ATGCCCACTTTCTATCCCAGACAAAGC | + |
| 623 | 86 | TGCATGTCATGATGTAT | - |
| 841 | 87 | GGACAAGACGACCCTGCC | - |
| 625 | 88 | CGTATTGCCTGTCAACAGGC | + |
| 631 | 89 | AGCGCCCACAAAGGCAGTAG | - |
| 842 | 90 | CCTCTTCAACATATTGGGG | + |
| 843 | 91 | CCAGGAACCGGAGCATGG | - |
| 859 | 92 | ACCAGTGGATAAGCTCGG | + |
| 904 | 93 | CGTGGTGTAGGCATTAATG | - |
| 862 | 94 | ATGTGGAGTGGGACCTTCC | + |
| 861 | 95 | CTCTGCTGTTATATGGGAGG | - |
| F4 | 96 | GTTGACGTCCATGCTCACTG | + |
| A4 | 97 | TTTCCACGTCTCCACTAGCG | - |
| 849 | 98 | GTGAGGACCACCGTCCGC | - |
| F1 | 99 | TTCCACCTCCAAAGTCCCCT | + |
| 2₁ | 100 | AGAACTTGCAGTCTGTCAAATGTGA | - |
| 621 | 101 | GGAAGAACAGAAACTGCCCATCAATGCACTAAGC | + |
| 2₀ | 102 | TGACGCCGCTGCTTTAACCT | - |
| 2₂ | 103 | TGCAAGCTTCCTCTACGGAT | - |
| 51 | 104 | AGGTTAAAGCAGCGGCGTCA | + |
| 50 | 105 | AGCTTCCCATCACGGCCAA | - |
| 502 | 106 | GATGGCTTTGTACGACGTG | + |
| 55 | 107 | GCACCTGCGATAGCCGCAGT | - |
| 852 | 108 | GTCCCTCACCGAGAGGCT | + |
| 853 | 109 | GATTGGAGGTAGATCAAGTG | - |
| 4 | 110 | TACGACTTGGAGCTCATAAC | + |
| 62 | 111 | AGCAAGACACACTCCAGTCA | + |
| 61 | 112 | GCCTATTGGCCTGGAGTGGTTAGC | - |

| | | | |
|---|---|---|---|
| a (+) indicates sense strand (-) indicates anti-sense strand | | | |

Amplified sequences were subsequently isolated, rendered blunt-ended and inserted into a pUC or pBluescript (Stratagene) cloning vectors by standard procedures as described in Example 1A(4).

### (3) Sequence Analysis of Cloned HCV-Hc59 cDNA

Clones were sequenced using the dideoxy chain termination method using a duPont automated sequencer Genesis 2000. In order to minimize sequencing errors due to PCR artifacts (misreading by Taq polymerase), three independent clones were isolated for each target sequence and were then sequenced. The resulting sequences were compared in order to derive the final consensus sequence representative of the HCV Hutch strain (HCV-H) genome. In some cases, several clones derived from independent studies encompassed the same genomic domain. The sequences of these clones provided further confirmatory data.

### (4) Characterization of cDNA Clones and Primary Structure of HCV-Hc59

Pairs of primers were selected as described above and in Example 1A(3) to amplify specific regions of the HCV-Hc59 genome to generate overlapping clones, the sequences of which would comprise the entire genome. The primer pairs used in specific PCR reactions are listed in Table 9 below. The resultant forty cDNA clones generated from the selected primer pairs are listed numerically beginning with zero and ending with 39 in the same table and correspond to the putative map location shown in Figure 1. The deduced size in base pairs of each isolated cDNA is also listed in Table 9.

**TABLE 9**

| PCR DERIVED HCV-HC59 CLONES | | |
|---|---|---|
| Clone #^{a} | Primer Pair^{b} | Insert Size (bp)^{c} |
| 0 | 1:22 | 309 |
| 1 | 21:22 | 268 |
| 2 | 690:694 | 224 |
| 3 | 693:691 | 216 |
| 4 | 15:18 | 170 |
| 5 | 23:18 | 378 |
| 6 | 15:17 | 618 |
| 7 | 717:567 | 548 |
| 8 | 801:8 | 346 |
| 9 | 568:745 | 205 |
| 10 | 626:627 | 597 |
| 11 | 617:652 | 173 |
| 12 | 628:652 | 119 |
| 13 | 628:629 | 390 |
| 14 | 701:652 | 314 |
| 15 | 654:653 | 106 |
| 16 | 654:500 | 572 |
| 17 | 688:633 | 590 |
| 18 | 846:831 | 537 |
| 19 | 830:831 | 432 |
| 20 | 795:794 | 313 |
| 21 | 464:40 | 134 |
| 22 | 463:788 | 347 |
| 23 | 571:623 | 241 |
| 24 | 571:841 | 362 |
| 25 | 625:631 | 482 |
| 26 | 842:843 | 568 |
| 27 | 859:904 | 320 |
| 28 | 862:861 | 390 |
| 29 | F4:A4 | 397 |
| 30 | F4:849 | 498 |
| 31 | F1:2₁ | 493 |
| 32 | 621:2₁ | 132 |
| 33 | 621:2₀ | 181 |
| 34 | 621:2₂ | 221 |
| 35 | 51:50 | 360' |
| 36 | 502:55 | 322 |
| 37 | 852:853 | 625 |
| 38 | 4:853 | 315 |
| 39 | 62:61 | 611 |

| | | |
|---|---|---|
| ^{a} Relative location on HCV-Hc59 genome shown in Figure 1. ^{b} Sense (+) and anti-sense (-) primer pairs having nucleotide sequences shown in Table 1 and in the Sequence Listings. ^{c} Deduced size in base pairs (bp) of the cloned insert produced by PCR using the indicated primer pair as described in Example 1A(3) and 8A(3). | | |

Comparison of the sequences of three independently isolated cDNA clones from the same genomic domain revealed very few nucleotide differences indicating that the virus stock was homogeneous. The sequence of the complete HCV-H genome was deduced, representing 9416 nucleotides, which is similar in length to that of previously isolated HCV genomes, HCV-1, HCV-J, and HCV-BK. See, Kato et al., supra; Choo et al., Proc. Natl. Acad. Sci., USA, 88:2451-2455 (1991); and Takamizawa et al., J. Virol., 65:1105-1113 (1991). The sequence has a high GC content (58.8%), and contains one large open reading frame beginning at nucleotide base number 342 and ending at nucleotide base number 9374 (SEQ ID NO:46) corresponding to a protein of 3011 amino acid residues (SEQ ID NO:46). The deduced nucleotide sequences of HCV-Hc59 have been deposited in GenBank having the accession number M67463.

HCV-Hc59 sequences from the 5' and 3' end terminal non-coding (NC) domains, respectively encompassing 341 and 42 nucleotides, were identified. The first 12 nucleotides and the last 20 nucleotides (SEQ ID NO:46-see features) correspond to the nucleotide primers used in the amplification process and, thus are not confirmed as HCV-H sequences. However, 5' non-coding sequences of previously reported HCV genomes are extremely conserved (>98%), making it likely that the 5' end sequence of HCV-H reported here is very similar if not identical to the one indicated. However, due to greater divergence among HCV- 3' non-coding sequences, the HCV-Hc59 3' end sequence remains subject to confirmation. When an oligo (dT) primer was used for cDNA synthesis followed by PCR amplification using different combinations of primers, no viral sequences were obtained. This result indicates that the viral genome lacks internal A-rich tracts at the 3' terminal end or a 3'-terminal poly (rA) sequence. Similarly, no sequences were amplified when A-rich primers complementary to the 3' end (U-rich) nucleotide sequence of the two reported Japanese isolates, HCV-J and HCV-BK, were used in the RT priming reaction, thus suggesting the absence of a U-rich terminal sequence in the genome of HCV-Hc59.

The large open reading frame of the HCV-Hc59 RNA genome is preceded by five AUG codons (cDNA = ATG - nucleotide base numbers 13, 32, 85, 96 and 214 as shown in SEQ ID NO:46) confirming the existence of hypothetical small open reading frames in the 5' NC region of HCV genomes. Several repeated sequences as shown in SEQ ID NO:46 listed as R₁ through R₅ in the features portion of the listing were identified in the 5' and the 3' NC regions, and in the C terminal of the putative NS5 domain. These sequences might correspond to important Cis acting elements involved in the regulation of viral replication.

The repeated sequences, R₂ and R₃, appear conserved among all HCV isolates. Although other repeated sequences have now been found in the terminal ends of HCV genomes, it is possible that sequences having a regulatory function would be sequences conserved among all HCV viruses, such as R₂ and R₃. The repeated sequence R₂ is particularly significant as it is represented by the highest copy number of four, is found within both the 5' and 3' terminal ends, and is localized upstream from a 3' terminal hairpin loop which may be involved in cyclization of viruses. Nothing is yet known about putative cyclization of HCV viruses. It is also possible that these very conserved self-complementary sequences may represent replicase recognition sites, possibly used for both the plus and minus strands of the viral genome.

As described in previous reports for other HCV isolates, (Kato et al., Proc. Natl. Acad. Sci. USA, 87:9524-9528 (1990); Choo et al., Proc. Natl. Acad. Sci. USA, 88:2451-2455 (1991): and Takamizawa et al., J. Virol., 65:1105-1113 (1991)) the HCV-Hc59 genome or protein shares only limited similarity with other known viral sequences, except for three domains: (1) a few stretches of nucleotides in the 5' NC sequence are conserved with pestiviruses identical to those reported by Choo et al., supra, for the American prototype HCV-1 (SEQ ID NO:46), (2) blocks of amino acids found in the putative NS3 domain (nucleotide base numbers 3693 to 5198; SEQ ID NO:46) corresponding to putative NTP-binding helicase and trypsin-like serine proteases are conserved with flaviviruses and pestiviruses; and (3) the GDD consensus sequence conserved among all viral-encoded RNA-dependent RNA polymerases (amino acid residues 2737 to 2739; SEQ ID NO:46). In addition, a total of nineteen putative N-glycosylation sites were located, essentially clustered between amino acid residues 196 and 647 in a similar fashion to the organization observed for the envelope proteins of pestiviruses as described by Meyers et al., Virol., 171:555-567 (1989); and Collett et al., Virol., 162:167-180 (1988).

### B. Comparison of Nucleotide and Protein Sequences of HCV-Hc59 and Heterologous HCV Isolates

A summary of the comparison between different genomic domains of HCV-Hc59 and the previously reported sequences for the American (HCV-1) or American-like (HC-J1) isolates, and for the Japanese isolates HC-J4, HCV-JH, HCV-J and HCV-BK is shown in Table 10. Sequence comparison is limited with HC-J1, HC-J4 and HC-JH as the complete sequence of the genome of these isolates has not yet been reported. The hypothetical map assignments for HCV-encoded proteins deduced from sequence and hydrophobicity profile similarity between HCV genomes and flaviviruses and/or pestiviruses were used for making the comparison. The references for the compared sequences are listed at the bottom of Table 10. Based on sequence comparisons to related viruses, the HCV genome is believed to encode at least 8 domains as indicated in Table 10 : the structural domain consisting of the nucleocapsid (C) and two envelope (E1 and E2) proteins, and the non-structural region consisting of five proteins, NS2, NS3, NS4a, NS4b, and NS5. Domain designations are based on the organization of related HCV strains for comparative purposes, and do not necessarily reflect the domains of HCV-Hc59 because of the present state of the art in characterizing the domains of HCV-Hc59.

**TABLE 10**

| HOMOLOGY OF NUCLEOTIDE AND DEDUCED AMINO ACID SEQUENCE BETWEEN HCV-Hc59 AND HETEROLOGOUS ISOLATES | | | | | | |
|---|---|---|---|---|---|---|
| Domain¹ | Isolate² | | | | | |
| | HCV-1 | HC-J1 | HC-J4 | MCV-JH | HCV-J | HCV-BK |
| 5'NC | | | | | | |
| -326-1 | | | | | | |
| % bp³ | 99.7 | 99.1 | 99.1 | 98.9 | 98.2 | 98.8 |
| C | | | | | | |
| 1-570 | | | | | | |
| bp | 98.4 | 98.9 | 90.0 | 90.3 | 91.0 | 90.3 |
| aa | 98.9 | 98.9 | 97.9 | 98.4 | 98.9 | 98.4 |
| E1 | | | | | | |
| 571-1140 | | | | | | |
| bp | 93.5 | 93.1 | 74.1 | 73.7 | 73.9 | 73.8 |
| aa | 94.1 | 93.2 | 78.9 | 79.4 | 78.8 | 77.9 |
| E2/NS1 | | | | | | |
| 1141-2197 | | | | | | |
| bp | 93.6 | 91.7 | 67.7 | 65.4 | 73.5 | 71.2 |
| aa | 92.9 | 88.7 | 70.7 | 65.6 | 79.3 | 80.4 |
| NS2 | | | | | | |
| 2198-3350 | | | | | | |
| bp | 93.8 | -- | -- | -- | 72.4 | 72.7 |
| aa | 95.1 | -- | -- | -- | 80.0 | 78.2 |
| NS3 | | | | | | |
| 3351-4856 | | | | | | |
| bp | 95.4 | -- | -- | -- | 80.1 | 78.9 |
| aa | 97.2 | -- | -- | -- | 92.2 | 92.6 |
| NS4a | | | | | | |
| 4857-5596 | | | | | | |
| bp | 95.8 | -- | -- | -- | 80.4 | 80.0 |
| aa | 95.5 | -- | -- | -- | 87.0 | 86.2 |
| NS4b | | | | | | |
| 5597-6049 | | | | | | |
| bp | 95.4 | -- | -- | -- | 76.9 | 77.7 |
| aa | 96.7 | -- | -- | -- | 84.8 | 85.4 |
| NS5 | | | | | | |
| 6050-9036 | | | | | | |
| bp | 95.9 | -- | -- | -- | 78.3 | 79.3 |
| aa | 96.7 | -- | -- | -- | 83.2 | 83.7 |
| 3'NC | | | | | | |
| 9037-9055 | | | | | | |
| bp | 83.3 | -- | -- | -- | 73.6 | 63.1 |

| | | | | | | |
|---|---|---|---|---|---|---|
| ¹ Nucleotide position for C and E1 deduced from Weiner et al., Virol., 180:842-848 (1991) and for E₂ and NS2-NS5 from Takamizawa et al., J. Virol., 65:1105-1113 (1991): ² The nucleotide positions are calculated from the AUG initiation codon where A is base number 1. ³ The percentage of homology in base pairs (bp) and amino acid (aa) is listed. | | | | | | |

The data indicate a very high degree of identity found in two genomic domains (5' NC and C) for all isolates despite geographical separation (90.0-98.9% nucleotide homology and 97.9 to 98.9% amino acid homology). A similar observation has been made in flaviviruses that are members of the same sero-related subgroup by Brinton et al., Virol., 162:290-299 (1988), whereas members of different antigenic subgroups share only low levels of homology in that region. Two sets of repeated sequences found in the 5' NC domain, R₂ and R₃ (SEQ ID NO:46), are conserved among all reported isolates. Two copies of the repeated sequence R₁ are also conserved between the two American isolates HCV-Hc59 and HCV-1 but only one copy is found in both Japanese isolates HCV-J and HCV-BK. The 5' NC sequence of these genomes does not extend far enough to encompass the second copy. The nucleotide sequence reported for the other HCV isolates does not extend far enough into the 5' NC to allow for comparison.

Regions of moderate identity were found throughout the non-structural domains, where a clear separation between the two groups (American/Japanese) isolates could be seen. Whereas 93.8 to 95.9% nucleotide identity was observed when HCV-Hc59 was compared with the first group, only 72.7 to 80.0% identity was found with the second group (95.1 to 97.2% and 78.2-92.6% amino acid identity, respectively). One region, found in the putative NS5 (amino acid residue position 2356 to 2379 of SEQ ID NO:46) and called Region V₃ as shown in Table 11 below reflected even a more striking divergence between the two subgroups of HCV isolates. This region showed 100% identity between the two American isolates (data not shown) but only 12.5% with either Japanese strains. Although most of the changes appear to be conservative changes and might not therefore result in functional modification of the protein, it would be of interest to assess whether this genomic region is immunologically active and if antigenic variation also exist between the two subgroups of HCV isolates.

**Table 11¹**

| **REGION V** |
|---|
| (Residues 386 to 411 of SEQ ID NO:46) |
| IsolateS² |
| HCV-Hc59: |
| |
| HCV-1: |
| |
| HC-J1: |
| |
| HCV-J: |
| |
| HCV-BK: |
| |
| HC-J4: |
| |
| HCV-JH: |
| |
| HCV-Hh-H77: |
| |
| HCV-Hh-H90: |
| |

| **REGION V₁** |
|---|
| (Residue 246 to 275 of SEQ ID NO:46) |
| HCV-Hc59: |
| |
| HCV-1: |
| |
| HC-J1: |
| |
| HCV-J: |
| |
| HCV-BK: |
| |
| HC-J4: |
| |
| HCV-JH: |
| |
| HCV-Hh-H77: |
| |
| HCV-Hh-H90: |
| |

| **REGION V₂** |
|---|
| (Residue 456 to 482 of SEQ ID NO:46) |
| HCV-Hc59: |
| |
| HCV-1: |
| |
| HC-J1: |
| |
| HCV-J: |
| |
| HCV-BK: |
| |
| HC-J4: |
| |
| HCV-Hh-H77: |
| |
| HCV-Hh-H90: |
| |

| **REGION V₃** |
|---|
| (Residue 2356 to 2379 of SEQ ID NO:46) |
| HCV-HC59: |
| |
| HCV-J: |
| |
| HCV-BK: |
| |

| |
|---|
| ¹ Alignment of the deduced amino acid residue sequence of Regions V, V₁, V₂, and V₃ of HCV-Hc59 with other American and Japanese isolates. ² Isolates: HCV-Hc59: American/Chimp 59; Inschauspe et al., Proc. Natl. Acad. Sci., USA, 1991; GenBank Accession Number M67463; HCV-1: American; Choo et al., Proc. Natl. Acad, Sci., USA, 88:2451-2455 (1991); GenBank Accession Number M62321; HCV-1: 5' termini - Han et al., Proc. Natl. Acad. Sci. USA, 88:1711-1715 (1991); Genbank Accession Number M58407; HCV-1: 3' termini - Han et al., supra; GenBank Accession Number M58406; HC-J1: American; Okamoto et al., Japan J. Exp. Med., 60:167-177 (1990); HCV-J: Japanese; Kato et al., Proc. Natl. Acad. Sci.. USA, 87:9524-9528 (1990); Genbank Accession Number D90208; HCV-BK: Japanese; Takamizawa et al., J. Virol., 65:1105-1113 (1991); Genbank Accession Number M58335; HC-J4: Japanese; Okamoto et al., supra; HCV-JH: Japanese; Takeuchi et al., Nucl. acids Res., 18:4626 (1990); HCV-Hh-H77 and H90: American/human; Ogata et al., Proc. Natl. Acad. Sci., USA, 88:3392-3396 (1991). |

Regions of greater divergence were found in the putative envelope E1 (nucleotide base number 571 to 1140) and E2 (nucleotide base number 1141 to 2197 as calculated from the AUG initiation codon), where 77.9 to 94.1% and 65.6 to 92.9% amino acid identity, respectively, was observed between HCV-Hc59 and the other isolates. In addition to the moderate and hypervariable regions identified by Weiner et al., Virol., 180:842-848 (1991) in E1 and E2 (amino acid residues 214 to 254 and 386 to 411, respectively) for which protein heterogeneity between HCV-Hc59 and other HCV isolates ranged from 70.7 to 97.6% for the moderate region (data not shown) and from 51.7 to 72.4% for Region V as shown in Table 11, two regions of high variability were identified. Both regions, Region V₁ and Region V₂ (amino acid residues 246 to 275 and 456-482, respectively) appeared very conserved among American or Japanese type HCV (96% identity) but showed striking heterogeneity when both groups were compared (55-58% protein identity, Table 11). In contrast to the observation made by Weiner et al., supra, who reported that approximately 50% of the amino acid changes observed in Region V between four American isolates and one Italian isolate are non-conservative changes, more than 85% of the changes observed in either Region V, V₁ or V₂ were found to consist of conservative changes. Although the function of these regions remain unknown, these data suggest that they are under immunological pressure and could be good candidates for targeting protective epitope domains that might be subtype specific in the case of Regions V₁ and V₂.

Thus, the genome of HCV-Hc59 shows an overall amino acid homology of 96% with the American prototype HCV-1 and 84.9% with both HCV-J and HCV-BK isolates. Three new regions of high variability were identified within E1, E2 and NS5 (Regions V₁, V₂ and V₃, respectively). In all three regions, sequence heterogeneity appears to be subgroup specific (i.e., American versus Japanese isolates), in particular for Region V₃ where up to 87.5% divergence was found between the two subgroups as shown in Tables 10 and 11. Sequence heterogeneity has been observed in the envelope/NS1 regions of flaviviruses (see, Meyers et al., Virol., 171:555-567 (1989); Collett et al., Virol., 165:191-199 (1988); and Hahn et al., Virol., 162:167-180 (1988) but not to the extent reported here for Regions V₁ and V₂, thus further suggesting that HCV structure is significantly divergent from this family of viruses. The fact that three of four variable regions of the HCV genome are located in the putative envelope domains confirm that these domains are under great immunological pressure possibly associated with evolutionary-linked molecular divergence. A high rate of nucleotide change (28.2%) in the putative E2/NS1 domain of HCV-H over an interval of thirteen years suggests significant evolution of the HCV genome in that domain. See Ogata et al., supra.

The cDNA sequence of the human prototype strain H of HCV (9416 nucleotides) is the subject of this invention. To date, this is the second nucleotide sequence of a HCV genome determined for a prototype strain, as the two reported Japanese sequences HCV-J and HCV-BK have been derived from clones isolated from a mixture of plasma therefore representing likely genomic sequences from multiple isolates. The data confirms that HCV exhibits a unique structure and organization more closely related to the pestiviruses than flaviviruses by the presence of stretches of nucleotides highly conserved in the 5' NC domain, putative small open reading frames preceding the initial AUG codon, and putative NTP-binding helicases or trypsin-like serine proteases.

### Description of SEQ ID NO:1-6 in the Sequence Listings

SEQ ID NO:1 contains the linear single-stranded nucleotide base sequence of a preferred DNA segment of the present invention that encodes portions of the structural proteins of the Hutch strain of NANBV. The base sequences are shown conventionally from left to right and in the direction of 5' terminus to 3' terminus using the single letter nucleotide base code (A=adenine, T=thymine, C=cytosine and G=guanine) with the position number of the first base residue in each row indicated to the left of the row showing the nucleotide base sequence.

The reading frame of the nucleotide sequence of SEQ ID NO:1 is indicated by placement of the deduced amino acid residue sequence of the protein for which it codes below the nucleotide sequence such that the triple letter code for each amino acid residue (Table of Correspondence) is located directly below the three bases (codon) coding for each residue. SEQ ID NO:1 also contains the linear amino acid residue sequence encoded by the nucleotide sequence of SEQ ID NO:1 and is shown conventionally from left to right and in the direction of amino terminus to carboxy terminus. The position number for every fifth amino acid residue is indicated below that amino acid residue sequence.

SEQ ID NO:2 contains the linear amino acid residue sequence of a preferred fusion protein designated CAP-N and is comprised of an amino-terminal polypeptide portion corresponding to residues 1 to 221 of glutathione-S-transferase, an intermediate polypeptide portion corresponding to residues 222 to 225 and defining a cleavage site for the protease Factor Xa, a linker portion corresponding to residues 226 to 234, a polypeptide portion corresponding to residues 235 to 308 defining a NANBV capsid antigen that has the amino acid residue sequence 1 to 74 in SEQ ID NO:1, and a carboxy-terminal linker portion corresponding to residues 309 to 315. SEQ ID NO:2 also contains the nucleotide base sequence of a linear single-stranded DNA segment that encodes the fusion protein described herein. The nomenclature and presentation of sequence information is as described for SEQ ID NO:1.

SEQ ID NO:3 contains the linear amino acid residue sequence of a preferred fusion protein designated CAP-A and comprised of an amino-terminal polypeptide portion corresponding to residues 1 to 220 of glutathione-S-transferase, an intermediate polypeptide portion corresponding to residues 221 to 226 and defining a cleavage site for the protease Thrombin, a polypeptide portion corresponding to residues 227 to 246 defining a portion of the NANBV capsid antigen that has the amino acid residue sequence 1 to 20 in SEQ ID NO:1, and a carboxy-terminal linker portion corresponding to residues 247 to 252. SEQ ID NO:3 also contains the nucleotide base sequence of a linear single-stranded DNA segment that encodes the fusion protein described therein. The nomenclature and presentation of sequence information is as described for SEQ ID NO:1.

SEQ ID NO:4 contains the linear amino acid residue sequence of a preferred fusion protein designated CAP-B and comprised of an amino-terminal polypeptide portion corresponding to residues 1 to 220 of glutathione-S-transferase, an intermediate polypeptide portion corresponding to residues 221 to 226 and defining a cleavage site for the protease Thrombin, a polypeptide portion corresponding to residues 227 to 246 defining a portion of the NANBV capsid antigen that has the amino acid residue sequence 21 to 40 in SEQ ID NO:1, and a carboxy-terminal linker portion corresponding to residues 247 to 252. SEQ ID NO:4 also contains the nucleotide base sequence of a linear single-stranded DNA segment that encodes the fusion protein described therein. The nomenclature and presentation of sequence information is as described for SEQ ID NO:1.

SEQ ID NO:5 contains the linear amino acid residue sequence of a preferred fusion protein designated CAP-C and comprised of an amino-terminal polypeptide portion corresponding to residues 1 to 220 of glutathione-S-transferase, an intermediate polypeptide portion corresponding to residues 221 tp 226 and defining a cleavage site for the protease Thrombin, a polypeptide portion corresponding to residues 227 to 246 defining a portion of the NANBV capsid antigen that has the amino acid residue sequence 41 to 60 in SEQ ID NO:1, and a carboxy-terminal linker portion corresponding to residues 247 to 252. SEQ ID NO:5 also contains the nucleotide base sequence of a linear single-stranded DNA segment that encodes the fusion protein described therein. The nomenclature and presentation of sequence information is as described for SEQ ID NO:1.

SEQ ID NO:6 contains the linear amino acid residue sequence of a preferred fusion protein designated CAP-A-B and comprised of an amino-terminal polypeptide portion corresponding to residues 1 to 220 of glutathione-S-transferase, an intermediate polypeptide portion corresponding to residues 221 to 226 and defining a cleavage site for the protease Thrombin, a polypeptide portion corresponding to residues 227 to 265 defining a portion of the NANBV capsid antigen that has the amino acid residue sequence 2 to 40 in SEQ ID NO:1, and a carboxy-terminal linker portion corresponding to residues 266 to 271. SEQ ID NO:6 also contains the nucleotide base sequence of a linear single-stranded DNA segment that encodes the fusion protein described therein. The nomenclature and presentation of sequence information is as described for SEQ ID NO:1.

## Claims

1. A method for detecting seroconversion associated with NANBV infection at early times after infection comprising:
(a) forming an aqueous immunoreaction admixture by admixing a body fluid sample with a NANBV capsid antigen together with C100-3 antigen;
(b) maintaining said aqueous immunoreaction admixture for a time period sufficient for allowing antibodies against the NANBV capsid antigen present in the body fluid sample to immunoreact with said NANBV capsid antigen to form an immunoreaction product; and
(c) detecting the presence of said immunoreaction products formed and thereby detecting early sero conversion.

2. The method of claim 1, wherein said detecting in step (c) comprises the steps of:
(i) admixing said immunoreaction product formed in step (b) with a labeled specific binding agent to form a labeling admixture, said labeled specific binding agent comprising a specific binding agent and a label;
(ii) maintaining said labeling admixture for a period sufficient for any of said immunoreaction product present to bind with said labeled product; and
(iii) detecting the presence of any of said labeled product formed, and thereby the presence of said immunoreaction product.

3. The method of claim 2, wherein said specific binding agent is selected from the group consisting of Protein A and at least one of the antibodies anti-human IgG and anti-human IgM.

4. The method of claim 2 or 3, wherein said label is selected from the group consisting of lanthanide chelate, biotin, enzyme and radioactive isotope.

5. Use of NANBV capsid antigen together with C100-3 antigen for the preparation of a diagnostic composition for diagnosing seroconversion associated with NANBV infection at early times after infection.

6. The method of any one of claims 1 to 4 or the use of claim 5, wherein said NANBV capsid antigen is affixed to a solid matrix.

7. The method of any one of claims 1 to 4 or 6 the use of claim 5 or 6, wherein said NANBV capsid antigen is comprised by a fusion protein.

8. The method of any one of claims 1 to 4, 6 or 7 or the use of any one of claims 5 to 7, wherein said NANBV capsid antigen is selected from the group consisting of
(a) a NANBV capsid antigen having the amino acid sequence from the residue 1 to 120 of SEQ ID NO:1;
(b) a NANBV capsid antigen having the amino acid sequence from the residue 1 to 74 of SEQ ID NO:1;
(c) a NANBV capsid antigen having the amino acid sequence from the residue 69 to 120 of SEQ ID NO:1.

## Patentansprüche

1. Verfahren zum Nachweis der Serokonversion im Zusammenhang mit einer NANBV-Infektion zu einem frühen Zeitpunkt nach der Infektion, umfassend:
(a) Bildung eines wässrigen lmmunreaktionsgemischs durch das Vermischen einer Körperflüssigkeitsprobe mit einem NANBV-Kapsidantigen zusammen mit dem C100-3-Antigen;
(b) Belassen des wässrigen lmmunreaktionsgemischs für einen Zeitraum, der ausreicht, um Antikörper gegen das NANBV-Kapsidantigen, welche in der Körperflüssigkeitsprobe vorliegen, mit dem NANBV-Kapsidantigen immunreagieren zu lassen, um ein lmmunreaktionsprodukt auszubilden; und
(c) Nachweisen des Vorliegens der ausgebildeten lmmunreaktionsprodukte und **dadurch** das Nachweisen der frühen Serokonversion.

2. Verfahren nach Anspruch 1, wobei das Nachweisen in Schritt (c) die folgenden Schritte umfasst:
(i) Vermischen des in Schritt (b) ausgebildeten lmmunreaktionsprodukts mit einem markierten spezifischen Bindungsagens, um ein Markierungsgemisch auszubilden, wobei das markierte spezifische Bindungsagens ein spezifisches Bindungsagens und einen Marker umfasst;
(ii) Halten des Markierungsgemischs für einen Zeitraum, der für jedes der vorhandenen lmmunreaktionsprodukte ausreicht, um an das markierte Produkt zu binden; und
(iii) Nachweisen des Vorhandenseins von einem der gebildeten markierten Produkte und **dadurch** der Anwesenheit des lmmunreaktionsprodukts.

3. Verfahren nach Anspruch 2, wobei das spezifische Bindungsagens ausgewählt ist aus der Gruppe bestehend aus Protein A und mindestens einem der Antikörper Anti-Mensch-lgG und Anti-Mensch-lgM.

4. Verfahren nach Anspruch 2 oder 3, wobei der Marker ausgewählt ist aus der Gruppe bestehend aus Lanthanid-Chelat, Biotin, Enzym und radioaktivem Isotop.

5. Verwendung von NANBV-Kapsidantigen zusammen mit dem C100-3-Antigen für die Herstellung einer diagnostischen Zusammensetzung zur Diagnose der Serokonversion im Zusammenhang mit NANBV-infektion zu einem frühen Zeitpunkt nach der Infektion.

6. Verfahren nach einem der Ansprüche 1 bis 4 oder Verwendung nach Anspruch 5, wobei das NANBV-Kapsidantigen an einer festen Matrix befestigt ist.

7. Verfahren nach einem der Ansprüche 1 bis 4 oder 6 oder Verwendung nach Anspruch 5 oder 6, wobei das NANBV-Kapsidantigen von einem Fusionsprotein umfasst ist.

8. Verfahren nach einem der Ansprüche 1 bis 4, 6 oder 7 oder Verwendung nach einem der Ansprüche 5 bis 7, wobei das NANBV-Kapsidantigen ausgewählt ist aus der Gruppe bestehend aus:
(a) einem NANBV-Kapsidantigen, welches die Aminosäuresequenz von Rest 1 bis 120 der SEQ ID NO:1 hat;
(b) einem NANBV-Kapsidantigen, welches die Aminosäuresequenz von Rest 1 bis 74 der SEQ ID NO:1 hat;
(c) einem NANBV-Kapsidantigen, welches die Aminosäuresequenz von Rest 69 bis 120 der SEQ ID NO:1 hat.

## Revendications

1. Procédé de détection de la séroconversion associée à l'infection par NANBV très tôt après l'infection comprenant :
(a) la formation d'un mélange aqueux d'immunoréaction par mélange d'un échantillon de fluide corporel avec un antigène de capside de NANBV conjointement avec l'antigène C100-3 ;
(b) le maintien dudit mélange aqueux d'immunoréaction sur une durée suffisante pour permettre aux anticorps contre l'antigène de capside de NANBV présent dans l'échantillon de fluide corporel d'immunoréagir avec ledit antigène de capside de NANBV pour former un produit d'immunoréaction ; et
(c) la détection de la présence desdits produits d'immunoréaction formés et de là, la détection précoce de la séroconversion.

2. Procédé selon la revendication 1, dans lequel ladite détection dans l'étape (c) comprend les étapes de :
(i) mélange dudit produit d'immunoréaction formé dans l'étape (b) avec un agent marqué de liaison spécifique pour former un mélange de marquage, ledit agent marqué de liaison spécifique comprenant un agent de liaison spécifique et un marqueur ;
(ii) le maintien dudit mélange de marquage sur une durée suffisante pour que n'importe lequel dudit produit d'immunoréaction présent se lie audit produit marqué ; et
(iii) la détection de la présence de n'importe lequel dudit produit marqué formé, et de là la présence dudit produit d'immunoréaction.

3. Procédé selon la revendication 2, dans lequel ledit agent de liaison spécifique est choisi parmi le groupe constitué de la Protéine A et d'au moins un des anticorps anti-lgG humaine et anti-lgM humaine.

4. Procédé selon la revendication 2 ou 3, dans lequel ledit marqueur est choisi parmi le groupe constitué des chélates de lanthanides, des biotines, des enzymes et des isotopes radioactifs.

5. Utilisation d'antigène de capside de NANBV conjointement avec l'antigène C100-3 pour la préparation d'une composition de diagnostic pour diagnostiquer la séroconversion associée à l'infection par NANBV très tôt après l'infection.

6. Procédé selon l'une quelconque des revendications 1 à 4 ou utilisation selon la revendication 5, dans lequel ledit antigène de capside de NANBV est fixé à une matrice solide.

7. Procédé selon l'une quelconque des revendications 1 à 4 ou 6 ou utilisation selon la revendication 5 ou 6, dans lesquels ledit antigène de capside de NANBV fait partie d'une protéine de fusion.

8. Procédé selon l'une quelconque des revendications 1 à 4, 6 ou 7, ou utilisation selon l'une quelconque des revendications 5 à 7, dans lesquels ledit antigène de capside de NANBV est choisi parmi le groupe constitué de
(a) un antigène de capside de NANBV ayant la séquence d'acides aminés du résidu 1 à 120 de SEQ ID n° 1 ;
(b) un antigène de capside de NANBV ayant la séquence d'acides aminés du résidu 1 à 74 de SEQ ID n° 1 ;
(c) un antigène de capside de NANBV ayant la séquence d'acides aminés du résidu 69 à 120 de SEQ ID n° 1.


### SEQUENCE LISTING

(1) GENERAL INFORMATION:
   (i) APPLICANT: Zebedee, Suzanne Inchauspe, Genevieve
      Nasoff, Marc
      Prince, Alfred
   (ii) TITLE OF INVENTION: NON-A, NON-B HEPATITIS VIRUS ANTIGEN, DIAGNOSTIC METHODS AND VACCINES
   (iii) NUMBER OF SEQUENCES: 137
   (iv) CORRESPONDENCE ADDRESS:
      (A) ADDRESSEE: DRESSLER, GOLDSMITH, SHORE, SUTKER & MILNAMOW, LTD.
      (B) STREET: 180 N. Stetson, Suite 4700
      (C) CITY: Chicago
      (D) STATE: IL
      (E) COUNTRY: USA
      (F) ZIP: 60601
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patentin Release #1.0, Version #1.25
   (vi) CURRENT APPLICATION DATA:
      (A) APPLICATION NUMBER: US
      (B) FILING DATE:
      (C) CLASSIFICATION:
   (vii) PRIOR APPLICATION DATA:
      (A) APPLICATION NUMBER: Us 07/616369
      (B) FILING DATE:21-NOV-1990
   (vii) PRIOR APPLICATION DATA:
      (A) APPLICATION NUMBER: US 07/573643
      (B) FILING DATE: 25-AUG-1 990
   (viii) ATTORNEY/AGENT INFORMATION:
      (A) NAME: Gamson, Edward P.
      (B) REGISTRATION NUMBER: 29,381
      (C) REFERENCE/DOCKET NUMBER: PHA0029P
   (ix) TELECOMMUNICATION INFORMATION:
      (A) TELEPHONE: 312-616-5400
      (B) TELEFAX: 312-616-5460
(2) INFORMATION FOR SEQ ID NO:1:
   (i) SEQUENCE CHARACTERISTICS:
      (A) LENGTH: 978 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO (ix) FEATURE:
      (A) NAME/KEY: CDS
      (B) LOCATION: 1..978
      (D) OTHER INFORMATION: /codon start= 1 /product= "NANBV Structural Antigen" /number= 1
   (xi) SEQUENCE DESCRIPTION: SEQ ID NO:1:
   ATG AGC ACG ATT CCC AAA CCT CAA AGA AAA ACC AAA CGT AAC ACC AAC     48
   Met Ser Thr Ile Pro Lys Pro Gln Arg Lys Thr Lys Arg Asn Thr Asn
    1               5                  10                  15
   CGT CGC CCA CAG GAC GTC AAG TTC CCG GGT GGC GGT CAG ATC GTT GGT     96
   Arg Arg Pro Gln Asp Val Lys Phe Pro Gly Gly Gly Gln Ile Val Gly
               20                  25                  30
   GGA GTT TAC TTG TTG CCG CGC AGG GGC CCT AGA TTG GGT GTG CGC GCG    144
   Gly Val Tyr Leu Leu Pro Arg Arg Gly Pro Arg Leu Gly Val Arg Ala
           35                  40                  45
   ACG AGG AAG ACT TCC GAG CGG TCG CAA CCT CGA GGT AGA CGT CAG CCT    192
   Thr Arg Lys Thr Ser Glu Arg Ser Gln Pro Arg Gly Arg Arg Gln Pro
       50                  55                  60
   ATC CCC AAG GCA CGT CGG CCC GAG GGC AGG ACC TGG GCT CAG CCC GGG    240
   Ile Pro Lys Ala Arg Arg Pro Glu Gly Arg Thr Trp Ala Gln Pro Gly
    65                 70                  75                  80
   TAC CCT TGG CCC CTC TAT GGC AAT GAG GGT TGC GGG TGG GCG GGA TGG    288
   Tyr Pro Trp Pro Leu Tyr Gly Asn Glu Gly Cys Gly Trp Ala Gly Trp
                   85                  90                  95
   CTC CTG TCT CCC CGT GGC TCT CGG CCT AGC TGG GGC CCC ACA GAC CCC    336
   Leu Leu Ser Pro Arg Gly Ser Arg Pro Ser Trp Gly Pro Thr Asp Pro
               100                 105                 110
   CGG CGT AGG TCG CGC AAT TTG GGT AAG GTC ATC GAT ACC CTT ACG TGC       384
   Arg Arg Arg Ser Arg Asn Leu Gly Lys Val Ile Asp Thr Leu Thr Cys
           115                 120                 125
   GGC TTC GCC GAC CTC ATG GGG TAC ATA CCG CTC GTC GGC GCC CCT CTT       432
   Gly Phe Ala Asp Leu Met Gly Tyr Ile Pro Leu Val Gly Ala Pro Leu
       130                 135                 140
   GGA GGC GCT GCC AGG GCC CTG GCG CAT GGC GTC CGG GTT CTG GAA GAC       480
   Gly Gly Ala Ala Arg Ala Leu Ala His Gly Val Arg Val Leu Glu Asp
   145                 150                 155                 160
   GGC GTG AAC TAT GCA ACA GGG AAC CTT CCT GGT TGC TCT TTC TCT ATC       528
   Gly Val Asn Tyr Ala Thr Gly Asn Leu Pro Gly Cys Ser Phe Ser Ile
                   165                 170                 175
   TTC CTT CTG GCC CTG CTC TCT TGC CTG ACT GTG CCC GCT TCA GCC TAC       576
   Phe Leu Leu Ala Leu Leu Ser Cys Leu Thr Val Pro Ala Ser Ala Tyr
               180                 185                 190
   CAA GTG CGC AAT TCC TCG GGG CTT TAC CAT GTC ACC AAT GAT TGC CCT       624
   Gln Val Arg Asn Ser Ser Gly Leu Tyr His Val Thr Asn Asp Cys Pro
           195                 200                 205
   AAC TCG AGT GTT GTG TAC GAG GCG GCC GAT GCC ATC CTG CAC ACT CCG       672
   Asn Ser Ser Val Val Tyr Glu Ala Ala Asp Ala Ile Leu His Thr Pro
       210                 215                 220
   GGG TGT GTC CCT TGC GTT CGC GAG GGT AAC GCC TCG AGG TGT TGG GTG       720
   Gly Cys Val Pro Cys Val Arg Glu Gly Asn Ala Ser Arg Cys Trp Val
   225                 230                 235                 240
   GCG GTG ACC CCC ACG GTG GCC ACC AGG GAC GGC AAA CTT CCC ACA ACG       768
   Ala Val Thr Pro Thr Val Ala Thr Arg Asp Gly Lys Leu Pro Thr Thr
                   245                 250                 255
   CAG CTT CGA CGT CAT ATC GAT CTG CTT GTC GGG AGC GCC ACC CTC TGC       816
   Gln Leu Arg Arg His Ile Asp Leu Leu Val Gly Ser Ala Thr Leu Cys
               260                 265                 270
   TCG GCC CTC TAC GTG GGG GAC CTG TGC GGG TCT GTC TTT CTC GTT GGT       864
   Ser Ala Leu Tyr Val Gly Asp Leu Cys Gly Ser Val Phe Leu Val Gly
           275                 280                 285
   CAA CTG TTT ACC TTC TCT CCC AGG CGC CAC TGG ACG ACG CAA GAC TGC       912
   Gln Leu Phe Thr Phe Ser Pro Arg Arg His Trp Thr Thr Gln Asp Cys
       290                 295                 300
   AAT TGT TCT ATC TAT CCC GGC CAT ATA ACG GGT CAT CGC ATG GCA TGG       960
   Asn Cys Ser Ile Tyr Pro Gly His Ile Thr Gly His Arg Met Ala Trp
   305                 310                 315                 320
   GAT ATG ATG ATG AAC TGG                                               978
   Asp Met Met Met Asn Trp
                   325
(2) INFORMATION FOR SEQ ID NO:2:
   (i) SEQUENCE CHARACTERISTICS:
      (A) LENGTH: 948 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..945
   (xi) SEQUENCE DESCRIPTION: SEQ ID NO:2:
   ATG TCC CCT ATA CTA GGT TAT TGG AAA ATT AAG GGC CTT GTG CAA CCC      48
   Met Ser Pro Ile Leu Gly Tyr Trp Lys Ile Lys Gly Leu Val Gln Pro
    1               5                  10                  15
   ACT CGA CTT CTT TTG GAA TAT CTT GAA GAA AAA TAT GAA GAG CAT TTG      96
   Thr Arg Leu Leu Leu Glu Tyr Leu Glu Glu Lys Tyr Glu Glu His Leu
               20                  25                  30
   TAT GAG CGC GAT GAA GGT GAT AAA TGG CGA AAC AAA AAG TTT GAA TTG     144
   Tyr Glu Arg Asp Glu Gly Asp Lys Trp Arg Asn Lys Lys Phe Glu Leu
           35                  40                  45
   GGT TTG GAG TTT CCC AAT CTT CCT TAT TAT ATT GAT GGT GAT GTT AAA     192
   Gly Leu Glu Phe Pro Asn Leu Pro Tyr Tyr Ile Asp Gly Asp Val Lys
       50                  55                  60
   TTA ACA CAG TCT ATG GCC ATC ATA CGT TAT ATA GCT GAC AAG CAC AAC     240
   Leu Thr Gln Ser Met Ala Ile Ile Arg Tyr Ile Ala Asp Lys His Asn
   65                  70                  75                  80
   ATG TTG GGT GGT TGT CCA AAA GAG CGT GCA GAG ATT TCA ATG CTT GAA     288
   Met Leu Gly Gly Cys Pro Lys Glu Arg Ala Glu Ile Ser Met Leu Glu
                       85                                90                              95
   GGA GCG GTT TTG GAT ATT AGA TAC GGT GTT TCG AGA ATT GCA TAT AGT        336
   Gly Ala Val Leu Asp Ile Arg Tyr Gly Val Ser Arg Ile Ala Tyr Ser
               100                 105                 110
   AAA GAC TTT GAA ACT CTC AAA GTT GAT TTT CTT AGC AAG CTA CCT GAA        384
   Lys Asp Phe Glu Thr Leu Lys Val Asp Phe Leu Ser Lys Leu Pro Glu
           115                 120                 125
   ATG CTG AAA ATG TTC GAA GAT CGT TTA TGT CAT AAA ACA TAT TTA AAT        432
   Met Leu Lys Met Phe Glu Asp Arg Leu Cys His Lys Thr Tyr Leu Asn
       130                 135                 140
   GGT GAT CAT GTA ACC CAT CCT GAC TTC ATG TTG TAT GAC GCT CTT GAT        480
   Gly Asp His Val Thr His Pro Asp Phe Met Leu Tyr Asp Ala Leu Asp
   145                 150                 155                 160
   GTT GTT TTA TAC ATG GAC CCA ATG TGC CTG GAT GCG TTC CCA AAA TTA        528
   Val Val Leu Tyr Met Asp Pro Met Cys Leu Asp Ala Phe Pro Lys Leu
                   165                 170                 175
   GTT TGT TTT AAA AAA CGT ATT GAA GCT ATC CCA CAA ATT GAT AAG TAC        576
   Val Cys Phe Lys Lys Arg Ile Glu Ala Ile Pro Gln Ile Asp Lys Tyr
               180                 185                 190
   TTG AAA TCC AGC AAG TAT ATA GCA TGG CCT TTG CAG GGC TGG CAA GCC        624
   Leu Lys Ser Ser Lys Tyr Ile Ala Trp Pro Leu Gln Gly Trp Gln Ala
           195                 200                 205
   ACG TTT GGT GGT GGC GAC CAT CCT CCA AAA TCG GAT CTG ATC GAA GGT        672
   Thr Phe Gly Gly Gly Asp His Pro Pro Lys Ser Asp Leu Ile Glu Gly
       210                 215                 220
   CGT GGG ATC CCC AAT TCG AGC TCG GTA CCC ATG AGC ACG ATT CCC AAA        720
   Arg Gly Ile Pro Asn Ser Ser Ser Val Pro Met Ser Thr Ile Pro Lys
   225                 230                 235                 240
   CCT CAA AGA AAA ACC AAA CGT AAC ACC AAC CGT CGC CCA CAG GAC GTC        768
   Pro Gln Arg Lys Thr Lys Arg Asn Thr Asn Arg Arg Pro Gln Asp Val
                   245                 250                 255
   AAG TTC CCG GGT GGC GGT CAG ATC GTT GGT GGA GTT TAC TTG TTG CCG        816
   Lys Phe Pro Gly Gly Gly Gln Ile Val Gly Gly Val Tyr Leu Leu Pro
               260                 265                 270
   CGC AGG GGC CCT AGA TTG GGT GTG CGC GCG ACG AGG AAG ACT TCC GAG        864
   Arg Arg Gly Pro Arg Leu Gly Val Arg Ala Thr Arg Lys Thr Ser Glu
           275                 280                 285
   CGG TCG CAA CCT CGA GGT AGA CGT CAG CCT ATC CCC AAG GCA CGT CGG        912
   Arg Ser Gln Pro Arg Gly Arg Arg Gln Pro Ile Pro Lys Ala Arg Arg
       290                 295                 300
   CCC GAG GGC AGG ACG GGG ATC GGG AAT TCA TCG TGA                        948
   Pro Glu Gly Arg Thr Gly Ile Gly Asn Ser Ser
   305                 310                 315
(2) INFORMATION FOR SEQ ID NO:3:
   (i) SEQUENCE CHARACTERISTICS:
      (A) LENGTH: 759 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO (ix) FEATURE:
      (A) NAME/KEY: CDS
      (B) LOCATION: 1..756
   (xi) SEQUENCE DESCRIPTION: SEQ ID NO:3:
   ATG TCC CCT ATA CTA GGT TAT TGG AAA ATT AAG GGC CTT GTG CAA CCC      48
   Met Ser Pro Ile Leu Gly Tyr Trp Lys Ile Lys Gly Leu Val Gln Pro
     1               5                  10                  15
   ACT CGA CTT CTT TTG GAA TAT CTT GAA GAA AAA TAT GAA GAG CAT TTG      96
   Thr Arg Leu Leu Leu Glu Tyr Leu Glu Glu Lys Tyr Glu Glu His Leu
                20                  25                  30
   TAT GAG CGC GAT GAA GGT GAT AAA TGG CGA AAC AAA AAG TTT GAA TTG     144
   Tyr Glu Arg Asp Glu Gly Asp Lys Trp Arg Asn Lys Lys Phe Glu Leu
            35                  40                  45
   GGT TTG GAG TTT CCC AAT CTT CCT TAT TAT ATT GAT GGT GAT GTT AAA     192
   Gly Leu Glu Phe Pro Asn Leu Pro Tyr Tyr Ile Asp Gly Asp Val Lys
        50                  55                  60
   TTA ACA CAG TCT ATG GCC ATC ATA CGT TAT ATA GCT GAC AAG CAC AAC     240
   Leu Thr Gln Ser Met Ala Ile Ile Arg Tyr Ile Ala Asp Lys His Asn
    65                  70                  75                  80
   ATG TTG GGT GGT TGT CCA AAA GAG CGT GCA GAG ATT TCA ATG CTT GAA     288
   Met Leu Gly Gly Cys Pro Lys Glu Arg Ala Glu Ile Ser Met Leu Glu
                    85                  90                  95
   GGA GCG GTT TTG GAT ATT AGA TAC GGT GTT TCG AGA ATT GCA TAT AGT        336
   Gly Ala Val Leu Asp Ile Arg Tyr Gly Val Ser Arg Ile Ala Tyr Ser
               100                 105                 110
   AAA GAC TTT GAA ACT CTC AAA GTT GAT TTT CTT AGC AAG CTA CCT GAA        384
   Lys Asp Phe Glu Thr Leu Lys Val Asp Phe Leu Ser Lys Leu Pro Glu
           115                 120                 125
   ATG CTG AAA ATG TTC GAA GAT CGT TTA TGT CAT AAA ACA TAT TTA AAT        432
   Met Leu Lys Met Phe Glu Asp Arg Leu Cys His Lys Thr Tyr Leu Asn
       130                 135                 140
   GGT GAT CAT GTA ACC CAT CCT GAC TTC ATG TTG TAT GAC GCT CTT GAT        480
   Gly Asp His Val Thr His Pro Asp Phe Met Leu Tyr Asp Ala Leu Asp
   145                 150                 155                 160
   GTT GTT TTA TAC ATG GAC CCA ATG TGC CTG GAT GCG TTC CCA AAA TTA        528
   Val Val Leu Tyr Met Asp Pro Met Cys Leu Asp Ala Phe Pro Lys Leu
                   165                 170                 175
   GTT TGT TTT AAA AAA CGT ATT GAA GCT ATC CCA CAA ATT GAT AAG TAC        576
   Val Cys Phe Lys Lys Arg Ile Glu Ala Ile Pro Gln Ile Asp Lys Tyr
               180                 185                 190
   TTG AAA TCC AGC AAG TAT ATA GCA TGG CCT TTG CAG GGC TGG CAA GCC        624
   Leu Lys Ser Ser Lys Tyr Ile Ala Trp Pro Leu Gln Gly Trp Gln Ala
           195                 200                 205
   ACG TTT GGT GGT GGC GAC CAT CCT CCA AAA TCG GAT CTG GTT CCG CGT        672
   Thr Phe Gly Gly Gly Asp His Pro Pro Lys Ser Asp Leu Val Pro Arg
       210                 215                 220
   GGA TCC ATG AGC ACG ATT CCC AAA CCT CAA AGA AAA ACC AAA CGT AAC        720
   Gly Ser Met Ser Thr Ile Pro Lys Pro Gln Arg Lys Thr Lys Arg Asn
   225                 230                 235                 240
   ACC AAC CGT CGC CCA CAG GAA TTC ATC GTG ACT GAC TGA                    759
   Thr Asn Arg Arg Pro Gln Glu Phe Ile Val Thr Asp
                   245                 250
(2) INFORMATION FOR SEQ ID NO:4:
   (i) SEQUENCE CHARACTERISTICS:
      (A) LENGTH: 759 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..756
   (xi) SEQUENCE DESCRIPTION: SEQ ID NO:4:
   ATG TCC CCT ATA CTA GGT TAT TGG AAA ATT AAG GGC CTT GTG CAA CCC      48
   Met Ser Pro Ile Leu Gly Tyr Trp Lys Ile Lys Gly Leu Val Gln Pro
    1               5                  10                  15
   ACT CGA CTT CTT TTG GAA TAT CTT GAA GAA AAA TAT GAA GAG CAT TTG      96
   Thr Arg Leu Leu Leu Glu Tyr Leu Glu Glu Lys Tyr Glu Glu His Leu
               20                  25                  30
   TAT GAG CGC GAT GAA GGT GAT AAA TGG CGA AAC AAA AAG TTT GAA TTG     144
   Tyr Glu Arg Asp Glu Gly Asp Lys Trp Arg Asn Lys Lys Phe Glu Leu
           35                  40                  45
   GGT TTG GAG TTT CCC AAT CTT CCT TAT TAT ATT GAT GGT GAT GTT AAA     192
   Gly Leu Glu Phe Pro Asn Leu Pro Tyr Tyr Ile Asp Gly Asp Val Lys
       50                  55                  60
   TTA ACA CAG TCT ATG GCC ATC ATA CGT TAT ATA GCT GAC AAG CAC AAC     240
   Leu Thr Gln Ser Met Ala Ile Ile Arg Tyr Ile Ala Asp Lys His Asn
   65                  70                  75                  80
   ATG TTG GGT GGT TGT CCA AAA GAG CGT GCA GAG ATT TCA ATG CTT GAA     288
   Met Leu Gly Gly Cys Pro Lys Glu Arg Ala Glu Ile Ser Met Leu Glu
                   85                  90                  95
   GGA GCG GTT TTG GAT ATT AGA TAC GGT GTT TCG AGA ATT GCA TAT AGT     336
   Gly Ala Val Leu Asp Ile Arg Tyr Gly Val Ser Arg Ile Ala Tyr Ser
               100                 105                 110
   AAA GAC TTT GAA ACT CTC AAA GTT GAT TTT CTT AGC AAG CTA CCT GAA     384
   Lys Asp Phe Glu Thr Leu Lys Val Asp Phe Leu Ser Lys Leu Pro Glu
           115                 120                 125
   ATG CTG AAA ATG TTC GAA GAT CGT TTA TGT CAT AAA ACA TAT TTA AAT     432
   Met Leu Lys Met Phe Glu Asp Arg Leu Cys His Lys Thr Tyr Leu Asn
       130                 135                 140
   GGT GAT CAT GTA ACC CAT CCT GAC TTC ATG TTG TAT GAC GCT CTT GAT     480
   Gly Asp His Val Thr His Pro Asp Phe Met Leu Tyr Asp Ala Leu Asp
   145                 150                 155                 160
   GTT GTT TTA TAC ATG GAC CCA ATG TGC CTG GAT GCG TTC CCA AAA TTA     528
   Val Val Leu Tyr Met Asp Pro Met Cys Leu Asp Ala Phe Pro Lys Leu
                   165                 170                 175
   GTT TGT TTT AAA AAA CGT ATT GAA GCT ATC CCA CAA ATT GAT AAG TAC     576
   Val Cys Phe Lys Lys Arg Ile Glu Ala Ile Pro Gln Ile Asp Lys Tyr
               180                 185                 190
   TTG AAA TCC AGC AAG TAT ATA GCA TGG CCT TTG CAG GGC TGG CAA GCC     624
   Leu Lys Ser Ser Lys Tyr Ile Ala Trp Pro Leu Gln Gly Trp Gln Ala
           195                 200                 205
   ACG TTT GGT GGT GGC GAC CAT CCT CCA AAA TCG GAT CTG GTT CCG CGT     672
   Thr Phe Gly Gly Gly Asp His Pro Pro Lys Ser Asp Leu Val Pro Arg
       210                 215                 220
   GGA TCC GAC GTC AAG TTC CCG GGT GGC GGT CAG ATC GTT GGT GGA GTT     720
   Gly Ser Asp Val Lys Phe Pro Gly Gly Gly Gln Ile Val Gly Gly Val
   225                 230                 235                 240
   TAC TTG TTG CCG CGC AGG GAA TTC ATC GTG ACT GAC TGA                 759
   Tyr Leu Leu Pro Arg Arg Glu Phe Ile Val Thr Asp
                   245                 250
(2) INFORMATION FOR SEQ ID NO:5:
   (i) SEQUENCE CHARACTERISTICS:
      (A) LENGTH: 759 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..756
   (xi) SEQUENCE DESCRIPTION: SEQ ID NO:5:
   ATG TCC CCT ATA CTA GGT TAT TGG AAA ATT AAG GGC CTT GTG CAA CCC        48
   Met Ser Pro Ile Leu Gly Tyr Trp Lys Ile Lys Gly Leu Val Gln Pro
     1               5                  10                  15
   ACT CGA CTT CTT TTG GAA TAT CTT GAA GAA AAA TAT GAA GAG CAT TTG        96
   Thr Arg Leu Leu Leu Glu Tyr Leu Glu Glu Lys Tyr Glu Glu His Leu
               20                  25                  30
   TAT GAG CGC GAT GAA GGT GAT AAA TGG CGA AAC AAA AAG TTT GAA TTG      144
   Tyr Glu Arg Asp Glu Gly Asp Lys Trp Arg Asn Lys Lys Phe Glu Leu
           35                  40                  45
   GGT TTG GAG TTT CCC AAT CTT CCT TAT TAT ATT GAT GGT GAT GTT AAA      192
   Gly Leu Glu Phe Pro Asn Leu Pro Tyr Tyr Ile Asp Gly Asp Val Lys
       50                  55                  60
   TTA ACA CAG TCT ATG GCC ATC ATA CGT TAT ATA GCT GAC AAG CAC AAC      240
   Leu Thr Gln Ser Met Ala Ile Ile Arg Tyr Ile Ala Asp Lys His Asn
   65                  70                  75                  80
   ATG TTG GGT GGT TGT CCA AAA GAG CGT GCA GAG ATT TCA ATG CTT GAA      288
   Met Leu Gly Gly Cys Pro Lys Glu Arg Ala Glu Ile Ser Met Leu Glu
                   85                  90                  95
   GGA GCG GTT TTG GAT ATT AGA TAC GGT GTT TCG AGA ATT GCA TAT AGT      336
   Gly Ala Val Leu Asp Ile Arg Tyr Gly Val Ser Arg Ile Ala Tyr Ser
               100                 105                 110
   AAA GAC TTT GAA ACT CTC AAA GTT GAT TTT CTT AGC AAG CTA CCT GAA      384
   Lys Asp Phe Glu Thr Leu Lys Val Asp Phe Leu Ser Lys Leu Pro Glu
           115                 120                 125
   ATG CTG AAA ATG TTC GAA GAT CGT TTA TGT CAT AAA ACA TAT TTA AAT      432
   Met Leu Lys Met Phe Glu Asp Arg Leu Cys His Lys Thr Tyr Leu Asn
       130                 135                 140
   GGT GAT CAT GTA ACC CAT CCT GAC TTC ATG TTG TAT GAC GCT CTT GAT      480
   Gly Asp His Val Thr His Pro Asp Phe Met Leu Tyr Asp Ala Leu Asp
   145                 150                 155                 160
   GTT GTT TTA TAC ATG GAC CCA ATG TGC CTG GAT GCG TTC CCA AAA TTA      528
   Val Val Leu Tyr Met Asp Pro Met Cys Leu Asp Ala Phe Pro Lys Leu
                   165                 170                 175
   GTT TGT TTT AAA AAA CGT ATT GAA GCT ATC CCA CAA ATT GAT AAG TAC      576
   Val Cys Phe Lys Lys Arg Ile Glu Ala Ile Pro Gln Ile Asp Lys Tyr
               180                 185                 190
   TTG AAA TCC AGC AAG TAT ATA GCA TGG CCT TTG CAG GGC TGG CAA GCC      624
   Leu Lys Ser Ser Lys Tyr Ile Ala Trp Pro Leu Gln Gly Trp Gln Ala
           195                 200                 205
   ACG TTT GGT GGT GGC GAC CAT CCT CCA AAA TCG GAT CTG GTT CCG CGT      672
   Thr Phe Gly Gly Gly Asp His Pro Pro Lys Ser Asp Leu Val Pro Arg
       210                 215                 220
   GGA TCC GGC CCT AGA TTG GGT GTG CGC GCG ACG AGG AAG ACT TCC GAG      720
   Gly Ser Gly Pro Arg Leu Gly Val Arg Ala Thr Arg Lys Thr Ser Glu
   225                 230                 235                 240
   CGG TCG CAA CCT CGA GGT GAA TTC ATC GTG ACT GAC TGA                  759
   Arg Ser Gln Pro Arg Gly Glu Phe Ile Val Thr Asp
           245                 250
(2) INFORMATION FOR SEQ ID NO:6:
   (i) SEQUENCE CHARACTERISTICS:
      (A) LENGTH: 816 base pairs'
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..813
   (xi) SEQUENCE DESCRIPTION: SEQ ID NO:6:
   ATG TCC CCT ATA CTA GGT TAT TGG AAA ATT AAG GGC CTT GTG CAA CCC        48
   Met Ser Pro Ile Leu Gly Tyr Trp Lys Ile Lys Gly Leu Val Gln Pro
     1               5                  10                  15
   ACT CGA CTT CTT TTG GAA TAT CTT GAA GAA AAA TAT GAA GAG CAT TTG        96
   Thr Arg Leu Leu Leu Glu Tyr Leu Glu Glu Lys Tyr Glu Glu His Leu
                20                  25                  30
   TAT GAG CGC GAT GAA GGT GAT AAA TGG CGA AAC AAA AAG TTT GAA TTG       144
   Tyr Glu Arg Asp Glu Gly Asp Lys Trp Arg Asn Lys Lys Phe Glu Leu
            35                  40                  45
   GGT TTG GAG TTT CCC AAT CTT CCT TAT TAT ATT GAT GGT GAT GTT AAA       192
   Gly Leu Glu Phe Pro Asn Leu Pro Tyr Tyr Ile Asp Gly Asp Val Lys
        50                  55                  60
   TTA ACA CAG TCT ATG GCC ATC ATA CGT TAT ATA GCT GAC AAG CAC AAC       240
   Leu Thr Gln Ser Met Ala Ile Ile Arg Tyr Ile Ala Asp Lys His Asn
    65                  70                  75                  80
   ATG TTG GGT GGT TGT CCA AAA GAG CGT GCA GAG ATT TCA ATG CTT GAA       288
   Met Leu Gly Gly Cys Pro Lys Glu Arg Ala Glu Ile Ser Met Leu Glu
                       85                      90                      95
   GGA GCG GTT TTG GAT ATT AGA TAC GGT GTT TCG AGA ATT GCA TAT AGT          336
   Gly Ala Val Leu Asp Ile Arg Tyr Gly Val Ser Arg Ile Ala Tyr Ser
               100                 105                     110
   AAA GAC TTT GAA ACT CTC AAA GTT GAT TTT CTT AGC AAG CTA CCT GAA          384
   Lys Asp Phe Glu Thr Leu Lys Val Asp Phe Leu Ser Lys Leu Pro Glu
           115                 120                     125
   ATG CTG AAA ATG TTC GAA GAT CGT TTA TGT CAT AAA ACA TAT TTA AAT          432
   Met Leu Lys Met Phe Glu Asp Arg Leu Cys His Lys Thr Tyr Leu Asn
       130                 135                     140
   GGT GAT CAT GTA ACC CAT CCT GAC TTC ATG TTG TAT GAC GCT CTT GAT          480
   Gly Asp His Val Thr His Pro Asp Phe Met Leu Tyr Asp Ala Leu Asp
   145                 150                     155                 160
   GTT GTT TTA TAC ATG GAC CCA ATG TGC CTG GAT GCG TTC CCA AAA TTA          528
   Val Val Leu Tyr Met Asp Pro Met Cys Leu Asp Ala Phe Pro Lys Leu
                   165                     170                 175
   GTT TGT TTT AAA AAA CGT ATT GAA GCT ATC CCA CAA ATT GAT AAG TAC          576
   Val Cys Phe Lys Lys Arg Ile Glu Ala Ile Pro Gln Ile Asp Lys Tyr
               180                 185                     190
   TTG AAA TCC AGC AAG TAT ATA GCA TGG CCT TTG CAG GGC TGG CAA GCC          624
   Leu Lys Ser Ser Lys Tyr Ile Ala Trp Pro Leu Gln Gly Trp Gln Ala
           195                 200                     205
   ACG TTT GGT GGT GGC GAC CAT CCT CCA AAA TCG GAT CTG GTT CCG CGT          672
   Thr Phe Gly Gly Gly Asp His Pro Pro Lys Ser Asp Leu Val Pro Arg
       210                 215                     220
   GGA TCC AGC ACG ATT CCC AAA CCT CAA AGA AAA ACC AAA CGT AAC ACC          720
   Gly Ser Ser Thr Ile Pro Lys Pro Gln Arg Lys Thr Lys Arg Asn Thr
   225                 230                     235                 240
   AAC CGT CGC CCA CAG GAC GTC AAG TTC CCG GGT GGC GGT CAG ATC GTT          768
   Asn Arg Arg Pro Gln Asp Val Lys Phe Pro Gly Gly Gly Gln Ile Val
                   245                     250                 255
   GGT GGA GTT TAC TTG TTG CCG CGC AGG GAA TTC ATC GTG ACT GAC              813
   Gly Gly Val Tyr Leu Leu Pro Arg Arg Glu Phe Ile Val Thr Asp
               260                 265                     270
   TGA                                                                      816
(2) INFORMATION FOR SEQ ID NO:7:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:7:
   GATCCATGAG CACGATTCCC AAACCTCAAA GAAAAACCAA ACGTAACACC AACCGTCGCC 60
   CACAGG 66
(2) INFORMATION FOR SEQ ID NO:8:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:8:
   AATTCCTGTG GGCGACGGTT GGTGTTACGT TTGGTTTTTC TTTGAGGTTT GGGAATCGTG    60
   CTCATG 66
(2) INFORMATION FOR SEQ ID NO:9:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:9:
   GATCCGACGT CAAGTTCCCG GGTGGCGGTC AGATCGTTGG TGGAGTTTAC TTGTTGCCGC 60
   GCAGGG 66
(2) INFORMATION FOR SEQ ID NO:10:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:10:
   AATTCCCTGC GCGGCAACAA GTAAACTCCA CCAACGATCT GACCGCCACC CGGGAACTTG 60
   ACGTCG 66
(2) INFORMATION FOR SEQ ID NO:11:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO (xi) SEQUENCE DESCRIPTION: SEQ ID NO:11:
(2) INFORMATION FOR SEQ ID NO:12:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:12:
   AATTCACCTC GAGGTTGCGA CCGCTCGGAA GTCTTCCTCG TCGCGCGCAC ACCCAATCTA 60
   GGGCCG 66
(2) INFORMATION FOR SEQ ID NO:13:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:13:
      GAATTCTTAC CTGCGCGGCA ACAAGTAAAC TC 32
(2) INFORMATION FOR SEQ ID NO:14:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:14:
      GCTGGATCCA GCACGATTCC CAAACCTCAA AG 32
(2) INFORMATION FOR SEQ ID NO:15:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:15:
      ATGAGCACGA TTCCCAAACC T 21
(2) INFORMATION FOR SEQ ID NO:16:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:16:
      GAGGAAGACTTCCGAGC 17
(2) INFORMATION FOR SEQ ID NO:17:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:17:
      GTCCTGCCCT CGGGCCG 17
(2) INFORMATION FOR SEQ ID NO:18:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:18:
      ACCCAAATTG CGCGACCTAC G 21
(2) INFORMATION FOR SEQ ID NO:19:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:19:
      TGGGTAAGGT CATCGATAC 19
(2) INFORMATION FOR SEQ ID NO:20:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:20:
      AAGGTCATCG ATACCCT 17
(2) INFORMATION FOR SEQ ID NO:21:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:21:
      AGATAGAGAA AGAGCAAC 18
(2) INFORMATION FOR SEQ ID NO:22:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:22:
      GGACCAGTTC ATCATCATAT AT 22
(2) INFORMATION FOR SEQ ID NO:23:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:23:
      CAGTTCATCA --TCATATCCCA 20
(2) INFORMATION FOR SEQ ID NO:24:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..15
      (D) OTHER INFORMATION: /product= "Linker Protein in GST-NANBV 693-691"
   (xi) SEQUENCE DESCRIPTION: SEQ ID NO:24:
   GGG ATC CCC AAT TCA                                                15
   Gly Ile Pro Asn Ser
     1               5
(2) INFORMATION FOR SEQ ID NO:25:
   (i) SEQUENCE CHARACTERISTICS:
      (A) LENGTH: 5 amino acids
      (B) TYPE: amino acid
      (D) TOPOLOGY: linear
   (ii) MOLECULE TYPE: protein
   (xi) SEQUENCE DESCRIPTION: SEQ ID NO:25:
   Gly Ile Pro Asn Ser
     1               5
(2) INFORMATION FOR SEQ ID NO:26:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..9
      (D) OTHER INFORMATION: /product= "Carboxy-terminal Linker Protein in GST-NANBV 693-691"
   (xi) SEQUENCE DESCRIPTION: SEQ ID NO:26:
   AAT TCA TCG TGA                                                        12
   Asn Ser Ser
     1
(2) INFORMATION FOR SEQ ID NO:27:
   (i) SEQUENCE CHARACTERISTICS:
      (A) LENGTH: 3 amino acids
      (B) TYPE: amino acid
      (D) TOPOLOGY: linear
   (ii) MOLECULE TYPE: protein
   (xi) SEQUENCE DESCRIPTION: SEQ ID NO:27:
   Asn Ser Ser
    1
(2) INFORMATION FOR SEQ ID NO:28:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..27
      (D) OTHER INFORMATION: /product= "Linker Protein in GST-NANBV 15-18"
   (xi) SEQUENCE DESCRIPTION: SEQ ID NO:28:
   GGG ATC CCC ATC GAA TTC CTG CAG CCC                                    27
   Gly Ile Pro Ile Glu Phe Leu Gln Pro
    1               5
(2) INFORMATION FOR SEQ ID NO:29:
   (i) SEQUENCE CHARACTERISTICS:
      (A) LENGTH: 9 amino acids
      (B) TYPE: amino acid
      (D) TOPOLOGY: linear
   (ii) MOLECULE TYPE: protein
   (xi) SEQUENCE DESCRIPTION: SEQ ID NO:29:
   Gly Ile Pro Ile Glu Phe Leu Gln Pro
    1               5
(2) INFORMATION FOR SEQ ID NO:30:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..21
      (D) OTHER INFORMATION: /product= "Carboxy-terntinal Linker Protein in GST-NANBV 15-18"
   (xi) SEQUENCE DESCRIPTION: SEQ ID NO:30:
   TGG GGG ATC GGG AAT TCA TCG TGA                                 24
   Trp Gly Ile Gly Asn Ser Ser
    1               5
(2) INFORMATION FOR SEQ ID NO:31:
   (i) SEQUENCE CHARACTERISTICS:
      (A) LENGTH: 7 amino acids
      (B) TYPE: amino acid
      (D) TOPOLOGY: linear
   (ii) MOLECULE TYPE: protein
   (xi) SEQUENCE DESCRIPTION: SEQ ID NO:31:
   Trp Gly Ile Gly Asn Ser Ser
    1               5
(2) INFORMATION FOR SEQ ID NO:32:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..24
      (D) OTHER INFORMATION: /product= "Linker Protein in GST-NANBV 15-17"
   (xi) SEQUENCE DESCRIPTION: SEQ ID NO:32:
   GGG ATC CCC AAT TCC TGC AGC CCT                                24
   Gly Ile Pro Asn Ser Cys Ser Pro
     1               5
(2) INFORMATION FOR SEQ ID NO:33:
   (i) SEQUENCE CHARACTERISTICS:
      (A) LENGTH: 8 amino acids
      (B) TYPE: amino acid
      (D) TOPOLOGY: linear
   • (ii) MOLECULE TYPE: protein
   (xi) SEQUENCE DESCRIPTION: SEQ ID NO:33:
   Gly Ile Pro Asn Ser Cys Ser Pro
     1               5
(2) INFORMATION FOR SEQ ID NO:34:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..18
      (D) OTHER INFORMATION: /product= "Carboxy-terminal Linker Protein in GST-NANBV 15-17"
   (xi) SEQUENCE DESCRIPTION: SEQ ID NO:34:
   GGG ATC GGG AAT TCA TCG TGA                                    21
   Gly Ile Gly Asn Ser Ser
     1               5
(2) INFORMATION FOR SEQ ID NO:35:
   (i) SEQUENCE CHARACTERISTICS:
      (A) LENGTH: 6 amino acids
      (B) TYPE: amino acid
      (D) TOPOLOGY: linear
   (ii) MOLECULE TYPE: protein
   (xi) SEQUENCE DESCRIPTION: SEQ ID NO:35:
   Gly Ile Gly Asn Ser Ser
    1               5
(2) INFORMATION FOR SEQ ID NO:36:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..15
      (D) OTHER INFORMATION: /product= "Thrombin Cleavage Site in GST-NANBV 15-17"
   (xi) SEQUENCE DESCRIPTION: SEQ ID NO:36:
   GTT CCG CGT GGA TCC                                              15
   Val Pro Arg Gly Ser
    1               5
(2) INFORMATION FOR SEQ ID NO:37:
   (i) SEQUENCE CHARACTERISTICS:
      (A) LENGTH: 5 amino acids
      (B) TYPE: amino acid
      (D) TOPOLOGY: linear
   (ii) MOLECULE TYPE: protein
   (xi) SEQUENCE DESCRIPTION: SEQ ID NO:37:
   Val Pro Arg Gly Ser
    1               5
(2) INFORMATION FOR SEQ ID NO:38:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..21
      (D) OTHER INFORMATION: /product= "Linker Protein in GST-NANBV 15-17
   (xi) SEQUENCE DESCRIPTION: SEQ ID NO:38:
   CCA TCG AAT TCC TGC AGC CCT                21
   Pro Ser Asn Ser Cys Ser Pro
   1               5
(2) INFORMATION FOR SEQ ID NO:39:
   (i) SEQUENCE CHARACTERISTICS:
      (A) LENGTH: 7 amino acids
      (B) TYPE: amino acid
      (D) TOPOLOGY: linear
   (ii) MOLECULE TYPE: protein
   (xi) SEQUENCE DESCRIPTION: SEQ ID NO:39:
   Pro Ser Asn Ser Cys Ser Pro
   1               5
(2) INFORMATION FOR SEQ ID NO:40:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..15
      (D) OTHER INFORMATION: /product= "Carboxy-terminal Linker Protein in GST-NANBV 15-17"
   (xi) SEQUENCE DESCRIPTION: SEQ ID NO:40:
   GGA ATT CAT CGT GAC TGA                                              18
   Gly Ile His Arg Asp
     1               5
(2) INFORMATION FOR SEQ ID NO:41:
   (i) SEQUENCE CHARACTERISTICS:
      (A) LENGTH: 5 amino acids
      (B) TYPE: amino acid
      (D) TOPOLOGY: linear
   (ii) MOLECULE TYPE: protein
   (xi) SEQUENCE DESCRIPTION: SEQ ID NO:41:
   Gly Ile His Arg Asp
     1               5
(2) INFORMATION FOR SEQ ID NO:42:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..27
      (D) OTHER INFORMATION: /product= "Linker Protein in GST-NANBV 690-691"
   (xi) SEQUENCE DESCRIPTION: SEQ ID NO:42:
   GGG ATC CCC AAT TCG AGC TCG GTA CCC                                  27
   Gly Ile Pro Asn Ser Ser Ser Val Pro
     1               5
(2) INFORMATION FOR SEQ ID NO:43:
   (i) SEQUENCE CHARACTERISTICS:
      (A) LENGTH: 9 amino acids
      (B) TYPE: amino acid
      (D) TOPOLOGY: linear
   (ii) MOLECULE TYPE: protein
   (xi) SEQUENCE DESCRIPTION: SEQ ID NO:43:
   Gly Ile Pro Asn Ser Ser Ser Val Pro
    1               5
(2) INFORMATION FOR SEQ ID NO:44:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..21
      (D) OTHER INFORMATION: /product= "Carboxy-terminal Linker Protein in GST-NANBV 690-691"
   (xi) SEQUENCE DESCRIPTION: SEQ ID NO:44:
   ACG GGG ATC GGG AAT TCA TCG TGA                                            24
   Thr Gly Ile Gly Asn Ser Ser
     1               5
(2) INFORMATION FOR SEQ ID NO:45:
   (i) SEQUENCE CHARACTERISTICS:
      (A) LENGTH: 7 amino acids
      (B) TYPE: amino acid
      (D) TOPOLOGY: linear
   (ii) MOLECULE TYPE: protein
   (xi) SEQUENCE DESCRIPTION: SEQ ID NO:45:
   Thr Gly Ile Gly Asn Ser Ser
   1 5
(2) INFORMATION FOR SEQ ID NO:46:
   (i) SEQUENCE CHARACTERISTICS:
      (A) LENGTH: 9416 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (ix) FEATURE:
      (A) NAME/KEY: CDS
      (B) LOCATION: 342..9374
   (ix) FEATURE:
      (A) NAME/KEY: misc_feature
      (B) LOCATION: 1..12
      (D) OTHER INFORMATION: /note= "Not confirmed as HCV-Hc59 Sequence"
   (ix) FEATURE:
      (A) NAME/KEY: misc_feature
      (B) LOCATION: 9397..9416
      (D) OTHER INFORMATION: /note= "Not confirmed as HCV-Hc59 Sequence"
   (ix) FEATURE:
      (A) NAME/KEY: repeat_unit
      (B) LOCATION: group(7..12, 42..47)
      (D) OTHER INFORMATION: /rpt_type= "other" /rpt_family= "1"
   (ix) FEATURE:
      (A) NAME/KEY: repeat_unit
      (B) LOCATION: group(23..28, 38..43, 9209..9214, 9391..9396)
      (D) OTHER INFORMATION: /rpt_type= "other" /rpt_family= "2"
   (ix) FEATURE:
      (A) NAME/KEY: repeat_unit
      (B) LOCATION: group(128..135, 315..322)
      (D) OTHER INFORMATION: /rpt_type= "other" /rpt_family= "3"
   (ix) FEATURE:
      (A) NAME/KEY: repeat_unit
      (B) LOCATION: group(9231..9237, 9245..9251, 9256..9262)
      (D) OTHER INFORMATION: /rpt_type= "other" /rpt_family= "4"
   (ix) FEATURE:
      (A) NAME/KEY: repeat_unit
      (B) LOCATION: group(9248..9253, 9221..9226, 9227..9232)
      (D) OTHER INFORMATION: /rpt_type= "other" /rpt_family= "5"
   (xi) SEQUENCE DESCRIPTION: SEQ ID NO:46:
   GCCAGCCCCC TGATGGGGGC GACACTCCAC CATGAATCAC TCCCCTGTGA GGAACTACTG     60
   TCTTCACGCA GAAAGCGTCT AGCCATGGCG TTAGTATGAG TGTCGTGCAG CCTCCAGGAC    120
   CCCCCCTCCC GGGAGAGCCA TAGTGGTCTG CGGAACCGGT GAGTACACCG GAATTGCCAG    180
   GACGACCGGG TCCTTTCTTG GATAAACCCG CTCAATGCCT GGAGATTTGG GCGTGCCCCC    240
   GCAAGACTGC TAGCCGAGTA GTGTTGGGTC GCGAAAGGCC TTGTGGTACT GCCTGATAGG    300
   GTGCTTGCGA GTGCCCCGGG AGGTCTCGTA GACCGTGCAC C ATG AGC ACG AAT        353
                                                 Met Ser Thr Asn
                                                  1
   CCT AAA CCT CAA AGA AAA ACC AAA CGT AAC ACC AAC CGT CGC CCA CAG      401
   Pro Lys Pro Gln Arg Lys Thr Lys Arg Asn Thr Asn Arg Arg Pro Gln
    5                  10                  15                  20
   GAC GTC AAG TTC CCG GGT GGC GGT CAG ATC GTT GGT GGA GTT TAC TTG      449
   Asp Val Lys Phe Pro Gly Gly Gly Gln Ile Val Gly Gly Val Tyr Leu
                   25                  30                  35
   TTG CCG CGC AGG GGC CCT AGA TTG GGT GTG CGC GCG ACG AGG AAG ACT      497
   Leu Pro Arg Arg Gly Pro Arg Leu Gly Val Arg Ala Thr Arg Lys Thr
               40                  45                  50
   TCC GAG CGG TCG CAA CCT CGA GGT AGA CGT CAG CCT ATC CCC AAG GCA      545
   Ser Glu Arg Ser Gln Pro Arg Gly Arg Arg Gln Pro Ile Pro Lys Ala
           55                  60                  65
   CGT CGG CCC GAG GGC AGG ACC TGG GCT CAG CCC GGG TAC CCT TGG CCC      593
   Arg Arg Pro Glu Gly Arg Thr Trp Ala Gln Pro Gly Tyr Pro Trp Pro
       70                  75                  80
   CTC TAT GGC AAT GAG GGT TGC GGG TGG GCG GGA TGG CTC CTG TCT CCC      641
   Leu Tyr Gly Asn Glu Gly Cys Gly Trp Ala Gly Trp Leu Leu Ser Pro
   85                  90                  95                  100
   CGT GGC TCT CGG CCT AGC TGG GGC CCC ACA GAC CCC CGG CGT AGG TCG      689
   Arg Gly Ser Arg Pro Ser Trp Gly Pro Thr Asp Pro Arg Arg Arg Ser
                   105                 110                 115
   CGC AAT TTG GGT AAG GTC ATC GAT ACC CTT ACG TGC GGC TTC GCC GAC      737
   Arg Asn Leu Gly Lys Val Ile Asp Thr Leu Thr Cys Gly Phe Ala Asp
               120                 125                 130
   CTC ATG GGG TAC ATA CCG CTC GTC GGC GCC CCT CTT GGA GGC GCT GCC      785
   Leu Met Gly Tyr Ile Pro Leu Val Gly Ala Pro Leu Gly Gly Ala Ala
           135                 140                 145
   AGG GCC CTG GCG CAT GGC GTC CGG GTT CTG GAA GAC GGC GTG AAC TAT        833
   Arg Ala Leu Ala His Gly Val Arg Val Leu Glu Asp Gly Val Asn Tyr
       150                 155                 160
   GCA ACA GGG AAC CTT CCT GGT TGC TCT TTC TCT ATC TTC CTT CTG GCC        881
   Ala Thr Gly Asn Leu Pro Gly Cys Ser Phe Ser Ile Phe Leu Leu Ala
   165                 170                 175                 180
   CTG CTC TCT TGC CTG ACT GTG CCC GCT TCA GCC TAC CAA GTG CGC AAT        929
   Leu Leu Ser Cys Leu Thr Val Pro Ala Ser Ala Tyr Gln Val Arg Asn
                   185                 190                 195
   TCC TCG GGG CTT TAC CAT GTC ACC AAT GAT TGC CCT AAC TCG AGT GTT        977
   Ser Ser Gly Leu Tyr His Val Thr Asn Asp Cys Pro Asn Ser Ser Val
               200                 205                 210
   GTG TAC GAG GCG GCC GAT GCC ATC CTG CAC ACT CCG GGG TGT GTC CCT       1025
   Val Tyr Glu Ala Ala Asp Ala Ile Leu His Thr Pro Gly Cys Val Pro
           215                 220                 225
   TGC GTT CGC GAG GGT AAC GCC TCG AGG TGT TGG GTG GCG GTG ACC CCC       1073
   Cys Val Arg Glu Gly Asn Ala Ser Arg Cys Trp Val Ala Val Thr Pro
       230                 235                 240
   ACG GTG GCC ACC AGG GAC GGC AAA CTC CCC ACA ACG CAG CTT CGA CGT       1121
   Thr Val Ala Thr Arg Asp Gly Lys Leu Pro Thr Thr Gln Leu Arg Arg
   245                 250                 255                 260
   CAT ATC GAT CTG CTT GTC GGG AGC GCC ACC CTC TGC TCG GCC CTC TAC       1169
   His Ile Asp Leu Leu Val Gly Ser Ala Thr Leu Cys Ser Ala Leu Tyr
                   265                 270                 275
   GTG GGG GAC CTG TGC GGG TCT GTC TTT CTT GTT GGT CAA CTG TTT ACC       1217
   Val Gly Asp Leu Cys Gly Ser Val Phe Leu Val Gly Gln Leu Phe Thr
               280                 285                 290
   TTC TCT CCC AGG CAC CAC TGG ACG ACG CAA GAC TGC AAT TGT TCT ATC       1265
   Phe Ser Pro Arg His His Trp Thr Thr Gln Asp Cys Asn Cys Ser Ile
           295                 300                 305
   TAT CCC GGC CAT ATA ACG GGT CAT CGC ATG GCA TGG AAT ATG ATG ATG       1313
   Tyr Pro Gly His Ile Thr Gly His Arg Met Ala Trp Asn Met Met Met
       310                 315                 320
   AAC TGG TCC CCT ACG GCA GCG TTG GTG GTA GCT CAG CTG CTC CGA ATC       1361
   Asn Trp Ser Pro Thr Ala Ala Leu Val Val Ala Gln Leu Leu Arg Ile
   325                 330                 335                 340
   CCA CAA GCC ATC ATG GAC ATG ATC GCT GGC GCC CAC TGG GGA GTC CTG       1409
   Pro Gln Ala Ile Met Asp Met Ile Ala Gly Ala His Trp Gly Val Leu
                   345                 350                 355
   GCG GGC ATA AAG TAT TTC TCC ATG GTG GGG AAC TGG GCG AAG GTC CTG      1457
   Ala Gly Ile Lys Tyr Phe Ser Met Val Gly Asn Trp Ala Lys Val Leu
               360                 365                 370
   GTA GTG CTG CTG CTA TTT GCC GGC GTC GAC GCG GAA ACC CAC GTC ACC      1505
   Val Val Leu Leu Leu Phe Ala Gly Val Asp Ala Glu Thr His Val Thr
           375                 380                 385
   GGG GGA AAT GCC GGC CGC ACC ACG GCT GGG CTT GTT GGT CTC CTT ACA      1553
   Gly Gly Asn Ala Gly Arg Thr Thr Ala Gly Leu Val Gly Leu Leu Thr
       390                 395                 400
   CCA GGC GCC AAG CAG AAC ATC CAA CTG ATC AAC ACC AAC GGC AGT TGG      1601
   Pro Gly Ala Lys Gln Asn Ile Gln Leu Ile Asn Thr Asn Gly Ser Trp
   405                 410                 415                 420
   CAC ATC AAT AGC ACG GCC TTG AAC TGC AAT GAA AGC CTT AAC ACC GGC      1649
   His Ile Asn Ser Thr Ala Leu Asn Cys Asn Glu Ser Leu Asn Thr Gly
                   425                 430                 435
   TGG TTA GCA GGG CTC TTC TAT CAG CAC AAA TTC AAC TCT TCA GGC TGT      1697
   Trp Leu Ala Gly Leu Phe Tyr Gln His Lys Phe Asn Ser Ser Gly Cys
               440                 445                 450
   CCT GAG AGG TTG GCC AGC TGC CGA CGC CTT ACC GAT TTT GCC CAG GGC      1745
   Pro Glu Arg Leu Ala Ser Cys Arg Arg Leu Thr Asp Phe Ala Gln Gly
           455                 460                 465
   TGG GGT CCT ATC AGT TAT GCC AAC GGA AGC GGC CTC GAC GAA CGC CCC      1793
   Trp Gly Pro Ile Ser Tyr Ala Asn Gly Ser Gly Leu Asp Glu Arg Pro
       470                 475                 480
   TAC TGC TGG CAC TAC CCT CCA AGA CCT TGT GGC ATT GTG CCC GCA AAG      1841
   Tyr Cys Trp His Tyr Pro Pro Arg Pro Cys Gly Ile Val Pro Ala Lys
   485                 490                 495                 500
   AGC GTG TGT GGC CCG GTA TAT TGC TTC ACT CCC AGC CCC GTG GTG GTG      1889
   Ser Val Cys Gly Pro Val Tyr Cys Phe Thr Pro Ser Pro Val Val Val
                   505                 510                 515
   GGA ACG ACC GAC AGG TCG GGC GCG CCT ACC TAC AGC TGG GGT GCA AAT      1937
   Gly Thr Thr Asp Arg Ser Gly Ala Pro Thr Tyr Ser Trp Gly Ala Asn
               520                 525                 530
   GAT ACG GAT GTC TTC GTC CTT AAC AAC ACC AGG CCA CCG CTG GGC AAT      1985
   Asp Thr Asp Val Phe Val Leu Asn Asn Thr Arg Pro Pro Leu Gly Asn
           535                 540                 545
   TGG TTC GGT TGT ACC TGG ATG AAC TCA ACT GGA TTC ACC AAA GTG TGC      2033
   Trp Phe Gly Cys Thr Trp Met Asn Ser Thr Gly Phe Thr Lys Val Cys
       550                 555                 560
   GGA GCG CCC CCT TGT GTC ATC GGA GGG GTG GGC AAC AAC ACC TTG CTC     2081
   Gly Ala Pro Pro Cys Val Ile Gly Gly Val Gly Asn Asn Thr Leu Leu
   565                 570                 575                 580
   TGC CCC ACT GAT TGC TTC CGC AAA TAT CCG GAA GCC ACA TAC TCT CGG     2129
   Cys Pro Thr Asp Cys Phe Arg Lys Tyr Pro Glu Ala Thr Tyr Ser Arg
                   585                 590                 595
   TGC GGC TCC GGT CCC AGG ATT ACA CCC AGG TGC ATG GTC GAC TAC CCG     2177
   Cys Gly Ser Gly Pro Arg Ile Thr Pro Arg Cys Met Val Asp Tyr Pro
               600                 605                 610
   TAT AGG CTT TGG CAC TAT CCT TGT ACC ATC AAT TAC ACC ATA TTC AAA     2225
   Tyr Arg Leu Trp His Tyr Pro Cys Thr Ile Asn Tyr Thr Ile Phe Lys
           615                 620                 625
   GTC AGG ATG TAC GTG GGA GGG GTC GAG CAC AGG CTG GAA GCG GCC TGC     2273
   Val Arg Met Tyr Val Gly Gly Val Glu His Arg Leu Glu Ala Ala Cys
       630                 635                 640
   AAC TGG ACG CGG GGC GAA CGC TGT GAT CTG GAA GAC AGG GAC AGG TCC     2321
   Asn Trp Thr Arg Gly Glu Arg Cys Asp Leu Glu Asp Arg Asp Arg Ser
   645                 650                 655                 660
   GAG CTC AGC CCG TTG CTG CTG TCC ACC ACA CAG TGG CAG GTC CTT CCG     2369
   Glu Leu Ser Pro Leu Leu Leu Ser Thr Thr Gln Trp Gln Val Leu Pro
                   665                 670                 675
   TGT TCT TTC ACG ACC CTG CCA GCC TTG TCC ACC GGC CTC ATC CAC CTC     2417
   Cys Ser Phe Thr Thr Leu Pro Ala Leu Ser Thr Gly Leu Ile His Leu
               680                 685                 690
   CAC CAG AAC ATT GTG GAC GTG CAG TAC TTG TAC GGG GTA GGG TCA AGC     2465
   His Gln Asn Ile Val Asp Val Gln Tyr Leu Tyr Gly Val Gly Ser Ser
           695                 700                 705
   ATC GCG TCC TGG GCC ATT AAG TGG GAG TAC GTC GTT CTC CTG TTC CTT     2513
   Ile Ala Ser Trp Ala Ile Lys Trp Glu Tyr Val Val Leu Leu Phe Leu
       710                 715                 720
   CTG CTT GCA GAC GCG CGC GTC TGT TCC TGC TTG TGG ATG ATG TTA CTC     2561
   Leu Leu Ala Asp Ala Arg Val Cys Ser Cys Leu Trp Met Met Leu Leu
   725                 730                 735                 740
   ATA TCC CAA GCG GAG GCG GCT TTG GAG AAC CTC GTA ATA CTC AAT GCA     2609
   Ile Ser Gln Ala Glu Ala Ala Leu Glu Asn Leu Val Ile Leu Asn Ala
                   745                 750                 755
   GCA TCC CTG GCC GGG ACG CAT GGT CTT GTG TCC TTC CTC GTG TTC TTC     2657
   Ala Ser Leu Ala Gly Thr His Gly Leu Val Ser Phe Leu Val Phe Phe
               760                 765                 770
   TGC TTT GCG TGG TAT CTG AAG GGT AGG TGG GTG CCC GGA GCG GTC TAC        2705
   Cys Phe Ala Trp Tyr Leu Lys Gly Arg Trp Val Pro Gly Ala Val Tyr
           775                 780                 785
   GCC CTC TAC GGG ATG TGG CCT CTC CTC CTG CTC CTG CTG GCG TTG CCT        2753
   Ala Leu Tyr Gly Met Trp Pro Leu Leu Leu Leu Leu Leu Ala Leu Pro
       790                 795                 800
   CAG CGG GCA TAC GCA CTG GAC ACG GAG GTG GCC GCG TCG TGT GGC GGC        2801
   Gln Arg Ala Tyr Ala Leu Asp Thr Glu Val Ala Ala Ser Cys Gly Gly
   805                 810                 815                 820
   GTT GTT CTT GTC GGG TTA ATG GCG CTG ACT CTG TCG CCA TAT TAC AAG        2849
   Val Val Leu Val Gly Leu Met Ala Leu Thr Leu Ser Pro Tyr Tyr Lys
                   825                 830                 835
   CGC TAT ATC AGC TGG TGC ATG TGG TGG CTT CAG TAT TTT CTG ACC AGA        2897
   Arg Tyr Ile Ser Trp Cys Met Trp Trp Leu Gln Tyr Phe Leu Thr Arg
               840                 845                 850
   GTA GAA GCG CAA CTG CAC GTG TGG GTT CCC CCC CTC AAC GTC CGG GGG        2945
   Val Glu Ala Gln Leu His Val Trp Val Pro Pro Leu Asn Val Arg Gly
           855                 860                 865
   GGG CGC GAT GCC GTC ATC TTA CTC ACG TGT GTA GTA CAC CCG GCC CTG        2993
   Gly Arg Asp Ala Val Ile Leu Leu Thr Cys Val Val His Pro Ala Leu
       870                 875                 880
   GTA TTT GAC ATC ACC AAA CTA CTC CTG GCC ATC TTC GGA CCC CTT TGG        3041
   Val Phe Asp Ile Thr Lys Leu Leu Leu Ala Ile Phe Gly Pro Leu Trp
   885                 890                 895                 900
   ATT CTT CAA GCC AGT TTG CTT AAA GTC CCC TAC TTC GTG CGC GTT CAA        3089
   Ile Leu Gln Ala Ser Leu Leu Lys Val Pro Tyr Phe Val Arg Val Gln
                   905                 910                 915
   GGC CTT CTC CGG ATC TGC GCG CTA GCG CGG AAG ATA GCC GGA GGT CAT        3137
   Gly Leu Leu Arg Ile Cys Ala Leu Ala Arg Lys Ile Ala Gly Gly His
               920                 925                 930
   TAC GTG CAA ATG GCC ATC ATC AAG TTA GGG GCG CTT ACT GGC ACC TGT        3185
   Tyr Val Gln Met Ala Ile Ile Lys Leu Gly Ala Leu Thr Gly Thr Cys
           935                 940                 945
   GTG TAT AAC CAT CTC GCT CCT CTT CGA GAC TGG GCG CAC AAC GGC CTG        3233
   Val Tyr Asn His Leu Ala Pro Leu Arg Asp Trp Ala His Asn Gly Leu
       950                 955                 960
   CGA GAT CTG GCC GTG GCT GTG GAA CCA GTC GTC TTC TCC CGA ATG GAG        3281
   Arg Asp Leu Ala Val Ala Val Glu Pro Val Val Phe Ser Arg Met Glu
   965                 970                 975                 980
   ACC AAG CTC ATC ACG TGG GGG GCA GAT ACC GCC GCG TGC GGT GAC ATC 3329
   Thr Lys Leu Ile Thr Trp Gly Ala Asp Thr Ala Ala Cys Gly Asp Ile
   985 990 995
   ATC AAC GGC TTG CCC GTC TCT GCC CGT AGG GGC CAG GAG ATA CTG CTT 3377
   Ile Asn Gly Leu Pro Val Ser Ala Arg Arg Gly Gln Glu Ile Leu Leu
   1000 1005 1010
   GGG CCA GCC GAC GGA ATG GTC TCC AAG GGG TGG AGG TTG CTG GCG CCC 3425
   Gly Pro Ala Asp Gly Met Val Ser Lys Gly Trp Arg Leu Leu Ala Pro
   1015 1020 1025
   ATC ACG GCG TAC GCC CAG CAG ACG AGA GGC CTC CTA GGG TGT ATA ATC 3473
   Ile Thr Ala Tyr Ala Gln Gln Thr Arg Gly Leu Leu Gly Cys Ile Ile
   1030 1035 1040
   ACC AGC CTG ACT GGC CGG GAC AAA AAC CAA GTG GAG GGT GAG GTC CAG 3521
   Thr Ser Leu Thr Gly Arg Asp Lys Asn Gln Val Glu Gly Glu Val Gln
   1045 1050 1055 1060
   ATC GTG TCA ACT GCT ACC CAG ACC TTC CTG GCA ACG TGC ATC AAT GGG 3569
   Ile Val Ser Thr Ala Thr Gln Thr Phe Leu Ala Thr Cys Ile Asn Gly
   1065 1070 1075
   GTA TGC TGG ACT GTC TAC CAC GGG GCC GGA ACG AGG ACC ATC GCA TCA 3617
   Val Cys Trp Thr Val Tyr His Gly Ala Gly Thr Arg Thr Ile Ala Ser
   1080 1085 1090
   CCC AAG GGT CCT GTC ATC CAG ACG TAT ACC AAT GTG GAT CAA GAC CTC 3665
   Pro Lys Gly Pro Val Ile Gln Thr Tyr Thr Asn Val Asp Gln Asp Leu
   1095 1100 1105
   GTG GGC TGG CCC GCT CCT CAA GGT TCC CGC TCA TTG ACA CCC TGC ACC 3713
   Val Gly Trp Pro Ala Pro Gln Gly Ser Arg Ser Leu Thr Pro Cys Thr
   1110 1115 1120
   TGC GGC TCC TCG GAC CTT TAC CTG GTC ACG AGG CAC GCC GAT GTC ATT 3761
   Cys Gly Ser Ser Asp Leu Tyr Leu Val Thr Arg His Ala Asp Val Ile
   1125 1130 1135 1140
   CCC GTG CGC CGG CGA GGT GAT AGC AGG GGT AGC CTG CTT TCG CCC CGG 3809
   Pro Val Arg Arg Arg Gly Asp Ser Arg Gly Ser Leu Leu Ser Pro Arg
   1145 1150 1155
   CCC ATT TCC TAC TTG AAA GGC TCC TCG GGG GGT CCG CTG TTG TGC CCC 3857
   Pro Ile Ser Tyr Leu Lys Gly Ser Ser Gly Gly Pro Leu Leu Cys Pro
   1160 1165 1170
   ACG GGA CAC GCC GTG GGC CTA TTC AGG GCC GCG GTG TGC ACC CGT GGA 3905
   Thr Gly His Ala Val Gly Leu Phe Arg Ala Ala Val Cys Thr Arg Gly
   1175 1180 1185
   GTG GCT AAG GCG GTG GAC TTT ATC CCT GTG GAG AAC CTA GAG ACA ACC      3953
   Val Ala Lys Ala Val Asp Phe Ile Pro Val Glu Asn Leu Glu Thr Thr
       1190                1195                1200
   ATG AGA TCC CCG GTG TTC ACG GAC AAC TCC TCT CCA CCA GCA GTG CCC      4001
   Met Arg Ser Pro Val Phe Thr Asp Asn Ser Ser Pro Pro Ala Val Pro
   1205                1210                1215                1220
   CAG AGC TTC CAG GTG GCC CAC CTG CAT GCT CCC ACC GGC AGC GGT AAG      4049
   Gln Ser Phe Gln Val Ala His Leu His Ala Pro Thr Gly Ser Gly Lys
                   1225                1230                1235
   AGC ACC AAG GTC CCG GCT GCG TAC GCA GCC AAG GGC TAC AAG GTG TTG      4097
   Ser Thr Lys Val Pro Ala Ala Tyr Ala Ala Lys Gly Tyr Lys Val Leu
               1240                1245                1250
   GTG CTC AAC CCC TCT GTT GCT GCA ACA CTG GGC TTT GGT GCT TAC ATG      4145
   Val Leu Asn Pro Ser Val Ala Ala Thr Leu Gly Phe Gly Ala Tyr Met
           1255                1260                1265
   TCC AAG GCC CAT GGG GTT GAT CCT AAT ATC AGG ACC GGG GTG AGA ACA      4193
   Ser Lys Ala His Gly Val Asp Pro Asn Ile Arg Thr Gly Val Arg Thr
       1270                1275                1280
   ATT ACC ACT GGC AGC CCC ATC ACG TAC TCC ACC TAC GGC AAG TTC CTT      4241
   Ile Thr Thr Gly Ser Pro Ile Thr Tyr Ser Thr Tyr Gly Lys Phe Leu
   1285                1290                1295                1300
   GCC GAC GCC GGG TGC TCA GGA GGT GCT TAT GAC ATA ATA ATT TGT GAC      4289
   Ala Asp Ala Gly Cys Ser Gly Gly Ala Tyr Asp Ile Ile Ile Cys Asp
                   1305                1310                1315
   GAG TGC CAC TCC ACG GAT GCC ACA TCC ATC TCG GGC ATC GGC ACT GTC      4337
   Glu Cys His Ser Thr Asp Ala Thr Ser Ile Ser Gly Ile Gly Thr Val
               1320                1325                1330
   CTT GAC CAA GCA GAG ACT GCG GGG GCG AGA CTG GTT GTG CTC GCC ACT      4385
   Leu Asp Gln Ala Glu Thr Ala Gly Ala Arg Leu Val Val Leu Ala Thr
           1335                1340                1345
   GCT ACC CCT CCG GGC TCC GTC ACT GTG TCC CAT CCT AAC ATC GAG GAG      4433
   Ala Thr Pro Pro Gly Ser Val Thr Val Ser His Pro Asn Ile Glu Glu
       1350                1355                1360
   GTT GCT CTG TCC ACC ACC GGA GAG ATC CCC TTT TAC GGC AAG GCT ATC      4481
   Val Ala Leu Ser Thr Thr Gly Glu Ile Pro Phe Tyr Gly Lys Ala Ile
   1365                1370                1375                1380
   CCC CTC GAG GTG ATC AAG GGG GGA AGA CAT CTC ATC TTC TGC CAC TCA      4529
   Pro Leu Glu Val Ile Lys Gly Gly Arg His Leu Ile Phe Cys His Ser
                   1385                1390                1395
   AAG AAG AAG TGC GAC GAG CTC GCC GCG AAG CTG GTC GCA TTG GGC ATC        4577
   Lys Lys Lys Cys Asp Glu Leu Ala Ala Lys Leu Val Ala Leu Gly Ile
               1400                1405                1410
   AAT GCC GTG GCC TAC TAC CGC GGT CTT GAC GTG TCT GTC ATC CCG ACC        4625
   Asn Ala Val Ala Tyr Tyr Arg Gly Leu Asp Val Ser Val Ile Pro Thr
           1415                1420                1425
   AGC GGC GAT GTT GTC GTC GTG TCG ACC GAT GCT CTC ATG ACT GGC TTT        4673
   Ser Gly Asp Val Val Val Val Ser Thr Asp Ala Leu Met Thr Gly Phe
       1430                1435                1440
   ACC GGC GAC TTC GAC TCT GTG ATA GAC TGC AAC ACG TGT GTC ACT CAG        4721
   Thr Gly Asp Phe Asp Ser Val Ile Asp Cys Asn Thr Cys Val Thr Gln
   1445                1450                1455                1460
   ACA GTC GAT TTT AGC CTT GAC CCT ACC TTT ACC ATT GAG ACA ACC ACG        4769
   Thr Val Asp Phe Ser Leu Asp Pro Thr Phe Thr Ile Glu Thr Thr Thr
                   1465                1470                1475
   CTC CCC CAG GAT GCT GTC TCC AGG ACT CAA CGC CGG GGC AGG ACT GGC        4817
   Leu Pro Gln Asp Ala Val Ser Arg Thr Gln Arg Arg Gly Arg Thr Gly
               1480                1485                1490
   AGG GGG AAG CCA GGC ATC TAT AGA TTT GTG GCA CCG GGG GAG CGC CCC        4865
   Arg Gly Lys Pro Gly Ile Tyr Arg Phe Val Ala Pro Gly Glu Arg Pro
           1495                1500                1505
   TCC GGC ATG TTC GAC TCG TCC GTC CTC TGT GAG TGC TAT GAC GCG GGC        4913
   Ser Gly Met Phe Asp Ser Ser Val Leu Cys Glu Cys Tyr Asp Ala Gly
       1510                1515                1520
   TGT GCT TGG TAT GAG CTC ACG CCC GCC GAG ACT ACA GTT AGG CTA CGA        4961
   Cys Ala Trp Tyr Glu Leu Thr Pro Ala Glu Thr Thr Val Arg Leu Arg
   1525                1530                1535                1540
   GCG TAC ATG AAC ACC CCG GGG CTT CCC GTG TGC CAG GAC CAT CTT GGA        5009
   Ala Tyr Met Asn Thr Pro Gly Leu Pro Val Cys Gln Asp His Leu Gly
                   1545                1550                1555
   TTT TGG GAG GGC GTC TTT ACG GGC CTC ACT CAT ATA GAT GCC CAC TTT        5057
   Phe Trp Glu Gly Val Phe Thr Gly Leu Thr His Ile Asp Ala His Phe
               1560                1565                1570
   CTA TCC CAG ACA AAG CAG AGT GGG GAG AAC TTT CCT TAC CTG GTA GCG        5105
   Leu Ser Gln Thr Lys Gln Ser Gly Glu Asn Phe Pro Tyr Leu Val Ala
           1575                1580                1585
   TAC CAA GCC ACC GTG TGC GCT AGG GCT CAA GCC CCT CCC CCA TCG TGG        5153
   Tyr Gln Ala Thr Val Cys Ala Arg Ala Gln Ala Pro Pro Pro Ser Trp
       1590                1595                1600
   GAC CAG ATG CGG AAG TGT TTG ATC CGC CTT AAA CCC ACC CTC CAT GGG      5201
   Asp Gln Met Arg Lys Cys Leu Ile Arg Leu Lys Pro Thr Leu His Gly
   1605                1610                1615                1620
   CCA ACA CCC CTG CTA TAC AGA CTG GGC GCT GTT CAG AAT GAA GTC ACC      5249
   Pro Thr Pro Leu Leu Tyr Arg Leu Gly Ala Val Gln Asn Glu Val Thr
                   1625                1630                1635
   CTG ACG CAC CCA ATC ACC AAA TAC ATC ATG ACA TGC ATG TCG GCC GAC      5297
   Leu Thr His Pro Ile Thr Lys Tyr Ile Met Thr Cys Met Ser Ala Asp
               1640                1645                1650
   CTG GAG GTC GTC ACG AGC ACC TGG GTG CTC GTT GGC GGC GTC CTG GCT      5345
   Leu Glu Val Val Thr Ser Thr Trp Val Leu Val Gly Gly Val Leu Ala
           1655                1660                1665
   GCT CTG GCC GCG TAT TGC CTG TCA ACA GGC TGC GTG GTC ATA GTG GGC      5393
   Ala Leu Ala Ala Tyr Cys Leu Ser Thr Gly Cys Val Val Ile Val Gly
       1670                1675                1680
   AGG ATC GTC TTG TCC GGG AAG CCG GCA ATT ATA CCT GAC AGG GAG GTT      5441
   Arg Ile Val Leu Ser Gly Lys Pro Ala Ile Ile Pro Asp Arg Glu Val
   1685                1690                1695                1700
   CTC TAC CAG GAG TTC GAT GAG ATG GAA GAG TGC TCT CAG CAC TTA CCG      5489
   Leu Tyr Gln Glu Phe Asp Glu Met Glu Glu Cys Ser Gln His Leu Pro
                   1705                1710                1715
   TAC ATC GAG CAA GGG ATG ATG CTC GCT GAG CAG TTC AAG CAG AAG GCC      5537
   Tyr Ile Glu Gln Gly Met Met Leu Ala Glu Gln Phe Lys Gln Lys Ala
               1720                1725                1730
   CTC GGC CTC CTG CAG ACC GCG TCC CGC CAT GCA GAG GTT ATC ACC CCT      5585
   Leu Gly Leu Leu Gln Thr Ala Ser Arg His Ala Glu Val Ile Thr Pro
           1735                1740                1745
   GCT GTC CAG ACC AAC TGG CAG AAA CTC GAG GTC TTT TGG GCG AAG CAC      5633
   Ala Val Gln Thr Asn Trp Gln Lys Leu Glu Val Phe Trp Ala Lys His
       1750                1755                1760
   ATG TGG AAT TTC ATC AGT GGG ATA CAA TAC TTG GCG GGC CTG TCA ACG      5681
   Met Trp Asn Phe Ile Ser Gly Ile Gln Tyr Leu Ala Gly Leu Ser Thr
   1765                1770                1775                1780
   CTG CCT GGT AAC CCC GCC ATT GCT TCA TTG ATG GCT TTT ACA GCT GCC      5729
   Leu Pro Gly Asn Pro Ala Ile Ala Ser Leu Met Ala Phe Thr Ala Ala
                   1785                1790                1795
   GTC ACC AGC CCA CTA ACC ACT GGC CAA ACC CTC CTC TTC AAC ATA TTG      5777
   Val Thr Ser Pro Leu Thr Thr Gly Gln Thr Leu Leu Phe Asn Ile Leu
               1800                1805                1810
   GGG GGG TGG GTG GCT GCC CAG CTC GCC GCC CCC GGT GCC GCT ACC GCC 5825
   Gly Gly Trp Val Ala Ala Gln Leu Ala Ala Pro Gly Ala Ala Thr Ala
   1815 1820 1825
   TTT GTG GGC GCT GGC TTA GCT GGC GCC GCA CTC GAC AGC GTT GGA CTG 5873
   Phe Val Gly Ala Gly Leu Ala Gly Ala Ala Leu Asp Ser Val Gly Leu
   1830 1835 1840
   GGG AAG GTC CTC GTG GAC ATT CTT GCA GGC TAT GGC GCG GGC GTG GCG 5921
   Gly Lys Val Leu Val Asp Ile Leu Ala Gly Tyr Gly Ala Gly Val Ala
   1845 1850 1855 1860
   GGA GCT CTT GTG GCA TTC AAG ATC ATG AGC GGT GAG GTC CCC TCC ACG 5969
   Gly Ala Leu Val Ala Phe Lys Ile Met Ser Gly Glu Val Pro Ser Thr
   1865 1870 1875
   GAG GAC CTG GTC AAT CTG CTG CCC GCC ATC CTC TCA CCT GGA GCC CTT 6017
   Glu Asp Leu Val Asn Leu Leu Pro Ala Ile Leu Ser Pro Gly Ala Leu
   1880 1885 1890
   GCA GTC GGT GTG GTC TTT GCA TCA ATA CTG CGC CGG CGT GTT GGC CCG 6065
   Ala Val Gly Val Val Phe Ala Ser Ile Leu Arg Arg Arg Val Gly Pro
   1895 1900 1905
   GGC GAG GGG GCA GTG CAA TGG ATG AAC CGG CTA ATA GCC TTC GCC TCC 6113
   Gly Glu Gly Ala Val Gln Trp Met Asn Arg Leu Ile Ala Phe Ala Ser
   1910 1915 1920
   CGG GGG AAC CAT GTT TCC CCC ACA CAC TAC GTG CCG GAG AGC GAT GCA 6161
   Arg Gly Asn His Val Ser Pro Thr His Tyr Val Pro Glu Ser Asp Ala
   1925 1930 1935 1940
   GCC GCC CGC GTC ACT GCC ATA CTC AGC AGC CTC ACT GTA ACC CAG CTC 6209
   Ala Ala Arg Val Thr Ala Ile Leu Ser Ser Leu Thr Val Thr Gln Leu
   1945 1950 1955
   CTG AGG CGA CTG CAT CAG TGG ATA AGC TCG GAG TGT ACC ACT CCA TGC 6257
   Leu Arg Arg Leu His Gln Trp Ile Ser Ser Glu Cys Thr Thr Pro Cys
   1960 1965 1970
   TCC GGT TCC TGG CTA AGG GAC ATC TGG GAC TGG ATA TGC GAG GTG CTG 6305
   Ser Gly Ser Trp Leu Arg Asp Ile Trp Asp Trp Ile Cys Glu Val Leu
   1975 1980 1985
   AGC GAC TTT AAG ACC TGG CTG AAA GCC AAG CTC ATG CCA CAA CTG CCT 6353
   Ser Asp Phe Lys Thr Trp Leu Lys Ala Lys Leu Met Pro Gln Leu Pro
   1990 1995 2000
   GGG ATT CCC TTT GTG TCC TGC CAG CGC GGG TAT AGG GGG GTC TGG CGA 6401
   Gly Ile Pro Phe Val Ser Cys Gln Arg Gly Tyr Arg Gly Val Trp Arg
   2005 2010 2015 2020
   GGA GAC GGC ATT ATG CAC ACT CGC TGC CAC TGT GGA GCT GAG ATC ACT       6449
   Gly Asp Gly Ile Met His Thr Arg Cys His Cys Gly Ala Glu Ile Thr
                   2025                2030                2035
   GGA CAT GTC AAA AAC GGG ACG ATG AGG ATC GTC GGT CCT AGG ACC TGC       6497
   Gly His Val Lys Asn Gly Thr Met Arg Ile Val Gly Pro Arg Thr Cys
               2040                2045                2050
   AAG AAC ATG TGG AGT GGG ACG TTC TTC ATT AAT GCC TAC ACC ACG GGC       6545
   Lys Asn Met Trp Ser Gly Thr Phe Phe Ile Asn Ala Tyr Thr Thr Gly
           2055                2060                2065
   CCC TGT ACT CCC CTT CCT GCG CCG AAC TAT AAG TTC GCG CTG TGG AGG       6593
   Pro Cys Thr Pro Leu Pro Ala Pro Asn Tyr Lys Phe Ala Leu Trp Arg
       2070                2075                2080
   GTG TCT GCA GAG GAA TAC GTG GAG ATA AGG CGG GTG GGG GAC TTC CAC       6641
   Val Ser Ala Glu Glu Tyr Val Glu Ile Arg Arg Val Gly Asp Phe His
   2085                2090                2095                2100
   TAC GTA TCG GGC ATG ACT ACT GAC AAT CTC AAA TGC CCG TGC CAG ATC       6689
   Tyr Val Ser Gly Met Thr Thr Asp Asn Leu Lys Cys Pro Cys Gln Ile
                   2105                2110                2115
   CCA TCG CCC GAA TTT TTC ACA GAA TTG GAC GGG GTG CGC CTA CAT AGG       6737
   Pro Ser Pro Glu Phe Phe Thr Glu Leu Asp Gly Val Arg Leu His Arg
               2120                2125                2130
   TTT GCG CCC CCT TGC AAG CCC TTG CTG CGG GAG GAG GTA TCT TTC AGA       6785
   Phe Ala Pro Pro Cys Lys Pro Leu Leu Arg Glu Glu Val Ser Phe Arg
           2135                2140                2145
   GTA GGA CTC CAC GAG TAC CCG GTG GGG TCG CAA TTA CCT TGC GAG CCC       6833
   Val Gly Leu His Glu Tyr Pro Val Gly Ser Gln Leu Pro Cys Glu Pro
       2150                2155                2160
   GAA CCG GAC GTA GCC GTG TTG ACG TCC ATG CTC ACT GAT CCC TCC CAT       6881
   Glu Pro Asp Val Ala Val Leu Thr Ser Met Leu Thr Asp Pro Ser His
   2165                2170                2175                2180
   ATA ACA GCA GAG GCG GCC GGG AGA AGG TTG GCG AGA GGG TCA CCC CCT       6929
   Ile Thr Ala Glu Ala Ala Gly Arg Arg Leu Ala Arg Gly Ser Pro Pro
                   2185                2190                2195
   TCT ATG GCC AGC TCC TCG GCT AGC CAG CTG TCC GCT CCA TCT CTC AAG       6977
   Ser Met Ala Ser Ser Ser Ala Ser Gln Leu Ser Ala Pro Ser Leu Lys
               2200                2205                2210
   GCA ACT TGC ACC GCC AAC CAT GAC TCC CCT GAC GCC GAG CTC ATA GAG       7025
   Ala Thr Cys Thr Ala Asn His Asp Ser Pro Asp Ala Glu Leu Ile Glu
           2215                2220                2225
   GCT AAC CTC CTG TGG AGG CAG GAG ATG GGC GGC AAC ATC ACC AGG GTT      7073
   Ala Asn Leu Leu Trp Arg Gln Glu Met Gly Gly Asn Ile Thr Arg Val
       2230                2235                2240
   GAG TCA GAG AAC AAA GTG GTG ATT CTG GAC TCC TTC GAT CCG CTT GTG      7121
   Glu Ser Glu Asn Lys Val Val Ile Leu Asp Ser Phe Asp Pro Leu Val
   2245                2250                2255                2260
   GCA GAG GAG GAT GAG CGG GAG GTC TCC GTA CCC GCA GAA ATT CTG CGG      7169
   Ala Glu Glu Asp Glu Arg Glu Val Ser Val Pro Ala Glu Ile Leu Arg
                   2265                2270                2275
   AAG TCT CGG AGA TTC GCC CCA GCC CTG CCC GTC TGG GCG CGG CCG GAC      7217
   Lys Ser Arg Arg Phe Ala Pro Ala Leu Pro Val Trp Ala Arg Pro Asp
               2280                2285                2290
   TAC AAC CCC CTG CTA GTA GAG ACG TGG AAA AAG CCT GAC TAC GAA CCA      7265
   Tyr Asn Pro Leu Leu Val Glu Thr Trp Lys Lys Pro Asp Tyr Glu Pro
           2295                2300                2305
   CCT GTG GTC CAT GGC TGC CCG CTA CCA CCT CCA CGG TCC CCT CCT GTG      7313
   Pro Val Val His Gly Cys Pro Leu Pro Pro Pro Arg Ser Pro Pro Val
       2310                2315                2320
   CCT CCG CCT CGG AAA AAG CGT ACG GTG GTC CTC ACC GAA TCA ACC CTA      7361
   Pro Pro Pro Arg Lys Lys Arg Thr Val Val Leu Thr Glu Ser Thr Leu
   2325                2330                2335                2340
   CCT ACT GCC TTG GCC GAG CTT GCC ACC AAA AGT TTT GGC AGC TCC TCA      7409
   Pro Thr Ala Leu Ala Glu Leu Ala Thr Lys Ser Phe Gly Ser Ser Ser
                   2345                2350                2355
   ACT TCC GGC ATT ACG GGC GAC AAT ACG ACA ACA TCC TCT GAG CCC GCC      7457
   Thr Ser Gly Ile Thr Gly Asp Asn Thr Thr Thr Ser Ser Glu Pro Ala
               2360                2365                2370
   CCT TCT GGC TGC CCC CCC GAC TCC GAC GTT GAG TCC TAT TCT TCC ATG      7505
   Pro Ser Gly Cys Pro Pro Asp Ser Asp Val Glu Ser Tyr Ser Ser Met
           2375                2380                2385
   CCC CCC CTG GAG GGG GAG CCT GGG GAT CCG GAT CTC AGC GAC GGG TCA      7553
   Pro Pro Leu Glu Gly Glu Pro Gly Asp Pro Asp Leu Ser Asp Gly Ser
       2390                2395                2400
   TGG TCG ACG GTC AGT AGT GGG GCC GAC ACG GAA GAT GTC GTG TGC TGC      7601
   Trp Ser Thr Val Ser Ser Gly Ala Asp Thr Glu Asp Val Val Cys Cys
   2405                2410                2415                2420
   TCA ATG TCT TAT TCC TGG ACA GGC GCA CTC GTC ACC CCG TGC GCT GCG      7649
   Ser Met Ser Tyr Ser Trp Thr Gly Ala Leu Val Thr Pro Cys Ala Ala
                   2425                2430                2435
   GAG GAA CAA AAA CTG CCC ATC AAC GCA CTG AGC AAC TCG TTG CTA CGC      7697
   Glu Glu Gln Lys Leu Pro Ile Asn Ala Leu Ser Asn Ser Leu Leu Arg
               2440                2445                2450
   CAT CAC AAT CTG GTG TAT TCC ACC ACT TCA CGC AGT GCT TGC CAA AGG      7745
   His His Asn Leu Val Tyr Ser Thr Thr Ser Arg Ser Ala Cys Gln Arg
           2455                2460                2465
   AAG AAG AAA GTC ACA TTT GAC AGA CTG CAA GTT CTG GAC AGC CAT TAC      7793
   Lys Lys Lys Val Thr Phe Asp Arg Leu Gln Val Leu Asp Ser His Tyr
       2470                2475                2480
   CAG GAC GTG CTC AAG GAG GTC AAA GCA GCG GCG TCA AAA GTG AAG GCT      7841
   Gln Asp Val Leu Lys Glu Val Lys Ala Ala Ala Ser Lys Val Lys Ala
   2485                2490                2495                2500
   AAC TTG CTA TCC GTA GAG GAA GCT TGC AGC CTG GCG CCC CCA CAT TCA      7889
   Asn Leu Leu Ser Val Glu Glu Ala Cys Ser Leu Ala Pro Pro His Ser
                   2505                2510                2515
   GCC AAA TCC AAG TTT GGC TAT GGG GCA AAA GAC GTC CGT TGC CAT GCC      7937
   Ala Lys Ser Lys Phe Gly Tyr Gly Ala Lys Asp Val Arg Cys His Ala
               2520                2525                2530
   AGA AAG GCC GTA GCC CAC ATC AAC TCC GTG TGG AAA GAC CTT CTG GAA      7985
   Arg Lys Ala Val Ala His Ile Asn Ser Val Trp Lys Asp Leu Leu Glu
           2535                2540                2545
   GAC AGT GTA ACA CCA ATA GAC ACT ACC ATC ATG GCC AAG AAC GAG GTT      8033
   Asp Ser Val Thr Pro Ile Asp Thr Thr Ile Met Ala Lys Asn Glu Val
       2550                2555                2560
   TTC TGC GTT CAG CCT GAG AAG GGG GGT CGT AAG CCA GCT CGT CTC ATC      8081
   Phe Cys Val Gln Pro Glu Lys Gly Gly Arg Lys Pro Ala Arg Leu Ile
   2565                2570                2575                2580
   GTG TTC CCC GAC CTG GGC GTG CGC GTG TGC GAG AAG ATG GCC CTG TAC      8129
   Val Phe Pro Asp Leu Gly Val Arg Val Cys Glu Lys Met Ala Leu Tyr
                   2585                2590                2595
   GAC GTG GTT AGC AAG CTC CCC TTG GCC GTG ATG GGA AGC TCC TAC GGA      8177
   Asp Val Val Ser Lys Leu Pro Leu Ala Val Met Gly Ser Ser Tyr Gly
               2600                2605                2610
   TTC CAA TAC TCA CCA GGA CAG CGG GTT GAA TTC CTC GTG CAA GCG TGG      8225
   Phe Gln Tyr Ser Pro Gly Gln Arg Val Glu Phe Leu Val Gln Ala Trp
           2615                2620                2625
   AAG TCC AAG AAG ACC CCG ATG GGG CTC TCG TAT GAT ACC CGC TGT TTT      8273
   Lys Ser Lys Lys Thr Pro Met Gly Leu Ser Tyr Asp Thr Arg Cys Phe
       2630                2635                2640
   GAC TCC ACA GTC ACT GAG AGC GAC ATC CGT ACG GAG GAG GCA ATT TAC        8321
   Asp Ser Thr Val Thr Glu Ser Asp Ile Arg Thr Glu Glu Ala Ile Tyr
   2645                2650                2655                2660
   CAA TGT TGT GAC CTG GAC CCC CAA GCC CGC GTG GCC ATC AAG TCC CTC        8369
   Gln Cys Cys Asp Leu Asp Pro Gln Ala Arg Val Ala Ile Lys Ser Leu
                   2665                2670                2675
   ACT GAG AGG CTT TAT GTT GGG GGC CCT CTT ACT AAT TCA AGG GGG GAA        8417
   Thr Glu Arg Leu Tyr Val Gly Gly Pro Leu Thr Asn Ser Arg Gly Glu
               2680                2685                2690
   AAC TGC GGC TAC CGC AGG TGC CGC GCG AGC AGA GTA CTG ACA ACT AGC        8465
   Asn Cys Gly Tyr Arg Arg Cys Arg Ala Ser Arg Val Leu Thr Thr Ser
           2695                2700                2705
   TGT GGT AAC ACC CTC ACT CGC TAC ATC AAG GCC CGG GCA GCC TGT CGA        8513
   Cys Gly Asn Thr Leu Thr Arg Tyr Ile Lys Ala Arg Ala Ala Cys Arg
       2710                2715                2720
   GCC GCA GGG CTC CAG GAC TGC ACC ATG CTC GTG TGT GGC GAC GAC TTA        8561
   Ala Ala Gly Leu Gln Asp Cys Thr Met Leu Val Cys Gly Asp Asp Leu
   2725                2730                2735                2740
   GTC GTT ATC TGT GAA AGT GCG GGG GTC CAG GAG GAC GCG GCG AGC CTG        8609
   Val Val Ile Cys Glu Ser Ala Gly Val Gln Glu Asp Ala Ala Ser Leu
                   2745                2750                2755
   AGA GCC TTC ACG GAG GCT ATG ACC AGG TAC TCC GCC CCC CCC GGG GAC        8657
   Arg Ala Phe Thr Glu Ala Met Thr Arg Tyr Ser Ala Pro Pro Gly Asp
               2760                2765                2770
   CCC CCA CAA CCA GAA TAC GAC TTG GAG CTT ATA ACA TCA TGC TCC TCC        8705
   Pro Pro Gln Pro Glu Tyr Asp Leu Glu Leu Ile Thr Ser Cys Ser Ser
           2775                2780                2785
   AAC GTG TCA GTC GCC CAC GAC GGC GCT GGA AAG AGG GTC TAC TAC CTT        8753
   Asn Val Ser Val Ala His Asp Gly Ala Gly Lys Arg Val Tyr Tyr Leu
       2790                2795                2800
   ACC CGT GAC CCT ACA ACC CCC CTC GCG AGA GCC GCG TGG GAG ACA GCA        8801
   Thr Arg Asp Pro Thr Thr Pro Leu Ala Arg Ala Ala Trp Glu Thr Ala
   2805                2810                2815                2820
   AGA CAC ACT CCA GTC AAT TCC TGG CTA GGC AAC ATA ATC ATG TTT GCC        8849
   Arg His Thr Pro Val Asn Ser Trp Leu Gly Asn Ile Ile Met Phe Ala
                   2825                2830                2835
   CCC ACA CTG TGG GCG AGG ATG ATA CTG ATG ACC CAC TTC TTT AGC GTC        8897
   Pro Thr Leu Trp Ala Arg Met Ile Leu Met Thr His Phe Phe Ser Val
               2840                2845                2850
   CTC ATA GCC AGG GAT CAG CTT GAA CAG GCT CTC AAC TGC GAG ATC TAC          8945
   Leu Ile Ala Arg Asp Gln Leu Glu Gln Ala Leu Asn Cys Glu Ile Tyr
           2855                2860                2865
   GGA GCC TGC TAC TCC ATA GAA CCA CTG GAT CTA CCT CCA ATC ATT CAA          8993
   Gly Ala Cys Tyr Ser Ile Glu Pro Leu Asp Leu Pro Pro Ile Ile Gln
       2870                2875                2880
   AGA CTC CAT GGC CTC AGC GCA TTT TCA CTC CAC AGT TAC TCT CCA GGT          9041
   Arg Leu His Gly Leu Ser Ala Phe Ser Leu His Ser Tyr Ser Pro Gly
   2885                2890                2895                2900
   GAA ATT AAT AGG GTG GCC GCA TGC CTC AGA AAA CTT GGG GTC CCG CCC          9089
   Glu Ile Asn Arg Val Ala Ala Cys Leu Arg Lys Leu Gly Val Pro Pro
                   2905                2910                2915
   TTG CGA GCT TGG AGA CAC CGG GCC TGG AGC GTC CGC GCT AGG CTT CTG          9137
   Leu Arg Ala Trp Arg His Arg Ala Trp Ser Val Arg Ala Arg Leu Leu
               2920                2925                2930
   GCC AGA GGA GGC AAG GCT GCC ATA TGT GGC AAG TAC CTC TTC AAC TGG          9185
   Ala Arg Gly Gly Lys Ala Ala Ile Cys Gly Lys Tyr Leu Phe Asn Trp
           2935                2940                2945
   GCA GTA AGA ACA AAG CTC AAA CTC ACT CCG ATA ACG GCC GCT GGC CGG          9233
   Ala Val Arg Thr Lys Leu Lys Leu Thr Pro Ile Thr Ala Ala Gly Arg
       2950                2955                2960
   CTG GAC TTG TCC GGC TGG TTC ACG GCT GGC TAC AGC GGG GGA GAC ATT          9281
   Leu Asp Leu Ser Gly Trp Phe Thr Ala Gly Tyr Ser Gly Gly Asp Ile
   2965                2970                2975                2980
   TAT CAC AGC GTG TCT CAT GCC CGG CCC CGC TGG TTC TGG TTT TGC CTA          9329
   Tyr His Ser Val Ser His Ala Arg Pro Arg Trp Phe Trp Phe Cys Leu
                   2985                2990                2995
   CTC CTG CTT GCT GCA GGG GTA GGC ATC TAC CTC CTC CCC AAC CGA              9374
   Leu Leu Leu Ala Ala Gly Val Gly Ile Tyr Leu Leu Pro Asn Arg
               3000                3005                3010
   TGAAGATTGG GCTAACCACT CCAGGCCAAT AGGCCATTCC CT                           9416
   ***
(2) INFORMATION FOR SEQ ID NO:47:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:47:
      CAGCCCCCTG ATGGGGGCGA C 21
(2) INFORMATION FOR SEQ ID NO:48:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:48:
      ACTCGCAAGC ACCCTATCA 19
(2) INFORMATION FOR SEQ ID NO:49:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:495:
      CTGTGAGGAA CTACTGTCT 19
(2) INFORMATION FOR SEQ ID NO:50:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:50:
      ATGAGCACGA ATCCTCAAAC CT 22
(2) INFORMATION FOR SEQ ID NO:51:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:51:
      GTCCTGCCCT CGGGCC 16
(2) INFORMATION FOR SEQ ID NO:52:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:52:
      CGAGGAAGAC TTCCGAGC 18
(2) INFORMATION FOR SEQ ID NO:53:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:53:
      ACCCAAATTG CGCGACCTAC 20
(2) INFORMATION FOR SEQ ID NO:54:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:54:
      TAAGGTCATC GATACCCT 18
(2) INFORMATION FOR SEQ ID NO:55:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:55:
      CAGTTCATCA TCATATCCCA 20
(2) INFORMATION FOR SEQ ID NO:56:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:56:
      GATAGAGAA AGAGCAAC 18
(2) INFORMATION FOR SEQ ID NO:57:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:57:
      AGACTTCCGA GCGGTCGCAA 20
(2) INFORMATION FOR SEQ ID NO:58:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:58:
      GACCTGTGCG GGTCTGTC 18
(2) INFORMATION FOR SEQ ID NO:59:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:59:
      GGGTCGGCAG CTGGCTAGCC TCTCA 25
(2) INFORMATION FOR SEQ ID NO:60:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:60:
      TCCTGGCGGG CATAGCGT 18
(2) INFORMATION FOR SEQ ID NO:61:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:61:
      CCCCAGCCCT GGTCAAAATC GGTAA 25
(2) INFORMATION FOR SEQ ID NO:62:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:62:

   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:63:
      CTCGGTCG TTCCCACCA 19
(2) INFORMATION FOR SEQ ID NO:64:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:64:
      CCGCGAAGAG TGTGTGTGGT 20
(2) INFORMATION FOR SEQ ID NO:65:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:65:
      CAATGTTCTG GTGGAGGTG 19
(2) INFORMATION FOR SEQ ID NO:66:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:66:
      GCCATTAAGT GGGAGTACGT CGTTCTCC 28
(2) INFORMATION FOR SEQ ID NO:67:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:67:
      CGAGGAAGGA TACAAGACC 19
(2) INFORMATION FOR SEQ ID NO:68:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:68:
      TGCTTGTGGA TGATGCTACT 20
(2) INFORMATION FOR SEQ ID NO:69:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:69:
      CACACGTGCA GTTGCGCT 18
(2) INFORMATION FOR SEQ ID NO:70:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:70:
      CTGCTGACCA CTACACAG 18
(2) INFORMATION FOR SEQ ID NO:71:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:71:
      GACCAGAGTG GAAGCGCAA 19
(2) INFORMATION FOR SEQ ID NO:72:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:72:
      TACCAGAGTC GGGTGTACAG 20
(2) INFORMATION FOR SEQ ID NO:73:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:73:
      CTAGGAGGCC CCTTGTCTGC 20
(2) INFORMATION FOR SEQ ID NO:74:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:74:
      CTCGGGCCAG CCGATGGA 18
(2) INFORMATION FOR SEQ ID NO:75:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:75:
      GGGGACCTCA TGGTTGTCT 19
(2) INFORMATION FOR SEQ ID NO:76:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:76:
      CCCGTGGAGT GGCTAAGG 18
(2) INFORMATION FOR SEQ ID NO:77:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:77:
      CTCCTCGATG TTGGGATGG 19
(2) INFORMATION FOR SEQ ID NO:78:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:78:
      CAGAGCTTCC AGGTGGCTC 19
(2) INFORMATION FOR SEQ ID NO:79:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:79:
      CGGGCTCCGT CACTGTG 17
(2) INFORMATION FOR SEQ ID NO:80:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:80:
      GTATTGCAGT CTATCACCGA G 21
(2) INFORMATION FOR SEQ ID NO:81:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:81:
      GGCTATACCG GCGACTTCGA 20
(2) INFORMATION FOR SEQ ID NO:82:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:82:
      CGTTGAGTGC GGGAGACAG 19
(2) INFORMATION FOR SEQ ID NO:83:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:83:
      TCACCATTGA GACAATCACG 20
(2) INFORMATION FOR SEQ ID NO:84:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:84:
      GTAAGGAAGG TTCTCCCCAC TC 22
(2) INFORMATION FOR SEQ ID NO:85:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:85:
      ATGCCCACTT TCTATCCCAG ACAAAGC 27
(2) INFORMATION FOR SEQ ID NO:86:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:86:
      TGCATGTCAT GATGTAT 17
(2) INFORMATION FOR SEQ ID NO:87:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:87:
      GGACAAGACG ACCCTGCC 18
(2) INFORMATION FOR SEQ ID NO:88:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:88:
      CGTATTGCCT GTCAACAGGC 20
(2) INFORMATION FOR SEQ ID NO:89:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:89:
      AGCGCCCACA AAGGCAGTAG 20
(2) INFORMATION FOR SEQ ID NO:90:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:90:
(2) INFORMATION FOR SEQ ID NO:91:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:91:
      CCAGGAACCG GAGCATGG 18
(2) INFORMATION FOR SEQ ID NO:92:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:92:
      ACCAGTGGAT AAGCTCGG 18
(2) INFORMATION FOR SEQ ID NO:93:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:93:
      CGTGGTGTAG GCATTAATG 19
(2) INFORMATION FOR SEQ ID NO:94:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:94:
      ATGTGGAGTG GGACCTTCC 19
(2) INFORMATION FOR SEQ ID NO:95:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:95:
      CTCTGCTGTT ATATGGGAGG 20
(2) INFORMATION FOR SEQ ID NO:96:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:96:
      GTTGACGTCC ATGCTCACTG 20
(2) INFORMATION FOR SEQ ID NO:97:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:97:
      TTTCCACGTC TCCACTAGCG 20
(2) INFORMATION FOR SEQ ID NO:98:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:98:
      GTGAGGACCA CCGTCCGC 18
(2) INFORMATION FOR SEQ ID NO:99:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:99:
      TTCCACCTCC AAAGTCCCCT 20
(2) INFORMATION FOR SEQ ID NO:100:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:100:
      AGAACTTGCA GTCTGTCAAA TGTGA 25
(2) INFORMATION FOR SEQ ID NO:101:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:101:
      GGAAGAACAG AAACTGCCCA TCAATGCACT AAGC 34
(2) INFORMATION FOR SEQ ID NO:102:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:102:
      TGACGCCGCT GCTTTAACCT 20
(2) INFORMATION FOR SEQ ID NO:103:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:103:
      TGCAAGCTTC CTCTACGGAT 20
(2) INFORMATION FOR SEQ ID NO:104:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:104:
      AGGTTAAAGC AGCGGCGTCA 20
(2) INFORMATION FOR SEQ ID NO:105:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:105:
      AGCTTCCCAT CACGGCCAA 19
(2) INFORMATION FOR SEQ ID NO:106:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:106:
      GATGGCTTTG TACGACGTG 19
(2) INFORMATION FOR SEQ ID NO:107:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:107:
      GCACCTGCGA TAGCCGCAGT 20
(2) INFORMATION FOR SEQ ID NO:108:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:108:
      GTCCCTCACC GAGAGGCT 18
(2) INFORMATION FOR SEQ ID NO:109:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:109:
      GATTGGAGGT AGATCAAGTG 20
(2) INFORMATION FOR SEQ ID NO:110:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:110:
      TACGACTTGG AGCTCATAAC 20
(2) INFORMATION FOR SEQ ID NO:111:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:111:
      AGCAAGACAC ACTCCAGTCA 20
(2) INFORMATION FOR SEQ ID NO:112:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (ix) FEATURE:
      (A) NAME/KEY: CDS
      (B) LOCATION: 1..21
      (D) OTHER INFORMATION: /product= "Carboxy-terminal Linker Protein in GST-NANBV 15-18"
   (xi) SEQUENCE DESCRIPTION: SEQ ID NO:30:
(2) INFORMATION FOR SEQ ID NO:31:
   (i) SEQUENCE CHARACTERISTICS:
      (A) LENGTH: 7 amino acids
      (B) TYPE: amino acid
      (D) TOPOLOGY: linear
   (ii) MOLECULE TYPE: protein
   (xi) SEQUENCE DESCRIPTION: SEQ ID NO:31:
   Trp Gly Ile Gly Asn Ser Ser
    1               5
(2) INFORMATION FOR SEQ ID NO:32:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..24
      (D) OTHER INFORMATION: /product= "Linker Protein in GST-NANBV 15-17"
   (xi) SEQUENCE DESCRIPTION: SEQ ID NO:32:
   GGG ATC CCC AAT TCC TGC AGC CCT                                  24
   Gly Ile Pro Asn Ser Cys Ser Pro
    1               5
   (iv) ANTI-SENSE: YES
   (xi) SEQUENCE DESCRIPTION: SEQ ID NO:112:
      GCCTATTGGC CTGGAGTGGT TAGC 24
(2) INFORMATION FOR SEQ ID NO:113:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide
   (xi) SEQUENCE DESCRIPTION: SEQ ID NO:113:
   His Val Thr Gly Gly Asn Ala Gly Arg Thr Thr Ala Gly Leu Val Gly
   1               5                   10                  15
   Leu Leu Thr Pro Gly Ala Lys Gln Asn Ile
               20                  25
(2) INFORMATION FOR SEQ ID NO:114:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide
   (xi) SEQUENCE DESCRIPTION: SEQ ID NO:114:
   His Val Thr Gly Gly Ser Ala Gly His Thr Val Ser Gly Phe Val Ser
   1               5                   10                  15
   Leu Leu Ala Pro Gly Ala Lys Gln Asn Val
               20                  25
(2) INFORMATION FOR SEQ ID NO:115:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide
   (xi) SEQUENCE DESCRIPTION: SEQ ID NO:115:
   His Val Thr Gly Gly Gln Ala Ala Arg Ala Met Ser Gly Leu Val Ser
   1 5 10 15
   Leu Phe Thr Pro Gly Ala Lys Gln Asn Ile
   20 25
(2) INFORMATION FOR SEQ ID NO:116:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide
   (xi) SEQUENCE DESCRIPTION: SEQ ID NO:116:
   His Val Thr Gly Gly Arg Val Ala Ser Ser Thr Gln Ser Leu Val Ser
   1 5 10 15
   Trp Leu Ser Gln Gly Pro Ser Gln Lys Ile
   20 25
(2) INFORMATION FOR SEQ ID NO:117:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide
   (xi) SEQUENCE DESCRIPTION: SEQ ID NO:117:
   His Val Thr Gly Gly Ala Gln Ala Lys Thr Thr Asn Arg Leu Val Ser
   1 5 10 15
   Met Phe Ala Ser Gly Pro Ser Gln Lys Ile
   20 25
(2) INFORMATION FOR SEQ ID NO:118:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide (xi) SEQUENCE DESCRIPTION: SEQ ID NO:118:
(2) INFORMATION FOR SEQ ID NO:119:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide
   (xi) SEQUENCE DESCRIPTION: SEQ ID NO:119:
   His Val Thr Gly Gly Val Gln Gly His Val Thr Ser Thr Leu Thr Ser
   1 5 10 15
   Leu Phe Arg Pro Gly Ala Ser Gln Lys Ile
   20 25
(2) INFORMATION FOR SEQ ID NO:120:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide
   (xi) SEQUENCE DESCRIPTION: SEQ ID NO:120:
   His Val Thr Gly Gly Ser Ala Gly Arg Thr Thr Ala Gly Leu Val Gly
   1 5 10 15
   Leu Leu Thr Pro Gly Ala Lys Gln Asn Ile
   20 25
(2) INFORMATION FOR SEQ ID NO:121:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide (xi) SEQUENCE DESCRIPTION: SEQ ID NO:121:
(2) INFORMATION FOR SEQ ID NO:122:
   (i) SEQUENCE CHARACTERISTICS:
      (A) LENGTH: 30 amino acids
      (B) TYPE: amino acid
      (D) TOPOLOGY: linear
   (ii) MOLECULE TYPE: peptide
   (xi) SEQUENCE DESCRIPTION: SEQ ID NO:122:
   Val Ala Thr Arg Asp Gly Lys Leu Pro Thr Thr Gln Leu Arg Arg His
   1 5 10 15
   Ile Asp Leu Leu Val Gly Ser Ala Thr Leu Cys Ser Ala Leu
   20 25 30
(2) INFORMATION FOR SEQ ID NO:123:
   (i) SEQUENCE CHARACTERISTICS:
      (A) LENGTH: 30 amino acids
      (B) TYPE: amino acid
      (D) TOPOLOGY: linear
   (ii) MOLECULE TYPE: peptide
   (xi) SEQUENCE DESCRIPTION: SEQ ID NO:123:
   Val Ala Thr Arg Asp Gly Lys Leu Pro Ala Thr Gln Leu Arg Arg His
   1 5 10 15
   Ile Asp Leu Leu Val Gly Ser Ala Thr Leu Cys Ser Ala Leu
   20 25 30
(2) INFORMATION FOR SEQ ID NO:124:
   (i) SEQUENCE CHARACTERISTICS:
      (A) LENGTH: 30 amino acids
      (B) TYPE: amino acid
      (D) TOPOLOGY: linear
   (ii) MOLECULE TYPE: peptide
   (xi) SEQUENCE DESCRIPTION: SEQ ID NO:124:
   Leu Ala Ala Arg Asn Ser Ser Ile Pro Thr Thr Thr Ile Arg Arg His
   1 5 10 15
   Val Asp Leu Leu Val Gly Ala Ala Ala Leu Cys Ser Ala Met
   20 25 30
(2) INFORMATION FOR SEQ ID NO:125:
   (i) SEQUENCE CHARACTERISTICS:
      (A) LENGTH: 30 amino acids
      (B) TYPE: amino acid
      (D) TOPOLOGY: linear
   (ii) MOLECULE TYPE: peptide
   (xi) SEQUENCE DESCRIPTION: SEQ ID NO:125:
   Leu Ala Ala Arg Asn Val Thr Ile Pro Thr Thr Thr Ile Arg Arg His
   1 5 10 15
   Val Asp Leu Leu Val Gly Ala Ala Ala Phe Cys Ser Ala Met
   20 25 30
(2) INFORMATION FOR SEQ ID NO:126:
   (i) SEQUENCE CHARACTERISTICS:
      (A) LENGTH: 30 amino acids
      (B) TYPE: amino acid
      (D) TOPOLOGY: linear
   (ii) MOLECULE TYPE: peptide
   (xi) SEQUENCE DESCRIPTION: SEQ ID NO:126:
   Leu Ala Ala Arg Asn Ala Ser Val Pro Thr Thr Thr Ile Arg Arg His
   1 5 10 15
   Val Asp Leu Leu Val Gly Ala Ala Ala Phe Cys Ser Ala Met
   20 25 30
(2) INFORMATION FOR SEQ ID NO:127:
   (i) SEQUENCE CHARACTERISTICS:
      (A) LENGTH: 30 amino acids
      (B) TYPE: amino acid
      (D) TOPOLOGY: linear
   (ii) MOLECULE TYPE: peptide
   (xi) SEQUENCE DESCRIPTION: SEQ ID NO:127:
   Leu Ala Ala Arg Asn Ala Ser Val Pro Thr Thr Thr Leu Arg Arg His
   1 5 10 15
   Val Asp Leu Leu Val Gly Thr Ala Ala Phe Cys Ser Ala Met
   20 25 30
(2) INFORMATION FOR SEQ ID NO:128:
   (i) SEQUENCE CHARACTERISTICS:
      (A) LENGTH: 27 amino acids
      (B) TYPE: amino acid
      (D) TOPOLOGY: linear
   (ii) MOLECULE TYPE: peptide
   (xi) SEQUENCE DESCRIPTION: SEQ ID NO:128:
   Leu Ala Ser Cys Arg Arg Leu Thr Asp Phe Ala Gln Gly Trp Gly Pro
   1 5 10 15
   Ile Ser Tyr Ala Asn Gly Ser Gly Leu Asp Glu
   20 25
(2) INFORMATION FOR SEQ ID NO:129:
   (i) SEQUENCE CHARACTERISTICS:
      (A) LENGTH: 27 amino acids
      (B) TYPE: amino acid
      (D) TOPOLOGY: linear
   (ii) MOLECULE TYPE: peptide
   (xi) SEQUENCE DESCRIPTION: SEQ ID NO:129:
   Leu Ala Ser Cys Arg Pro Leu Thr Asp Phe Asp Gln Gly Trp Gly Pro
   1 5 10 15
   Ile Ser Tyr Ala Asn Gly Ser Gly Pro Asp Gln
   20 25
(2) INFORMATION FOR SEQ ID NO:130:
   (i) SEQUENCE CHARACTERISTICS:
      (A) LENGTH: 27 amino acids
      (B) TYPE: amino acid
      (D) TOPOLOGY: linear
   (ii) MOLECULE TYPE: peptide (xi) SEQUENCE DESCRIPTION: SEQ ID NO:130:
(2) INFORMATION FOR SEQ ID NO:131:
   (i) SEQUENCE CHARACTERISTICS:
      (A) LENGTH: 27 amino acids
      (B) TYPE: amino acid
      (D) TOPOLOGY: linear
   (ii) MOLECULE TYPE: peptide
   (xi) SEQUENCE DESCRIPTION: SEQ ID NO:131:
   Met Ala Ser Cys Arg Pro Ile Asp Glu Phe Ala Gln Gly Trp Gly Pro
   1 5 10 15
   Ile Thr His Asp Met Pro Glu Ser Ser Asp Gln
   20 25
(2) INFORMATION FOR SEQ ID NO:132:
   (i) SEQUENCE CHARACTERISTICS:
      (A) LENGTH: 27 amino acids
      (B) TYPE: amino acid
      (D) TOPOLOGY: linear
   (ii) MOLECULE TYPE: peptide
   (xi) SEQUENCE DESCRIPTION: SEQ ID NO:132:
   Met Ala Gln Cys Arg Thr Ile Asp Lys Phe Asp Gln Gly Trp Gly Pro
   1 5 10 15
   Ile Thr Tyr Ala Glu Ser Ser Arg Ser Asp Gln
   20 25
(2) INFORMATION FOR SEQ ID NO:122:
   (i) SEQUENCE CHARACTERISTICS:
      (A) LENGTH: 27 amino acids
      (B) TYPE: amino acid
      (D) TOPOLOGY: linear
   (ii) MOLECULE TYPE: peptide (xi) SEQUENCE DESCRIPTION: SEQ ID NO:133:
(2) INFORMATION FOR SEQ ID NO:134:
   (i) SEQUENCE CHARACTERISTICS:
      (A) LENGTH: 27 amino acids
      (B) TYPE: amino acid
      (D) TOPOLOGY: linear
   (ii) MOLECULE TYPE: peptide
   (xi) SEQUENCE DESCRIPTION: SEQ ID NO:134:
   Leu Ala Ser Cys Arg Arg Leu Thr Asp Phe Asp Gln Gly Trp Gly Pro
   1 5 10 15
   Ile Ser Tyr Ala Asn Gly Ser Gly Pro Asp Glu
   20 25
(2) INFORMATION FOR SEQ ID NO:135:
   (i) SEQUENCE CHARACTERISTICS:
      (A) LENGTH: 24 amino acids
      (B) TYPE: amino acid
      (D) TOPOLOGY: linear
   (ii) MOLECULE TYPE: peptide
   (xi) SEQUENCE DESCRIPTION: SEQ ID NO:135:
   Ser Thr Ser Gly Ile Thr Gly Asp Asn Thr Thr Thr Ser Ser Glu Pro
   1 5 10 15
   Ala Pro Ser Gly Cys Pro Pro Asp
   20
(2) INFORMATION FOR SEQ ID NO:136:
   (i) SEQUENCE CHARACTERISTICS:
      (A) LENGTH: 24 amino acids
      (B) TYPE: amino acid
      (D) TOPOLOGY: linear
   (ii) MOLECULE TYPE: peptide
   (xi) SEQUENCE DESCRIPTION: SEQ ID NO:136:
   Gly Ser Ser Ala Val Asp Ser Gly Thr Ala Thr Gly Pro Pro Asp Gln
   1 5 10 15
   Ala Ser Asp Asp Gly Asp Lys Gly
   20
(2) INFORMATION FOR SEQ ID NO:137:
   (i) SEQUENCE CHARACTERISTICS:
      (A) LENGTH: 24 amino acids
      (B) TYPE: amino acid
      (D) TOPOLOGY: linear
   (ii) MOLECULE TYPE: peptide
   (xi) SEQUENCE DESCRIPTION: SEQ ID NO:137:
   Glu Ser Ser Ala Val Asp Ser Gly Thr Ala Thr Ala Leu Pro Asp Gln
   1 5 10 15
   Ala Ser Asp Asp Gly Asp Lys Gly
   20
(2) INFORMATION FOR SEQ ID NO:1:
   (i) SEQUENCE CHARACTERISTICS:
      (A) LENGTH: 978 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..978
      (D) OTHER INFORMATION: /codon_start= 1 /product= "NANBV Structural Antigen" /numbe= 1
   (xi) SEQUENCE DESCRIPTION: SEQ ID NO:1:
   ATG AGC ACG ATT CCC AAA CCT CAA AGA AAA ACC AAA CGT AAC ACC AAC      48
   Met Ser Thr Ile Pro Lys Pro Gln Arg Lys Thr Lys Arg Asn Thr Asn
    1               5                  10                  15
   CGT CGC CCA CAG GAC GTC AAG TTC CCG GGT GGC GGT CAG ATC GTT GGT      96
   Arg Arg Pro Gln Asp Val Lys Phe Pro Gly Gly Gly Gln Ile Val Gly
               20                  25                  30
   GGA GTT TAC TTG TTG CCG CGC AGG GGC CCT AGA TTG GGT GTG CGC GCG     144
   Gly Val Tyr Leu Leu Pro Arg Arg Gly Pro Arg Leu Gly Val Arg Ala
           35                  40                  45
   ACG AGG AAG ACT TCC GAG CGG TCG CAA CCT CGA GGT AGA CGT CAG CCT     192
   Thr Arg Lys Thr Ser Glu Arg Ser Gln Pro Arg Gly Arg Arg Gln Pro
       50                  55                  60
   ATC CCC AAG GCA CGT CGG CCC GAG GGC AGG ACC TGG GCT CAG CCC GGG     240
   Ile Pro Lys Ala Arg Arg Pro Glu Gly Arg Thr Trp Ala Gln Pro Gly
   65                  70                  75                  80
   TAC CCT TGG CCC CTC TAT GGC AAT GAG GGT TGC GGG TGG GCG GGA TGG     288
   Tyr Pro Trp Pro Leu Tyr Gly Asn Glu Gly Cys Gly Trp Ala Gly Trp
                   85                  90                  95
   CTC CTG TCT CCC CGT GGC TCT CGG CCT AGC TGG GGC CCC ACA GAC CCC     336
   Leu Leu Ser Pro Arg Gly Ser Arg Pro Ser Trp Gly Pro Thr Asp Pro
               100                 105                 110
   CGG CGT AGG TCG CGC AAT TTG GGT AAG GTC ATC GAT ACC CTT ACG TGC     384
   Arg Arg Arg Ser Arg Asn Leu Gly Lys Val Ile Asp Thr Leu Thr Cys
           115                 120                 125
   GGC TTC GCC GAC CTC ATG GGG TAC ATA CCG CTC GTC GGC GCC CCT CTT     432
   Gly Phe Ala Asp Leu Met Gly Tyr Ile Pro Leu Val Gly Ala Pro Leu
       130                 135                 140
   GGA GGC GCT GCC AGG GCC CTG GCG CAT GGC GTC CGG GTT CTG GAA GAC     480
   Gly Gly Ala Ala Arg Ala Leu Ala His Gly Val Arg Val Leu Glu Asp
   145                 150                 155                 160
   GGC GTG AAC TAT GCA ACA GGG AAC CTT CCT GGT TGC TCT TTC TCT ATC     528
   Gly Val Asn Tyr Ala Thr Gly Asn Leu Pro Gly Cys Ser Phe Ser Ile
                   165                 170                 175
   TTC CTT CTG GCC CTG CTC TCT TGC CTG ACT GTG CCC GCT TCA GCC TAC     576
   Phe Leu Leu Ala Leu Leu Ser Cys Leu Thr Val Pro Ala Ser Ala Tyr
               180                 185                 190
   CAA GTG CGC AAT TCC TCG GGG CTT TAC CAT GTC ACC AAT GAT TGC CCT     624
   Gln Val Arg Asn Ser Ser Gly Leu Tyr His Val Thr Asn Asp Cys Pro
           195                 200                 205
   AAC TCG AGT GTT GTG TAC GAG GCG GCC GAT GCC ATC CTG CAC ACT CCG     672
   Asn Ser Ser Val Val Tyr Glu Ala Ala Asp Ala Ile Leu His Thr Pro
       210                 215                 220
   GGG TGT GTC CCT TGC GTT CGC GAG GGT AAC GCC TCG AGG TGT TGG GTG     720
   Gly Cys Val Pro Cys Val Arg Glu Gly Asn Ala Ser Arg Cys Trp Val
   225                 230                 235                 240
   GCG GTG ACC CCC ACG GTG GCC ACC AGG GAC GGC AAA CTT CCC ACA ACG     768
   Ala Val Thr Pro Thr Val Ala Thr Arg Asp Gly Lys Leu Pro Thr Thr
                   245                 250                 255
   CAG CTT CGA CGT CAT ATC GAT CTG CTT GTC GGG AGC GCC ACC CTC TGC     816
   Gln Leu Arg Arg His Ile Asp Leu Leu Val Gly Ser Ala Thr Leu Cys
               260                 265                 270
   TCG GCC CTC TAC GTG GGG GAC CTG TGC GGG TCT GTC TTT CTC GTT GGT     864
   Ser Ala Leu Tyr Val Gly Asp Leu Cys Gly Ser Val Phe Leu Val Gly
           275                 280                 285
   CAA CTG TTT ACC TTC TCT CCC AGG CGC CAC TGG ACG ACG CAA GAC TGC     912
   Gln Leu Phe Thr Phe Ser Pro Arg Arg His Trp Thr Thr Gln Asp Cys
       290                 295                 300
   AAT TGT TCT ATC TAT CCC GGC CAT ATA ACG GGT CAT CGC ATG GCA TGG     960
   Asn Cys Ser Ile Tyr Pro Gly His Ile Thr Gly His Arg Met Ala Trp
   305                 310                 315                 320
   GAT ATG ATG ATG AAC TGG                                             978
   Asp Met Met Met Asn Trp
                   325
(2) INFORMATION FOR SEQ ID NO:2:
   (i) SEQUENCE CHARACTERISTICS:
      (A) LENGTH: 948 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..945
   (xi) SEQUENCE DESCRIPTION: SEQ ID NO:2:
(2) INFORMATION FOR SEQ ID NO:3:
   (i) SEQUENCE CHARACTERISTICS:
      (A) LENGTH: 759 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..756
   (xi) SEQUENCE DESCRIPTION: SEQ ID NO:3:
(2) INFORMATION FOR SEQ ID NO:4:
   (i) SEQUENCE CHARACTERISTICS:
      (A) LENGTH: 759 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..756
   (xi) SEQUENCE DESCRIPTION: SEQ ID NO:4:
   ATG TCC CCT ATA CTA GGT TAT TGG AAA ATT AAG GGC CTT GTG CAA CCC      48
   Met Ser Pro Ile Leu Gly Tyr Trp Lys Ile Lys Gly Leu Val Gln Pro
    1               5                  10                  15
   ACT CGA CTT CTT TTG GAA TAT CTT GAA GAA AAA TAT GAA GAG CAT TTG      96
   Thr Arg Leu Leu Leu Glu Tyr Leu Glu Glu Lys Tyr Glu Glu His Leu
               20                  25                  30
   TAT GAG CGC GAT GAA GGT GAT AAA TGG CGA AAC AAA AAG TTT GAA TTG     144
   Tyr Glu Arg Asp Glu Gly Asp Lys Trp Arg Asn Lys Lys Phe Glu Leu
           35                  40                  45
   GGT TTG GAG TTT CCC AAT CTT CCT TAT TAT ATT GAT GGT GAT GTT AAA     192
   Gly Leu Glu Phe Pro Asn Leu Pro Tyr Tyr Ile Asp Gly Asp Val Lys
       50                  55                  60
   TTA ACA CAG TCT ATG GCC ATC ATA CGT TAT ATA GCT GAC AAG CAC AAC     240
   Leu Thr Gln Ser Met Ala Ile Ile Arg Tyr Ile Ala Asp Lys His Asn
   65                  70                  75                  80
   ATG TTG GGT GGT TGT CCA AAA GAG CGT GCA GAG ATT TCA ATG CTT GAA     288
   Met Leu Gly Gly Cys Pro Lys Glu Arg Ala Glu Ile Ser Met Leu Glu
                   85                  90                  95
   GGA GCG GTT TTG GAT ATT AGA TAC GGT GTT TCG AGA ATT GCA TAT AGT     336
   Gly Ala Val Leu Asp Ile Arg Tyr Gly Val Ser Arg Ile Ala Tyr Ser
               100                 105                 110
   AAA GAC TTT GAA ACT CTC AAA GTT GAT TTT CTT AGC AAG CTA CCT GAA     384
   Lys Asp Phe Glu Thr Leu Lys Val Asp Phe Leu Ser Lys Leu Pro Glu
           115                 120                 125
   ATG CTG AAA ATG TTC GAA GAT CGT TTA TGT CAT AAA ACA TAT TTA AAT     432
   Met Leu Lys Met Phe Glu Asp Arg Leu Cys His Lys Thr Tyr Leu Asn
       130                 135                 140
   GGT GAT CAT GTA ACC CAT CCT GAC TTC ATG TTG TAT GAC GCT CTT GAT     480
   Gly Asp His Val Thr His Pro Asp Phe Met Leu Tyr Asp Ala Leu Asp
   145                 150                 155                 160
   GTT GTT TTA TAC ATG GAC CCA ATG TGC CTG GAT GCG TTC CCA AAA TTA     528
   Val Val Leu Tyr Met Asp Pro Met Cys Leu Asp Ala Phe Pro Lys Leu
                   165                 170                 175
   GTT TGT TTT AAA AAA CGT ATT GAA GCT ATC CCA CAA ATT GAT AAG TAC     576
   Val Cys Phe Lys Lys Arg Ile Glu Ala Ile Pro Gln Ile Asp Lys Tyr
               180                 185                 190
   TTG AAA TCC AGC AAG TAT ATA GCA TGG CCT TTG CAG GGC TGG CAA GCC     624
   Leu Lys Ser Ser Lys Tyr Ile Ala Trp Pro Leu Gln Gly Trp Gln Ala
           195                 200                 205
   ACG TTT GGT GGT GGC GAC CAT CCT CCA AAA TCG GAT CTG GTT CCG CGT     672
   Thr Phe Gly Gly Gly Asp His Pro Pro Lys Ser Asp Leu Val Pro Arg
       210                 215                 220
   GGA TCC GAC GTC AAG TTC CCG GGT GGC GGT CAG ATC GTT GGT GGA GTT     720
   Gly Ser Asp Val Lys Phe Pro Gly Gly Gly Gln Ile Val Gly Gly Val
   225                 230                 235                 240
   TAC TTG TTG CCG CGC AGG GAA TTC ATC GTG ACT GAC TGA                 759
   Tyr Leu Leu Pro Arg Arg Glu Phe Ile Val Thr Asp
                   245                 250
(2) INFORMATION FOR SEQ ID NO:5:
   (i) SEQUENCE CHARACTERISTICS:
      (A) LENGTH: 759 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..756
   (xi) SEQUENCE DESCRIPTION: SEQ ID NO:5:
   ATG TCC CCT ATA CTA GGT TAT TGG AAA ATT AAG GGC CTT GTG CAA CCC        48
   Met Ser Pro Ile Leu Gly Tyr Trp Lys Ile Lys Gly Leu Val Gln Pro
     1               5                  10                  15
   ACT CGA CTT CTT TTG GAA TAT CTT GAA GAA AAA TAT GAA GAG CAT TTG        96
   Thr Arg Leu Leu Leu Glu Tyr Leu Glu Glu Lys Tyr Glu Glu His Leu
                20                  25                  30
   TAT GAG CGC GAT GAA GGT GAT AAA TGG CGA AAC AAA AAG TTT GAA TTG      144
   Tyr Glu Arg Asp Glu Gly Asp Lys Trp Arg Asn Lys Lys Phe Glu Leu
            35                  40                  45
   GGT TTG GAG TTT CCC AAT CTT CCT TAT TAT ATT GAT GGT GAT GTT AAA      192
   Gly Leu Glu Phe Pro Asn Leu Pro Tyr Tyr Ile Asp Gly Asp Val Lys
        50                  55                  60
   TTA ACA CAG TCT ATG GCC ATC ATA CGT TAT ATA GCT GAC AAG CAC AAC      240
   Leu Thr Gln Ser Met Ala Ile Ile Arg Tyr Ile Ala Asp Lys His Asn
    65                  70                  75                  80
   ATG TTG GGT GGT TGT CCA AAA GAG CGT GCA GAG ATT TCA ATG CTT GAA      288
   Met Leu Gly Gly Cys Pro Lys Glu Arg Ala Glu Ile Ser Met Leu Glu
                    85                  90                  95
   GGA GCG GTT TTG GAT ATT AGA TAC GGT GTT TCG AGA ATT GCA TAT AGT      336
   Gly Ala Val Leu Asp Ile Arg Tyr Gly Val Ser Arg Ile Ala Tyr Ser
               100                 105                 110
   AAA GAC TTT GAA ACT CTC AAA GTT GAT TTT CTT AGC AAG CTA CCT GAA      384
   Lys Asp Phe Glu Thr Leu Lys Val Asp Phe Leu Ser Lys Leu Pro Glu
           115                 120                 125
   ATG CTG AAA ATG TTC GAA GAT CGT TTA TGT CAT AAA ACA TAT TTA AAT      432
   Met Leu Lys Met Phe Glu Asp Arg Leu Cys His Lys Thr Tyr Leu Asn
       130                 135                 140
   GGT GAT CAT GTA ACC CAT CCT GAC TTC ATG TTG TAT GAC GCT CTT GAT      480
   Gly Asp His Val Thr His Pro Asp Phe Met Leu Tyr Asp Ala Leu Asp
   145                 150                 155                 160
   GTT GTT TTA TAC ATG GAC CCA ATG TGC CTG GAT GCG TTC CCA AAA TTA      528
   Val Val Leu Tyr Met Asp Pro Met Cys Leu Asp Ala Phe Pro Lys Leu
                   165                 170                 175
   GTT TGT TTT AAA AAA CGT ATT GAA GCT ATC CCA CAA ATT GAT AAG TAC      576
   Val Cys Phe Lys Lys Arg Ile Glu Ala Ile Pro Gln Ile Asp Lys Tyr
               180                 185                 190
   TTG AAA TCC AGC AAG TAT ATA GCA TGG CCT TTG CAG GGC TGG CAA GCC      624
   Leu Lys Ser Ser Lys Tyr Ile Ala Trp Pro Leu Gln Gly Trp Gln Ala
           195                 200                 205
   ACG TTT GGT GGT GGC GAC CAT CCT CCA AAA TCG GAT CTG GTT CCG CGT      672
   Thr Phe Gly Gly Gly Asp His Pro Pro Lys Ser Asp Leu Val Pro Arg
       210                 215                 220
   GGA TCC GGC CCT AGA TTG GGT GTG CGC GCG ACG AGG AAG ACT TCC GAG      720
   Gly Ser Gly Pro Arg Leu Gly Val Arg Ala Thr Arg Lys Thr Ser Glu
   225                 230                 235                 240
   CGG TCG CAA CCT CGA GGT GAA TTC ATC GTG ACT GAC TGA                  759
   Arg Ser Gln Pro Arg Gly Glu Phe Ile Val Thr Asp
                   245                 250
(2) INFORMATION FOR SEQ ID NO:6:
   (i) SEQUENCE CHARACTERISTICS:
      (A) LENGTH: 816 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..813
   (xi) SEQUENCE DESCRIPTION: SEQ ID NO:6:
   ATG TCC CCT ATA CTA GGT TAT TGG AAA ATT AAG GGC CTT GTG CAA CCC          48
   Met Ser Pro Ile Leu Gly Tyr Trp Lys Ile Lys Gly Leu Val Gln Pro
     1               5                  10                  15
   ACT CGA CTT CTT TTG GAA TAT CTT GAA GAA AAA TAT GAA GAG CAT TTG          96
   Thr Arg Leu Leu Leu Glu Tyr Leu Glu Glu Lys Tyr Glu Glu His Leu
                20                  25                  30
   TAT GAG CGC GAT GAA GGT GAT AAA TGG CGA AAC AAA AAG TTT GAA TTG         144
   Tyr Glu Arg Asp Glu Gly Asp Lys Trp Arg Asn Lys Lys Phe Glu Leu
            35                  40                  45
   GGT TTG GAG TTT CCC AAT CTT CCT TAT TAT ATT GAT GGT GAT GTT AAA         192
   Gly Leu Glu Phe Pro Asn Leu Pro Tyr Tyr Ile Asp Gly Asp Val Lys
        50                  55                  60
   TTA ACA CAG TCT ATG GCC ATC ATA CGT TAT ATA GCT GAC AAG CAC AAC         240
   Leu Thr Gln Ser Met Ala Ile Ile Arg Tyr Ile Ala Asp Lys His Asn
    65                  70                  75                  80
   ATG TTG GGT GGT TGT CCA AAA GAG CGT GCA GAG ATT TCA ATG CTT GAA         288
   Met Leu Gly Gly Cys Pro Lys Glu Arg Ala Glu Ile Ser Met Leu Glu
                         85                       90                       95
   GGA GCG GTT TTG GAT ATT AGA TAC GGT GTT TCG AGA ATT GCA TAT AGT      336
   Gly Ala Val Leu Asp Ile Arg Tyr Gly Val Ser Arg Ile Ala Tyr Ser
               100                     105                     110
   AAA GAC TTT GAA ACT CTC AAA GTT GAT TTT CTT AGC AAG CTA CCT GAA      384
   Lys Asp Phe Glu Thr Leu Lys Val Asp Phe Leu Ser Lys Leu Pro Glu
           115                     120                     125
   ATG CTG AAA ATG TTC GAA GAT CGT TTA TGT CAT AAA ACA TAT TTA AAT      432
   Met Leu Lys Met Phe Glu Asp Arg Leu Cys His Lys Thr Tyr Leu Asn
       130                     135                     140
   GGT GAT CAT GTA ACC CAT CCT GAC TTC ATG TTG TAT GAC GCT CTT GAT      480
   Gly Asp His Val Thr His Pro Asp Phe Met Leu Tyr Asp Ala Leu Asp
   145                     150                     155                     160
   GTT GTT TTA TAC ATG GAC CCA ATG TGC CTG GAT GCG TTC CCA AAA TTA      528
   Val Val Leu Tyr Met Asp Pro Met Cys Leu Asp Ala Phe Pro Lys Leu
                   165                     170                     175
   GTT TGT TTT AAA AAA CGT ATT GAA GCT ATC CCA CAA ATT GAT AAG TAC      576
   Val Cys Phe Lys Lys Arg Ile Glu Ala Ile Pro Gln Ile Asp Lys Tyr
               180                     185                     190
   TTG AAA TCC AGC AAG TAT ATA GCA TGG CCT TTG CAG GGC TGG CAA GCC      624
   Leu Lys Ser Ser Lys Tyr Ile Ala Trp Pro Leu Gln Gly Trp Gln Ala
           195                     200                     205
   ACG TTT GGT GGT GGC GAC CAT CCT CCA AAA TCG GAT CTG GTT CCG CGT      672
   Thr Phe Gly Gly Gly Asp His Pro Pro Lys Ser Asp Leu Val Pro Arg
       210                     215                     220
   GGA TCC AGC ACG ATT CCC AAA CCT CAA AGA AAA ACC AAA CGT AAC ACC      720
   Gly Ser Ser Thr Ile Pro Lys Pro Gln Arg Lys Thr Lys Arg Asn Thr
   225                     230                     235                     240
   AAC CGT CGC CCA CAG GAC GTC AAG TTC CCG GGT GGC GGT CAG ATC GTT      768
   Asn Arg Arg Pro Gln Asp Val Lys Phe Pro Gly Gly Gly Gln Ile Val
                   245                     250                     255
   GGT GGA GTT TAC TTG TTG CCG CGC AGG GAA TTC ATC GTG ACT GAC          813
   Gly Gly Val Tyr Leu Leu Pro Arg Arg Glu Phe Ile Val Thr Asp
               260                     265                     270
   TGA                                                                  816
(2) INFORMATION FOR SEQ ID NO:7:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:7:
   GATCCATGAG CACGATTCCC AAACCTCAAA GAAAAACCAA ACGTAACACC AACCGTCGCC 60
   CACAGG 66
(2) INFORMATION FOR SEQ ID NO:8:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:8:
   AATTCCTGTG GGCGACGGTT GGTGTTACGT TTGGTTTTTC TTTGAGGTTT GGGAATCGTG 60
   CTCATG 66
(2) INFORMATION FOR SEQ ID NO:9:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:9:
   GATCCGACGT CAAGTTCCCG GGTGGCGGTC AGATCGTTGG TGGAGTTTAC TTGTTGCCGC 60
   GCAGGG 66
(2) INFORMATION FOR SEQ ID NO:10:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:10:
   AATTCCCTGC GCGGCAACAA GTAAACTCCA CCAACGATCT GACCGCCACC CGGGAACTTG 60
   ACGTCG 66
(2) INFORMATION FOR SEQ ID NO:11:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:11:
   GATCCGGCCC TAGATTGGGT GTGCGCGCGA CGAGGAAGAC TTCCGAGCGG TCGCAACCTC 60
   GAGGTG 66
(2) INFORMATION FOR SEQ ID NO:12:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:12:
   AATTCACCTC GAGGTTGCGA CCGCTCGGAA GTCTTCCTCG TCGCGCGCAC ACCCAATCTA 60
   GGGCCG 66
(2) INFORMATION FOR SEQ ID NO:13:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:13:
      GAATTCTTAC CTGCGCGGCA ACAAGTAAAC TC 32
(2) INFORMATION FOR SEQ ID NO:14:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:14:
      GCTGGATCCA GCACGATTCC CAAACCTCAA AG 32
(2) INFORMATION FOR SEQ ID NO:15:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii), HYPOTHETICAL: NO
   (iv) ANTI-SENSE: NO
   (xi) SEQUENCE DESCRIPTION: SEQ ID NO:15:
      ATGAGCACGA TTCCCAAACC T 21
(2) INFORMATION FOR SEQ ID NO:16:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:16:
      GAGGAAGACTTCCGAGC 17
(2) INFORMATION FOR SEQ ID NO:17:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:17:
      GTCCTGCCCT CGGGCCG 17
(2) INFORMATION FOR SEQ ID NO:18:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:18:
      ACCCAAATTG CGCGACCTAC G 21
(2) INFORMATION FOR SEQ ID NO:19:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:19:
      TGGGTAAGGT CATCGATAC 19
(2) INFORMATION FOR SEQ ID NO:20:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:20:
      AAGGTCATCG ATACCCT 17
(2) INFORMATION FOR SEQ ID NO:21:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:21:
      AGATAGAGAA AGAGCAAG 18
(2) INFORMATION FOR SEQ ID NO:22:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:22:
      GGACCAGTTC ATCATCATAT AT 22
(2) INFORMATION FOR SEQ ID NO:23:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:23:
      CAGTTCATCA TCATATCCCA 20
(2) INFORMATION FOR SEQ ID NO:24:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..15
      (D) OTHER INFORMATION: /product= "Linker Protein in GST-NANBV 693-691"
   (xi) SEQUENCE DESCRIPTION: SEQ ID NO:24:
   GGG ATC CCC AAT TCA                                          15
   Gly Ile Pro Asn Ser
     1               5
(2) INFORMATION FOR SEQ ID NO:25:
   (i) SEQUENCE CHARACTERISTICS:
      (A) LENGTH: 5 amino acids
      (B) TYPE: amino acid
      (D) TOPOLOGY: linear
   (ii) MOLECULE TYPE: protein
   (xi) SEQUENCE DESCRIPTION: SEQ ID NO:25:
   Gly Ile Pro Asn Ser
     1               5
(2) INFORMATION FOR SEQ ID NO:26:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..9
      (D) OTHER INFORMATION: /product= "Carboxy-terminal Linker Protein in GST-NANBV 693-691" "
   (xi) SEQUENCE DESCRIPTION: SEQ ID NO:26:
   AAT TCA TCG TGA                                              12
   Asn Ser Ser
     1
(2) INFORMATION FOR SEQ ID NO:27:
   (i) SEQUENCE CHARACTERISTICS:
      (A) LENGTH: 3 amino acids
      (B) TYPE: amino acid
      (D) TOPOLOGY: linear
   (ii) MOLECULE TYPE: protein
   (xi) SEQUENCE DESCRIPTION: SEQ ID NO:27:
   Asn Ser Ser
   1
(2) INFORMATION FOR SEQ ID NO:28:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..27
      (D) OTHER INFORMATION: /product= "Linker Protein in GST-NANBV 15-18"
   (xi) SEQUENCE DESCRIPTION: SEQ ID NO:28:
   GGG ATC CCC ATC GAA TTC CTG CAG CCC                                  27
   Gly Ile Pro Ile Glu Phe Leu Gln Pro
     1             5
(2) INFORMATION FOR SEQ ID NO:29:
   (i) SEQUENCE CHARACTERISTICS:
      (A) LENGTH: 9 amino acids
      (B) TYPE: amino acid
      (D) TOPOLOGY: linear
   (ii) MOLECULE TYPE: protein
   (xi) SEQUENCE DESCRIPTION: SEQ ID NO:29:
   Gly Ile Pro Ile Glu Phe Leu Gln Pro
     1             5
(2) INFORMATION FOR SEQ ID NO:30:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..21
      (D) OTHER INFORMATION: /product= "Carboxy-terminal Linker Protein in GST-NANBV 15-18"
   (xi) SEQUENCE DESCRIPTION: SEQ ID NO:30:
   TGG GGG ATC GGG AAT TCA TCG TGA                                          24
   Trp Gly Ile Gly Asn Ser Ser
     1                 5
(2) INFORMATION FOR SEQ ID NO:31:
   (i) SEQUENCE CHARACTERISTICS:
      (A) LENGTH: 7 amino acids
      (B) TYPE: amino acid
      (D) TOPOLOGY: linear
   (ii) MOLECULE TYPE: protein
   (xi) SEQUENCE DESCRIPTION: SEQ ID NO:31:
   Trp Gly Ile Gly Asn Ser Ser
     1                 5
(2) INFORMATION FOR SEQ ID NO:32:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..24
      (D) OTHER INFORMATION: /product= "Linker Protein in GST-NANBV 15-17"
   (xi) SEQUENCE DESCRIPTION: SEQ ID NO:32:
   GGG ATC CCC AAT TCC TGC AGC CCT                                          24
   Gly Ile Pro Asn Ser Cys Ser Pro
     1                 5
(2) INFORMATION FOR SEQ ID NO:33:
   (i) SEQUENCE CHARACTERISTICS:
      (A) LENGTH: 8 amino acids
      (B) TYPE: amino acid
      (D) TOPOLOGY: linear
   (ii) MOLECULE TYPE: protein
   (xi) SEQUENCE DESCRIPTION: SEQ ID NO:33:
   Gly Ile Pro Asn Ser Cys Ser Pro
    1               5
(2) INFORMATION FOR SEQ ID NO:34:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..18
      (D) OTHER INFORMATION: /product= "Carboxy-terminal Linker Protein in GST-NANBV 15-17"
   (xi) SEQUENCE DESCRIPTION: SEQ ID NO:34:
   GGG ATC GGG AAT TCA TCG TGA                                    21
   Gly Ile Gly Asn Ser Ser
    1               5
(2) INFORMATION FOR SEQ ID NO:35:
   (i) SEQUENCE CHARACTERISTICS:
      (A) LENGTH: 6 amino acids
      (B) TYPE: amino acid
      (D) TOPOLOGY: linear
   (ii) MOLECULE TYPE: protein
   (xi) SEQUENCE DESCRIPTION: SEQ ID NO:35:
   Gly Ile Gly Asn Ser Ser
    1               5
(2) INFORMATION FOR SEQ ID NO:36:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..15
      (D) OTHER INFORMATION: /product= "Thrombin Cleavage Site in GST-NANBV 15-17"
   (xi) SEQUENCE DESCRIPTION: SEQ ID NO:36:
   GTT CCG CGT GGA TCC                                              15
   Val Pro Arg Gly Ser
     1               5
(2) INFORMATION FOR SEQ ID NO:37:
   (i) SEQUENCE CHARACTERISTICS:
      (A) LENGTH: 5 amino acids
      (B) TYPE: amino acid
      (D) TOPOLOGY: linear
   (ii) MOLECULE TYPE: protein
   (xi) SEQUENCE DESCRIPTION: SEQ ID NO:37:
   Val Pro Arg Gly Ser
     1               5
(2) INFORMATION FOR SEQ ID NO:38:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..21
      (D) OTHER INFORMATION: /product= "Linker Protein in GST-NANBV 15-17" (xi) SEQUENCE DESCRIPTION: SEQ ID NO:38:
(2) INFORMATION FOR SEQ ID NO:39:
   (i) SEQUENCE CHARACTERISTICS:
      (A) LENGTH: 7 amino acids
      (B) TYPE: amino acid
      (D) TOPOLOGY: linear
   (ii) MOLECULE TYPE: protein
   (xi) SEQUENCE DESCRIPTION: SEQ ID NO:39:
   Pro Ser Asn Ser Cys Ser Pro
   1               5
(2) INFORMATION FOR SEQ ID NO:40:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..15
      (D) OTHER INFORMATION: /product= "Carboxy-terminal Linker Protein in GST-NANBV 15-17"
   (xi) SEQUENCE DESCRIPTION: SEQ ID NO:40:
   GGA ATT CAT CGT GAC TGA                                             18
   Gly Ile His Arg Asp
     1               5
(2) INFORMATION FOR SEQ ID NO:41:
   (i) SEQUENCE CHARACTERISTICS:
      (A) LENGTH: 5 amino acids
      (B) TYPE: amino acid
      (D) TOPOLOGY: linear
   (ii) MOLECULE TYPE: protein
   (xi) SEQUENCE DESCRIPTION: SEQ ID NO:41:
   Gly Ile His Arg Asp
    1               5
(2) INFORMATION FOR SEQ ID NO:42:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..27
      (D) OTHER INFORMATION: /product= "Linker Protein in GST-NANBV 690-691"
   (xi) SEQUENCE DESCRIPTION: SEQ ID NO:42:
   GGG ATC CCC AAT TCG AGC TCG GTA CCC                                27
   Gly Ile Pro Asn Ser Ser Ser Val Pro
     1               5
(2) INFORMATION FOR SEQ ID NO:43:
   (i) SEQUENCE CHARACTERISTICS:
      (A) LENGTH: 9 amino acids
      (B) TYPE: amino acid
      (D) TOPOLOGY: linear
   (ii) MOLECULE TYPE: protein
   (xi) SEQUENCE DESCRIPTION: SEQ ID NO:43:
   Gly Ile Pro Asn Ser Ser Ser Val Pro
     1               5
(2) INFORMATION FOR SEQ ID NO:44:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..21
      (D) OTHER INFORMATION: /product= "Carboxy-terminal Linker Protein in GST-NANBV 690-691"
   (xi) SEQUENCE DESCRIPTION: SEQ ID NO:44:
   ACG GGG ATC GGG AAT TCA TCG TGA                                      24
   Thr Gly Ile Gly Asn Ser Ser
     1               5
(2) INFORMATION FOR SEQ ID NO:45:
   (i) SEQUENCE CHARACTERISTICS:
      (A) LENGTH: 7 amino acids
      (B) TYPE: amino acid
      (D) TOPOLOGY: linear
   (ii) MOLECULE TYPE: protein
   (xi) SEQUENCE DESCRIPTION: SEQ ID NO:45:
   Thr Gly Ile Gly Asn Ser Ser
     1               5
(2) INFORMATION FOR SEQ ID NO:46:
   (i) SEQUENCE CHARACTERISTICS:
      (A) LENGTH: 9416 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (ix) FEATURE:
      (A) NAME/KEY: CDS
      (B) LOCATION: 342..9374
   (ix) FEATURE:
      (A) NAME/KEY: misc_feature
      (B) LOCATION: 1..12
      (D) OTHER INFORMATION: /note= "Not confirmed as HCV-Hc59 Sequence"
   (ix) FEATURE:
      (A) NAME/KEY: misc_feature
      (B) LOCATION: 9397..9416
      (D) OTHER INFORMATION: /note= "Not confirmed as HCV-Hc59 Sequence"
   (ix) FEATURE:
      (A) NAME/KEY: repeat_unit
      (B) LOCATION: group(7..12, 42..47)
      (D) OTHER INFORMATION: /rpt_type= "other" /rpt_family= "1"
   (ix) FEATURE:
      (A) NAME/KEY: repeat_unit
      (B) LOCATION: group(23..28, 38..43, 9209..9214, 9391..9396)
      (D) OTHER INFORMATION: /rpt_type= "other" /rpt_family= "2"
   (ix) FEATURE:
      (A) NAME/KEY: repeat_unit
      (B) LOCATION: group(128..135, 315..322)
      (D) OTHER INFORMATION: /rpt_type= "other" /rpt_family= "3"
   (ix) FEATURE:
      (A) NAME/KEY: repeat_unit
      (B) LOCATION: group(9231..9237, 9245..9251, 9256..9262)
      (D) OTHER INFORMATION: /rpt_type= "other" /rpt_family= "4"
   (ix) FEATURE:
      (A) NAME/KEY: repeat_unit,
      (B) LOCATION: group(9248..9253, 9221..9226, 9227..9232)
      (D) OTHER INFORMATION: /rpt_type= "other" /rpt_family= "5"
   (xi) SEQUENCE DESCRIPTION: SEQ ID NO:46:
   GCCAGCCCCC TGATGGGGGC GACACTCCAC CATGAATCAC TCCCCTGTGA GGAACTACTG      60
   TCTTCACGCA GAAAGCGTCT AGCCATGGCG TTAGTATGAG TGTCGTGCAG CCTCCAGGAC     120
   CCCCCCTCCC GGGAGAGCCA TAGTGGTCTG CGGAACCGGT GAGTACACCG GAATTGCCAG     180
   GACGACCGGG TCCTTTCTTG GATAAACCCG CTCAATGCCT GGAGATTTGG GCGTGCCCCC     240
   GCAAGACTGC TAGCCGAGTA GTGTTGGGTC GCGAAAGGCC TTGTGGTACT GCCTGATAGG     300
   GTGCTTGCGA GTGCCCCGGG AGGTCTCGTA GACCGTGCAC C ATG AGC ACG AAT        353
                                                 Met Ser Thr Asn
                                                   1
   CCT AAA CCT CAA AGA AAA ACC AAA CGT AAC ACC AAC CGT CGC CCA CAG      401
   Pro Lys Pro Gln Arg Lys Thr Lys Arg Asn Thr Asn Arg Arg Pro Gln
     5                  10                  15                  20
   GAC GTC AAG TTC CCG GGT GGC GGT CAG ATC GTT GGT GGA GTT TAC TTG      449
   Asp Val Lys Phe Pro Gly Gly Gly Gln Ile Val Gly Gly Val Tyr Leu
                    25                  30                  35
   TTG CCG CGC AGG GGC CCT AGA TTG GGT GTG CGC GCG ACG AGG AAG ACT      497
   Leu Pro Arg Arg Gly Pro Arg Leu Gly Val Arg Ala Thr Arg Lys Thr
                40                  45                  50
   TCC GAG CGG TCG CAA CCT CGA GGT AGA CGT CAG CCT ATC CCC AAG GCA      545
   Ser Glu Arg Ser Gln Pro Arg Gly Arg Arg Gln Pro Ile Pro Lys Ala
            55                  60                  65
   CGT CGG CCC GAG GGC AGG ACC TGG GCT CAG CCC GGG TAC CCT TGG CCC      593
   Arg Arg Pro Glu Gly Arg Thr Trp Ala Gln Pro Gly Tyr Pro Trp Pro
        70                  75                  80
   CTC TAT GGC AAT GAG GGT TGC GGG TGG GCG GGA TGG CTC CTG TCT CCC      641
   Leu Tyr Gly Asn Glu Gly Cys Gly Trp Ala Gly Trp Leu Leu Ser Pro
    85                  90                  95                 100
   CGT GGC TCT CGG CCT AGC TGG GGC CCC ACA GAC CCC CGG CGT AGG TCG      689
   Arg Gly Ser Arg Pro Ser Trp Gly Pro Thr Asp Pro Arg Arg Arg Ser
                   105                 110                 115
   CGC AAT TTG GGT AAG GTC ATC GAT ACC CTT ACG TGC GGC TTC GCC GAC      737
   Arg Asn Leu Gly Lys Val Ile Asp Thr Leu Thr Cys Gly Phe Ala Asp
               120                 125                 130
   CTC ATG GGG TAC ATA CCG CTC GTC GGC GCC CCT CTT GGA GGC GCT GCC      785
   Leu Met Gly Tyr Ile Pro Leu Val Gly Ala Pro Leu Gly Gly Ala Ala
           135                 140                 145
   AGG GCC CTG GCG CAT GGC GTC CGG GTT CTG GAA GAC GGC GTG AAC TAT        833
   Arg Ala Leu Ala His Gly Val Arg Val Leu Glu Asp Gly Val Asn Tyr
       150                 155                 160
   GCA ACA GGG AAC CTT CCT GGT TGC TCT TTC TCT ATC TTC CTT CTG GCC        881
   Ala Thr Gly Asn Leu Pro Gly Cys Ser Phe Ser Ile Phe Leu Leu Ala
   165                 170                 175                 180
   CTG CTC TCT TGC CTG ACT GTG CCC GCT TCA GCC TAC CAA GTG CGC AAT        929
   Leu Leu Ser Cys Leu Thr Val Pro Ala Ser Ala Tyr Gln Val Arg Asn
                   185                 190                 195
   TCC TCG GGG CTT TAC CAT GTC ACC AAT GAT TGC CCT AAC TCG AGT GTT        977
   Ser Ser Gly Leu Tyr His Val Thr Asn Asp Cys Pro Asn Ser Ser Val
               200                 205                 210
   GTG TAC GAG GCG GCC GAT GCC ATC CTG CAC ACT CCG GGG TGT GTC CCT       1025
   Val Tyr Glu Ala Ala Asp Ala Ile Leu His Thr Pro Gly Cys Val Pro
           215                 220                 225
   TGC GTT CGC GAG GGT AAC GCC TCG AGG TGT TGG GTG GCG GTG ACC CCC       1073
   Cys Val Arg Glu Gly Asn Ala Ser Arg Cys Trp Val Ala Val Thr Pro
       230                 235                 240
   ACG GTG GCC ACC AGG GAC GGC AAA CTC CCC ACA ACG CAG CTT CGA CGT       1121
   Thr Val Ala Thr Arg Asp Gly Lys Leu Pro Thr Thr Gln Leu Arg Arg
   245                 250                 255                 260
   CAT ATC GAT CTG CTT GTC GGG AGC GCC ACC CTC TGC TCG GCC CTC TAC       1169
   His Ile Asp Leu Leu Val Gly Ser Ala Thr Leu Cys Ser Ala Leu Tyr
                   265                 270                 275
   GTG GGG GAC CTG TGC GGG TCT GTC TTT CTT GTT GGT CAA CTG TTT ACC       1217
   Val Gly Asp Leu Cys Gly Ser Val Phe Leu Val Gly Gln Leu Phe Thr
               280                 285                 290
   TTC TCT CCC AGG CAC CAC TGG ACG ACG CAA GAC TGC AAT TGT TCT ATC       1265
   Phe Ser Pro Arg His His Trp Thr Thr Gln Asp Cys Asn Cys Ser Ile
           295                 300                 305
   TAT CCC GGC CAT ATA ACG GGT CAT CGC ATG GCA TGG AAT ATG ATG ATG       1313
   Tyr Pro Gly His Ile Thr Gly His Arg Met Ala Trp Asn Met Met Met
       310                 315                 320
   AAC TGG TCC CCT ACG GCA GCG TTG GTG GTA GCT CAG CTG CTC CGA ATC       1361
   Asn Trp Ser Pro Thr Ala Ala Leu Val Val Ala Gln Leu Leu Arg Ile
   325                 330                 335                 340
   CCA CAA GCC ATC ATG GAC ATG ATC GCT GGC GCC CAC TGG GGA GTC CTG       1409
   Pro Gln Ala Ile Met Asp Met Ile Ala Gly Ala His Trp Gly Val Leu
                   345                 350                 355
   GCG GGC ATA AAG TAT TTC TCC ATG GTG GGG AAC TGG GCG AAG GTC CTG      1457
   Ala Gly Ile Lys Tyr Phe Ser Met Val Gly Asn Trp Ala Lys Val Leu
               360                 365                 370
   GTA GTG CTG CTG CTA TTT GCC GGC GTC GAC GCG GAA ACC CAC GTC ACC      1505
   Val Val Leu Leu Leu Phe Ala Gly Val Asp Ala Glu Thr His Val Thr
           375                 380                 385
   GGG GGA AAT GCC GGC CGC ACC ACG GCT GGG CTT GTT GGT CTC CTT ACA      1553
   Gly Gly Asn Ala Gly Arg Thr Thr Ala Gly Leu Val Gly Leu Leu Thr
       390                 395                 400
   CCA GGC GCC AAG CAG AAC ATC CAA CTG ATC AAC ACC AAC GGC AGT TGG      1601
   Pro Gly Ala Lys Gln Asn Ile Gln Leu Ile Asn Thr Asn Gly Ser Trp
   405                 410                 415                 420
   CAC ATC AAT AGC ACG GCC TTG AAC TGC AAT GAA AGC CTT AAC ACC GGC      1649
   His Ile Asn Ser Thr Ala Leu Asn Cys Asn Glu Ser Leu Asn Thr Gly
                   425                 430                 435
   TGG TTA GCA GGG CTC TTC TAT CAG CAC AAA TTC AAC TCT TCA GGC TGT      1697
   Trp Leu Ala Gly Leu Phe Tyr Gln His Lys Phe Asn Ser Ser Gly Cys
               440                 445                 450
   CCT GAG AGG TTG GCC AGC TGC CGA CGC CTT ACC GAT TTT GCC CAG GGC      1745
   Pro Glu Arg Leu Ala Ser Cys Arg Arg Leu Thr Asp Phe Ala Gln Gly
           455                 460                 465
   TGG GGT CCT ATC AGT TAT GCC AAC GGA AGC GGC CTC GAC GAA CGC CCC      1793
   Trp Gly Pro Ile Ser Tyr Ala Asn Gly Ser Gly Leu Asp Glu Arg Pro
       470                 475                 480
   TAC TGC TGG CAC TAC CCT CCA AGA CCT TGT GGC ATT GTG CCC GCA AAG      1841
   Tyr Cys Trp His Tyr Pro Pro Arg Pro Cys Gly Ile Val Pro Ala Lys
   485                 490                 495                 500
   AGC GTG TGT GGC CCG GTA TAT TGC TTC ACT CCC AGC CCC GTG GTG GTG      1889
   Ser Val Cys Gly Pro Val Tyr Cys Phe Thr Pro Ser Pro Val Val Val
                   505                 510                 515
   GGA ACG ACC GAC AGG TCG GGC GCG CCT ACC TAC AGC TGG GGT GCA AAT      1937
   Gly Thr Thr Asp Arg Ser Gly Ala Pro Thr Tyr Ser Trp Gly Ala Asn
               520                 525                 530
   GAT ACG GAT GTC TTC GTC CTT AAC AAC ACC AGG CCA CCG CTG GGC AAT      1985
   Asp Thr Asp Val Phe Val Leu Asn Asn Thr Arg Pro Pro Leu Gly Asn
           535                 540                 545
   TGG TTC GGT TGT ACC TGG ATG AAC TCA ACT GGA TTC ACC AAA GTG TGC      2033
   Trp Phe Gly Cys Thr Trp Met Asn Ser Thr Gly Phe Thr Lys Val Cys
       550                 555                 560
   GGA GCG CCC CCT TGT GTC ATC GGA GGG GTG GGC AAC AAC ACC TTG CTC 2081
   Gly Ala Pro Pro Cys Val Ile Gly Gly Val Gly Asn Asn Thr Leu Leu
   565 570 575 580
   TGC CCC ACT GAT TGC TTC CGC AAA TAT CCG GAA GCC ACA TAC TCT CGG 2129
   Cys Pro Thr Asp Cys Phe Arg Lys Tyr Pro Glu Ala Thr Tyr Ser Arg
   585 590 595
   TGC GGC TCC GGT CCC AGG ATT ACA CCC AGG TGC ATG GTC GAC TAC CCG 2177
   Cys Gly Ser Gly Pro Arg Ile Thr Pro Arg Cys Met Val Asp Tyr Pro
   600 605 610
   TAT AGG CTT TGG CAC TAT CCT TGT ACC ATC AAT TAC ACC ATA TTC AAA 2225
   Tyr Arg Leu Trp His Tyr Pro Cys Thr Ile Asn Tyr Thr Ile Phe Lys
   615 620 625
   GTC AGG ATG TAC GTG GGA GGG GTC GAG CAC AGG CTG GAA GCG GCC TGC 2273
   Val Arg Met Tyr Val Gly Gly Val Glu His Arg Leu Glu Ala Ala Cys
   630 635 640
   AAC TGG ACG CGG GGC GAA CGC TGT GAT CTG GAA GAC AGG GAC AGG TCC 2321
   Asn Trp Thr Arg Gly Glu Arg Cys Asp Leu Glu Asp Arg Asp Arg Ser
   645 650 655 660
   GAG CTC AGC CCG TTG CTG CTG TCC ACC ACA CAG TGG CAG GTC CTT CCG 2369
   Glu Leu Ser Pro Leu Leu Leu Ser Thr Thr Gln Trp Gln Val Leu Pro
   665 670 675
   TGT TCT TTC ACG ACC CTG CCA GCC TTG TCC ACC GGC CTC ATC CAC CTC 2417
   Cys Ser Phe Thr Thr Leu Pro Ala Leu Ser Thr Gly Leu Ile His Leu
   680 685 690
   CAC CAG AAC ATT GTG GAC GTG CAG TAC TTG TAC GGG GTA GGG TCA AGC 2465
   His Gln Asn Ile Val Asp Val Gln Tyr Leu Tyr Gly Val Gly Ser Ser
   695 700 705
   ATC GCG TCC TGG GCC ATT AAG TGG GAG TAC GTC GTT CTC CTG TTC CTT 2513
   Ile Ala Ser Trp Ala Ile Lys Trp Glu Tyr Val Val Leu Leu Phe Leu
   710 715 720
   CTG CTT GCA GAC GCG CGC GTC TGT TCC TGC TTG TGG ATG ATG TTA CTC 2561
   Leu Leu Ala Asp Ala Arg Val Cys Ser Cys Leu Trp Met Met Leu Leu
   725 730 735 740
   ATA TCC CAA GCG GAG GCG GCT TTG GAG AAC CTC GTA ATA CTC AAT GCA 2609
   Ile Ser Gln Ala Glu Ala Ala Leu Glu Asn Leu Val Ile Leu Asn Ala
   745 750 755
   GCA TCC CTG GCC GGG ACG CAT GGT CTT GTG TCC TTC CTC GTG TTC TTC 2657
   Ala Ser Leu Ala Gly Thr His Gly Leu Val Ser Phe Leu Val Phe Phe
   760 765 770
   TGC TTT GCG TGG TAT CTG AAG GGT AGG TGG GTG CCC GGA GCG GTC TAC        2705
   Cys Phe Ala Trp Tyr Leu Lys Gly Arg Trp Val Pro Gly Ala Val Tyr
           775                 780                 785
   GCC CTC TAC GGG ATG TGG CCT CTC CTC CTG CTC CTG CTG GCG TTG CCT        2753
   Ala Leu Tyr Gly Met Trp Pro Leu Leu Leu Leu Leu Leu Ala Leu Pro
       790                 795                 800
   CAG CGG GCA TAC GCA CTG GAC ACG GAG GTG GCC GCG TCG TGT GGC GGC        2801
   Gln Arg Ala Tyr Ala Leu Asp Thr Glu Val Ala Ala Ser Cys Gly Gly
   805                 810                 815                 820
   GTT GTT CTT GTC GGG TTA ATG GCG CTG ACT CTG TCG CCA TAT TAC AAG        2849
   Val Val Leu Val Gly Leu Met Ala Leu Thr Leu Ser Pro Tyr Tyr Lys
                   825                 830                 835
   CGC TAT ATC AGC TGG TGC ATG TGG TGG CTT CAG TAT TTT CTG ACC AGA        2897
   Arg Tyr Ile Ser Trp Cys Met Trp Trp Leu Gln Tyr Phe Leu Thr Arg
               840                 845                 850
   GTA GAA GCG CAA CTG CAC GTG TGG GTT CCC CCC CTC AAC GTC CGG GGG        2945
   Val Glu Ala Gln Leu His Val Trp Val Pro Pro Leu Asn Val Arg Gly
           855                 860                 865
   GGG CGC GAT GCC GTC ATC TTA CTC ACG TGT GTA GTA CAC CCG GCC CTG        2993
   Gly Arg Asp Ala Val Ile Leu Leu Thr Cys Val Val His Pro Ala Leu
       870                 875                 880
   GTA TTT GAC ATC ACC AAA CTA CTC CTG GCC ATC TTC GGA CCC CTT TGG        3041
   Val Phe Asp Ile Thr Lys Leu Leu Leu Ala Ile Phe Gly Pro Leu Trp
   885                 890                 895                 900
   ATT CTT CAA GCC AGT TTG CTT AAA GTC CCC TAC TTC GTG CGC GTT CAA        3089
   Ile Leu Gln Ala Ser Leu Leu Lys Val Pro Tyr Phe Val Arg Val Gln
                   905                 910                 915
   GGC CTT CTC CGG ATC TGC GCG CTA GCG CGG AAG ATA GCC GGA GGT CAT        3137
   Gly Leu Leu Arg Ile Cys Ala Leu Ala Arg Lys Ile Ala Gly Gly His
               920                 925                 930
   TAC GTG CAA ATG GCC ATC ATC AAG TTA GGG GCG CTT ACT GGC ACC TGT        3185
   Tyr Val Gln Met Ala Ile Ile Lys Leu Gly Ala Leu Thr Gly Thr Cys
           935                 940                 945
   GTG TAT AAC CAT CTC GCT CCT CTT CGA GAC TGG GCG CAC AAC GGC CTG        3233
   Val Tyr Asn His Leu Ala Pro Leu Arg Asp Trp Ala His Asn Gly Leu
       950                 955                 960
   CGA GAT CTG GCC GTG GCT GTG GAA CCA GTC GTC TTC TCC CGA ATG GAG        3281
   Arg Asp Leu Ala Val Ala Val Glu Pro Val Val Phe Ser Arg Met Glu
   965                 970                 975                 980
   ACC AAG CTC ATC ACG TGG GGG GCA GAT ACC GCC GCG TGC GGT GAC ATC 3329
   Thr Lys Leu Ile Thr Trp Gly Ala Asp Thr Ala Ala Cys Gly Asp Ile
   985 990 995
   ATC AAC GGC TTG CCC GTC TCT GCC CGT AGG GGC CAG GAG ATA CTG CTT 3377
   Ile Asn Gly Leu Pro Val Ser Ala Arg Arg Gly Gln Glu Ile Leu Leu
   1000 1005 1010
   GGG CCA GCC GAC GGA ATG GTC TCC AAG GGG TGG AGG TTG CTG GCG CCC 3425
   Gly Pro Ala Asp Gly Met Val Ser Lys Gly Trp Arg Leu Leu Ala Pro
   1015 1020 1025
   ATC ACG GCG TAC GCC CAG CAG ACG AGA GGC CTC CTA GGG TGT ATA ATC 3473
   Ile Thr Ala Tyr Ala Gln Gln Thr Arg Gly Leu Leu Gly Cys Ile Ile
   1030 1035 1040
   ACC AGC CTG ACT GGC CGG GAC AAA AAC CAA GTG GAG GGT GAG GTC CAG 3521
   Thr Ser Leu Thr Gly Arg Asp Lys Asn Gln Val Glu Gly Glu Val Gln
   1045 1050 1055 1060
   ATC GTG TCA ACT GCT ACC CAG ACC TTC CTG GCA ACG TGC ATC AAT GGG 3569
   Ile Val Ser Thr Ala Thr Gln Thr Phe Leu Ala Thr Cys Ile Asn Gly
   1065 1070 1075
   GTA TGC TGG ACT GTC TAC CAC GGG GCC GGA ACG AGG ACC ATC GCA TCA 3617
   Val Cys Trp Thr Val Tyr His Gly Ala Gly Thr Arg Thr Ile Ala Ser
   1080 1085 1090
   CCC AAG GGT CCT GTC ATC CAG ACG TAT ACC AAT GTG GAT CAA GAC CTC 3665
   Pro Lys Gly Pro Val Ile Gln Thr Tyr Thr Asn Val Asp Gln Asp Leu
   1095 1100 1105
   GTG GGC TGG CCC GCT CCT CAA GGT TCC CGC TCA TTG ACA CCC TGC ACC 3713
   Val Gly Trp Pro Ala Pro Gln Gly Ser Arg Ser Leu Thr Pro Cys Thr
   1110 1115 1120
   TGC GGC TCC TCG GAC CTT TAC CTG GTC ACG AGG CAC GCC GAT GTC ATT 3761
   Cys Gly Ser Ser Asp Leu Tyr Leu Val Thr Arg His Ala Asp Val Ile
   1125 1130 1135 1140
   CCC GTG CGC CGG CGA GGT GAT AGC AGG GGT AGC CTG CTT TCG CCC CGG 3809
   Pro Val Arg Arg Arg Gly Asp Ser Arg Gly Ser Leu Leu Ser Pro Arg
   1145 1150 1155
   CCC ATT TCC TAC TTG AAA GGC TCC TCG GGG GGT CCG CTG TTG TGC CCC 3857
   Pro Ile Ser Tyr Leu Lys Gly Ser Ser Gly Gly Pro Leu Leu Cys Pro
   1160 1165 1170
   ACG GGA CAC GCC GTG GGC CTA TTC AGG GCC GCG GTG TGC ACC CGT GGA 3905
   Thr Gly His Ala Val Gly Leu Phe Arg Ala Ala Val Cys Thr Arg Gly
   1175 1180 1185
   GTG GCT AAG GCG GTG GAC TTT ATC CCT GTG GAG AAC CTA GAG ACA ACC 3953
   Val Ala Lys Ala Val Asp Phe Ile Pro Val Glu Asn Leu Glu Thr Thr
   1190 1195 1200
   ATG AGA TCC CCG GTG TTC ACG GAC AAC TCC TCT CCA CCA GCA GTG CCC 4001
   Met Arg Ser Pro Val Phe Thr Asp Asn Ser Ser Pro Pro Ala Val Pro
   1205 1210 1215 1220
   CAG AGC TTC CAG GTG GCC CAC CTG CAT GCT CCC ACC GGC AGC GGT AAG 4049
   Gln Ser Phe Gln Val Ala His Leu His Ala Pro Thr Gly Ser Gly Lys
   1225 1230 1235
   AGC ACC AAG GTC CCG GCT GCG TAC GCA GCC AAG GGC TAC AAG GTG TTG 4097
   Ser Thr Lys Val Pro Ala Ala Tyr Ala Ala Lys Gly Tyr Lys Val Leu
   1240 1245 1250
   GTG CTC AAC CCC TCT GTT GCT GCA ACA CTG GGC TTT GGT GCT TAC ATG 4145
   Val Leu Asn Pro Ser Val Ala Ala Thr Leu Gly Phe Gly Ala Tyr Met
   1255 1260 1265
   TCC AAG GCC CAT GGG GTT GAT CCT AAT ATC AGG ACC GGG GTG AGA ACA 4193
   Ser Lys Ala His Gly Val Asp Pro Asn Ile Arg Thr Gly Val Arg Thr
   1270 1275 1280
   ATT ACC ACT GGC AGC CCC ATC ACG TAC TCC ACC TAC GGC AAG TTC CTT 4241
   Ile Thr Thr Gly Ser Pro Ile Thr Tyr Ser Thr Tyr Gly Lys Phe Leu
   1285 1290 1295 1300
   GCC GAC GCC GGG TGC TCA GGA GGT GCT TAT GAC ATA ATA ATT TGT GAC 4289
   Ala Asp Ala Gly Cys Ser Gly Gly Ala Tyr Asp Ile Ile Ile Cys Asp
   1305 1310 1315
   GAG TGC CAC TCC ACG GAT GCC ACA TCC ATC TCG GGC ATC GGC ACT GTC 4337
   Glu Cys His Ser Thr Asp Ala Thr Ser Ile Ser Gly Ile Gly Thr Val
   1320 1325 1330
   CTT GAC CAA GCA GAG ACT GCG GGG GCG AGA CTG GTT GTG CTC GCC ACT 4385
   Leu Asp Gln Ala Glu Thr Ala Gly Ala Arg Leu Val Val Leu Ala Thr
   1335 1340 1345
   GCT ACC CCT CCG GGC TCC GTC ACT GTG TCC CAT CCT AAC ATC GAG GAG 4433
   Ala Thr Pro Pro Gly Ser Val Thr Val Ser His Pro Asn Ile Glu Glu
   1350 1355 1360
   GTT GCT CTG TCC ACC ACC GGA GAG ATC CCC TTT TAC GGC AAG GCT ATC 4481
   Val Ala Leu Ser Thr Thr Gly Glu Ile Pro Phe Tyr Gly Lys Ala Ile
   1365 1370 1375 1380
   CCC CTC GAG GTG ATC AAG GGG GGA AGA CAT CTC ATC TTC TGC CAC TCA 4529
   Pro Leu Glu Val Ile Lys Gly Gly Arg His Leu Ile Phe Cys His Ser
   1385 1390 1395
   AAG AAG AAG TGC GAC GAG CTC GCC GCG AAG CTG GTC GCA TTG GGC ATC 4577
   Lys Lys Lys Cys Asp Glu Leu Ala Ala Lys Leu Val Ala Leu Gly Ile
   1400 1405 1410
   AAT GCC GTG GCC TAC TAC CGC GGT CTT GAC GTG TCT GTC ATC CCG ACC 4625
   Asn Ala Val Ala Tyr Tyr Arg Gly Leu Asp Val Ser Val Ile Pro Thr
   1415 1420 1425
   AGC GGC GAT GTT GTC GTC GTG TCG ACC GAT GCT CTC ATG ACT GGC TTT 4673
   Ser Gly Asp Val Val Val Val Ser Thr Asp Ala Leu Met Thr Gly Phe
   1430 1435 1440
   ACC GGC GAC TTC GAC TCT GTG ATA GAC TGC AAC ACG TGT GTC ACT CAG 4721
   Thr Gly Asp Phe Asp Ser Val Ile Asp Cys Asn Thr Cys Val Thr Gln
   1445 1450 1455 1460
   ACA GTC GAT TTT AGC CTT GAC CCT ACC TTT ACC ATT GAG ACA ACC ACG 4769
   Thr Val Asp Phe Ser Leu Asp Pro Thr Phe Thr Ile Glu Thr Thr Thr
   1465 1470 1475
   CTC CCC CAG GAT GCT GTC TCC AGG ACT CAA CGC CGG GGC AGG ACT GGC 4817
   Leu Pro Gln Asp Ala Val Ser Arg Thr Gln Arg Arg Gly Arg Thr Gly
   1480 1485 1490
   AGG GGG AAG CCA GGC ATC TAT AGA TTT GTG GCA CCG GGG GAG CGC CCC 4865
   Arg Gly Lys Pro Gly Ile Tyr Arg Phe Val Ala Pro Gly Glu Arg Pro
   1495 1500 1505
   TCC GGC ATG TTC GAC TCG TCC GTC CTC TGT GAG TGC TAT GAC GCG GGC 4913
   Ser Gly Met Phe Asp Ser Ser Val Leu Cys Glu Cys Tyr Asp Ala Gly
   1510 1515 1520
   TGT GCT TGG TAT GAG CTC ACG CCC GCC GAG ACT ACA GTT AGG CTA CGA 4961
   Cys Ala Trp Tyr Glu Leu Thr Pro Ala Glu Thr Thr Val Arg Leu Arg
   1525 1530 1535 1540
   GCG TAC ATG AAC ACC CCG GGG CTT CCC GTG TGC CAG GAC CAT CTT GGA 5009
   Ala Tyr Met Asn Thr Pro Gly Leu Pro Val Cys Gln Asp His Leu Gly
   1545 1550 1555
   TTT TGG GAG GGC GTC TTT ACG GGC CTC ACT CAT ATA GAT GCC CAC TTT 5057
   Phe Trp Glu Gly Val Phe Thr Gly Leu Thr His Ile Asp Ala His Phe
   1560 1565 1570
   CTA TCC CAG ACA AAG CAG AGT GGG GAG AAC TTT CCT TAC CTG GTA GCG 5105
   Leu Ser Gln Thr Lys Gln Ser Gly Glu Asn Phe Pro Tyr Leu Val Ala
   1575 1580 1585
   TAC CAA GCC ACC GTG TGC GCT AGG GCT CAA GCC CCT CCC CCA TCG TGG 5153
   Tyr Gln Ala Thr Val Cys Ala Arg Ala Gln Ala Pro Pro Pro Ser Trp
   1590 1595 1600
   GAC CAG ATG CGG AAG TGT TTG ATC CGC CTT AAA CCC ACC CTC CAT GGG      5201
   Asp Gln Met Arg Lys Cys Leu Ile Arg Leu Lys Pro Thr Leu His Gly
   1605                1610                1615                1620
   CCA ACA CCC CTG CTA TAC AGA CTG GGC GCT GTT CAG AAT GAA GTC ACC      5249
   Pro Thr Pro Leu Leu Tyr Arg Leu Gly Ala Val Gln Asn Glu Val Thr
                   1625                1630                1635
   CTG ACG CAC CCA ATC ACC AAA TAC ATC ATG ACA TGC ATG TCG GCC GAC      5297
   Leu Thr His Pro Ile Thr Lys Tyr Ile Met Thr Cys Met Ser Ala Asp
               1640                1645                1650
   CTG GAG GTC GTC ACG AGC ACC TGG GTG CTC GTT GGC GGC GTC CTG GCT      5345
   Leu Glu Val Val Thr Ser Thr Trp Val Leu Val Gly Gly Val Leu Ala
           1655                1660                1665
   GCT CTG GCC GCG TAT TGC CTG TCA ACA GGC TGC GTG GTC ATA GTG GGC      5393
   Ala Leu Ala Ala Tyr Cys Leu Ser Thr Gly Cys Val Val Ile Val Gly
       1670                1675                1680
   AGG ATC GTC TTG TCC GGG AAG CCG GCA ATT ATA CCT GAC AGG GAG GTT      5441
   Arg Ile Val Leu Ser Gly Lys Pro Ala Ile Ile Pro Asp Arg Glu Val
   1685                1690                1695                1700
   CTC TAC CAG GAG TTC GAT GAG ATG GAA GAG TGC TCT CAG CAC TTA CCG      5489
   Leu Tyr Gln Glu Phe Asp Glu Met Glu Glu Cys Ser Gln His Leu Pro
                   1705                1710                1715
   TAC ATC GAG CAA GGG ATG ATG CTC GCT GAG CAG TTC AAG CAG AAG GCC      5537
   Tyr Ile Glu Gln Gly Met Met Leu Ala Glu Gln Phe Lys Gln Lys Ala
               1720                1725                1730
   CTC GGC CTC CTG CAG ACC GCG TCC CGC CAT GCA GAG GTT ATC ACC CCT      5585
   Leu Gly Leu Leu Gln Thr Ala Ser Arg His Ala Glu Val Ile Thr Pro
           1735                1740                1745
   GCT GTC CAG ACC AAC TGG CAG AAA CTC GAG GTC TTT TGG GCG AAG CAC      5633
   Ala Val Gln Thr Asn Trp Gln Lys Leu Glu Val Phe Trp Ala Lys His
       1750                1755                1760
   ATG TGG AAT TTC ATC AGT GGG ATA CAA TAC TTG GCG GGC CTG TCA ACG      5681
   Met Trp Asn Phe Ile Ser Gly Ile Gln Tyr Leu Ala Gly Leu Ser Thr
   1765                1770                1775                1780
   CTG CCT GGT AAC CCC GCC ATT GCT TCA TTG ATG GCT TTT ACA GCT GCC      5729
   Leu Pro Gly Asn Pro Ala Ile Ala Ser Leu Met Ala Phe Thr Ala Ala
                   1785                1790                1795
   GTC ACC AGC CCA CTA ACC ACT GGC CAA ACC CTC CTC TTC AAC ATA TTG      5777
   Val Thr Ser Pro Leu Thr Thr Gly Gln Thr Leu Leu Phe Asn Ile Leu
               1800                1805                1810
   GGG GGG TGG GTG GCT GCC CAG CTC GCC GCC CCC GGT GCC GCT ACC GCC        5825
   Gly Gly Trp Val Ala Ala Gln Leu Ala Ala Pro Gly Ala Ala Thr Ala
           1815                1820                1825
   TTT GTG GGC GCT GGC TTA GCT GGC GCC GCA CTC GAC AGC GTT GGA CTG        5873
   Phe Val Gly Ala Gly Leu Ala Gly Ala Ala Leu Asp Ser Val Gly Leu
       1830                1835                1840
   GGG AAG GTC CTC GTG GAC ATT CTT GCA GGC TAT GGC GCG GGC GTG GCG        5921
   Gly Lys Val Leu Val Asp Ile Leu Ala Gly Tyr Gly Ala Gly Val Ala
   1845                1850                1855                1860
   GGA GCT CTT GTG GCA TTC AAG ATC ATG AGC GGT GAG GTC CCC TCC ACG        5969
   Gly Ala Leu Val Ala Phe Lys Ile Met Ser Gly Glu Val Pro Ser Thr
                   1865                1870                1875
   GAG GAC CTG GTC AAT CTG CTG CCC GCC ATC CTC TCA CCT GGA GCC CTT        6017
   Glu Asp Leu Val Asn Leu Leu Pro Ala Ile Leu Ser Pro Gly Ala Leu
               1880                1885                1890
   GCA GTC GGT GTG GTC TTT GCA TCA ATA CTG CGC CGG CGT GTT GGC CCG        6065
   Ala Val Gly Val Val Phe Ala Ser Ile Leu Arg Arg Arg Val Gly Pro
           1895                1900                1905
   GGC GAG GGG GCA GTG CAA TGG ATG AAC CGG CTA ATA GCC TTC GCC TCC        6113
   Gly Glu Gly Ala Val Gln Trp Met Asn Arg Leu Ile Ala Phe Ala Ser
       1910                1915                1920
   CGG GGG AAC CAT GTT TCC CCC ACA CAC TAC GTG CCG GAG AGC GAT GCA        6161
   Arg Gly Asn His Val Ser Pro Thr His Tyr Val Pro Glu Ser Asp Ala
   1925                1930                1935                1940
   GCC GCC CGC GTC ACT GCC ATA CTC AGC AGC CTC ACT GTA ACC CAG CTC        6209
   Ala Ala Arg Val Thr Ala Ile Leu Ser Ser Leu Thr Val Thr Gln Leu
                   1945                1950                1955
   CTG AGG CGA CTG CAT CAG TGG ATA AGC TCG GAG TGT ACC ACT CCA TGC        6257
   Leu Arg Arg Leu His Gln Trp Ile Ser Ser Glu Cys Thr Thr Pro Cys
               1960                1965                1970
   TCC GGT TCC TGG CTA AGG GAC ATC TGG GAC TGG ATA TGC GAG GTG CTG        6305
   Ser Gly Ser Trp Leu Arg Asp Ile Trp Asp Trp Ile Cys Glu Val Leu
           1975                1980                1985
   AGC GAC TTT AAG ACC TGG CTG AAA GCC AAG CTC ATG CCA CAA CTG CCT        6353
   Ser Asp Phe Lys Thr Trp Leu Lys Ala Lys Leu Met Pro Gln Leu Pro
       1990                1995                2000
   GGG ATT CCC TTT GTG TCC TGC CAG CGC GGG TAT AGG GGG GTC TGG CGA        6401
   Gly Ile Pro Phe Val Ser Cys Gln Arg Gly Tyr Arg Gly Val Trp Arg
   2005                2010                2015                2020
   GGA GAC GGC ATT ATG CAC ACT CGC TGC CAC TGT GGA GCT GAG ATC ACT        6449
   Gly Asp Gly Ile Met His Thr Arg Cys His Cys Gly Ala Glu Ile Thr
                   2025                2030                2035
   GGA CAT GTC AAA AAC GGG ACG ATG AGG ATC GTC GGT CCT AGG ACC TGC        6497
   Gly His Val Lys Asn Gly Thr Met Arg Ile Val Gly Pro Arg Thr Cys
               2040                2045                2050
   AAG AAC ATG TGG AGT GGG ACG TTC TTC ATT AAT GCC TAC ACC ACG GGC        6545
   Lys Asn Met Trp Ser Gly Thr Phe Phe Ile Asn Ala Tyr Thr Thr Gly
           2055                2060                2065
   CCC TGT ACT CCC CTT CCT GCG CCG AAC TAT AAG TTC GCG CTG TGG AGG        6593
   Pro Cys Thr Pro Leu Pro Ala Pro Asn Tyr Lys Phe Ala Leu Trp Arg
       2070                2075                2080
   GTG TCT GCA GAG GAA TAC GTG GAG ATA AGG CGG GTG GGG GAC TTC CAC        6641
   Val Ser Ala Glu Glu Tyr Val Glu Ile Arg Arg Val Gly Asp Phe His
   2085                2090                2095                2100
   TAC GTA TCG GGC ATG ACT ACT GAC AAT CTC AAA TGC CCG TGC CAG ATC        6689
   Tyr Val Ser Gly Met Thr Thr Asp Asn Leu Lys Cys Pro Cys Gln Ile
                   2105                2110                2115
   CCA TCG CCC GAA TTT TTC ACA GAA TTG GAC GGG GTG CGC CTA CAT AGG        6737
   Pro Ser Pro Glu Phe Phe Thr Glu Leu Asp Gly Val Arg Leu His Arg
               2120                2125                2130
   TTT GCG CCC CCT TGC AAG CCC TTG CTG CGG GAG GAG GTA TCT TTC AGA        6785
   Phe Ala Pro Pro Cys Lys Pro Leu Leu Arg Glu Glu Val Ser Phe Arg
           2135                2140                2145
   GTA GGA CTC CAC GAG TAC CCG GTG GGG TCG CAA TTA CCT TGC GAG CCC        6833
   Val Gly Leu His Glu Tyr Pro Val Gly Ser Gln Leu Pro Cys Glu Pro
       2150                2155                2160
   GAA CCG GAC GTA GCC GTG TTG ACG TCC ATG CTC ACT GAT CCC TCC CAT        6881
   Glu Pro Asp Val Ala Val Leu Thr Ser Met Leu Thr Asp Pro Ser His
   2165                2170                2175                2180
   ATA ACA GCA GAG GCG GCC GGG AGA AGG TTG GCG AGA GGG TCA CCC CCT        6929
   Ile Thr Ala Glu Ala Ala Gly Arg Arg Leu Ala Arg Gly Ser Pro Pro
                   2185                2190                2195
   TCT ATG GCC AGC TCC TCG GCT AGC CAG CTG TCC GCT CCA TCT CTC AAG        6977
   Ser Met Ala Ser Ser Ser Ala Ser Gln Leu Ser Ala Pro Ser Leu Lys
               2200                2205                2210
   GCA ACT TGC ACC GCC AAC CAT GAC TCC CCT GAG GCC GAG CTC ATA GAG        7025
   Ala Thr Cys Thr Ala Asn His Asp Ser Pro Asp Ala Glu Leu Ile Glu
           2215                2220                2225
   GCT AAC CTC CTG TGG AGG CAG GAG ATG GGC GGC AAC ATC ACC AGG GTT      7073
   Ala Asn Leu Leu Trp Arg Gln Glu Met Gly Gly Asn Ile Thr Arg Val
       2230                2235                2240
   GAG TCA GAG AAC AAA GTG GTG ATT CTG GAC TCC TTC GAT CCG CTT GTG      7121
   Glu Ser Glu Asn Lys Val Val Ile Leu Asp Ser Phe Asp Pro Leu Val
   2245                2250                2255                2260
   GCA GAG GAG GAT GAG CGG GAG GTC TCC GTA CCC GCA GAA ATT CTG CGG      7169
   Ala Glu Glu Asp Glu Arg Glu Val Ser Val Pro Ala Glu Ile Leu Arg
                   2265                2270                2275
   AAG TCT CGG AGA TTC GCC CCA GCC CTG CCC GTC TGG GCG CGG CCG GAC      7217
   Lys Ser Arg Arg Phe Ala Pro Ala Leu Pro Val Trp Ala Arg Pro Asp
               2280                2285                2290
   TAC AAC CCC CTG CTA GTA GAG ACG TGG AAA AAG CCT GAC TAC GAA CCA      7265
   Tyr Asn Pro Leu Leu Val Glu Thr Trp Lys Lys Pro Asp Tyr Glu Pro
           2295                2300                2305
   CCT GTG GTC CAT GGC TGC CCG CTA CCA CCT CCA CGG TCC CCT CCT GTG      7313
   Pro Val Val His Gly Cys Pro Leu Pro Pro Pro Arg Ser Pro Pro Val
       2310                2315                2320
   CCT CCG CCT CGG AAA AAG CGT ACG GTG GTC CTC ACC GAA TCA ACC CTA      7361
   Pro Pro Pro Arg Lys Lys Arg Thr Val Val Leu Thr Glu Ser Thr Leu
   2325                2330                2335                2340
   CCT ACT GCC TTG GCC GAG CTT GCC ACC AAA AGT TTT GGC AGC TCC TCA      7409
   Pro Thr Ala Leu Ala Glu Leu Ala Thr Lys Ser Phe Gly Ser Ser Ser
                   2345                2350                2355
   ACT TCC GGC ATT ACG GGC GAC AAT ACG ACA ACA TCC TCT GAG CCC GCC      7457
   Thr Ser Gly Ile Thr Gly Asp Asn Thr Thr Thr Ser Ser Glu Pro Ala
               2360                2365                2370
   CCT TCT GGC TGC CCC CCC GAC TCC GAC GTT GAG TCC TAT TCT TCC ATG      7505
   Pro Ser Gly Cys Pro Pro Asp Ser Asp Val Glu Ser Tyr Ser Ser Met
           2375                2380                2385
   CCC CCC CTG GAG GGG GAG CCT GGG GAT CCG GAT CTC AGC GAC GGG TCA      7553
   Pro Pro Leu Glu Gly Glu Pro Gly Asp Pro Asp Leu Ser Asp Gly Ser
       2390                2395                2400
   TGG TCG ACG GTC AGT AGT GGG GCC GAC ACG GAA GAT GTC GTG TGC TGC      7601
   Trp Ser Thr Val Ser Ser Gly Ala Asp Thr Glu Asp Val Val Cys Cys
   2405                2410                2415                2420
   TCA ATG TCT TAT TCC TGG ACA GGC GCA CTC GTC ACC CCG TGC GCT GCG      7649
   Ser Met Ser Tyr Ser Trp Thr Gly Ala Leu Val Thr Pro Cys Ala Ala
                   2425                2430                2435
   GAG GAA CAA AAA CTG CCC ATC AAC GCA CTG AGC AAC TCG TTG CTA CGC      7697
   Glu Glu Gln Lys Leu Pro Ile Asn Ala Leu Ser Asn Ser Leu Leu Arg
               2440                2445                2450
   CAT CAC AAT CTG GTG TAT TCC ACC ACT TCA CGC AGT GCT TGC CAA AGG      7745
   His His Asn Leu Val Tyr Ser Thr Thr Ser Arg Ser Ala Cys Gln Arg
           2455                2460                2465
   AAG AAG AAA GTC ACA TTT GAC AGA CTG CAA GTT CTG GAC AGC CAT TAC      7793
   Lys Lys Lys Val Thr Phe Asp Arg Leu Gln Val Leu Asp Ser His Tyr
       2470                2475                2480
   CAG GAC GTG CTC AAG GAG GTC AAA GCA GCG GCG TCA AAA GTG AAG GCT      7841
   Gln Asp Val Leu Lys Glu Val Lys Ala Ala Ala Ser Lys Val Lys Ala
   2485                2490                2495                2500
   AAC TTG CTA TCC GTA GAG GAA GCT TGC AGC CTG GCG CCC CCA CAT TCA      7889
   Asn Leu Leu Ser Val Glu Glu Ala Cys Ser Leu Ala Pro Pro His Ser
                   2505                2510                2515
   GCC AAA TCC AAG TTT GGC TAT GGG GCA AAA GAC GTC CGT TGC CAT GCC      7937
   Ala Lys Ser Lys Phe Gly Tyr Gly Ala Lys Asp Val Arg Cys His Ala
               2520                2525                2530
   AGA AAG GCC GTA GCC CAC ATC AAC TCC GTG TGG AAA GAC CTT CTG GAA      7985
   Arg Lys Ala Val Ala His Ile Asn Ser Val Trp Lys Asp Leu Leu Glu
           2535                2540                2545
   GAC AGT GTA ACA CCA ATA GAC ACT ACC ATC ATG GCC AAG AAC GAG GTT      8033
   Asp Ser Val Thr Pro Ile Asp Thr Thr Ile Met Ala Lys Asn Glu Val
       2550                2555                2560
   TTC TGC GTT CAG CCT GAG AAG GGG GGT CGT AAG CCA GCT CGT CTC ATC      8081
   Phe Cys Val Gln Pro Glu Lys Gly Gly Arg Lys Pro Ala Arg Leu Ile
   2565                2570                2575                2580
   GTG TTC CCC GAC CTG GGC GTG CGC GTG TGC GAG AAG ATG GCC CTG TAC      8129
   Val Phe Pro Asp Leu Gly Val Arg Val Cys Glu Lys Met Ala Leu Tyr
                   2585                2590                2595
   GAC GTG GTT AGC AAG CTC CCC TTG GCC GTG ATG GGA AGC TCC TAC GGA      8177
   Asp Val Val Ser Lys Leu Pro Leu Ala Val Met Gly Ser Ser Tyr Gly
               2600                2605                2610
   TTC CAA TAC TCA CCA GGA CAG CGG GTT GAA TTC CTC GTG CAA GCG TGG      8225
   Phe Gln Tyr Ser Pro Gly Gln Arg Val Glu Phe Leu Val Gln Ala Trp
           2615                2620                2625
   AAG TCC AAG AAG ACC CCG ATG GGG CTC TCG TAT GAT ACC CGC TGT TTT      8273
   Lys Ser Lys Lys Thr Pro Met Gly Leu Ser Tyr Asp Thr Arg Cys Phe
       2630                2635                2640
   GAC TCC ACA GTC ACT GAG AGC GAC ATC CGT ACG GAG GAG GCA ATT TAC        8321
   Asp Ser Thr Val Thr Glu Ser Asp Ile Arg Thr Glu Glu Ala Ile Tyr
   2645                2650                2655                2660
   CAA TGT TGT GAC CTG GAC CCC CAA GCC CGC GTG GCC ATC AAG TCC CTC        8369
   Gln Cys Cys Asp Leu Asp Pro Gln Ala Arg Val Ala Ile Lys Ser Leu
                   2665                2670                2675
   ACT GAG AGG CTT TAT GTT GGG GGC CCT CTT ACT AAT TCA AGG GGG GAA        8417
   Thr Glu Arg Leu Tyr Val Gly Gly Pro Leu Thr Asn Ser Arg Gly Glu
               2680                2685                2690
   AAC TGC GGC TAC CGC AGG TGC CGC GCG AGC AGA GTA CTG ACA ACT AGC        8465
   Asn Cys Gly Tyr Arg Arg Cys Arg Ala Ser Arg Val Leu Thr Thr Ser
           2695                2700                2705
   TGT GGT AAC ACC CTC ACT CGC TAC ATC AAG GCC CGG GCA GCC TGT CGA        8513
   Cys Gly Asn Thr Leu Thr Arg Tyr Ile Lys Ala Arg Ala Ala Cys Arg
       2710                2715                2720
   GCC GCA GGG CTC CAG GAC TGC ACC ATG CTC GTG TGT GGC GAC GAC TTA        8561
   Ala Ala Gly Leu Gln Asp Cys Thr Met Leu Val Cys Gly Asp Asp Leu
   2725                2730                2735                2740
   GTC GTT ATC TGT GAA AGT GCG GGG GTC CAG GAG GAC GCG GCG AGC CTG        8609
   Val Val Ile Cys Glu Ser Ala Gly Val Gln Glu Asp Ala Ala Ser Leu
                   2745                2750                2755
   AGA GCC TTC ACG GAG GCT ATG ACC AGG TAC TCC GCC CCC CCC GGG GAC        8657
   Arg Ala Phe Thr Glu Ala Met Thr Arg Tyr Ser Ala Pro Pro Gly Asp
               2760                2765                2770
   CCC CCA CAA CCA GAA TAC GAC TTG GAG CTT ATA ACA TCA TGC TCC TCC        8705
   Pro Pro Gln Pro Glu Tyr Asp Leu Glu Leu Ile Thr Ser Cys Ser Ser
           2775                2780                2785
   AAC GTG TCA GTC GCC CAC GAC GGC GCT GGA AAG AGG GTC TAC TAC CTT        8753
   Asn Val Ser Val Ala His Asp Gly Ala Gly Lys Arg Val Tyr Tyr Leu
       2790                2795                2800
   ACC CGT GAC CCT ACA ACC CCC CTC GCG AGA GCC GCG TGG GAG ACA GCA        8801
   Thr Arg Asp Pro Thr Thr Pro Leu Ala Arg Ala Ala Trp Glu Thr Ala
   2805                2810                2815                2820
   AGA CAC ACT CCA GTC AAT TCC TGG CTA GGC AAC ATA ATC ATG TTT GCC        8849
   Arg His Thr Pro Val Asn Ser Trp Leu Gly Asn Ile Ile Met Phe Ala
                   2825                2830                2835
   CCC ACA CTG TGG GCG AGG ATG ATA CTG ATG ACC CAC TTC TTT AGC GTC        8897
   Pro Thr Leu Trp Ala Arg Met Ile Leu Met Thr His Phe Phe Ser Val
               2840                2845                2850
   CTC ATA GCC AGG GAT CAG CTT GAA CAG GCT CTC AAC TGC GAG ATC TAC      8945
   Leu Ile Ala Arg Asp Gln Leu Glu Gln Ala Leu Asn Cys Glu Ile Tyr
           2855                2860                2865
   GGA GCC TGC TAC TCC ATA GAA CCA CTG GAT CTA CCT CCA ATC ATT CAA      8993
   Gly Ala Cys Tyr Ser Ile Glu Pro Leu Asp Leu Pro Pro Ile Ile Gln
       2870                2875                2880
   AGA CTC CAT GGC CTC AGC GCA TTT TCA CTC CAC AGT TAC TCT CCA GGT      9041
   Arg Leu His Gly Leu Ser Ala Phe Ser Leu His Ser Tyr Ser Pro Gly
   2885                2890                2895                2900
   GAA ATT AAT AGG GTG GCC GCA TGC CTC AGA AAA CTT GGG GTC CCG CCC      9089
   Glu Ile Asn Arg Val Ala Ala Cys Leu Arg Lys Leu Gly Val Pro Pro
                   2905                2910                2915
   TTG CGA GCT TGG AGA CAC CGG GCC TGG AGC GTC CGC GCT AGG CTT CTG      9137
   Leu Arg Ala Trp Arg His Arg Ala Trp Ser Val Arg Ala Arg Leu Leu
               2920                2925                2930
   GCC AGA GGA GGC AAG GCT GCC ATA TGT GGC AAG TAC CTC TTC AAC TGG      9185
   Ala Arg Gly Gly Lys Ala Ala Ile Cys Gly Lys Tyr Leu Phe Asn Trp
           2935                2940                2945
   GCA GTA AGA ACA AAG CTC AAA CTC ACT CCG ATA ACG GCC GCT GGC CGG      9233
   Ala Val Arg Thr Lys Leu Lys Leu Thr Pro Ile Thr Ala Ala Gly Arg
       2950                2955                2960
   CTG GAC TTG TCC GGC TGG TTC ACG GCT GGC TAC AGC GGG GGA GAC ATT      9281
   Leu Asp Leu Ser Gly Trp Phe Thr Ala Gly Tyr Ser Gly Gly Asp Ile
   2965                2970                2975                2980
   TAT CAC AGC GTG TCT CAT GCC CGG CCC CGC TGG TTC TGG TTT TGC CTA      9329
   Tyr His Ser Val Ser His Ala Arg Pro Arg Trp Phe Trp Phe Cys Leu
                   2985                2990                2995
   CTC CTG CTT GCT GCA GGG GTA GGC ATC TAC CTC CTC CCC AAC CGA          9374
   Leu Leu Leu Ala Ala Gly Val Gly Ile Tyr Leu Leu Pro Asn Arg
               3000                3005                3010
   TGAAGATTGG GCTAACCACT CCAGGCCAAT AGGCCATTCC CT                       9416
   ***
(2) INFORMATION FOR SEQ ID NO:47:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:47:
      CAGCCCCCTG ATGGGGGCGA C 21
(2) INFORMATION FOR SEQ ID NO:48:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:48:
      ACTCGCAAGC ACCCTATCA 19
(2) INFORMATION FOR SEQ ID NO:49:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:495:
      CTGTGAGGAA CTACTGTCT 19
(2) INFORMATION FOR SEQ ID NO:50:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:50:
      ATGAGCACGA ATCCTCAAAC CT 22
(2) INFORMATION FOR SEQ ID NO:51:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:51:
      GTCCTGCCCT CGGGCC 16
(2) INFORMATION FOR SEQ ID NO:52:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:52:
      CGAGGAAGACTTCCGAGC 18
(2) INFORMATION FOR SEQ ID NO:53:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:53:
      ACCCAAATTG CGCGACCTAC 20
(2) INFORMATION FOR SEQ ID NO:54:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:54:
      TAAGGTCATC GATACCCT 18
(2) INFORMATION FOR SEQ ID NO:55:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:55:
      CAGTTCATCA TCATATCCCA 20
(2) INFORMATION FOR SEQ ID NO:56:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:56:
      GATAGAGAA AGAGCAAC 18
(2) INFORMATION FOR SEQ ID NO:57:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:57:
      AGACTTCCGA GCGGTCGCAA 20
(2) INFORMATION FOR SEQ ID NO:58:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO (xi) SEQUENCE DESCRIPTION: SEQ ID NO:58:
      GACCTGTGCG GGTCTGTC 18
(2) INFORMATION FOR SEQ ID NO:59:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:59:
      GGGTCGGCAG CTGGCTAGCC TCTCA 25
(2) INFORMATION FOR SEQ ID NO:60:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:60:
      TCCTGGCGGG CATAGCGT 18
(2) INFORMATION FOR SEQ ID NO:61:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:61:
      CCCCAGCCCT GGTCAAAATC GGTAA 25
(2) INFORMATION FOR SEQ ID NO:62:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:62:

   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:63:
      CTCGGTCG TTCCCACCA 19
(2) INFORMATION FOR SEQ ID NO:64:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:64:
      CCGCGAAGAG TGTGTGTGGT 20
(2) INFORMATION FOR SEQ ID NO:65:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:65:
      CAATGTTCTG GTGGAGGTG 19
(2) INFORMATION FOR SEQ ID NO:66:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:66:
      GCCATTAAGT GGGAGTACGT CGTTCTCC 28
(2) INFORMATION FOR SEQ ID NO:67:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:67:
      CGAGGAAGGA TACAAGACC 19
(2) INFORMATION FOR SEQ ID NO:68:
   (i) SEQUENCE CHARACTERISTICS:
      (A), LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:68:
      TGCTTGTGGA TGATGCTACT 20
(2) INFORMATION FOR SEQ ID NO:69:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:69:
      CACACGTGCA GTTGCGCT 18
(2) INFORMATION FOR SEQ ID NO:70:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:70:
      CTGCTGACCA CTACACAG 18
(2) INFORMATION FOR SEQ ID NO:71:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO (xi) SEQUENCE DESCRIPTION: SEQ ID NO:71:
      GACCAGAGTG GAAGCGCAA 19
(2) INFORMATION FOR SEQ ID NO:72:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:72:
      TACCAGAGTC GGGTGTACAG 20
(2) INFORMATION FOR SEQ ID NO:73:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:73:
      CTAGGAGGCC CCTTGTCTGC 20
(2) INFORMATION FOR SEQ ID NO:74:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:74:
      CTCGGGCCAG CCGATGGA 18
(2) INFORMATION FOR SEQ ID NO:75:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:75:
      GGGGACCTCA TGGTTGTCT 19
(2) INFORMATION FOR SEQ ID NO:76:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:76:
      CCCGTGGAGT GGCTAAGG 18
(2) INFORMATION FOR SEQ ID NO:77:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:77:
      CTCCTCGATG TTGGGATGG 19
(2) INFORMATION FOR SEQ ID NO:78:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:78:
      CAGAGCTTCC AGGTGGCTC 19
(2) INFORMATION FOR SEQ ID NO:79:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:79:
      CGGGCTCCGT CACTGTG 17
(2) INFORMATION FOR SEQ ID NO:80:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:80:
      GTATTGCAGT CTATCACCGA G 21
(2) INFORMATION FOR SEQ ID NO:81:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:81:
      GGCTATACCG GCGACTTCGA 20
(2) INFORMATION FOR SEQ ID NO:82:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:82:
      CGTTGAGTGC GGGAGACAG 19
(2) INFORMATION FOR SEQ ID NO:83:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:83:
      TCACCATTGA GACAATCACG 20
(2) INFORMATION FOR SEQ ID NO:84:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:84:
      GTAAGGAAGG TTCTCCCCAC TC 22
(2) INFORMATION FOR SEQ ID NO:85:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:85:
      ATGCCCACTT TCTATCCCAG ACAAAGC 27
(2) INFORMATION FOR SEQ ID NO:86:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:86:
      TGCATGTCAT GATGTAT 17
(2) INFORMATION FOR SEQ ID NO:87:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:87:
      GGACAAGACG ACCCTGCC 18
(2) INFORMATION FOR SEQ ID NO:88:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO (xi) SEQUENCE DESCRIPTION: SEQ ID NO:88:
      CGTATTGCCT GTCAACAGGC 20
(2) INFORMATION FOR SEQ ID No:89:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:89:
      ACCGCCCACA AAGGCAGTAG 20
(2) INFORMATION FOR SEQ ID NO:90:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:90:
(2) INFORMATION FOR SEQ ID NO:91:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES (xi) SEQUENCE DESCRIPTION: SEQ ID NO:91:
      CCAGGAACCG GAGCATGG 18
(2) INFORMATION FOR SEQ ID NO:92:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:92:
      ACCAGTGGAT AAGCTCGG 18
(2) INFORMATION FOR SEQ ID NO:93:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:93:
      CGTGGTGTAG GCATTAATG 19
(2) INFORMATION FOR SEQ ID NO:94:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:94:
      ATGTGGAGTG GGACCTTCC 19
(2) INFORMATION FOR SEQ ID NO:95:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES (xi) SEQUENCE DESCRIPTION: SEQ ID NO:95:
      CTCTGCTGTT ATATGGGAGG 20
(2) INFORMATION FOR SEQ ID NO:96:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:96:
      GTTGACGTCC ATGCTCACTG 20
(2) INFORMATION FOR SEQ ID NO:97:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:97:
      TTTCCACGTC TCCACTAGCG 20
(2) INFORMATION FOR SEQ ID NO:98:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:98:
      GTGAGGACCA CCGTCCGC 18
(2) INFORMATION FOR SEQ ID NO:99:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:99:
      TTCCACCTCC AAAGTCCCCT 20
(2) INFORMATION FOR SEQ ID NO:100:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:100:
      AGAACTTGCA GTCTGTCAAA TGTGA 25
(2) INFORMATION FOR SEQ ID NO:101:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:101:
      GGAAGAACAG AAACTGCCCA TCAATGCACT AAGC 34
(2) INFORMATION FOR SEQ ID NO:102:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:102:
      TGACGCCGCT GCTTTAACCT 20
(2) INFORMATION FOR SEQ ID NO:103:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES (xi) SEQUENCE DESCRIPTION: SEQ ID NO:103:
      TGCAAGCTTC CTCTACGGAT 20
(2) INFORMATION FOR SEQ ID NO:104:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:104:
      AGGTTAAAGC AGCGGCGTCA 20
(2) INFORMATION FOR SEQ ID NO:105:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:105:
      AGCTTCCCAT CACGGCCAA 19
(2) INFORMATION FOR SEQ ID NO:106:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:106:
      GATGGCTTTG TACGACGTG 19
(2) INFORMATION FOR SEQ ID NO:107:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:107:
      GCACCTGCGA TAGCCGCAGT 20
(2) INFORMATION FOR SEQ ID NO:108:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:108:
      GTCCCTCACC GAGAGGCT 18
(2) INFORMATION FOR SEQ ID NO:109:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:109:
      GATTGGAGGT AGATCAAGTG 20
(2) INFORMATION FOR SEQ ID NO:110:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:110:
      TACGACTTGG AGCTCATAAC 20
(2) INFORMATION FOR SEQ ID NO:111:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:111:
      AGCAAGACAC ACTCCAGTCA 20
(2) INFORMATION FOR SEQ ID NO:112:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:112:
      GCCTATTGGC CTGGAGTGGTTAGC 24
(2) INFORMATION FOR SEQ ID NO:113:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide
   (xi) SEQUENCE DESCRIPTION: SEQ ID NO:113:
   His Val Thr Gly Gly Asn Ala Gly Arg Thr Thr Ala Gly Leu Val Gly
   1               5                   10                  15
   Leu Leu Thr Pro Gly Ala Lys Gln Asn Ile
               20                  25
(2) INFORMATION FOR SEQ ID NO:114:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide
   (xi) SEQUENCE DESCRIPTION: SEQ ID NO:114:
   His Val Thr Gly Gly Ser Ala Gly His Thr Val Ser Gly Phe Val Ser
   1 5 10 15
   Leu Leu Ala Pro Gly Ala Lys Gln Asn Val
   20 25
(2) INFORMATION FOR SEQ ID NO:115:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide
   (xi) SEQUENCE DESCRIPTION: SEQ ID NO:115:
   His Val Thr Gly Gly Gln Ala Ala Arg Ala Met Ser Gly Leu Val Ser
   1 5 10 15
   Leu Phe Thr Pro Gly Ala Lys Gln Asn Ile
   20 25
(2) INFORMATION FOR SEQ ID NO:116:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide
   (xi) SEQUENCE DESCRIPTION: SEQ ID NO:116:
   His Val Thr Gly Gly Arg Val Ala Ser Ser Thr Gln Ser Leu Val Ser
   1 5 10 15
   Trp Leu Ser Gln Gly Pro Ser Gln Lys Ile
   20 25
(2) INFORMATION FOR SEQ ID NO:117:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide
   (xi) SEQUENCE DESCRIPTION: SEQ ID NO:117:
   His Val Thr Gly Gly Ala Gln Ala Lys Thr Thr Asn Arg Leu Val Ser
   1               5                   10                  15
   Met Phe Ala Ser Gly Pro Ser Gln Lys Ile
               20                  25
(2) INFORMATION FOR SEQ ID NO:118:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide
   (xi) SEQUENCE DESCRIPTION: SEQ ID NO:118:
   Tyr Thr Ser Gly Gly Ala Ala Ser His Thr Thr Ser Thr Leu Ala Ser
   1               5                   10                  15
   Leu Phe Ser Pro Gly Ala Ser Arg Asn Ile
               20                  25
(2) INFORMATION FOR SEQ-ID NO:119:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide
   (xi) SEQUENCE DESCRIPTION: SEQ ID NO:119:
(2) INFORMATION FOR SEQ ID NO:120:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide
   (xi) SEQUENCE DESCRIPTION: SEQ ID NO:120:
   His Val Thr Gly Gly Ser Ala Gly Arg Thr Thr Ala Gly Leu Val Gly
   1 5 10 15
   Leu Leu Thr Pro Gly Ala Lys Gln Asn Ile
   20 25
(2) INFORMATION FOR SEQ ID NO:121:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide
   (xi) SEQUENCE DESCRIPTION: SEQ ID NO:121:
   His Val Thr Gly Gly Ser Ala Gly Arg Ser Val Leu Gly Ile Ala Ser
   1 5 10 15
   Phe Leu Thr Arg Gly Pro Lys Gln Asn Ile
   20 25
(2) INFORMATION FOR SEQ ID NO:122:
   (i) SEQUENCE CHARACTERISTICS:
      (A) LENGTH: 30 amino acids
      (B) TYPE: amino acid
      (D) TOPOLOGY: linear
   (ii) MOLECULE TYPE: peptide
   (xi) SEQUENCE DESCRIPTION: SEQ ID NO:122:
   Val Ala Thr Arg Asp Gly Lys Leu Pro Thr Thr Gln Leu Arg Arg His
   1 5 10 15
   Ile Asp Leu Leu Val Gly Ser Ala Thr Leu Cys Ser Ala Leu
   20 25 30
(2) INFORMATION FOR SEQ ID NO:123:
   (i) SEQUENCE CHARACTERISTICS:
      (A) LENGTH: 30 amino acids
      (B) TYPE: amino acid
      (D) TOPOLOGY: linear
   (ii) MOLECULE TYPE: peptide
   (xi) SEQUENCE DESCRIPTION: SEQ ID NO:123:
   Val Ala Thr Arg Asp Gly Lys Leu Pro Ala Thr Gln Leu Arg Arg His
   1               5                   10                  15
   Ile Asp Leu Leu Val Gly Ser Ala Thr Leu Cys Ser Ala Leu
               20                  25                  30
(2) INFORMATION FOR SEQ ID NO:124:
   (i) SEQUENCE CHARACTERISTICS:
      (A) LENGTH: 30 amino acids
      (B) TYPE: amino acid
      (D) TOPOLOGY: linear
   (ii) MOLECULE TYPE: peptide (xi) SEQUENCE DESCRIPTION: SEQ ID NO:124:
(2) INFORMATION FOR SEQ ID NO:125:
   (i) SEQUENCE CHARACTERISTICS:
      (A) LENGTH: 30 amino acids
      (B) TYPE: amino acid
      (D) TOPOLOGY: linear
   (ii) MOLECULE TYPE: peptide
   (xi) SEQUENCE DESCRIPTION: SEQ ID NO:125:
   Leu Ala Ala Arg Asn Val Thr Ile Pro Thr Thr Thr Ile Arg Arg His
   1               5                   10                  15
   Val Asp Leu Leu Val Gly Ala Ala Ala Phe Cys Ser Ala Met
               20                  25                  30
(2) INFORMATION FOR SEQ ID NO:126:
   (i) SEQUENCE CHARACTERISTICS:
      (A) LENGTH: 30 amino acids
      (B) TYPE: amino acid
      (D) TOPOLOGY: linear
   (ii) MOLECULE TYPE: peptide
   (xi) SEQUENCE DESCRIPTION: SEQ ID NO:126:
   Leu Ala Ala Arg Asn Ala Ser Val Pro Thr Thr Thr Ile Arg Arg His
   1 5 10 15
   Val Asp Leu Leu Val Gly Ala Ala Ala Phe Cys Ser Ala Met
   20 25 30
(2) INFORMATION FOR SEQ ID NO:127:
   (i) SEQUENCE CHARACTERISTICS:
      (A) LENGTH: 30 amino acids
      (B) TYPE: amino acid
      (D) TOPOLOGY: linear
   (ii) MOLECULE TYPE: peptide (xi) SEQUENCE DESCRIPTION: SEQ ID NO:127:
(2) INFORMATION FOR SEQ ID NO:128:
   (i) SEQUENCE CHARACTERISTICS:
      (A) LENGTH: 27 amino acids
      (B) TYPE: amino acid
      (D) TOPOLOGY: linear (ii) MOLECULE TYPE: peptide (xi) SEQUENCE DESCRIPTION: SEQ ID NO:128:
(2) INFORMATION FOR SEQ ID NO:129:
   (i) SEQUENCE CHARACTERISTICS:
      (A) LENGTH: 27 amino acids
      (B) TYPE: amino acid
      (D) TOPOLOGY: linear
   (ii) MOLECULE TYPE: peptide
   (xi) SEQUENCE DESCRIPTION: SEQ ID NO:129:
   Leu Ala Ser Cys Arg Pro Leu Thr Asp Phe Asp Gln Gly Trp Gly Pro
   1 5 10 15
   Ile Ser Tyr Ala Asn Gly Ser Gly Pro Asp Gln
   20 25
(2) INFORMATION FOR SEQ ID NO:130:
   (i) SEQUENCE CHARACTERISTICS:
      (A) LENGTH: 27 amino acids
      (B) TYPE: amino acid
      (D) TOPOLOGY: linear
   (ii) MOLECULE TYPE: peptide
   (xi) SEQUENCE DESCRIPTION: SEQ ID NO:130:
   Leu Ala Ser Cys Arg Arg Leu Thr Asp Phe Asp Gln Gly Trp Gly Pro
   1 5 10 15
   Ile Ser His Ala Asn Gly Ser Gly Pro Asp Gln
   20 25
(2) INFORMATION FOR SEQ ID NO:131:
   (i) SEQUENCE CHARACTERISTICS:
      (A) LENGTH: 27 amino acids
      (B) TYPE: amino acid
      (D) TOPOLOGY: linear
   (ii) MOLECULE TYPE: peptide
   (xi) SEQUENCE DESCRIPTION: SEQ ID NO:131:
   Met Ala Ser Cys Arg Pro Ile Asp Glu Phe Ala Gln Gly Trp Gly Pro
   1 5 10 15
   Ile Thr His Asp Met Pro Glu Ser Ser Asp Gln
   20 25
(2) INFORMATION FOR SEQ ID NO:132:
   (i) SEQUENCE CHARACTERISTICS:
      (A) LENGTH: 27 amino acids
      (B) TYPE: amino acid
      (D) TOPOLOGY: linear
   (ii) MOLECULE TYPE: peptide
   (xi) SEQUENCE DESCRIPTION: SEQ ID NO:132:
   Met Ala Gln Cys Arg Thr Ile Asp Lys Phe Asp Gln Gly Trp Gly Pro
   1 5 10 15
   Ile Thr Tyr Ala Glu Ser Ser Arg Ser Asp Gln
   20 25
(2) INFORMATION FOR SEQ ID NO:122:
   (i) SEQUENCE CHARACTERISTICS:
      (A) LENGTH: 27 amino acids
      (B) TYPE: amino acid
      (D) TOPOLOGY: linear
   (ii) MOLECULE TYPE: peptide
   (xi) SEQUENCE DESCRIPTION: SEQ ID NO:133:
(2) INFORMATION FOR SEQ ID NO:134:
   (i) SEQUENCE CHARACTERISTICS:
      (A) LENGTH: 27 amino acids
      (B) TYPE: amino acid
      (D) TOPOLOGY: linear
   (ii) MOLECULE TYPE: peptide
   (xi) SEQUENCE DESCRIPTION: SEQ ID NO:134:
   Leu Ala Ser Cys Arg Arg Leu Thr Asp Phe Asp Gln Gly Trp Gly Pro
   1 5 10 15
   Ile Ser Tyr Ala Asn Gly Ser Gly Pro Asp Glu
   20 25
(2) INFORMATION FOR SEQ ID NO:135:
   (i) SEQUENCE CHARACTERISTICS:
      (A) LENGTH: 24 amino acids
      (B) TYPE: amino acid
      (D) TOPOLOGY: linear
   (ii) MOLECULE TYPE: peptide
   (xi) SEQUENCE DESCRIPTION: SEQ ID NO:135:
   Ser Thr Ser Gly Ile Thr Gly Asp Asn Thr Thr Thr Ser Ser Glu Pro
   1 5 10 15
   Ala Pro Ser Gly Cys Pro Pro Asp
   20
(2) INFORMATION FOR SEQ ID NO:136:
   (i) SEQUENCE CHARACTERISTICS:
      (A) LENGTH: 24 amino acids
      (B) TYPE: amino acid
      (D) TOPOLOGY: linear
   (ii) MOLECULE TYPE: peptide
   (xi) SEQUENCE DESCRIPTION: SEQ ID NO:136:
   Gly Ser Ser Ala Val Asp Ser Gly Thr Ala Thr Gly Pro Pro Asp Gln
   1 5 10 15
   Ala Ser Asp Asp Gly Asp Lys Gly
   20
(2) INFORMATION FOR SEQ ID NO:137:
   (I) SEQUENCE CHARACTERISTICS:
      (A) LENGTH: 24 amino acids
      (B) TYPE: amino acid
      (D) TOPOLOGY: linear
   (ii) MOLECULE TYPE: peptide
   (xi) SEQUENCE DESCRIPTION: SEQ ID NO:137:
   Glu Ser Ser Ala Val Asp Ser Gly Thr Ala Thr Ala Leu Pro Asp Gln
   1 5 10 15
   Ala Ser Asp Asp Gly Asp Lys Gly
   20

### SEQUENCE LISTING

(1) GENERAL INFORMATION:
   (i) APPLICANT: Zebedee, Suzanne
      Inchauspe, Genevieve
      Nasoff, Marc
      Prince, Alfred
   (ii) TITLE OF INVENTION: NON-A, NON-B HEPATITIS VIRUS ANTIGEN, DIAGNOSTIC METHODS AND VACCINES
   (iii) NUMBER OF SEQUENCES: 137
   (iv) CORRESPONDENCE ADDRESS:
      (A) ADDRESSEE: DRESSLER, GOLDSMITH, SHORE, SUTKER & MILNAMOW, LTD.
      (B) STREET: 180 N. Stetson, Suite 4700
      (C) CITY: Chicago
      (D) STATE: IL
      (E) COUNTRY: USA
      (F) ZIP: 60601
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patentln Release #1.0, Version #1.25
   (vi) CURRENT APPLICATION DATA:
      (A) APPLICATION NUMBER: US
      (B) FILING DATE:
      (C) CLASSIFICATION:
   (vii) PRIOR APPLICATION DATA:
      (A) APPLICATION NUMBER: US 07/616369
      (B) FILING DATE:21-NOV-1990
   (vii) PRIOR APPLICATION DATA:
      (A) APPLICATION NUMBER: US 07/573643
      (B) FILING DATE: 25-AUG-1990
   (viii) ATTORNEY/AGENT INFORMATION:
      (A) NAME: Gamson, Edward P.
      (B) REGISTRATION NUMBER: 29,381
      (C) REFERENCE/DOCKET NUMBER: PHA0029P
   (ix) TELECOMMUNICATION INFORMATION:
      (A) TELEPHONE: 312-616-5400
      (B) TELEFAX: 312-616-5460
(2) INFORMATION FOR SEQ ID NO:1:
   (i) SEQUENCE CHARACTERISTICS:
      (A) LENGTH: 978 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..978
      (D) OTHER INFORMATION: /codon start= 1 /product= "NANBV Structural Antigen" /number= 1
   (xi) SEQUENCE DESCRIPTION: SEQ ID NO:1:
   ATG AGC ACG ATT CCC AAA CCT CAA AGA AAA ACC AAA CGT AAC ACC AAC        48
   Met Ser Thr Ile Pro Lys Pro Gln Arg Lys Thr Lys Arg Asn Thr Asn
     1               5                  10                  15
   CGT CGC CCA CAG GAC GTC AAG TTC CCG GGT GGC GGT CAG ATC GTT GGT        96
   Arg Arg Pro Gln Asp Val Lys Phe Pro Gly Gly Gly Gln Ile Val Gly
                20                  25                  30
   GGA GTT TAC TTG TTG CCG CGC AGG GGC CCT AGA TTG GGT GTG CGC GCG       144
   Gly Val Tyr Leu Leu Pro Arg Arg Gly Pro Arg Leu Gly Val Arg Ala
            35                  40                  45
   ACG AGG AAG ACT TCC GAG CGG TCG CAA CCT CGA GGT AGA CGT CAG CCT       192
   Thr Arg Lys Thr Ser Glu Arg Ser Gln Pro Arg Gly Arg Arg Gln Pro
        50                  55                  60
   ATC CCC AAG GCA CGT CGG CCC GAG GGC AGG ACC TGG GCT CAG CCC GGG       240
   Ile Pro Lys Ala Arg Arg Pro Glu Gly Arg Thr Trp Ala Gln Pro Gly
    65                  70                  75                  80
   TAC CCT TGG CCC CTC TAT GGC AAT GAG GGT TGC GGG TGG GCG GGA TGG       288
   Tyr Pro Trp Pro Leu Tyr Gly Asn Glu Gly Cys Gly Trp Ala Gly Trp
                    85                  90                  95
   CTC CTG TCT CCC CGT GGC TCT CGG CCT AGC TGG GGC CCC ACA GAC CCC       336
   Leu Leu Ser Pro Arg Gly Ser Arg Pro Ser Trp Gly Pro Thr Asp Pro
               100                 105                 110
   CGG CGT AGG TCG CGC AAT TTG GGT AAG GTC ATC GAT ACC CTT ACG TGC      384
   Arg Arg Arg Ser Arg Asn Leu Gly Lys Val Ile Asp Thr Leu Thr Cys
           115                 120                 125
   GGC TTC GCC GAC CTC ATG GGG TAC ATA CCG CTC GTC GGC GCC CCT CTT      432
   Gly Phe Ala Asp Leu Met Gly Tyr Ile Pro Leu Val Gly Ala Pro Leu
       130                 135                 140
   GGA GGC GCT GCC AGG GCC CTG GCG CAT GGC GTC CGG GTT CTG GAA GAC      480
   Gly Gly Ala Ala Arg Ala Leu Ala His Gly Val Arg Val Leu Glu Asp
   145                 150                 155                 160
   GGC GTG AAC TAT GCA ACA GGG AAC CTT CCT GGT TGC TCT TTC TCT ATC      528
   Gly Val Asn Tyr Ala Thr Gly Asn Leu Pro Gly Cys Ser Phe Ser Ile
                   165                 170                 175
   TTC CTT CTG GCC CTG CTC TCT TGC CTG ACT GTG CCC GCT TCA GCC TAC      576
   Phe Leu Leu Ala Leu Leu Ser Cys Leu Thr Val Pro Ala Ser Ala Tyr
               180                 185                 190
   CAA GTG CGC AAT TCC TCG GGG CTT TAC CAT GTC ACC AAT GAT TGC CCT      624
   Gln Val Arg Asn Ser Ser Gly Leu Tyr His Val Thr Asn Asp Cys Pro
           195                 200                 205
   AAC TCG AGT GTT GTG TAC GAG GCG GCC GAT GCC ATC CTG CAC ACT CCG      672
   Asn Ser Ser Val Val Tyr Glu Ala Ala Asp Ala Ile Leu His Thr Pro
       210                 215                 220
   GGG TGT GTC CCT TGC GTT CGC GAG GGT AAC GCC TCG AGG TGT TGG GTG      720
   Gly Cys Val Pro Cys Val Arg Glu Gly Asn Ala Ser Arg Cys Trp Val
   225                 230                 235                 240
   GCG GTG ACC CCC ACG GTG GCC ACC AGG GAC GGC AAA CTT CCC ACA ACG      768
   Ala Val Thr Pro Thr Val Ala Thr Arg Asp Gly Lys Leu Pro Thr Thr
                   245                 250                 255
   CAG CTT CGA CGT CAT ATC GAT CTG CTT GTC GGG AGC GCC ACC CTC TGC      816
   Gln Leu Arg Arg His Ile Asp Leu Leu Val Gly Ser Ala Thr Leu Cys
               260                 265                 270
   TCG GCC CTC TAC GTG GGG GAC CTG TGC GGG TCT GTC TTT CTC GTT GGT      864
   Ser Ala Leu Tyr Val Gly Asp Leu Cys Gly Ser Val Phe Leu Val Gly
           275                 280                 285
   CAA CTG TTT ACC TTC TCT CCC AGG CGC CAC TGG ACG ACG CAA GAC TGC      912
   Gln Leu Phe Thr Phe Ser Pro Arg Arg His Trp Thr Thr Gln Asp Cys
       290                 295                 300
   AAT TGT TCT ATC TAT CCC GGC CAT ATA ACG GGT CAT CGC ATG GCA TGG      960
   Asn Cys Ser Ile Tyr Pro Gly His Ile Thr Gly His Arg Met Ala Trp
   305                 310                 315                 320
   GAT ATG ATG ATG AAC TGG                                              978
   Asp Met Met Met Asn Trp
                   325
(2) INFORMATION FOR SEQ ID NO:2:
   (i) SEQUENCE CHARACTERISTICS:
      (A) LENGTH: 948 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..945
   (xi) SEQUENCE DESCRIPTION: SEQ ID NO:2:
   ATG TCC CCT ATA CTA GGT TAT TGG AAA ATT AAG GGC CTT GTG CAA CCC        48
   Met Ser Pro Ile Leu Gly Tyr Trp Lys Ile Lys Gly Leu Val Gln Pro
     1               5                  10                  15
   ACT CGA CTT CTT TTG GAA TAT CTT GAA GAA AAA TAT GAA GAG CAT TTG        96
   Thr Arg Leu Leu Leu Glu Tyr Leu Glu Glu Lys Tyr Glu Glu His Leu
                20                  25                  30
   TAT GAG CGC GAT GAA GGT GAT AAA TGG CGA AAC AAA AAG TTT GAA TTG       144
   Tyr Glu Arg Asp Glu Gly Asp Lys Trp Arg Asn Lys Lys Phe Glu Leu
            35                  40                  45
   GGT TTG GAG TTT CCC AAT CTT CCT TAT TAT ATT GAT GGT GAT GTT AAA       192
   Gly Leu Glu Phe Pro Asn Leu Pro Tyr Tyr Ile Asp Gly Asp Val Lys
        50                  55                  60
   TTA ACA CAG TCT ATG GCC ATC ATA CGT TAT ATA GCT GAC AAG CAC AAC       240
   Leu Thr Gln Ser Met Ala Ile Ile Arg Tyr Ile Ala Asp Lys His Asn
    65                  70                  75                  80
   ATG TTG GGT GGT TGT CCA AAA GAG CGT GCA GAG ATT TCA ATG CTT GAA       288
   Met Leu Gly Gly Cys Pro Lys Glu Arg Ala Glu Ile Ser Met Leu Glu
                           85                  90                  95
   GGA GCG GTT TTG GAT ATT AGA TAC GGT GTT TCG AGA ATT GCA TAT AGT          336
   Gly Ala Val Leu Asp Ile Arg Tyr Gly Val Ser Arg Ile Ala Tyr Ser
               100                 105                 110
   AAA GAC TTT GAA ACT CTC AAA GTT GAT TTT CTT AGC AAG CTA CCT GAA          384
   Lys Asp Phe Glu Thr Leu Lys Val Asp Phe Leu Ser Lys Leu Pro Glu
           115                 120                 125
   ATG CTG AAA ATG TTC GAA GAT CGT TTA TGT CAT AAA ACA TAT TTA AAT          432
   Met Leu Lys Met Phe Glu Asp Arg Leu Cys His Lys Thr Tyr Leu Asn
       130                 135                 140
   GGT GAT CAT GTA ACC CAT CCT GAC TTC ATG TTG TAT GAC GCT CTT GAT          480
   Gly Asp His Val Thr His Pro Asp Phe Met Leu Tyr Asp Ala Leu Asp
   145                 150                 155                 160
   GTT GTT TTA TAC ATG GAC CCA ATG TGC CTG GAT GCG TTC CCA AAA TTA          528
   Val Val Leu Tyr Met Asp Pro Met Cys Leu Asp Ala Phe Pro Lys Leu
                   165                 170                 175
   GTT TGT TTT AAA AAA CGT ATT GAA GCT ATC CCA CAA ATT GAT AAG TAC          576
   Val Cys Phe Lys Lys Arg Ile Glu Ala Ile Pro Gln Ile Asp Lys Tyr
               180                 185                 190
   TTG AAA TCC AGC AAG TAT ATA GCA TGG CCT TTG CAG GGC TGG CAA GCC          624
   Leu Lys Ser Ser Lys Tyr Ile Ala Trp Pro Leu Gln Gly Trp Gln Ala
           195                 200                 205
   ACG TTT GGT GGT GGC GAC CAT CCT CCA AAA TCG GAT CTG ATC GAA GGT          672
   Thr Phe Gly Gly Gly Asp His Pro Pro Lys Ser Asp Leu Ile Glu Gly
       210                 215                 220
   CGT GGG ATC CCC AAT TCG AGC TCG GTA CCC ATG AGC ACG ATT CCC AAA          720
   Arg Gly Ile Pro Asn Ser Ser Ser Val Pro Met Ser Thr Ile Pro Lys
   225                 230                 235                 240
   CCT CAA AGA AAA ACC AAA CGT AAC ACC AAC CGT CGC CCA CAG GAC GTC          768
   Pro Gln Arg Lys Thr Lys Arg Asn Thr Asn Arg Arg Pro Gln Asp Val
                   245                 250                 255
   AAG TTC CCG GGT GGC GGT CAG ATC GTT GGT GGA GTT TAC TTG TTG CCG          816
   Lys Phe Pro Gly Gly Gly Gln Ile Val Gly Gly Val Tyr Leu Leu Pro
               260                 265                 270
   CGC AGG GGC CCT AGA TTG GGT GTG CGC GCG ACG AGG AAG ACT TCC GAG          864
   Arg Arg Gly Pro Arg Leu Gly Val Arg Ala Thr Arg Lys Thr Ser Glu
           275                 280                 285
   CGG TCG CAA CCT CGA GGT AGA CGT CAG CCT ATC CCC AAG GCA CGT CGG          912
   Arg Ser Gln Pro Arg Gly Arg Arg Gln Pro Ile Pro Lys Ala Arg Arg
       290                 295                 300
   CCC GAG GGC AGG ACG GGG ATC GGG AAT TCA TCG TGA                          948
   Pro Glu Gly Arg Thr Gly Ile Gly Asn Ser Ser
   305                 310                 315
(2) INFORMATION FOR SEQ ID NO:3:
   (i) SEQUENCE CHARACTERISTICS:
      (A) LENGTH: 759 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..756
   (xi) SEQUENCE DESCRIPTION: SEQ ID NO:3:
   ATG TCC CCT ATA CTA GGT TAT TGG AAA ATT AAG GGC CTT GTG CAA CCC      48
   Met Ser Pro Ile Leu Gly Tyr Trp Lys Ile Lys Gly Leu Val Gln Pro
    1               5                  10                  15
   ACT CGA CTT CTT TTG GAA TAT CTT GAA GAA AAA TAT GAA GAG CAT TTG      96
   Thr Arg Leu Leu Leu Glu Tyr Leu Glu Glu Lys Tyr Glu Glu His Leu
               20                  25                  30
   TAT GAG CGC GAT GAA GGT GAT AAA TGG CGA AAC AAA AAG TTT GAA TTG     144
   Tyr Glu Arg Asp Glu Gly Asp Lys Trp Arg Asn Lys Lys Phe Glu Leu
           35                  40                  45
   GGT TTG GAG TTT CCC AAT CTT CCT TAT TAT ATT GAT GGT GAT GTT AAA     192
   Gly Leu Glu Phe Pro Asn Leu Pro Tyr Tyr Ile Asp Gly Asp Val Lys
       50                  55                  60
   TTA ACA CAG TCT ATG GCC ATC ATA CGT TAT ATA GCT GAC AAG CAC AAC     240
   Leu Thr Gln Ser Met Ala Ile Ile Arg Tyr Ile Ala Asp Lys His Asn
   65                  70                  75                  80
   ATG TTG GGT GGT TGT CCA AAA GAG CGT GCA GAG ATT TCA ATG CTT GAA     288
   Met Leu Gly Gly Cys Pro Lys Glu Arg Ala Glu Ile Ser Met Leu Glu
                   85                  90                  95
   GGA GCG GTT TTG GAT ATT AGA TAC GGT GTT TCG AGA ATT GCA TAT AGT      336
   Gly Ala Val Leu Asp Ile Arg Tyr Gly Val Ser Arg Ile Ala Tyr Ser
               100                 105                 110
   AAA GAC TTT GAA ACT CTC AAA GTT GAT TTT CTT AGC AAG CTA CCT GAA      384
   Lys Asp Phe Glu Thr Leu Lys Val Asp Phe Leu Ser Lys Leu Pro Glu
           115                 120                 125
   ATG CTG AAA ATG TTC GAA GAT CGT TTA TGT CAT AAA ACA TAT TTA AAT      432
   Met Leu Lys Met Phe Glu Asp Arg Leu Cys His Lys Thr Tyr Leu Asn
       130                 135                 140
   GGT GAT CAT GTA ACC CAT CCT GAC TTC ATG TTG TAT GAC GCT CTT GAT      480
   Gly Asp His Val Thr His Pro Asp Phe Met Leu Tyr Asp Ala Leu Asp
   145                 150                 155                 160
   GTT GTT TTA TAC ATG GAC CCA ATG TGC CTG GAT GCG TTC CCA AAA TTA      528
   Val Val Leu Tyr Met Asp Pro Met Cys Leu Asp Ala Phe Pro Lys Leu
                   165                 170                 175
   GTT TGT TTT AAA AAA CGT ATT GAA GCT ATC CCA CAA ATT GAT AAG TAC      576
   Val Cys Phe Lys Lys Arg Ile Glu Ala Ile Pro Gln Ile Asp Lys Tyr
               180                 185                 190
   TTG AAA TCC AGC AAG TAT ATA GCA TGG CCT TTG CAG GGC TGG CAA GCC      624
   Leu Lys Ser Ser Lys Tyr Ile Ala Trp Pro Leu Gln Gly Trp Gln Ala
           195                 200                 205
   ACG TTT GGT GGT GGC GAC CAT CCT CCA AAA TCG GAT CTG GTT CCG CGT      672
   Thr Phe Gly Gly Gly Asp His Pro Pro Lys Ser Asp Leu Val Pro Arg
       210                 215                 220
   GGA TCC ATG AGC ACG ATT CCC AAA CCT CAA AGA AAA ACC AAA CGT AAC      720
   Gly Ser Met Ser Thr Ile Pro Lys Pro Gln Arg Lys Thr Lys Arg Asn
   225                 230                 235                 240
   ACC AAC CGT CGC CCA CAG GAA TTC ATC GTG ACT GAC TGA                  759
   Thr Asn Arg Arg Pro Gln Glu Phe Ile Val Thr Asp
                   245                 250
(2) INFORMATION FOR SEQ ID NO:4:
   (i) SEQUENCE CHARACTERISTICS:
      (A) LENGTH: 759 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..756
   (xi) SEQUENCE DESCRIPTION: SEQ ID NO:4:
   ATG TCC CCT ATA CTA GGT TAT TGG AAA ATT AAG GGC CTT GTG CAA CCC      48
   Met Ser Pro Ile Leu Gly Tyr Trp Lys Ile Lys Gly Leu Val Gln Pro
     1               5                  10                  15
   ACT CGA CTT CTT TTG GAA TAT CTT GAA GAA AAA TAT GAA GAG CAT TTG      96
   Thr Arg Leu Leu Leu Glu Tyr Leu Glu Glu Lys Tyr Glu Glu His Leu
                    20                  25                  30
   TAT GAG CGC GAT GAA GGT GAT AAA TGG CGA AAC AAA AAG TTT GAA TTG     144
   Tyr Glu Arg Asp Glu Gly Asp Lys Trp Arg Asn Lys Lys Phe Glu Leu
                35                  40                  45
   GGT TTG GAG TTT CCC AAT CTT CCT TAT TAT ATT GAT GGT GAT GTT AAA     192
   Gly Leu Glu Phe Pro Asn Leu Pro Tyr Tyr Ile Asp Gly Asp Val Lys
            50                  55                  60
   TTA ACA CAG TCT ATG GCC ATC ATA CGT TAT ATA GCT GAC AAG CAC AAC     240
   Leu Thr Gln Ser Met Ala Ile Ile Arg Tyr Ile Ala Asp Lys His Asn
    65                  70                  75                      80
   ATG TTG GGT GGT TGT CCA AAA GAG CGT GCA GAG ATT TCA ATG CTT GAA     288
   Met Leu Gly Gly Cys Pro Lys Glu Arg Ala Glu Ile Ser Met Leu Glu
                    85                  90                  95
   GGA GCG GTT TTG GAT ATT AGA TAC GGT GTT TCG AGA ATT GCA TAT AGT     336
   Gly Ala Val Leu Asp Ile Arg Tyr Gly Val Ser Arg Ile Ala Tyr Ser
               100                 105                 110
   AAA GAC TTT GAA ACT CTC AAA GTT GAT TTT CTT AGC AAG CTA CCT GAA     384
   Lys Asp Phe Glu Thr Leu Lys Val Asp Phe Leu Ser Lys Leu Pro Glu
           115                 120                 125
   ATG CTG AAA ATG TTC GAA GAT CGT TTA TGT CAT AAA ACA TAT TTA AAT     432
   Met Leu Lys Met Phe Glu Asp Arg Leu Cys His Lys Thr Tyr Leu Asn
       130                 135                 140
   GGT GAT CAT GTA ACC CAT CCT GAC TTC ATG TTG TAT GAC GCT CTT GAT     480
   Gly Asp His Val Thr His Pro Asp Phe Met Leu Tyr Asp Ala Leu Asp
   145                 150                 155                     160
   GTT GTT TTA TAC ATG GAC CCA ATG TGC CTG GAT GCG TTC CCA AAA TTA     528
   Val Val Leu Tyr Met Asp Pro Met Cys Leu Asp Ala Phe Pro Lys Leu
                   165                 170                 175
   GTT TGT TTT AAA AAA CGT ATT GAA GCT ATC CCA CAA ATT GAT AAG TAC     576
   Val Cys Phe Lys Lys Arg Ile Glu Ala Ile Pro Gln Ile Asp Lys Tyr
               180                 185                 190
   TTG AAA TCC AGC AAG TAT ATA GCA TGG CCT TTG CAG GGC TGG CAA GCC     624
   Leu Lys Ser Ser Lys Tyr Ile Ala Trp Pro Leu Gln Gly Trp Gln Ala
           195                 200                 205
   ACG TTT GGT GGT GGC GAC CAT CCT CCA AAA TCG GAT CTG GTT CCG CGT     672
   Thr Phe Gly Gly Gly Asp His Pro Pro Lys Ser Asp Leu Val Pro Arg
       210                 215                 220
   GGA TCC GAC GTC AAG TTC CCG GGT GGC GGT CAG ATC GTT GGT GGA GTT     720
   Gly Ser Asp Val Lys Phe Pro Gly Gly Gly Gln Ile Val Gly Gly Val
   225                 230                 235                 240
   TAC TTG TTG CCG CGC AGG GAA TTC ATC GTG ACT GAC TGA                 759
   Tyr Leu Leu Pro Arg Arg Glu Phe Ile Val Thr Asp
                   245                 250
(2) INFORMATION FOR SEQ ID NO:5:
   (i) SEQUENCE CHARACTERISTICS:
      (A) LENGTH: 759 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..756
   (xi) SEQUENCE DESCRIPTION: SEQ ID NO:5:
   ATG TCC CCT ATA CTA GGT TAT TGG AAA ATT AAG GGC CTT GTG CAA CCC        48
   Met Ser Pro Ile Leu Gly Tyr Trp Lys Ile Lys Gly Leu Val Gln Pro
     1               5                  10                  15
   ACT CGA CTT CTT TTG GAA TAT CTT GAA GAA AAA TAT GAA GAG CAT TTG        96
   Thr Arg Leu Leu Leu Glu Tyr Leu Glu Glu Lys Tyr Glu Glu His Leu
               20                  25                  30
   TAT GAG CGC GAT GAA GGT GAT AAA TGG CGA AAC AAA AAG TTT GAA TTG      144
   Tyr Glu Arg Asp Glu Gly Asp Lys Trp Arg Asn Lys Lys Phe Glu Leu
           35                  40                  45
   GGT TTG GAG TTT CCC AAT CTT CCT TAT TAT ATT GAT GGT GAT GTT AAA      192
   Gly Leu Glu Phe Pro Asn Leu Pro Tyr Tyr Ile Asp Gly Asp Val Lys
       50                  55                  60
   TTA ACA CAG TCT ATG GCC ATC ATA CGT TAT ATA GCT GAC AAG CAC AAC      240
   Leu Thr Gln Ser Met Ala Ile Ile Arg Tyr Ile Ala Asp Lys His Asn
   65                  70                  75                  80
   ATG TTG GGT GGT TGT CCA AAA GAG CGT GCA GAG ATT TCA ATG CTT GAA      288
   Met Leu Gly Gly Cys Pro Lys Glu Arg Ala Glu Ile Ser Met Leu Glu
                   85                  90                  95
   GGA GCG GTT TTG GAT ATT AGA TAC GGT GTT TCG AGA ATT GCA TAT AGT      336
   Gly Ala Val Leu Asp Ile Arg Tyr Gly Val Ser Arg Ile Ala Tyr Ser
               100                 105                 110
   AAA GAC TTT GAA ACT CTC AAA GTT GAT TTT CTT AGC AAG CTA CCT GAA      384
   Lys Asp Phe Glu Thr Leu Lys Val Asp Phe Leu Ser Lys Leu Pro Glu
           115                 120                 125
   ATG CTG AAA ATG TTC GAA GAT CGT TTA TGT CAT AAA ACA TAT TTA AAT      432
   Met Leu Lys Met Phe Glu Asp Arg Leu Cys His Lys Thr Tyr Leu Asn
       130                 135                 140
   GGT GAT CAT GTA ACC CAT CCT GAC TTC ATG TTG TAT GAC GCT CTT GAT      480
   Gly Asp His Val Thr His Pro Asp Phe Met Leu Tyr Asp Ala Leu Asp
   145                 150                 155                 160
   GTT GTT TTA TAC ATG GAC CCA ATG TGC CTG GAT GCG TTC CCA AAA TTA      528
   Val Val Leu Tyr Met Asp Pro Met Cys Leu Asp Ala Phe Pro Lys Leu
                   165                 170                 175
   GTT TGT TTT AAA AAA CGT ATT GAA GCT ATC CCA CAA ATT GAT AAG TAC      576
   Val Cys Phe Lys Lys Arg Ile Glu Ala Ile Pro Gln Ile Asp Lys Tyr
               180                 185                 190
   TTG AAA TCC AGC AAG TAT ATA GCA TGG CCT TTG CAG GGC TGG CAA GCC      624
   Leu Lys Ser Ser Lys Tyr Ile Ala Trp Pro Leu Gln Gly Trp Gln Ala
           195                 200                 205
   ACG TTT GGT GGT GGC GAC CAT CCT CCA AAA TCG GAT CTG GTT CCG CGT      672
   Thr Phe Gly Gly Gly Asp His Pro Pro Lys Ser Asp Leu Val Pro Arg
       210                 215                 220
   GGA TCC GGC CCT AGA TTG GGT GTG CGC GCG ACG AGG AAG ACT TCC GAG      720
   Gly Ser Gly Pro Arg Leu Gly Val Arg Ala Thr Arg Lys Thr Ser Glu
   225                 230                 235                 240
   CGG TCG CAA CCT CGA GGT GAA TTC ATC GTG ACT GAC TGA                  759
   Arg Ser Gln Pro Arg Gly Glu Phe Ile Val Thr Asp
                   245                 250
(2) INFORMATION FOR SEQ ID NO:6:
   (i) SEQUENCE CHARACTERISTICS:
      (A) LENGTH: 816 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..813
   (xi) SEQUENCE DESCRIPTION: SEQ ID NO:6:
   ATG TCC CCT ATA CTA GGT TAT TGG AAA ATT AAG GGC CTT GTG CAA CCC        48
   Met Ser Pro Ile Leu Gly Tyr Trp Lys Ile Lys Gly Leu Val Gln Pro
    1               5                  10                  15
   ACT CGA CTT CTT TTG GAA TAT CTT GAA GAA AAA TAT GAA GAG CAT TTG        96
   Thr Arg Leu Leu Leu Glu Tyr Leu Glu Glu Lys Tyr Glu Glu His Leu
               20                  25                  30
   TAT GAG CGC GAT GAA GGT GAT AAA TGG CGA AAC AAA AAG TTT GAA TTG       144
   Tyr Glu Arg Asp Glu Gly Asp Lys Trp Arg Asn Lys Lys Phe Glu Leu
           35                  40                  45
   GGT TTG GAG TTT CCC AAT CTT CCT TAT TAT ATT GAT GGT GAT GTT AAA       192
   Gly Leu Glu Phe Pro Asn Leu Pro Tyr Tyr Ile Asp Gly Asp Val Lys
       50                  55                  60
   TTA ACA CAG TCT ATG GCC ATC ATA CGT TAT ATA GCT GAC AAG CAC AAC       240
   Leu Thr Gln Ser Met Ala Ile Ile Arg Tyr Ile Ala Asp Lys His Asn
   65                  70                  75                  80
   ATG TTG GGT GGT TGT CCA AAA GAG CGT GCA GAG ATT TCA ATG CTT GAA       288
   Met Leu Gly Gly Cys Pro Lys Glu Arg Ala Glu Ile Ser Met Leu Glu
                       85                       90                       95
   GGA GCG GTT TTG GAT ATT AGA TAC GGT GTT TCG AGA ATT GCA TAT AGT        336
   Gly Ala Val Leu Asp Ile Arg Tyr Gly Val Ser Arg Ile Ala Tyr Ser
               100                     105                     110
   AAA GAC TTT GAA ACT CTC AAA GTT GAT TTT CTT AGC AAG CTA CCT GAA        384
   Lys Asp Phe Glu Thr Leu Lys Val Asp Phe Leu Ser Lys Leu Pro Glu
           115                     120                     125
   ATG CTG AAA ATG TTC GAA GAT CGT TTA TGT CAT AAA ACA TAT TTA AAT        432
   Met Leu Lys Met Phe Glu Asp Arg Leu Cys His Lys Thr Tyr Leu Asn
       130                     135                     140
   GGT GAT CAT GTA ACC CAT CCT GAC TTC ATG TTG TAT GAC GCT CTT GAT        480
   Gly Asp His Val Thr His Pro Asp Phe Met Leu Tyr Asp Ala Leu Asp
   145                     150                     155                     160
   GTT GTT TTA TAC ATG GAC CCA ATG TGC CTG GAT GCG TTC CCA AAA TTA        528
   Val Val Leu Tyr Met Asp Pro Met Cys Leu Asp Ala Phe Pro Lys Leu
                   165                     170                     175
   GTT TGT TTT AAA AAA CGT ATT GAA GCT ATC CCA CAA ATT GAT AAG TAC        576
   Val Cys Phe Lys Lys Arg Ile Glu Ala Ile Pro Gln Ile Asp Lys Tyr
               180                     185                     190
   TTG AAA TCC AGC AAG TAT ATA GCA TGG CCT TTG CAG GGC TGG CAA GCC        624
   Leu Lys Ser Ser Lys Tyr Ile Ala Trp Pro Leu Gln Gly Trp Gln Ala
           195                     200                     205
   ACG TTT GGT GGT GGC GAC CAT CCT CCA AAA TCG GAT CTG GTT CCG CGT        672
   Thr Phe Gly Gly Gly Asp His Pro Pro Lys Ser Asp Leu Val Pro Arg
       210                     215                     220
   GGA TCC AGC ACG ATT CCC AAA CCT CAA AGA AAA ACC AAA CGT AAC ACC        720
   Gly Ser Ser Thr Ile Pro Lys Pro Gln Arg Lys Thr Lys Arg Asn Thr
   225                     230                     235                     240
   AAC CGT CGC CCA CAG GAC GTC AAG TTC CCG GGT GGC GGT CAG ATC GTT        768
   Asn Arg Arg Pro Gln Asp Val Lys Phe Pro Gly Gly Gly Gln Ile Val
                   245                     250                     255
   GGT GGA GTT TAC TTG TTG CCG CGC AGG GAA TTC ATC GTG ACT GAC            813
   Gly Gly Val Tyr Leu Leu Pro Arg Arg Glu Phe Ile Val Thr Asp
               260                     265                     270
   TGA                                                                    816
(2) INFORMATION FOR SEQ ID NO:7:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:7:
   GATCCATGAG CACGATTCCC AAACCTCAAA GAAAAACCAA ACGTAACACC AACCGTCGCC 60
   CACAGG 66
(2) INFORMATION FOR SEQ ID NO:8:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:8:
   AATTCCTGTG GGCGACGGTT GGTGTTACGT TTGGTTTTTC TTTGAGGTTT GGGAATCGTG 60
   CTCATG 66
(2) INFORMATION FOR SEQ ID NO:9:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:9:
   GATCCGACGT CAAGTTCCCG GGTGGCGGTC AGATCGTTGG TGGAGTTTAC TTGTTGCCGC 60
   GCAGGG 66
(2) INFORMATION FOR SEQ ID NO:10:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:10:
   AATTCCCTGC GCGGCAACAA GTAAACTCCA CCAACGATCT GACCGCCACC CGGGAACTTG 60
   ACGTCG 66
(2) INFORMATION FOR SEQ ID NO:11:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:11:
   GATCCGGCCC TAGATTGGGT GTGCGCGCGA CGAGGAAGAC TTCCGAGCGG TCGCAACCTC 60
   GAGGTG 66
(2) INFORMATION FOR SEQ ID NO:12:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:12:
   AATTCACCTC GAGGTTGCGA CCGCTCGGAA GTCTTCCTCG TCGCGCGCAC ACCCAATCTA 60
   GGGCCG 66
(2) INFORMATION FOR SEQ ID NO:13:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:13:
      GAATTCTTAC CTGCGCGGCA ACAAGTAAAC TC 32
(2) INFORMATION FOR SEQ ID NO:14:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:14:
      GCTGGATCCA GCACGATTCC CAAACCTCAA AG 32
(2) INFORMATION FOR SEQ ID NO:15:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:15:
      ATGAGCACGA TTCCCAAACC T 21
(2) INFORMATION FOR SEQ ID NO:16:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:16:
      GAGGAAGACT TCCGAGC 17
(2) INFORMATION FOR SEQ ID NO:17:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:17:
      GTCCTGCCCT CGGGCCG 17
(2) INFORMATION FOR SEQ ID NO:18:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:18:
      ACCCAAATTG CGCGACCTAC G 21
(2) INFORMATION FOR SEQ ID NO:19:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:19:
      TGGGTAAGGT CATCGATAC 19
(2) INFORMATION FOR SEQ ID NO:20:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:20:
      AAGGTCATCG ATACCCT 17
(2) INFORMATION FOR SEQ ID NO:21:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:21:
      AGATAGAGAA AGAGCAAC 18
(2) INFORMATION FOR SEQ ID NO:22:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:22:
      GGACCAGTTC ATCATCATAT AT 22
(2) INFORMATION FOR SEQ ID NO:23:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:23:
      CAGTTCATCA TCATATCCCA 20
(2) INFORMATION FOR SEQ ID NO:24:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..15
      (D) OTHER INFORMATION: /product= "Linker Protein in GST-NANBV 693-691"
   (xi) SEQUENCE DESCRIPTION: SEQ ID NO:24:
   GGG ATC CCC AAT TCA                                                15
   Gly Ile Pro Asn Ser
     1               5
(2) INFORMATION FOR SEQ ID NO:25:
   (i) SEQUENCE CHARACTERISTICS:
      (A) LENGTH: 5 amino acids
      (B) TYPE: amino acid
      (D) TOPOLOGY: linear
   (ii) MOLECULE TYPE: protein
   (xi) SEQUENCE DESCRIPTION: SEQ ID NO:25:
   Gly Ile Pro Asn Ser
     1               5
(2) INFORMATION FOR SEQ ID NO:26:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..9
      (D) OTHER INFORMATION: /product= "Carboxy-terminal Linker Protein in GST-NANBV 693-691"
   (xi) SEQUENCE DESCRIPTION: SEQ ID NO:26:
   AAT TCA TCG TGA                                                    12
   Asn Ser Ser
     1
(2) INFORMATION FOR SEQ ID NO:27:
   (i) SEQUENCE CHARACTERISTICS:
      (A) LENGTH: 3 amino acids
      (B) TYPE: amino acid
      (D) TOPOLOGY: linear
   (ii) MOLECULE TYPE: protein
   (xi) SEQUENCE DESCRIPTION: SEQ ID NO:27:
   Asn Ser Ser
   1
(2) INFORMATION FOR SEQ ID NO:28:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..27
      (D) OTHER INFORMATION: /product= "Linker Protein in GST-NANBV 15-18"
   (xi) SEQUENCE DESCRIPTION: SEQ ID NO:28:
   GGG ATC CCC ATC GAA TTC CTG CAG CCC                                        27
   Gly Ile Pro Ile Glu Phe Leu Gln Pro
     1               5
(2) INFORMATION FOR SEQ ID NO:29:
   (i) SEQUENCE CHARACTERISTICS:
      (A) LENGTH: 9 amino acids
      (B) TYPE: amino acid
      (D) TOPOLOGY: linear
   (ii) MOLECULE TYPE: protein
   (xi) SEQUENCE DESCRIPTION: SEQ ID NO:29:
   Gly Ile Pro Ile Glu Phe Leu Gln Pro
     1               5
(2) INFORMATION FOR SEQ ID NO:30:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
(2) INFORMATION FOR SEQ ID NO:33:
   (i) SEQUENCE CHARACTERTSTICS:
      (A) LENGTH: 8 amino acids
      (B) TYPE: amino acid
      (D) TOPOLOGY: linear
   (ii) MOLECULE TYPE: protein
   (xi) SEQUENCE DESCRIPTION: SEQ ID NO:33:
   Gly Ile Pro Asn Ser Cys Ser Pro
    1               5
(2) INFORMATION FOR SEQ ID NO:34:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..18
      (D) OTHER INFORMATION: /product= "Carboxy-terminal Linker Protein in GST-NANBV 15-17"
   (xi) SEQUENCE DESCRIPTION: SEQ ID NO:34:
   GGG ATC GGG AAT TCA TCG TGA                                                    21
   Gly Ile Gly Asn Ser Ser
    1               5
(2) INFORMATION FOR SEQ ID NO:35:
   (i) SEQUENCE CHARACTERISTICS:
      (A) LENGTH: 6 amino acids
      (B) TYPE: amino acid
      (D) TOPOLOGY: linear
   (ii) MOLECULE TYPE: protein
   (xi) SEQUENCE DESCRIPTION: SEQ ID NO:35:
   Gly Ile Gly Asn Ser Ser
    1               5
(2) INFORMATION FOR SEQ ID NO:36:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..15
      (D) OTHER INFORMATION: /product= "Thrombin Cleavage Site in GST-NANBV 15-17"
   (xi) SEQUENCE DESCRIPTION: SEQ ID NO:36:
   GTT CCG CGT GGA TCC                                   15
   Val Pro Arg Gly Ser
     1               5
(2) INFORMATION FOR SEQ ID NO:37:
   (i) SEQUENCE CHARACTERISTICS:
      (A) LENGTH: 5 amino acids
      (B) TYPE: amino acid
      (D) TOPOLOGY: linear
   (ii) MOLECULE TYPE: protein
   (xi) SEQUENCE DESCRIPTION: SEQ ID NO:37:
   Val Pro Arg Gly Ser
     1               5
(2) INFORMATION FOR SEQ ID NO:38:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..21
      (D) OTHER INFORMATION: /product= "Linker Protein in GST-NANBV 15-17"
   (xi) SEQUENCE DESCRIPTION: SEQ ID NO:38:
   CCA TCG AAT TCC TGC AGC CCT                                       21
   Pro Ser Asn Ser Cys Ser Pro
     1               5
(2) INFORMATION FOR SEQ ID NO:39:
   (i) SEQUENCE CHARACTERISTICS:
      (A) LENGTH: 7 amino acids
      (B) TYPE: amino acid
      (D) TOPOLOGY: linear
   (ii) MOLECULE TYPE: protein
   (xi) SEQUENCE DESCRIPTION: SEQ ID NO:39:
   Pro Ser Asn Ser Cys Ser Pro
     1               5
(2) INFORMATION FOR SEQ ID NO:40:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..15
      (D) OTHER INFORMATION: /product= "Carboxy-terminal Linker Protein in GST-NANBV 15-17"
   (xi) SEQUENCE DESCRIPTION: SEQ ID NO:40:
   GGA ATT CAT CGT GAC TGA                                                    18
   Gly Ile His Arg Asp
     1               5
(2) INFORMATION FOR SEQ ID NO:41:
   (i) SEQUENCE CHARACTERISTICS:
      (A) LENGTH: 5 amino acids
      (B) TYPE: amino acid
      (D) TOPOLOGY: linear
   (ii) MOLECULE TYPE: protein
   (xi) SEQUENCE DESCRIPTION: SEQ ID NO:41:
   Gly Ile His Arg Asp
     1               5
(2) INFORMATION FOR SEQ ID NO:42:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..27
      (D) OTHER INFORMATION: /product= "Linker Protein in GST-NANBV 690-691"
   (xi) SEQUENCE DESCRIPTION: SEQ ID NO:42:
   GGG ATC CCC AAT TCG AGC TCG GTA CCC                                        27
   Gly Ile Pro Asn Ser Ser Ser Val Pro
     1               5
(2) INFORMATION FOR SEQ ID NO:43:
   (i) SEQUENCE CHARACTERISTICS:
      (A) LENGTH: 9 amino acids
      (B) TYPE: amino acid
      (D) TOPOLOGY: linear
   (ii) MOLECULE TYPE: protein
   (xi) SEQUENCE DESCRIPTION: SEQ ID NO:43:
   Gly Ile Pro Asn Ser Ser Ser Val Pro
   1 5
(2) INFORMATION FOR SEQ ID NO:44:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (ix) FEATURE:
      (A) NAME/KEY: CDS
      (B) LOCATION: 1..21
      (D) OTHER INFORMATION: /product= "Carboxy-terminal Linker Protein in GST-NANBV 690-691"
   (xi) SEQUENCE DESCRIPTION: SEQ ID NO:44:
   ACG GGG ATC GGG AAT TCA TCG TGA                                    24
   Thr Gly Ile Gly Asn Ser Ser
    1               5
(2) INFORMATION FOR SEQ ID NO:45:
   (i) SEQUENCE CHARACTERISTICS:
      (A) LENGTH: 7 amino acids
      (B) TYPE: amino acid
      (D) TOPOLOGY: linear
   (ii) MOLECULE TYPE: protein
   (xi) SEQUENCE DESCRIPTION: SEQ ID NO:45:
   Thr Gly Ile Gly Asn Ser Ser
   1 5
(2) INFORMATION FOR SEQ ID NO:46:
   (i) SEQUENCE CHARACTERISTICS:
      (A) LENGTH: 9416 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (ix) FEATURE:
      (A) NAME/KEY: CDS
      (B) LOCATION: 342..9374
   (ix) FEATURE:
      (A) NAME/KEY: misc_feature
      (B) LOCATION: 1..12
      (D) OTHER INFORMATION: /note= "Not confirmed as HCV-Hc59 Sequence"
   (ix) FEATURE:
      (A) NAME/KEY: misc_feature
      (B) LOCATION: 9397..9416
      (D) OTHER INFORMATION: /note= "Not confirmed as HCV-Hc59 Sequence"
   (ix) FEATURE:
      (A) NAME/KEY: repeat_unit
      (B) LOCATION: group(7..12, 42..47)
      (D) OTHER INFORMATION: /rpt_type= "other" /rpt_family= "1"
   (ix) FEATURE:
      (A) NAME/KEY: repeat_unit
      (B) LOCATION: group(23..28, 38..43, 9209..9214, 9391..9396)
      (D) OTHER INFORMATION: /rpt_type= "other" /rpt_family= "2"
   (ix) FEATURE:
      (A) NAME/KEY: repeat_unit
      (B) LOCATION: group(128..135, 315..322)
      (D) OTHER INFORMATION: /rpt_type= "other" /rpt_family= "3"
   (ix) FEATURE:
      (A) NAME/KEY: repeat_unit
      (B) LOCATION: group(9231..9237, 9245..9251, 9256..9262)
      (D) OTHER INFORMATION: /rpt_type= "other" /rpt_family= "4"
   (ix) FEATURE:
      (A) NAME/KEY: repeat_unit
      (B) LOCATION: group(9248..9253, 9221..9226, 9227..9232)
      (D) OTHER INFORMATION: /rpt_type= "other" /rpt_family= "5"
   (xi) SEQUENCE DESCRIPTION: SEQ ID NO:46:
   GCCAGCCCCC TGATGGGGGC GACACTCCAC CATGAATCAC TCCCCTGTGA GGAACTACTG    60
   TCTTCACGCA GAAAGCGTCT AGCCATGGCG TTAGTATGAG TGTCGTGCAG CCTCCAGGAC   120
   CCCCCCTCCC GGGAGAGCCA TAGTGGTCTG CGGAACCGGT GAGTACACCG GAATTGCCAG   180
   GACGACCGGG TCCTTTCTTG GATAAACCCG CTCAATGCCT GGAGATTTGG GCGTGCCCCC   240
   GCAAGACTGC TAGCCGAGTA GTGTTGGGTC GCGAAAGGCC TTGTGGTACT GCCTGATAGG   300
   GTGCTTGCGA GTGCCCCGGG AGGTCTCGTA GACCGTGCAC C ATG AGC ACG AAT      353
                                                 Met Ser Thr Asn
                                                   1
   CCT AAA CCT CAA AGA AAA ACC AAA CGT AAC ACC AAC CGT CGC CCA CAG     401
   Pro Lys Pro Gln Arg Lys Thr Lys Arg Asn Thr Asn Arg Arg Pro Gln
     5                  10                  15                  20
   GAC GTC AAG TTC CCG GGT GGC GGT CAG ATC GTT GGT GGA GTT TAC TTG     449
   Asp Val Lys Phe Pro Gly Gly Gly Gln Ile Val Gly Gly Val Tyr Leu
                    25                  30                  35
   TTG CCG CGC AGG GGC CCT AGA TTG GGT GTG CGC GCG ACG AGG AAG ACT     497
   Leu Pro Arg Arg Gly Pro Arg Leu Gly Val Arg Ala Thr Arg Lys Thr
                40                  45                  50
   TCC GAG CGG TCG CAA CCT CGA GGT AGA CGT CAG CCT ATC CCC AAG GCA     545
   Ser Glu Arg Ser Gln Pro Arg Gly Arg Arg Gln Pro Ile Pro Lys Ala
            55                  60                  65
   CGT CGG CCC GAG GGC AGG ACC TGG GCT CAG CCC GGG TAC CCT TGG CCC     593
   Arg Arg Pro Glu Gly Arg Thr Trp Ala Gln Pro Gly Tyr Pro Trp Pro
        70                  75                  80
   CTC TAT GGC AAT GAG GGT TGC GGG TGG GCG GGA TGG CTC CTG TCT CCC     641
   Leu Tyr Gly Asn Glu Gly Cys Gly Trp Ala Gly Trp Leu Leu Ser Pro
    85                  90                  95                 100
   CGT GGC TCT CGG CCT AGC TGG GGC CCC ACA GAC CCC CGG CGT AGG TCG     689
   Arg Gly Ser Arg Pro Ser Trp Gly Pro Thr Asp Pro Arg Arg Arg Ser
                   105                 110                 115
   CGC AAT TTG GGT AAG GTC ATC GAT ACC CTT ACG TGC GGC TTC GCC GAC     737
   Arg Asn Leu Gly Lys Val Ile Asp Thr Leu Thr Cys Gly Phe Ala Asp
               120                 125                 130
   CTC ATG GGG TAC ATA CCG CTC GTC GGC GCC CCT CTT GGA GGC GCT GCC     785
   Leu Met Gly Tyr Ile Pro Leu Val Gly Ala Pro Leu Gly Gly Ala Ala
           135                 140                 145
   AGG GCC CTG GCG CAT GGC GTC CGG GTT CTG GAA GAC GGC GTG AAC TAT      833
   Arg Ala Leu Ala His Gly Val Arg Val Leu Glu Asp Gly Val Asn Tyr
       150                 155                 160
   GCA ACA GGG AAC CTT CCT GGT TGC TCT TTC TCT ATC TTC CTT CTG GCC      881
   Ala Thr Gly Asn Leu Pro Gly Cys Ser Phe Ser Ile Phe Leu Leu Ala
   165                 170                 175                 180
   CTG CTC TCT TGC CTG ACT GTG CCC GCT TCA GCC TAC CAA GTG CGC AAT      929
   Leu Leu Ser Cys Leu Thr Val Pro Ala Ser Ala Tyr Gln Val Arg Asn
                   185                 190                 195
   TCC TCG GGG CTT TAC CAT GTC ACC AAT GAT TGC CCT AAC TCG AGT GTT      977
   Ser Ser Gly Leu Tyr His Val Thr Asn Asp Cys Pro Asn Ser Ser Val
               200                 205                 210
   GTG TAC GAG GCG GCC GAT GCC ATC CTG CAC ACT CCG GGG TGT GTC CCT     1025
   Val Tyr Glu Ala Ala Asp Ala Ile Leu His Thr Pro Gly Cys Val Pro
           215                 220                 225
   TGC GTT CGC GAG GGT AAC GCC TCG AGG TGT TGG GTG GCG GTG ACC CCC     1073
   Cys Val Arg Glu Gly Asn Ala Ser Arg Cys Trp Val Ala Val Thr Pro
       230                 235                 240
   ACG GTG GCC ACC AGG GAC GGC AAA CTC CCC ACA ACG CAG CTT CGA CGT     1121
   Thr Val Ala Thr Arg Asp Gly Lys Leu Pro Thr Thr Gln Leu Arg Arg
   245                 250                 255                 260
   CAT ATC GAT CTG CTT GTC GGG AGC GCC ACC CTC TGC TCG GCC CTC TAC     1169
   His Ile Asp Leu Leu Val Gly Ser Ala Thr Leu Cys Ser Ala Leu Tyr
                   265                 270                 275
   GTG GGG GAC CTG TGC GGG TCT GTC TTT CTT GTT GGT CAA CTG TTT ACC     1217
   Val Gly Asp Leu Cys Gly Ser Val Phe Leu Val Gly Gln Leu Phe Thr
               280                 285                 290
   TTC TCT CCC AGG CAC CAC TGG ACG ACG CAA GAC TGC AAT TGT TCT ATC     1265
   Phe Ser Pro Arg His His Trp Thr Thr Gln Asp Cys Asn Cys Ser Ile
           295                 300                 305
   TAT CCC GGC CAT ATA ACG GGT CAT CGC ATG GCA TGG AAT ATG ATG ATG     1313
   Tyr Pro Gly His Ile Thr Gly His Arg Met Ala Trp Asn Met Met Met
       310                 315                 320
   AAC TGG TCC CCT ACG GCA GCG TTG GTG GTA GCT CAG CTG CTC CGA ATC     1361
   Asn Trp Ser Pro Thr Ala Ala Leu Val Val Ala Gln Leu Leu Arg Ile
   325                 330                 335                 340
   CCA CAA GCC ATC ATG GAC ATG ATC GCT GGC GCC CAC TGG GGA GTC CTG     1409
   Pro Gln Ala Ile Met Asp Met Ile Ala Gly Ala His Trp Gly Val Leu
                   345                 350                 355
   GCG GGC ATA AAG TAT TTC TCC ATG GTG GGG AAC TGG GCG AAG GTC CTG        1457
   Ala Gly Ile Lys Tyr Phe Ser Met Val Gly Asn Trp Ala Lys Val Leu
               360                 365                 370
   GTA GTG CTG CTG CTA TTT GCC GGC GTC GAC GCG GAA ACC CAC GTC ACC        1505
   Val Val Leu Leu Leu Phe Ala Gly Val Asp Ala Glu Thr His Val Thr
           375                 380                 385
   GGG GGA AAT GCC GGC CGC ACC ACG GCT GGG CTT GTT GGT CTC CTT ACA        1553
   Gly Gly Asn Ala Gly Arg Thr Thr Ala Gly Leu Val Gly Leu Leu Thr
       390                 395                 400
   CCA GGC GCC AAG CAG AAC ATC CAA CTG ATC AAC ACC AAC GGC AGT TGG        1601
   Pro Gly Ala Lys Gln Asn Ile Gln Leu Ile Asn Thr Asn Gly Ser Trp
   405                 410                 415                 420
   CAC ATC AAT AGC ACG GCC TTG AAC TGC AAT GAA AGC CTT AAC ACC GGC        1649
   His Ile Asn Ser Thr Ala Leu Asn Cys Asn Glu Ser Leu Asn Thr Gly
                   425                 430                 435
   TGG TTA GCA GGG CTC TTC TAT CAG CAC AAA TTC AAC TCT TCA GGC TGT        1697
   Trp Leu Ala Gly Leu Phe Tyr Gln His Lys Phe Asn Ser Ser Gly Cys
               440                 445                 450
   CCT GAG AGG TTG GCC AGC TGC CGA CGC CTT ACC GAT TTT GCC CAG GGC        1745
   Pro Glu Arg Leu Ala Ser Cys Arg Arg Leu Thr Asp Phe Ala Gln Gly
           455                 460                 465
   TGG GGT CCT ATC AGT TAT GCC AAC GGA AGC GGC CTC GAC GAA CGC CCC        1793
   Trp Gly Pro Ile Ser Tyr Ala Asn Gly Ser Gly Leu Asp Glu Arg Pro
       470                 475                 480
   TAC TGC TGG CAC TAC CCT CCA AGA CCT TGT GGC ATT GTG CCC GCA AAG        1841
   Tyr Cys Trp His Tyr Pro Pro Arg Pro Cys Gly Ile Val Pro Ala Lys
   485                 490                 495                 500
   AGC GTG TGT GGC CCG GTA TAT TGC TTC ACT CCC AGC CCC GTG GTG GTG        1889
   Ser Val Cys Gly Pro Val Tyr Cys Phe Thr Pro Ser Pro Val Val Val
                   505                 510                 515
   GGA ACG ACC GAC AGG TCG GGC GCG CCT ACC TAC AGC TGG GGT GCA AAT        1937
   Gly Thr Thr Asp Arg Ser Gly Ala Pro Thr Tyr Ser Trp Gly Ala Asn
               520                 525                 530
   GAT ACG GAT GTC TTC GTC CTT AAC AAC ACC AGG CCA CCG CTG GGC AAT        1985
   Asp Thr Asp Val Phe Val Leu Asn Asn Thr Arg Pro Pro Leu Gly Asn
           535                 540                 545
   TGG TTC GGT TGT ACC TGG ATG AAC TCA ACT GGA TTC ACC AAA GTG TGC        2033
   Trp Phe Gly Cys Thr Trp Met Asn Ser Thr Gly Phe Thr Lys Val Cys
       550                 555                 560
   GGA GCG CCC CCT TGT GTC ATC GGA GGG GTG GGC AAC AAC ACC TTG CTC 2081
   Gly Ala Pro Pro Cys Val Ile Gly Gly Val Gly Asn Asn Thr Leu Leu
   565 570 575 580
   TGC CCC ACT GAT TGC TTC CGC AAA TAT CCG GAA GCC ACA TAC TCT CGG 2129
   Cys Pro Thr Asp Cys Phe Arg Lys Tyr Pro Glu Ala Thr Tyr Ser Arg
   585 590 595
   TGC GGC TCC GGT CCC AGG ATT ACA CCC AGG TGC ATG GTC GAC TAC CCG 2177
   Cys Gly Ser Gly Pro Arg Ile Thr Pro Arg Cys Met Val Asp Tyr Pro
   600 605 610
   TAT AGG CTT TGG CAC TAT CCT TGT ACC ATC AAT TAC ACC ATA TTC AAA 2225
   Tyr Arg Leu Trp His Tyr Pro Cys Thr Ile Asn Tyr Thr Ile Phe Lys
   615 620 625
   GTC AGG ATG TAC GTG GGA GGG GTC GAG CAC AGG CTG GAA GCG GCC TGC 2273
   Val Arg Met Tyr Val Gly Gly Val Glu His Arg Leu Glu Ala Ala Cys
   630 635 640
   AAC TGG ACG CGG GGC GAA CGC TGT GAT CTG GAA GAC AGG GAC AGG TCC 2321
   Asn Trp Thr Arg Gly Glu Arg Cys Asp Leu Glu Asp Arg Asp Arg Ser
   645 650 655 660
   GAG CTC AGC CCG TTG CTG CTG TCC ACC ACA CAG TGG CAG GTC CTT CCG 2369
   Glu Leu Ser Pro Leu Leu Leu Ser Thr Thr Gln Trp Gln Val Leu Pro
   665 670 675
   TGT TCT TTC ACG ACC CTG CCA GCC TTG TCC ACC GGC CTC ATC CAC CTC 2417
   Cys Ser Phe Thr Thr Leu Pro Ala Leu Ser Thr Gly Leu Ile His Leu
   680 685 690
   CAC CAG AAC ATT GTG GAC GTG CAG TAC TTG TAC GGG GTA GGG TCA AGC 2465
   His Gln Asn Ile Val Asp Val Gln Tyr Leu Tyr Gly Val Gly Ser Ser
   695 700 705
   ATC GCG TCC TGG GCC ATT AAG TGG GAG TAC GTC GTT CTC CTG TTC CTT 2513
   Ile Ala Ser Trp Ala Ile Lys Trp Glu Tyr Val Val Leu Leu Phe Leu
   710 715 720
   CTG CTT GCA GAC GCG CGC GTC TGT TCC TGC TTG TGG ATG ATG TTA CTC 2561
   Leu Leu Ala Asp Ala Arg Val Cys Ser Cys Leu Trp Met Met Leu Leu
   725 730 735 740
   ATA TCC CAA GCG GAG GCG GCT TTG GAG AAC CTC GTA ATA CTC AAT GCA 2609
   Ile Ser Gln Ala Glu Ala Ala Leu Glu Asn Leu Val Ile Leu Asn Ala
   745 750 755
   GCA TCC CTG GCC GGG ACG CAT GGT CTT GTG TCC TTC CTC GTG TTC TTC 2657
   Ala Ser Leu Ala Gly Thr His Gly Leu Val Ser Phe Leu Val Phe Phe
   760 765 770
   TGC TTT GCG TGG TAT CTG AAG GGT AGG TGG GTG CCC GGA GCG GTC TAC        2705
   Cys Phe Ala Trp Tyr Leu Lys Gly Arg Trp Val Pro Gly Ala Val Tyr
           775                 780                 785
   GCC CTC TAC GGG ATG TGG CCT CTC CTC CTG CTC CTG CTG GCG TTG CCT        2753
   Ala Leu Tyr Gly Met Trp Pro Leu Leu Leu Leu Leu Leu Ala Leu Pro
       790                 795                 800
   CAG CGG GCA TAC GCA CTG GAC ACG GAG GTG GCC GCG TCG TGT GGC GGC        2801
   Gln Arg Ala Tyr Ala Leu Asp Thr Glu Val Ala Ala Ser Cys Gly Gly
   805                 810                 815                 820
   GTT GTT CTT GTC GGG TTA ATG GCG CTG ACT CTG TCG CCA TAT TAC AAG        2849
   Val Val Leu Val Gly Leu Met Ala Leu Thr Leu Ser Pro Tyr Tyr Lys
                   825                 830                 835
   CGC TAT ATC AGC TGG TGC ATG TGG TGG CTT CAG TAT TTT CTG ACC AGA        2897
   Arg Tyr Ile Ser Trp Cys Met Trp Trp Leu Gln Tyr Phe Leu Thr Arg
               840                 845                 850
   GTA GAA GCG CAA CTG CAC GTG TGG GTT CCC CCC CTC AAC GTC CGG GGG        2945
   Val Glu Ala Gln Leu His Val Trp Val Pro Pro Leu Asn Val Arg Gly
           855                 860                 865
   GGG CGC GAT GCC GTC ATC TTA CTC ACG TGT GTA GTA CAC CCG GCC CTG        2993
   Gly Arg Asp Ala Val Ile Leu Leu Thr Cys Val Val His Pro Ala Leu
       870                 875                 880
   GTA TTT GAC ATC ACC AAA CTA CTC CTG GCC ATC TTC GGA CCC CTT TGG        3041
   Val Phe Asp Ile Thr Lys Leu Leu Leu Ala Ile Phe Gly Pro Leu Trp
   885                 890                 895                 900
   ATT CTT CAA GCC AGT TTG CTT AAA GTC CCC TAC TTC GTG CGC GTT CAA        3089
   Ile Leu Gln Ala Ser Leu Leu Lys Val Pro Tyr Phe Val Arg Val Gln
                   905                 910                 915
   GGC CTT CTC CGG ATC TGC GCG CTA GCG CGG AAG ATA GCC GGA GGT CAT        3137
   Gly Leu Leu Arg Ile Cys Ala Leu Ala Arg Lys Ile Ala Gly Gly His
               920                 925                 930
   TAC GTG CAA ATG GCC ATC ATC AAG TTA GGG GCG CTT ACT GGC ACC TGT        3185
   Tyr Val Gln Met Ala Ile Ile Lys Leu Gly Ala Leu Thr Gly Thr Cys
           935                 940                 945
   GTG TAT AAC CAT CTC GCT CCT CTT CGA GAC TGG GCG CAC AAC GGC CTG        3233
   Val Tyr Asn His Leu Ala Pro Leu Arg Asp Trp Ala His Asn Gly Leu
       950                 955                 960
   CGA GAT CTG GCC GTG GCT GTG GAA CCA GTC GTC TTC TCC CGA ATG GAG        3281
   Arg Asp Leu Ala Val Ala Val Glu Pro Val Val Phe Ser Arg Met Glu
   965                 970                 975                 980
   ACC AAG CTC ATC ACG TGG GGG GCA GAT ACC GCC GCG TGC GGT GAC ATC 3329
   Thr Lys Leu Ile Thr Trp Gly Ala Asp Thr Ala Ala Cys Gly Asp Ile
   985 990 995
   ATC AAC GGC TTG CCC GTC TCT GCC CGT AGG GGC CAG GAG ATA CTG CTT 3377
   Ile Asn Gly Leu Pro Val Ser Ala Arg Arg Gly Gln Glu Ile Leu Leu
   1000 1005 1010
   GGG CCA GCC GAC GGA ATG GTC TCC AAG GGG TGG AGG TTG CTG GCG CCC 3425
   Gly Pro Ala Asp Gly Met Val Ser Lys Gly Trp Arg Leu Leu Ala Pro
   1015 1020 1025
   ATC ACG GCG TAC GCC CAG CAG ACG AGA GGC CTC CTA GGG TGT ATA ATC 3473
   Ile Thr Ala Tyr Ala Gln Gln Thr Arg Gly Leu Leu Gly Cys Ile Ile
   1030 1035 1040
   ACC AGC CTG ACT GGC CGG GAC AAA AAC CAA GTG GAG GGT GAG GTC CAG 3521
   Thr Ser Leu Thr Gly Arg Asp Lys Asn Gln Val Glu Gly Glu Val Gln
   1045 1050 1055 1060
   ATC GTG TCA ACT GCT ACC CAG ACC TTC CTG GCA ACG TGC ATC AAT GGG 3569
   Ile Val Ser Thr Ala Thr Gln Thr Phe Leu Ala Thr Cys Ile Asn Gly
   1065 1070 1075
   GTA TGC TGG ACT GTC TAC CAC GGG GCC GGA ACG AGG ACC ATC GCA TCA 3617
   Val Cys Trp Thr Val Tyr His Gly Ala Gly Thr Arg Thr Ile Ala Ser
   1080 1085 1090
   CCC AAG GGT CCT GTC ATC CAG ACG TAT ACC AAT GTG GAT CAA GAC CTC 3665
   Pro Lys Gly Pro Val Ile Gln Thr Tyr Thr Asn Val Asp Gln Asp Leu
   1095 1100 1105
   GTG GGC TGG CCC GCT CCT CAA GGT TCC CGC TCA TTG ACA CCC TGC ACC 3713
   Val Gly Trp Pro Ala Pro Gln Gly Ser Arg Ser Leu Thr Pro Cys Thr
   1110 1115 1120
   TGC GGC TCC TCG GAC CTT TAC CTG GTC ACG AGG CAC GCC GAT GTC ATT 3761
   Cys Gly Ser Ser Asp Leu Tyr Leu Val Thr Arg His Ala Asp Val Ile
   1125 1130 1135 1140
   CCC GTG CGC CGG CGA GGT GAT AGC AGG GGT AGC CTG CTT TCG CCC CGG 3809
   Pro Val Arg Arg Arg Gly Asp Ser Arg Gly Ser Leu Leu Ser Pro Arg
   1145 1150 1155
   CCC ATT TCC TAC TTG AAA GGC TCC TCG GGG GGT CCG CTG TTG TGC CCC 3857
   Pro Ile Ser Tyr Leu Lys Gly Ser Ser Gly Gly Pro Leu Leu Cys Pro
   1160 1165 1170
   ACG GGA CAC GCC GTG GGC CTA TTC AGG GCC GCG GTG TGC ACC CGT GGA 3905
   Thr Gly His Ala Val Gly Leu Phe Arg Ala Ala Val Cys Thr Arg Gly
   1175 1180 1185
   GTG GCT AAG GCG GTG GAC TTT ATC CCT GTG GAG AAC CTA GAG ACA ACC      3953
   Val Ala Lys Ala Val Asp Phe Ile Pro Val Glu Asn Leu Glu Thr Thr
       1190                1195                1200
   ATG AGA TCC CCG GTG TTC ACG GAC AAC TCC TCT CCA CCA GCA GTG CCC      4001
   Met Arg Ser Pro Val Phe Thr Asp Asn Ser Ser Pro Pro Ala Val Pro
   1205                1210                1215                1220
   CAG AGC TTC CAG GTG GCC CAC CTG CAT GCT CCC ACC GGC AGC GGT AAG      4049
   Gln Ser Phe Gln Val Ala His Leu His Ala Pro Thr Gly Ser Gly Lys
                   1225                1230                1235
   AGC ACC AAG GTC CCG GCT GCG TAC GCA GCC AAG GGC TAC AAG GTG TTG      4097
   Ser Thr Lys Val Pro Ala Ala Tyr Ala Ala Lys Gly Tyr Lys Val Leu
               1240                1245                1250
   GTG CTC AAC CCC TCT GTT GCT GCA ACA CTG GGC TTT GGT GCT TAC ATG      4145
   Val Leu Asn Pro Ser Val Ala Ala Thr Leu Gly Phe Gly Ala Tyr Met
           1255                1260                1265
   TCC AAG GCC CAT GGG GTT GAT CCT AAT ATC AGG ACC GGG GTG AGA ACA      4193
   Ser Lys Ala His Gly Val Asp Pro Asn Ile Arg Thr Gly Val Arg Thr
       1270                1275                1280
   ATT ACC ACT GGC AGC CCC ATC ACG TAC TCC ACC TAC GGC AAG TTC CTT      4241
   Ile Thr Thr Gly Ser Pro Ile Thr Tyr Ser Thr Tyr Gly Lys Phe Leu
   1285                1290                1295                1300
   GCC GAC GCC GGG TGC TCA GGA GGT GCT TAT GAC ATA ATA ATT TGT GAC      4289
   Ala Asp Ala Gly Cys Ser Gly Gly Ala Tyr Asp Ile Ile Ile Cys Asp
                   1305                1310                1315
   GAG TGC CAC TCC ACG GAT GCC ACA TCC ATC TCG GGC ATC GGC ACT GTC      4337
   Glu Cys His Ser Thr Asp Ala Thr Ser Ile Ser Gly Ile Gly Thr Val
               1320                1325                1330
   CTT GAC CAA GCA GAG ACT GCG GGG GCG AGA CTG GTT GTG CTC GCC ACT      4385
   Leu Asp Gln Ala Glu Thr Ala Gly Ala Arg Leu Val Val Leu Ala Thr
           1335                1340                1345
   GCT ACC CCT CCG GGC TCC GTC ACT GTG TCC CAT CCT AAC ATC GAG GAG      4433
   Ala Thr Pro Pro Gly Ser Val Thr Val Ser His Pro Asn Ile Glu Glu
       1350                1355                1360
   GTT GCT CTG TCC ACC ACC GGA GAG ATC CCC TTT TAC GGC AAG GCT ATC      4481
   Val Ala Leu Ser Thr Thr Gly Glu Ile Pro Phe Tyr Gly Lys Ala Ile
   1365                1370                1375                1380
   CCC CTC GAG GTG ATC AAG GGG GGA AGA CAT CTC ATC TTC TGC CAC TCA      4529
   Pro Leu Glu Val Ile Lys Gly Gly Arg His Leu Ile Phe Cys His Ser
                   1385                1390                1395
   AAG AAG AAG TGC GAC GAG CTC GCC GCG AAG CTG GTC GCA TTG GGC ATC 4577
   Lys Lys Lys Cys Asp Glu Leu Ala Ala Lys Leu Val Ala Leu Gly Ile
   1400 1405 1410
   AAT GCC GTG GCC TAC TAC CGC GGT CTT GAC GTG TCT GTC ATC CCG ACC 4625
   Asn Ala Val Ala Tyr Tyr Arg Gly Leu Asp Val Ser Val Ile Pro Thr
   1415 1420 1425
   AGC GGC GAT GTT GTC GTC GTG TCG ACC GAT GCT CTC ATG ACT GGC TTT 4673
   Ser Gly Asp Val Val Val Val Ser Thr Asp Ala Leu Met Thr Gly Phe
   1430 1435 1440
   ACC GGC GAC TTC GAC TCT GTG ATA GAC TGC AAC ACG TGT GTC ACT CAG 4721
   Thr Gly Asp Phe Asp Ser Val Ile Asp Cys Asn Thr Cys Val Thr Gln
   1445 1450 1455 1460
   ACA GTC GAT TTT AGC CTT GAC CCT ACC TTT ACC ATT GAG ACA ACC ACG 4769
   Thr Val Asp Phe Ser Leu Asp Pro Thr Phe Thr Ile Glu Thr Thr Thr
   1465 1470 1475
   CTC CCC CAG GAT GCT GTC TCC AGG ACT CAA CGC CGG GGC AGG ACT GGC 4817
   Leu Pro Gln Asp Ala Val Ser Arg Thr Gln Arg Arg Gly Arg Thr Gly
   1480 1485 1490
   AGG GGG AAG CCA GGC ATC TAT AGA TTT GTG GCA CCG GGG GAG CGC CCC 4865
   Arg Gly Lys Pro Gly Ile Tyr Arg Phe Val Ala Pro Gly Glu Arg Pro
   1495 1500 1505
   TCC GGC ATG TTC GAC TCG TCC GTC CTC TGT GAG TGC TAT GAC GCG GGC 4913
   Ser Gly Met Phe Asp Ser Ser Val Leu Cys Glu Cys Tyr Asp Ala Gly
   1510 1515 1520
   TGT GCT TGG TAT GAG CTC ACG CCC GCC GAG ACT ACA GTT AGG CTA CGA 4961
   Cys Ala Trp Tyr Glu Leu Thr Pro Ala Glu Thr Thr Val Arg Leu Arg
   1525 1530 1535 1540
   GCG TAC ATG AAC ACC CCG GGG CTT CCC GTG TGC CAG GAC CAT CTT GGA 5009
   Ala Tyr Met Asn Thr Pro Gly Leu Pro Val Cys Gln Asp His Leu Gly
   1545 1550 1555
   TTT TGG GAG GGC GTC TTT ACG GGC CTC ACT CAT ATA GAT GCC CAC TTT 5057
   Phe Trp Glu Gly Val Phe Thr Gly Leu Thr His Ile Asp Ala His Phe
   1560 1565 1570
   CTA TCC CAG ACA AAG CAG AGT GGG GAG AAC TTT CCT TAC CTG GTA GCG 5105
   Leu Ser Gln Thr Lys Gln Ser Gly Glu Asn Phe Pro Tyr Leu Val Ala
   1575 1580 1585
   TAC CAA GCC ACC GTG TGC GCT AGG GCT CAA GCC CCT CCC CCA TCG TGG 5153
   Tyr Gln Ala Thr Val Cys Ala Arg Ala Gln Ala Pro Pro Pro Ser Trp
   1590 1595 1600
   GAC CAG ATG CGG AAG TGT TTG ATC CGC CTT AAA CCC ACC CTC CAT GGG 5201
   Asp Gln Met Arg Lys Cys Leu Ile Arg Leu Lys Pro Thr Leu His Gly
   1605 1610 1615 1620
   CCA ACA CCC CTG CTA TAC AGA CTG GGC GCT GTT CAG AAT GAA GTC ACC 5249
   Pro Thr Pro Leu Leu Tyr Arg Leu Gly Ala Val Gln Asn Glu Val Thr
   1625 1630 1635
   CTG ACG CAC CCA ATC ACC AAA TAC ATC ATG ACA TGC ATG TCG GCC GAC 5297
   Leu Thr His Pro Ile Thr Lys Tyr Ile Met Thr Cys Met Ser Ala Asp
   1640 1645 1650
   CTG GAG GTC GTC ACG AGC ACC TGG GTG CTC GTT GGC GGC GTC CTG GCT 5345
   Leu Glu Val Val Thr Ser Thr Trp Val Leu Val Gly Gly Val Leu Ala
   1655 1660 1665
   GCT CTG GCC GCG TAT TGC CTG TCA ACA GGC TGC GTG GTC ATA GTG GGC 5393
   Ala Leu Ala Ala Tyr Cys Leu Ser Thr Gly Cys Val Val Ile Val Gly
   1670 1675 1680
   AGG ATC GTC TTG TCC GGG AAG CCG GCA ATT ATA CCT GAC AGG GAG GTT 5441
   Arg Ile Val Leu Ser Gly Lys Pro Ala Ile Ile Pro Asp Arg Glu Val
   1685 1690 1695 1700
   CTC TAC CAG GAG TTC GAT GAG ATG GAA GAG TGC TCT CAG CAC TTA CCG 5489
   Leu Tyr Gln Glu Phe Asp Glu Met Glu Glu Cys Ser Gln His Leu Pro
   1705 1710 1715
   TAC ATC GAG CAA GGG ATG ATG CTC GCT GAG CAG TTC AAG CAG AAG GCC 5537
   Tyr Ile Glu Gln Gly Met Met Leu Ala Glu Gln Phe Lys Gln Lys Ala
   1720 1725 1730
   CTC GGC CTC CTG CAG ACC GCG TCC CGC CAT GCA GAG GTT ATC ACC CCT 5585
   Leu Gly Leu Leu Gln Thr Ala Ser Arg His Ala Glu Val Ile Thr Pro
   1735 1740 1745
   GCT GTC CAG ACC AAC TGG CAG AAA CTC GAG GTC TTT TGG GCG AAG CAC 5633
   Ala Val Gln Thr Asn Trp Gln Lys Leu Glu Val Phe Trp Ala Lys His
   1750 1755 1760
   ATG TGG AAT TTC ATC AGT GGG ATA CAA TAC TTG GCG GGC CTG TCA ACG 5681
   Met Trp Asn Phe Ile Ser Gly Ile Gln Tyr Leu Ala Gly Leu Ser Thr
   1765 1770 1775 1780
   CTG CCT GGT AAC CCC GCC ATT GCT TCA TTG ATG GCT TTT ACA GCT GCC 5729
   Leu Pro Gly Asn Pro Ala Ile Ala Ser Leu Met Ala Phe Thr Ala Ala
   1785 1790 1795
   GTC ACC AGC CCA CTA ACC ACT GGC CAA ACC CTC CTC TTC AAC ATA TTG 5777
   Val Thr Ser Pro Leu Thr Thr Gly Gln Thr Leu Leu Phe Asn Ile Leu
   1800 1805 1810
   GGG GGG TGG GTG GCT GCC CAG CTC GCC GCC CCC GGT GCC GCT ACC GCC      5825
   Gly Gly Trp Val Ala Ala Gln Leu Ala Ala Pro Gly Ala Ala Thr Ala
           1815                1820                1825
   TTT GTG GGC GCT GGC TTA GCT GGC GCC GCA CTC GAC AGC GTT GGA CTG      5873
   Phe Val Gly Ala Gly Leu Ala Gly Ala Ala Leu Asp Ser Val Gly Leu
       1830                1835                1840
   GGG AAG GTC CTC GTG GAC ATT CTT GCA GGC TAT GGC GCG GGC GTG GCG      5921
   Gly Lys Val Leu Val Asp Ile Leu Ala Gly Tyr Gly Ala Gly Val Ala
   1845                1850                1855                1860
   GGA GCT CTT GTG GCA TTC AAG ATC ATG AGC GGT GAG GTC CCC TCC ACG      5969
   Gly Ala Leu Val Ala Phe Lys Ile Met Ser Gly Glu Val Pro Ser Thr
                   1865                1870                1875
   GAG GAC CTG GTC AAT CTG CTG CCC GCC ATC CTC TCA CCT GGA GCC CTT      6017
   Glu Asp Leu Val Asn Leu Leu Pro Ala Ile Leu Ser Pro Gly Ala Leu
               1880                1885                1890
   GCA GTC GGT GTG GTC TTT GCA TCA ATA CTG CGC CGG CGT GTT GGC CCG      6065
   Ala Val Gly Val Val Phe Ala Ser Ile Leu Arg Arg Arg Val Gly Pro
           1895                1900                1905
   GGC GAG GGG GCA GTG CAA TGG ATG AAC CGG CTA ATA GCC TTC GCC TCC      6113
   Gly Glu Gly Ala Val Gln Trp Met Asn Arg Leu Ile Ala Phe Ala Ser
       1910                1915                1920
   CGG GGG AAC CAT GTT TCC CCC ACA CAC TAC GTG CCG GAG AGC GAT GCA      6161
   Arg Gly Asn His Val Ser Pro Thr His Tyr Val Pro Glu Ser Asp Ala
   1925                1930                1935                1940
   GCC GCC CGC GTC ACT GCC ATA CTC AGC AGC CTC ACT GTA ACC CAG CTC      6209
   Ala Ala Arg Val Thr Ala Ile Leu Ser Ser Leu Thr Val Thr Gln Leu
                   1945                1950                1955
   CTG AGG CGA CTG CAT CAG TGG ATA AGC TCG GAG TGT ACC ACT CCA TGC      6257
   Leu Arg Arg Leu His Gln Trp Ile Ser Ser Glu Cys Thr Thr Pro Cys
               1960                1965                1970
   TCC GGT TCC TGG CTA AGG GAC ATC TGG GAC TGG ATA TGC GAG GTG CTG      6305
   Ser Gly Ser Trp Leu Arg Asp Ile Trp Asp Trp Ile Cys Glu Val Leu
           1975                1980                1985
   AGC GAC TTT AAG ACC TGG CTG AAA GCC AAG CTC ATG CCA CAA CTG CCT      6353
   Ser Asp Phe Lys Thr Trp Leu Lys Ala Lys Leu Met Pro Gln Leu Pro
       1990                1995                2000
   GGG ATT CCC TTT GTG TCC TGC CAG CGC GGG TAT AGG GGG GTC TGG CGA      6401
   Gly Ile Pro Phe Val Ser Cys Gln Arg Gly Tyr Arg Gly Val Trp Arg
   2005                2010                2015                2020
   GGA GAC GGC ATT ATG CAC ACT CGC TGC CAC TGT GGA GCT GAG ATC ACT        6449
   Gly Asp Gly Ile Met His Thr Arg Cys His Cys Gly Ala Glu Ile Thr
                   2025                2030                2035
   GGA CAT GTC AAA AAC GGG ACG ATG AGG ATC GTC GGT CCT AGG ACC TGC        6497
   Gly His Val Lys Asn Gly Thr Met Arg Ile Val Gly Pro Arg Thr Cys
               2040                2045                2050
   AAG AAC ATG TGG AGT GGG ACG TTC TTC ATT AAT GCC TAC ACC ACG GGC        6545
   Lys Asn Met Trp Ser Gly Thr Phe Phe Ile Asn Ala Tyr Thr Thr Gly
           2055                2060                2065
   CCC TGT ACT CCC CTT CCT GCG CCG AAC TAT AAG TTC GCG CTG TGG AGG        6593
   Pro Cys Thr Pro Leu Pro Ala Pro Asn Tyr Lys Phe Ala Leu Trp Arg
       2070                2075                2080
   GTG TCT GCA GAG GAA TAC GTG GAG ATA AGG CGG GTG GGG GAC TTC CAC        6641
   Val Ser Ala Glu Glu Tyr Val Glu Ile Arg Arg Val Gly Asp Phe His
   2085                2090                2095                2100
   TAC GTA TCG GGC ATG ACT ACT GAC AAT CTC AAA TGC CCG TGC CAG ATC        6689
   Tyr Val Ser Gly Met Thr Thr Asp Asn Leu Lys Cys Pro Cys Gln Ile
                   2105                2110                2115
   CCA TCG CCC GAA TTT TTC ACA GAA TTG GAC GGG GTG CGC CTA CAT AGG        6737
   Pro Ser Pro Glu Phe Phe Thr Glu Leu Asp Gly Val Arg Leu His Arg
               2120                2125                2130
   TTT GCG CCC CCT TGC AAG CCC TTG CTG CGG GAG GAG GTA TCT TTC AGA        6785
   Phe Ala Pro Pro Cys Lys Pro Leu Leu Arg Glu Glu Val Ser Phe Arg
           2135                2140                2145
   GTA GGA CTC CAC GAG TAC CCG GTG GGG TCG CAA TTA CCT TGC GAG CCC        6833
   Val Gly Leu His Glu Tyr Pro Val Gly Ser Gln Leu Pro Cys Glu Pro
       2150                2155                2160
   GAA CCG GAC GTA GCC GTG TTG ACG TCC ATG CTC ACT GAT CCC TCC CAT        6881
   Glu Pro Asp Val Ala Val Leu Thr Ser Met Leu Thr Asp Pro Ser His
   2165                2170                2175                2180
   ATA ACA GCA GAG GCG GCC GGG AGA AGG TTG GCG AGA GGG TCA CCC CCT        6929
   Ile Thr Ala Glu Ala Ala Gly Arg Arg Leu Ala Arg Gly Ser Pro Pro
                   2185                2190                2195
   TCT ATG GCC AGC TCC TCG GCT AGC CAG CTG TCC GCT CCA TCT CTC AAG        6977
   Ser Met Ala Ser Ser Ser Ala Ser Gln Leu Ser Ala Pro Ser Leu Lys
               2200                2205                2210
   GCA ACT TGC ACC GCC AAC CAT GAC TCC CCT GAC GCC GAG CTC ATA GAG        7025
   Ala Thr Cys Thr Ala Asn His Asp Ser Pro Asp Ala Glu Leu Ile Glu
           2215                2220                2225
   GCT AAC CTC CTG TGG AGG CAG GAG ATG GGC GGC AAC ATC ACC AGG GTT     7073
   Ala Asn Leu Leu Trp Arg Gln Glu Met Gly Gly Asn Ile Thr Arg Val
       2230                2235                2240
   GAG TCA GAG AAC AAA GTG GTG ATT CTG GAC TCC TTC GAT CCG CTT GTG     7121
   Glu Ser Glu Asn Lys Val Val Ile Leu Asp Ser Phe Asp Pro Leu Val
   2245                2250                2255                2260
   GCA GAG GAG GAT GAG CGG GAG GTC TCC GTA CCC GCA GAA ATT CTG CGG     7169
   Ala Glu Glu Asp Glu Arg Glu Val Ser Val Pro Ala Glu Ile Leu Arg
                   2265                2270                2275
   AAG TCT CGG AGA TTC GCC CCA GCC CTG CCC GTC TGG GCG CGG CCG GAC     7217
   Lys Ser Arg Arg Phe Ala Pro Ala Leu Pro Val Trp Ala Arg Pro Asp
               2280                2285                2290
   TAC AAC CCC CTG CTA GTA GAG ACG TGG AAA AAG CCT GAC TAC GAA CCA     7265
   Tyr Asn Pro Leu Leu Val Glu Thr Trp Lys Lys Pro Asp Tyr Glu Pro
           2295                2300                2305
   CCT GTG GTC CAT GGC TGC CCG CTA CCA CCT CCA CGG TCC CCT CCT GTG     7313
   Pro Val Val His Gly Cys Pro Leu Pro Pro Pro Arg Ser Pro Pro Val
       2310                2315                2320
   CCT CCG CCT CGG AAA AAG CGT ACG GTG GTC CTC ACC GAA TCA ACC CTA     7361
   Pro Pro Pro Arg Lys Lys Arg Thr Val Val Leu Thr Glu Ser Thr Leu
   2325                2330                2335                2340
   CCT ACT GCC TTG GCC GAG CTT GCC ACC AAA AGT TTT GGC AGC TCC TCA     7409
   Pro Thr Ala Leu Ala Glu Leu Ala Thr Lys Ser Phe Gly Ser Ser Ser
                   2345                2350                2355
   ACT TCC GGC ATT ACG GGC GAC AAT ACG ACA ACA TCC TCT GAG CCC GCC     7457
   Thr Ser Gly Ile Thr Gly Asp Asn Thr Thr Thr Ser Ser Glu Pro Ala
               2360                2365                2370
   CCT TCT GGC TGC CCC CCC GAC TCC GAC GTT GAG TCC TAT TCT TCC ATG     7505
   Pro Ser Gly Cys Pro Pro Asp Ser Asp Val Glu Ser Tyr Ser Ser Met
           2375                2380                2385
   CCC CCC CTG GAG GGG GAG CCT GGG GAT CCG GAT CTC AGC GAC GGG TCA     7553
   Pro Pro Leu Glu Gly Glu Pro Gly Asp Pro Asp Leu Ser Asp Gly Ser
       2390                2395                2400
   TGG TCG ACG GTC AGT AGT GGG GCC GAC ACG GAA GAT GTC GTG TGC TGC     7601
   Trp Ser Thr Val Ser Ser Gly Ala Asp Thr Glu Asp Val Val Cys Cys
   2405                2410                2415                2420
   TCA ATG TCT TAT TCC TGG ACA GGC GCA CTC GTC ACC CCG TGC GCT GCG     7649
   Ser Met Ser Tyr Ser Trp Thr Gly Ala Leu Val Thr Pro Cys Ala Ala
                   2425                2430                2435
   GAG GAA CAA AAA CTG CCC ATC AAC GCA CTG AGC AAC TCG TTG CTA CGC        7697
   Glu Glu Gln Lys Leu Pro Ile Asn Ala Leu Ser Asn Ser Leu Leu Arg
               2440                2445                2450
   CAT CAC AAT CTG GTG TAT TCC ACC ACT TCA CGC AGT GCT TGC CAA AGG        7745
   His His Asn Leu Val Tyr Ser Thr Thr Ser Arg Ser Ala Cys Gln Arg
           2455                2460                2465
   AAG AAG AAA GTC ACA TTT GAC AGA CTG CAA GTT CTG GAC AGC CAT TAC        7793
   Lys Lys Lys Val Thr Phe Asp Arg Leu Gln Val Leu Asp Ser His Tyr
       2470                2475                2480
   CAG GAC GTG CTC AAG GAG GTC AAA GCA GCG GCG TCA AAA GTG AAG GCT        7841
   Gln Asp Val Leu Lys Glu Val Lys Ala Ala Ala Ser Lys Val Lys Ala
   2485                2490                2495                2500
   AAC TTG CTA TCC GTA GAG GAA GCT TGC AGC CTG GCG CCC CCA CAT TCA        7889
   Asn Leu Leu Ser Val Glu Glu Ala Cys Ser Leu Ala Pro Pro His Ser
                   2505                2510                2515
   GCC AAA TCC AAG TTT GGC TAT GGG GCA AAA GAC GTC CGT TGC CAT GCC        7937
   Ala Lys Ser Lys Phe Gly Tyr Gly Ala Lys Asp Val Arg Cys His Ala
               2520                2525                2530
   AGA AAG GCC GTA GCC CAC ATC AAC TCC GTG TGG AAA GAC CTT CTG GAA        7985
   Arg Lys Ala Val Ala His Ile Asn Ser Val Trp Lys Asp Leu Leu Glu
           2535                2540                2545
   GAC AGT GTA ACA CCA ATA GAC ACT ACC ATC ATG GCC AAG AAC GAG GTT        8033
   Asp Ser Val Thr Pro Ile Asp Thr Thr Ile Met Ala Lys Asn Glu Val
       2550                2555                2560
   TTC TGC GTT CAG CCT GAG AAG GGG GGT CGT AAG CCA GCT CGT CTC ATC        8081
   Phe Cys Val Gln Pro Glu Lys Gly Gly Arg Lys Pro Ala Arg Leu Ile
   2565                2570                2575                2580
   GTG TTC CCC GAC CTG GGC GTG CGC GTG TGC GAG AAG ATG GCC CTG TAC        8129
   Val Phe Pro Asp Leu Gly Val Arg Val Cys Glu Lys Met Ala Leu Tyr
                   2585                2590                2595
   GAC GTG GTT AGC AAG CTC CCC TTG GCC GTG ATG GGA AGC TCC TAC GGA        8177
   Asp Val Val Ser Lys Leu Pro Leu Ala Val Met Gly Ser Ser Tyr Gly
               2600                2605                2610
   TTC CAA TAC TCA CCA GGA CAG CGG GTT GAA TTC CTC GTG CAA GCG TGG        8225
   Phe Gln Tyr Ser Pro Gly Gln Arg Val Glu Phe Leu Val Gln Ala Trp
           2615                2620                2625
   AAG TCC AAG AAG ACC CCG ATG GGG CTC TCG TAT GAT ACC CGC TGT TTT        8273
   Lys Ser Lys Lys Thr Pro Met Gly Leu Ser Tyr Asp Thr Arg Cys Phe
       2630                2635                2640
   GAC TCC ACA GTC ACT GAG AGC GAC ATC CGT ACG GAG GAG GCA ATT TAC 8321
   Asp Ser Thr Val Thr Glu Ser Asp Ile Arg Thr Glu Glu Ala Ile Tyr
   2645 2650 2655 2660
   CAA TGT TGT GAC CTG GAC CCC CAA GCC CGC GTG GCC ATC AAG TCC CTC 8369
   Gln Cys Cys Asp Leu Asp Pro Gln Ala Arg Val Ala Ile Lys Ser Leu
   2665 2670 2675
   ACT GAG AGG CTT TAT GTT GGG GGC CCT CTT ACT AAT TCA AGG GGG GAA 8417
   Thr Glu Arg Leu Tyr Val Gly Gly Pro Leu Thr Asn Ser Arg Gly Glu
   2680 2685 2690
   AAC TGC GGC TAC CGC AGG TGC CGC GCG AGC AGA GTA CTG ACA ACT AGC 8465
   Asn Cys Gly Tyr Arg Arg Cys Arg Ala Ser Arg Val Leu Thr Thr Ser
   2695 2700 2705
   TGT GGT AAC ACC CTC ACT CGC TAC ATC AAG GCC CGG GCA GCC TGT CGA 8513
   Cys Gly Asn Thr Leu Thr Arg Tyr Ile Lys Ala Arg Ala Ala Cys Arg
   2710 2715 2720
   GCC GCA GGG CTC CAG GAC TGC ACC ATG CTC GTG TGT GGC GAC GAC TTA 8561
   Ala Ala Gly Leu Gln Asp Cys Thr Met Leu Val Cys Gly Asp Asp Leu
   2725 2730 2735 2740
   GTC GTT ATC TGT GAA AGT GCG GGG GTC CAG GAG GAC GCG GCG AGC CTG 8609
   Val Val Ile Cys Glu Ser Ala Gly Val Gln Glu Asp Ala Ala Ser Leu
   2745 2750 2755
   AGA GCC TTC ACG GAG GCT ATG ACC AGG TAC TCC GCC CCC CCC GGG GAC 8657
   Arg Ala Phe Thr Glu Ala Met Thr Arg Tyr Ser Ala Pro Pro Gly Asp
   2760 2765 2770
   CCC CCA CAA CCA GAA TAC GAC TTG GAG CTT ATA ACA TCA TGC TCC TCC 8705
   Pro Pro Gln Pro Glu Tyr Asp Leu Glu Leu Ile Thr Ser Cys Ser Ser
   2775 2780 2785
   AAC GTG TCA GTC GCC CAC GAC GGC GCT GGA AAG AGG GTC TAC TAC CTT 8753
   Asn Val Ser Val Ala His Asp Gly Ala Gly Lys Arg Val Tyr Tyr Leu
   2790 2795 2800
   ACC CGT GAC CCT ACA ACC CCC CTC GCG AGA GCC GCG TGG GAG ACA GCA 8801
   Thr Arg Asp Pro Thr Thr Pro Leu Ala Arg Ala Ala Trp Glu Thr Ala
   2805 2810 2815 2820
   AGA CAC ACT CCA GTC AAT TCC TGG CTA GGC AAC ATA ATC ATG TTT GCC 8849
   Arg His Thr Pro Val Asn Ser Trp Leu Gly Asn Ile Ile Met Phe Ala
   2825 2830 2835
   CCC ACA CTG TGG GCG AGG ATG ATA CTG ATG ACC CAC TTC TTT AGC GTC 8897
   Pro Thr Leu Trp Ala Arg Met Ile Leu Met Thr His Phe Phe Ser Val
   2840 2845 2850
   CTC ATA GCC AGG GAT CAG CTT GAA CAG GCT CTC AAC TGC GAG ATC TAC       8945
   Leu Ile Ala Arg Asp Gln Leu Glu Gln Ala Leu Asn Cys Glu Ile Tyr
           2855                2860                2865
   GGA GCC TGC TAC TCC ATA GAA CCA CTG GAT CTA CCT CCA ATC ATT CAA       8993
   Gly Ala Cys Tyr Ser Ile Glu Pro Leu Asp Leu Pro Pro Ile Ile Gln
       2870                2875                2880
   AGA CTC CAT GGC CTC AGC GCA TTT TCA CTC CAC AGT TAC TCT CCA GGT       9041
   Arg Leu His Gly Leu Ser Ala Phe Ser Leu His Ser Tyr Ser Pro Gly
   2885                2890                2895                2900
   GAA ATT AAT AGG GTG GCC GCA TGC CTC AGA AAA CTT GGG GTC CCG CCC       9089
   Glu Ile Asn Arg Val Ala Ala Cys Leu Arg Lys Leu Gly Val Pro Pro
                   2905                2910                2915
   TTG CGA GCT TGG AGA CAC CGG GCC TGG AGC GTC CGC GCT AGG CTT CTG       9137
   Leu Arg Ala Trp Arg His Arg Ala Trp Ser Val Arg Ala Arg Leu Leu
               2920                2925                2930
   GCC AGA GGA GGC AAG GCT GCC ATA TGT GGC AAG TAC CTC TTC AAC TGG       9185
   Ala Arg Gly Gly Lys Ala Ala Ile Cys Gly Lys Tyr Leu Phe Asn Trp
           2935                2940                2945
   GCA GTA AGA ACA AAG CTC AAA CTC ACT CCG ATA ACG GCC GCT GGC CGG       9233
   Ala Val Arg Thr Lys Leu Lys Leu Thr Pro Ile Thr Ala Ala Gly Arg
       2950                2955                2960
   CTG GAC TTG TCC GGC TGG TTC ACG GCT GGC TAC AGC GGG GGA GAC ATT       9281
   Leu Asp Leu Ser Gly Trp Phe Thr Ala Gly Tyr Ser Gly Gly Asp Ile
   2965                2970                2975                2980
   TAT CAC AGC GTG TCT CAT GCC CGG CCC CGC TGG TTC TGG TTT TGC CTA       9329
   Tyr His Ser Val Ser His Ala Arg Pro Arg Trp Phe Trp Phe Cys Leu
                   2985                2990                2995
   CTC CTG CTT GCT GCA GGG GTA GGC ATC TAC CTC CTC CCC AAC CGA           9374
   Leu Leu Leu Ala Ala Gly Val Gly Ile Tyr Leu Leu Pro Asn Arg
               3000                3005                3010
   TGAAGATTGG GCTAACCACT CCAGGCCAAT AGGCCATTCC CT                        9416
   ***
(2) INFORMATION FOR SEQ ID NO:47:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:47:
      CAGCCCCCTG ATGGGGGCGA C 21
(2) INFORMATION FOR SEQ ID NO:48:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:48:
      ACTCGCAAGC ACCCTATCA 19
(2) INFORMATION FOR SEQ ID NO:49:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:495:
      CTGTGAGGAA CTACTGTCT 19
(2) INFORMATION FOR SEQ ID NO:50:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:50:
      ATGAGCACGA ATCCTCAAAC CT 22
(2) INFORMATION FOR SEQ ID NO:51:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:51:
      GTCCTGCCCT CGGGCC 16
(2) INFORMATION FOR SEQ ID NO:52:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:52:
      CGAGGAAGAC TTCCGAGC 18
(2) INFORMATION FOR SEQ ID NO:53:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:53:
      ACCCAAATTG CGCGACCTAC 20
(2) INFORMATION FOR SEQ ID NO:54:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:54:
      TAAGGTCATC GATACCCT 18
(2) INFORMATION FOR SEQ ID NO:55:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:55:
      CAGTTCATCA TCATATCCCA 20
(2) INFORMATION FOR SEQ ID NO:56:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:56:
      GATAGAGAA AGAGCAAC 18
(2) INFORMATION FOR SEQ ID NO:57:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:57:
      AGACTTCCGA GCGGTCGCAA 20
(2) INFORMATION FOR SEQ ID NO:58:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:58:
      GACCTGTGCG GGTCTGTC 18
(2) INFORMATION FOR SEQ ID NO:59:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:59:
      GGGTCGGCAG CTGGCTAGCC TCTCA 25
(2) INFORMATION FOR SEQ ID NO:60:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:60:
      TCCTGGCGGG CATAGCGT 18
(2) INFORMATION FOR SEQ ID NO:61:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:61:
      CCCCAGCCCT GGTCAAAATC GGTAA 25
(2) INFORMATION FOR SEQ ID NO:62:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:62:

   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:63:
      CTGTCGGTCG TTCCCACCA 19
(2) INFORMATION FOR SEQ ID NO:64:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:64:
      CCGCGAAGAG TGTGTGTGGT 20
(2) INFORMATION FOR SEQ ID NO:65:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:65:
      CAATGTTCTG GTGGAGGTG 19
(2) INFORMATION FOR SEQ ID NO:66:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:66:
      GCCATTAAGT GGGAGTACGT CGTTCTCC 28
(2) INFORMATION FOR SEQ ID NO:67:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:67:
      CGAGGAAGGA TACAAGACC 19
(2) INFORMATION FOR SEQ ID NO:68:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:68:
      TGCTTGTGGA TGATGCTACT 20
(2) INFORMATION FOR SEQ ID NO:69:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:69:
      CACACGTGCA GTTGCGCT 18
(2) INFORMATION FOR SEQ ID NO:70:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:70:
      CTGCTGACCA CTACACAG 18
(2) INFORMATION FOR SEQ ID NO:71:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:71:
      GACCAGAGTG GAAGCGCAA 19
(2) INFORMATION FOR SEQ ID NO:72:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:72:
      TACCAGAGTC GGGTGTACAG 20
(2) INFORMATION FOR SEQ ID NO:73:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:73:
      CTAGGAGGCC CCTTGTCTGC 20
(2) INFORMATION FOR SEQ ID NO:74:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:74:
      CTCGGGCCAG CCGATGGA 18
(2) INFORMATION FOR SEQ ID NO:75:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:75:
      GGGGACCTCA TGGTTGTCT 19
(2) INFORMATION FOR SEQ ID NO:76:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:76:
      CCCGTGGAGT GGCTAAGG 18
(2) INFORMATION FOR SEQ ID NO:77:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:77:
      CTCCTCGATG TTGGGATGG 19
(2) INFORMATION FOR SEQ ID NO:78:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:78:
      CAGAGCTTCC AGGTGGCTC 19
(2) INFORMATION FOR SEQ ID NO:79:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:79:
      CGGGCTCCGT CACTGTG 17
(2) INFORMATION FOR SEQ ID NO:80:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:80:
      GTATTGCAGT CTATCACCGA G 21
(2) INFORMATION FOR SEQ ID NO:81:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:81:
      GGCTATACCG GCGACTTCGA 20
(2) INFORMATION FOR SEQ ID NO:82:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:82:
      CGTTGAGTGC GGGAGACAG 19
(2) INFORMATION FOR SEQ ID NO:83:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:83:
      TCACCATTGA GACAATCACG 20
(2) INFORMATION FOR SEQ ID NO:84:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:84:
      GTAAGGAAGG TTCTCCCCAC TC 22
(2) INFORMATION FOR SEQ ID NO:85:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:85:
      ATGCCCACTT TCTATCCCAG ACAAAGC 27
(2) INFORMATION FOR SEQ ID NO:86:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:86:
      TGCATGTCAT GATGTAT 17
(2) INFORMATION FOR SEQ ID NO:87:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:87:
      GGACAAGACG ACCCTGCC 18
(2) INFORMATION FOR SEQ ID NO:88:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:88:
      CGTATTGCCT GTCAACAGGC 20
(2) INFORMATION FOR SEQ ID NO:89:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:89:
      AGCGCCCACA AAGGCAGTAG 20
(2) INFORMATION FOR SEQ ID NO:90:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:90:
(2) INFORMATION FOR SEQ ID NO:91:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:91:
      CCAGGAACCG GAGCATGG 18
(2) INFORMATION FOR SEQ ID NO:92:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:92:
      ACCAGTGGAT AAGCTCGG 18
(2) INFORMATION FOR SEQ ID NO:93:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:93:
      CGTGGTGTAG GCATTAATG 19
(2) INFORMATION FOR SEQ ID NO:94:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:94:
      ATGTGGAGTG GGACCTTCC 19
(2) INFORMATION FOR SEQ ID NO:95:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:95:
      CTCTGCTGTT ATATGGGAGG 20
(2) INFORMATION FOR SEQ ID NO:96:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:96:
      GTTGACGTCC ATGCTCACTG 20
(2) INFORMATION FOR SEQ ID NO:97:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:97:
      TTTCCACGTC TCCACTAGCG 20
(2) INFORMATION FOR SEQ ID NO:98:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:98:
      GTGAGGACCA CCGTCCGC 18
(2) INFORMATION FOR SEQ ID NO:99:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:99:
      TTCCACCTCC AAAGTCCCCT 20
(2) INFORMATION FOR SEQ ID NO:100:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:100:
      AGAACTTGCA GTCTGTCAAA TGTGA 25
(2) INFORMATION FOR SEQ ID NO:101:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:101:
      GGAAGAACAG AAACTGCCCA TCAATGCACT AAGC 34
(2) INFORMATION FOR SEQ ID NO:102:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:102:
      TGACGCCGCT GCTTTAACCT 20
(2) INFORMATION FOR SEQ ID NO:103:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:103:
      TGCAAGCTTC CTCTACGGAT 20
(2) INFORMATION FOR SEQ ID NO:104:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:104:
      AGGTTAAAGC AGCGGCGTCA 20
(2) INFORMATION FOR SEQ ID NO:105:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:105:
      AGCTTCCCAT CACGGCCAA 19
(2) INFORMATION FOR SEQ ID NO:106:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:106:
      GATGGCTTTG TACGACGTG 19
(2) INFORMATION FOR SEQ ID NO:107:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:107:
      GCACCTGCGA TAGCCGCAGT 20
(2) INFORMATION FOR SEQ ID NO:108:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:108:
      GTCCCTCACC GAGAGGCT 18
(2) INFORMATION FOR SEQ ID NO:109:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:109:
      GATTGGAGGT AGATCAAGTG 20
(2) INFORMATION FOR SEQ ID NO:110:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:110:
      TACGACTTGG AGCTCATAAC 20
(2) INFORMATION FOR SEQ ID NO:111:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO: 111:
      AGCAAGACAC ACTCCAGTCA 20
(2) INFORMATION FOR SEQ ID NO:112:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: YES
   (xi) SEQUENCE DESCRIPTION: SEQ ID NO:112:
      GCCTATTGGC CTGGAGTGGTTAGC 24
(2) INFORMATION FOR SEQ ID NO:113:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide
   (xi) SEQUENCE DESCRIPTION: SEQ ID NO:113:
   His Val Thr Gly Gly Asn Ala Gly Arg Thr Thr Ala Gly Leu Val Gly
   1               5                   10                  15
   Leu Leu Thr Pro Gly Ala Lys Gln Asn Ile
               20                  25
(2) INFORMATION FOR SEQ ID NO:114:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide
   (xi) SEQUENCE DESCRIPTION: SEQ ID NO:114:
   His Val Thr Gly Gly Ser Ala Gly His Thr Val Ser Gly Phe Val Ser
   1               5                   10                  15
   Leu Leu Ala Pro Gly Ala Lys Gln Asn Val
               20                  25
(2) INFORMATION FOR SEQ ID NO:115:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide
   (xi) SEQUENCE DESCRIPTION: SEQ ID NO:115:
   His Val Thr Gly Gly Gln Ala Ala Arg Ala Met Ser Gly Leu Val Ser
   1               5                   10                  15
   Leu Phe Thr Pro Gly Ala Lys Gln Asn Ile
               20                  25
(2) INFORMATION FOR SEQ ID NO:116:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide
   (xi) SEQUENCE DESCRIPTION: SEQ ID NO:116:
   His Val Thr Gly Gly Arg Val Ala Ser Ser Thr Gln Ser Leu Val Ser
   1               5                   10                  15
   Trp Leu Ser Gln Gly Pro Ser Gln Lys Ile
               20                  25
(2) INFORMATION FOR SEQ ID NO:117:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide
   (xi) SEQUENCE DESCRIPTION: SEQ ID NO:117:
   His Val Thr Gly Gly Ala Gln Ala Lys Thr Thr Asn Arg Leu Val Ser
   1               5                   10                  15
   Met Phe Ala Ser Gly Pro Ser Gln Lys Ile
               20                  25
(2) INFORMATION FOR SEQ ID NO:118:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide (xi) SEQUENCE DESCRIPTION: SEQ ID NO:118:
(2) INFORMATION FOR SEQ ID NO:119:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide
   (xi) SEQUENCE DESCRIPTION: SEQ ID NO:119:
   His Val Thr Gly Gly Val Gln Gly His Val Thr Ser Thr Leu Thr Ser
   1               5                   10                  15
   Leu Phe Arg Pro Gly Ala Ser Gln Lys Ile
               20                  25
(2) INFORMATION FOR SEQ ID NO:120:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide
   (xi) SEQUENCE DESCRIPTION: SEQ ID NO:120:
   His Val Thr Gly Gly Ser Ala Gly Arg Thr Thr Ala Gly Leu Val Gly
   1               5                   10                  15
   Leu Leu Thr Pro Gly Ala Lys Gln Asn Ile
               20                  25
(2) INFORMATION FOR SEQ ID NO:121:
   (i) SEQUENCE CHARACTERISTICS:
      (A) LENGTH: 26 amino acids
      (B) TYPE: amino acid
      (D) TOPOLOGY: linear
   (ii) MOLECULE TYPE: peptide
   (xi) SEQUENCE DESCRIPTION: SEQ ID NO:121:
   His Val Thr Gly Gly Ser Ala Gly Arg Ser Val Leu Gly Ile Ala Ser
   1               5                   10                  15
   Phe Leu Thr Arg Gly Pro Lys Gln Asn Ile
               20                  25
(2) INFORMATION FOR SEQ ID NO:122:
   (i) SEQUENCE CHARACTERISTICS:
      (A) LENGTH: 30 amino acids
      (B) TYPE: amino acid
      (D) TOPOLOGY: linear
   (ii) MOLECULE TYPE: peptide
   (xi) SEQUENCE DESCRIPTION: SEQ ID NO:122:
   Val Ala Thr Arg Asp Gly Lys Leu Pro Thr Thr Gln Leu Arg Arg His
   1 5 10 15
   Ile Asp Leu Leu Val Gly Ser Ala Thr Leu Cys Ser Ala Leu
   20 25 30
(2) INFORMATION FOR SEQ ID NO:123:
   (i) SEQUENCE CHARACTERISTICS:
      (A) LENGTH: 30 amino acids
      (B) TYPE: amino acid
      (D) TOPOLOGY: linear
   (ii) MOLECULE TYPE: peptide
   (xi) SEQUENCE DESCRIPTION: SEQ ID NO:123:
   Val Ala Thr Arg Asp Gly Lys Leu Pro Ala Thr Gln Leu Arg Arg His
   1 5 10 15
   Ile Asp Leu Leu Val Gly Ser Ala Thr Leu Cys Ser Ala Leu
   20 25 30
(2) INFORMATION FOR SEQ ID NO:124:
   (i) SEQUENCE CHARACTERISTICS:
      (A) LENGTH: 30 amino acids
      (B) TYPE: amino acid
      (D) TOPOLOGY: linear
   (ii) MOLECULE TYPE: peptide
   (xi) SEQUENCE DESCRIPTION: SEQ ID NO:124:
   Leu Ala Ala Arg Asn Ser Ser Ile Pro Thr Thr Thr Ile Arg Arg His
   1 5 10 15
   Val Asp Leu Leu Val Gly Ala Ala Ala Leu Cys Ser Ala Met
   20 25 30
(2) INFORMATION FOR SEQ ID NO:125:
   (i) SEQUENCE CHARACTERISTICS:
      (A) LENGTH: 30 amino acids
      (B) TYPE: amino acid
      (D) TOPOLOGY: linear
   (ii) MOLECULE TYPE: peptide
   (xi) SEQUENCE DESCRIPTION: SEQ ID NO:125:
   Leu Ala Ala Arg Asn Val Thr Ile Pro Thr Thr Thr Ile Arg Arg His
   1               5                   10                  15
   Val Asp Leu Leu Val Gly Ala Ala Ala Phe Cys Ser Ala Met
               20                  25                  30
(2) INFORMATION FOR SEQ ID NO:126:
   (i) SEQUENCE CHARACTERISTICS:
      (A) LENGTH: 30 amino acids
      (B) TYPE: amino acid
      (D) TOPOLOGY: linear
   (ii) MOLECULE TYPE: peptide
   (xi) SEQUENCE DESCRIPTION: SEQ ID NO:126:
   Leu Ala Ala Arg Asn Ala Ser Val Pro Thr Thr Thr Ile Arg Arg His
   1               5                   10                  15
   Val Asp Leu Leu Val Gly Ala Ala Ala Phe Cys Ser Ala Met
               20                  25                  30
(2) INFORMATION FOR SEQ ID NO:127:
   (i) SEQUENCE CHARACTERISTICS:
      (A) LENGTH: 30 amino acids
      (B) TYPE: amino acid
      (D) TOPOLOGY: linear
   (ii) MOLECULE TYPE: peptide
   (xi) SEQUENCE DESCRIPTION: SEQ ID NO:127:
   Leu Ala Ala Arg Asn Ala Ser Val Pro Thr Thr Thr Leu Arg Arg His
   1               5                   10                  15
   Val Asp Leu Leu Val Gly Thr Ala Ala Phe Cys Ser Ala Met
               20                  25                  30
(2) INFORMATION FOR SEQ ID NO:128:
   (i) SEQUENCE CHARACTERISTICS:
      (A) LENGTH: 27 amino acids
      (B) TYPE: amino acid
      (D) TOPOLOGY: linear
   (ii) MOLECULE TYPE: peptide
   (xi) SEQUENCE DESCRIPTION: SEQ ID NO:128:
   Leu Ala Ser Cys Arg Arg Leu Thr Asp Phe Ala Gln Gly Trp Gly Pro
   1               5                   10                  15
   Ile Ser Tyr Ala Asn Gly Ser Gly Leu Asp Glu
               20                  25
(2) INFORMATION FOR SEQ ID NO:129:
   (i) SEQUENCE CHARACTERISTICS:
      (A) LENGTH: 27 amino acids
      (B) TYPE: amino acid
      (D) TOPOLOGY: linear
   (ii) MOLECULE TYPE: peptide
   (xi) SEQUENCE DESCRIPTION: SEQ ID NO:129:
   Leu Ala Ser Cys Arg Pro Leu Thr Asp Phe Asp Gln Gly Trp Gly Pro
   1               5                   10                  15
   Ile Ser Tyr Ala Asn Gly Ser Gly Pro Asp Gln
               20                  25
(2) INFORMATION FOR SEQ ID NO:130:
   (i) SEQUENCE CHARACTERISTICS:
      (A) LENGTH: 27 amino acids
      (B) TYPE: amino acid
      (D) TOPOLOGY: linear
   (ii) MOLECULE TYPE: peptide
   (xi) SEQUENCE DESCRIPTION: SEQ ID NO:130:
   Leu Ala Ser Cys Arg Arg Leu Thr Asp Phe Asp Gln Gly Trp Gly Pro
   1 5 10 15
   Ile Ser His Ala Asn Gly Ser Gly Pro Asp Gln
   20 25
(2) INFORMATION FOR SEQ ID NO:131:
   (i) SEQUENCE CHARACTERISTICS:
      (A) LENGTH: 27 amino acids
      (B) TYPE: amino acid
      (D) TOPOLOGY: linear
   (ii) MOLECULE TYPE: peptide
   (xi) SEQUENCE DESCRIPTION: SEQ ID NO:131:
   Met Ala Ser Cys Arg Pro Ile Asp Glu Phe Ala Gln Gly Trp Gly Pro
   1 5 10 15
   Ile Thr His Asp Met Pro Glu Ser Ser Asp Gln
   20 25
(2) INFORMATION FOR SEQ ID NO:132:
   (i) SEQUENCE CHARACTERISTICS:
      (A) LENGTH: 27 amino acids
      (B) TYPE: amino acid
      (D) TOPOLOGY: linear
   (ii) MOLECULE TYPE: peptide
   (xi) SEQUENCE DESCRIPTION: SEQ ID NO:132:
   Met Ala Gln Cys Arg Thr Ile Asp Lys Phe Asp Gln Gly Trp Gly Pro
   1 5 10 15
   Ile Thr Tyr Ala Glu Ser Ser Arg Ser Asp Gln
   20 25
(2) INFORMATION FOR SEQ ID NO:122:
   (i) SEQUENCE CHARACTERISTICS:
      (A) LENGTH: 27 amino acids
      (B) TYPE: amino acid
      (D) TOPOLOGY: linear
   (ii) MOLECULE TYPE: peptide
   (xi) SEQUENCE DESCRIPTION: SEQ ID NO:133:
(2) INFORMATION FOR SEQ ID NO:134:
   (i) SEQUENCE CHARACTERISTICS:
      (A) LENGTH: 27 amino acids
      (B) TYPE: amino acid
      (D) TOPOLOGY: linear
   (ii) MOLECULE TYPE: peptide
   (xi) SEQUENCE DESCRIPTION: SEQ ID NO:134:
   Leu Ala Ser Cys Arg Arg Leu Thr Asp Phe Asp Gln Gly Trp Gly Pro
   1               5                   10                  15
   Ile Ser Tyr Ala Asn Gly Ser Gly Pro Asp Glu
               20                  25
(2) INFORMATION FOR SEQ ID NO:135:
   (i) SEQUENCE CHARACTERISTICS:
      (A) LENGTH: 24 amino acids
      (B) TYPE: amino acid
      (D) TOPOLOGY: linear
   (ii) MOLECULE TYPE: peptide
   (xi) SEQUENCE DESCRIPTION: SEQ ID NO:135:
   Ser Thr Ser Gly Ile Thr Gly Asp Asn Thr Thr Thr Ser Ser Glu Pro
   1               5                   10                  15
   Ala Pro Ser Gly Cys Pro Pro Asp
               20
(2) INFORMATION FOR SEQ ID NO:136:
   (i) SEQUENCE CHARACTERISTICS:
      (A) LENGTH: 24 amino acids
      (B) TYPE: amino acid
      (D) TOPOLOGY: linear
   (ii) MOLECULE TYPE: peptide
   (xi) SEQUENCE DESCRIPTION: SEQ ID NO:136:
   Gly Ser Ser Ala Val Asp Ser Gly Thr Ala Thr Gly Pro Pro Asp Gln
   1               5                   10                  15
   Ala Ser Asp Asp Gly Asp Lys Gly
               20
(2) INFORMATION FOR SEQ ID NO:137:
   (i) SEQUENCE CHARACTERISTICS:
      (A) LENGTH: 24 amino acids
      (B) TYPE: amino acid
      (D) TOPOLOGY: linear
   (ii) MOLECULE TYPE: peptide
   (xi) SEQUENCE DESCRIPTION: SEQ ID NO:137:
   Glu Ser Ser Ala Val Asp Ser Gly Thr Ala Thr Ala Leu Pro Asp Gln
   1               5                   10                  15
   Ala Ser Asp Asp Gly Asp Lys Gly
               20